# EUROPEAN PATENT SPECIFICATION

(11) **EP 2 630 155 B1**
(45) Date of publication and mention of the grant of the patent: **15.04.2020**
(21) Application number: 11778762.2
(22) Date of filing: 22.10.2011
(51) Int. Cl.: A61K 39/145, A61K 39/12, A61K 39/00

(54) **NOVEL HEMAGGLUTININ 5 (H5) PROTEINS FOR THE TREATMENT AND PREVENTION OF INFLUENZA INFECTIONS**
NEUARTIGE HÄMAGGLUTININ 5 (H5) -PROTEINE ZUR BEHANDLUNG UND VORBEUGUNG VON INFLUENZAINFEKTIONEN
NOUVELLES PROTÉINES D'HÉMAGGLUTININE 5 (H5) POUR LE TRAITEMENT ET LA PRÉVENTION D'INFECTIONS PAR LA GRIPPE

(30) Priority: 22.10.2010 EP 10188576
(43) Date of publication of application: 28.08.2013
(73) Proprietor: Boehringer Ingelheim Vetmedica GmbH, 55216 Ingelheim am Rhein (DE)
(72) Inventor: GONZALEZ-HERNANDEZ, Paulino Carlos, Jalisco 45650 (MX); LEON KEMPIS, Maria del Rocio, Missouri 64506 (US); REALPE-QUINTERO, Mauricio Alberto, Jalisco 45650 (MX); STADLER, Konrad, 55216 Ingelheim am Rhein (DE); VAUGHN, Eric Martin, MO 64506 (US)
(74) Representative: Simon, Elke Anna Maria
(86) International application number: PCT/US2011/057406
(87) International publication number: WO 2012/054907

(56) References cited:
- WO-A2-2007/149715
- WO-A2-2012/047941
- DATABASE UniProt [Online] 1 September 2009 (2009-09-01), "SubName: Full=Hemagglutinin; Flags: Precursor;", XP002629615, retrieved from EBI accession no. UNIPROT:C6EMS4 Database accession no. C6EMS4
- DATABASE UniProt [Online] 1 September 2009 (2009-09-01), "SubName: Full=Hemagglutinin; Flags: Precursor;", XP002629616, retrieved from EBI accession no. UNIPROT:C6EMR1 Database accession no. C6EMR1
- DATABASE UniProt [Online] 24 November 2009 (2009-11-24), "SubName: Full=Hemagglutinin;", XP002629617, retrieved from EBI accession no. UNIPROT:D0FHE8 Database accession no. D0FHE8
- DATABASE UniProt [Online] 29 May 2007 (2007-05-29), "SubName: Full=Hemagglutinin;", XP002629618, retrieved from EBI accession no. UNIPROT:A5A5I7 Database accession no. A5A5I7
- CRAWFORD J ET AL: "Baculovirus-derived hemagglutinin vaccines protect against lethal influenza infections by avian H5 and H7 subtypes", VACCINE, ELSEVIER LTD, GB, vol. 17, no. 18, 4 May 1999 (1999-05-04), pages 2265-2274, XP004165019, ISSN: 0264-410X, DOI: DOI:10.1016/S0264-410X(98)00494-0
- DATABASE UniProt [Online] 24 November 2009 (2009-11-24), "SubName: Full=Hemagglutinin;", XP002668586, retrieved from EBI accession no. UNIPROT:D0FHF2 Database accession no. D0FHF2
- DATABASE UniProt [Online] 24 November 2009 (2009-11-24), "SubName: Full=Hemagglutinin; Flags: Fragment;", XP002668587, retrieved from EBI accession no. UNIPROT:D0FHG6 Database accession no. D0FHG6
- E M LABIB: "C6EMS4", , 10 August 2010 (2010-08-10), XP055314852, Retrieved from the Internet: URL:http://www.uniprot.org/uniprot/C6EMS4. txt?version=8 [retrieved on 2016-10-28]

## Description

### FIELD OF THE INVENTION

The present disclosure relates to the field of medicine, preferably to the field of infectious diseases. In particular the present disclosure relates to influenza proteins, nucleic acid molecules and vectors encoding those proteins, and vaccines. Most particularly, the present disclosure relates to the use of any of such proteins, nucleic acid molecules, vectors, or vaccines for the treatment and/or prevention of influenza infections, preferably of infections with H5N1 of North African origin, and furthermore for the prevention of intra- and inter-species transmission of influenza virus.

### BACKGROUND OF THE INVENTION

Influenza infection remains an important infection in animals and humans. Influenza is caused by viruses that undergo continuous antigenic changes/modifications and that possess an animal reservoir. Thus new epidemics and pandemics may occur in the future, and eradication of the disease will be difficult to achieve. Influenza viruses are well known in the art and described more in detail for example by P. Palese, Nature Medicine, vol. 10, no. 12, pp. S 82 to S 86 of December 2004*,* with further references. Briefly, the genome of the influenza A virus consists of eight single-stranded segments, and the viral particle(s) has two major glycoproteins on its surface: hemagglutinin (H) and neuraminidase (N). With at least 16 different hemagglutinin (H1 to H16) and 9 different neuraminidase (N1 to N9) subtypes, there is a considerable antigenic variation among influenza viruses.

Influenza virus of type H5N1 Fowl Plague virus has been demonstrated to infect poultry, pigs, and man. The viruses can also be transmitted directly from avian species to humans (Claas et al., Lancet 1998, 351: 472*;* Suarez et al., J. Virol. 1998, 72: 6678*;* Subbarao et al., Science 1998, 279: 393*;* Shortridge, Vaccine 1999, 17 (Suppl. 1): S26-S29). Mortality in known human clinical cases approaches about 50%.

Over the last century, pigs have been an important vector for influenza pandemics. Pigs, camels, and seals, preferably pigs, can serve as a "mixing chamber" for avian influenza viruses, and therefore represent a potential risk factor for overcoming the species hurdles from poultry, the naturally reservoir of influenza viruses, to mammals. This "mixing" normally occurs by double infections of the susceptible animals, e.g. pigs, with both, an established mammalian (porcine), as well as, an avian influenza virus. This double infection may create new recombinant viruses that may be the cause of human or porcine pandemics. Recent evidence would, however, indicate that a recombination of current avian H5 strains with mammalian influenza viruses will not result in highly virulent recombinants. On the other hand, avian influenza virus can infect pigs and by spontaneous mutations can become adapted to pigs. The critical hurdle will be overcome as soon as the virus can cause horizontal infections within a pig (or other mammalian) population.

Yet, a major part of Southeast Asian pigs have been infected with avian (H5) influenza virus strains originating from neighbouring poultry husbandry. As those infections have so far been sub-clinical, they can only be diagnosed by laboratory methods and thus are frequently overlooked. There is a high risk that those sub-clinically-infected pigs will serve as an opportunity for the virus to adapt to the mammalian system, spread within the porcine population, and also infect human beings.

Current influenza vaccines include a subunit vaccine (Babai et al., Vaccine 1999, 17(9-10):1223-1238*;* Crawford et al., Vaccine 1999, 17(18):2265-2274*;* Johansson et al., Vaccine 1999, 17(15-16):2073-2080) attenuated vaccine (Horimoto et al., Vaccine 2004, 22(17-18):2244-2247), DNA vaccine (Watabe et al., Vaccine 2001, 19(31):4434-4444) and inactivated influenza vaccine (Cao et al., Vaccine 1992, 10(4):238-242), with the latter being the most widely used on a commercial scale (Lipatov et al., J Virol 2004, 78(17):8951-8959). WO2012047941 A2 discloses several H5 HA polypeptide sequences.

Subunit vaccines, recombinant hemagglutinin and neuraminidase (Babai et al., Vaccine 1999, 17(9-10):1223-1238*;* Crawford et al., Vaccine 1999, 17(18):2265-2274*;* Johansson et al., Vaccine 1999, 17(15-16):2073-2080) may be an attractive alternative to the inactivated vaccine, although none are currently in use as commercial vaccines. The preparation of such vaccines is obviously safer than for an inactivated vaccine. Moreover, subunit vaccines do not generate antibody responses to internal influenza viral proteins and thus allow distinction between vaccinated and infected animals (Crawford et al., Vaccine 1999, 17(18):2265-2274).

Hemagglutinin protein is the receptor-binding and membrane fusion glycoprotein of influenza virus and the target for infectivity-neutralizing antibodies. The entire hemagglutinin protein (HA) from the H5N1 is composed of 568 amino acids, with a molecular weight of 56 kDa. The HA molecule consists of HA1 and HA2 subunits, with the HA1 subunit mediating initial contact with the cell membrane and HA2 being responsible for membrane fusion (Chizmadzhev, Bioelectrochemistry 2004, 63(1-2):129-136).

Baculovirus/insect cell systems have been used to express hemagglutinin genes isolated from avian influenza subtypes (Babai et al., Vaccine 1999, 17(9-10):1223-1238*;* Crawford et al., Vaccine 1999, 17(18):2265-2274*;* Johansson et al., Vaccine 1999, 17(15-16):2073-2080*);* Nwe et al., BMC Mircobiology 2006, 6(16):doi:10.1186/1471-2180-6-16). However, the recombinant proteins seems not to be protective in any case, or only less effective at least for some species (Treanor et al., Vaccine 2001, 19: 1732-1737).

Further, since 2006 H5N1 has spread from Asia to North Africa, and particular H5N1 strains have developed in North African countries, such as Egypt and Sudan, which are referred to herein as "H5N1 of North African origin". Infection caused by any of these H5N1 of North African origin cannot adequately be treated or prevented by conventional medications or vaccines against H5N1. Thus, there is a particular need for proteins, nucleic acid molecules, vectors, and vaccines for the specific treatment and prevention of influenza infections caused by H5N1 strains of North African origin.

Moreover, there is a need to increase availability of improved vaccines and new vaccination approaches, in particular in North African countries, to provide better approaches to control influenza infections and to have a positive impact on disease load.

### SUMMARY AND DESCRIPTION OF THE INVENTION

Before the embodiments of the present invention it shall be noted that as used herein and in the appended claims, the singular forms "a", "an", and "the" include plural reference unless the context clearly dictates otherwise. Thus, for example, reference to "a preparation" includes a plurality of such preparations; reference to "the carrier" is a reference to one or more carriers and equivalents thereof known to those skilled in the art, and so forth. Unless defined otherwise, all technical and scientific terms used herein have the same meanings as commonly understood by one of ordinary skill in the art to which this invention belongs. All given ranges and values may vary by 1 to 5 % unless indicated otherwise or known otherwise by the person skilled in the art, therefore, the term "about" was omitted from the description. Although any methods and materials similar or equivalent to those described herein can be used in the practice or testing of the present invention, the preferred methods, devices, and materials are now described. Nothing herein is to be construed as an admission that the invention is not entitled to antedate such disclosure by virtue of prior invention.

The solution to the above technical problem is achieved by the description and the embodiments characterized in the claims.

Highly Pathogenic Avian Influenza (HPAI) H5N1 is widespread in North Africa, in particular in Egypt, in different compartments of the poultry industry. In the work underlying the disclosure, only viruses belonging to Clade 2.2.1 have been detected. These viruses may be divided in two subpopulations (Subclades A and B) which are genetically and antigenically distinct. Subclades A and B are co-circulating in intensively reared poultry and continuing to evolve. Both subclades appear to evolve rapidly and the emergence of antigenic variants is possible.

Sequencing analyses were conducted, all the HA (hemagglutinin) sequences of the viruses analysed grouped in clade 2.2.1. Data analysis points out that 2.2.1 viruses circulating in North Africa are genetically and antigenically heterogeneous. Two main genetic groups could be identified within clade 2.2.1 in North Africa, named subclade A and subclade B.

However, compared to subclade A, subclade B viruses are the most prevalent in the specimens examined. In fact, out of 67 sequences examined, 50 (75%) were in subclade B and 17 (25%) in subclade A. Subclade A is heterogeneous and includes viruses isolated in North Africa. This group of viruses, herein named A1, is evolving and it appears to become the dominant group within clade A. Subclade B is a monophyletic group of viruses under continuing, divergent evolution. Separate branches can be identified within this subclade. At present, subclade B shows distinct genetic characteristics and can be classified as a new emergent distinct clade, according to the WHO nomenclature.

Preliminary analysis based on subclade B viruses indicated that several additional AA (amino acid) mutations (13 to 23,) have occurred in this subclade. Laboratory tests based on cross-hemagglutination inhibition suggest that subclade B is antigenically different from A, supporting the genetic data.

In fact, sequence analysis conducted on two prototype strains circulating, clade A and clade B, revealed twelve amino acid differences in the HA molecule. Ten of twelve were located at the receptor binding site (RBS) with an impact on antigenicity (as revealed by hemagglutination inhibition (HI) assay) and host receptor binding affinity.

The disclosure is based on the surprising finding that particular proteins, nucleic acid molecules, and vectors based on said information received may serve as effective vaccines for the treatment and prevention of influenza infections caused by H5N1 viruses of North African origin.

### Influenza proteins and nucleic acid molecules coding for those

The present invention provides a vaccine comprising
a) H5 protein of influenza virus, wherein the H5 protein consists of or comprises any one of the sequences as set forth in SEQ ID NOs: 2 to 12, or 35 or 36 or 40;
b) a pharmaceutical acceptable carrier and/or excipient.

The present invention further provides the vaccine as described herein for use in a method of treating or preventing infections with Subclade A H5N1 virus of North African origin.

In one aspect, the present disclosure relates to a H5 protein of influenza virus, wherein the H5 protein has
(a) amino acids 113D, 126H, 145(-), 156R, 160F, 167T, and 181N, wherein the term "145(-)", or modification 145(-), respectively, means that the amino acid at position 145 of H5 is deleted, or
(b) amino acids 87P, 145L, 172T, 201E, 206I, 208K, 254T, 341G and 421K, or
(c) amino acids 145L, 172T, 254V,and
wherein the numbering of the amino acid positions of the H5 protein refers to the amino acid position or amino acid positions, respectively, as exemplarily given in SEQ ID NO:1. In the context of the disclosure, the H5 protein according to (a) is a Subclade A protein, and the H5 protein according to (b) or (c) is a Subclade B protein.

Within the context of the disclosure, it is understood that the term "amino acid" in particular refers to an amino acid residue or, respectively, to an amino acid which has been covalently linked via peptide bonds to two further amino acids or, if the amino acid is Nor C-terminally located in the peptide sequence, to one further amino acid.

The present disclosure also relates to a H5 protein, which consists of or comprises an amino acid sequence which is at least 95%, preferably 96%, more preferably 97%, still more preferably 98%, yet more preferably 99% or in particular preferrably 100% homologous with any one of the sequences as set forth in SEQ ID NOs: 2 to 40. Preferably, the H5 protein consists of or comprises an amino acid sequence which is at least 95%, preferably 96%, more preferably 97%, still more preferably 98%, yet more preferably 99% or in particular preferrably 100% homologous with any one of the sequences as set forth in SEQ ID NOs: 8, 13, or 40. In particular, H5 proteins that comprise or consist of the amino acid sequence set forth in SEQ ID NO: 13 are preferred.

According to the disclosure, the proteins consisting of or comprising an amino acid sequence being at least 95%, preferably 96%, more preferably 97%, still more preferably 98%, yet more preferably 99% or in particular preferrably 100% homologous with the sequence set forth in SEQ ID NO:40 are favoured, since due to the combination of the characteristics of both Subclade A and B, such a protein or proteins may serve as an effective universal vaccine against influenza infections caused by H5N1 viruses of North African origin.

In one preferred aspect, the H5 protein according to the disclosure has the amino acid 239N. The term "239N" exemplarily means, that the amino acid at position 239-numbering according to the amino acid positions of SEQ ID NO:1-shall code for the amino acid Asparagine (N). In other words, if reference is made to "H5 protein having the amino acid 239N", a H5 amino acid molecule that normally codes for Serine at amino acid position 239-numbering according to the amino acid positions of SEQ ID NO:1-that amino acid shall be substituted by an Asparagine (N).

In a particular preferred aspect, the H5 protein according to the disclosure does not comprise the influenza A H5N1 hemagglutinin signal sequence, wherein the H5 protein influenza A H5N1 hemagglutinin signal sequence preferably comprises or consists of the first 16 N-terminal amino acids (one letter code) MEKIVLLLAIVSLVKS (SEQ ID NO:44) or M1 E2 K3 14 V5 L6 L7 L8 A9 I10 V11 S12 L13 V14 K15 S16, respectively, wherein the numbering of said first N-terminal 16 amino acid positions refers to the amino acid positions 1 to 16 as exemplarily given in SEQ ID NO 1, namely referring to the sequence (three letter code) Met Glu Lys Ile Val Leu Leu Leu Ala Ile Val Ser Leu Val Lys Ser (SEQ ID NO:44) as set forth in SEQ ID NO:1.

In a further preferred aspect the H5 protein according to the disclosure thus consists of or comprises an amino acid sequence which is at least 95%, preferably 96%, more preferably 97%, still more preferably 98%, yet more preferably 99% or in particular preferably 100% homologous with any one of the sequences as set forth in SEQ ID NOs: 2 to 40, wherein the sequences as set forth in SEQ ID NOs: 2 to 40 start with amino acid D17, and wherein the numbering of the first N-terminal amino acid position (D17) refers to the amino acid position 17 as exemplarily given in SEQ ID NO 1.

Table B provides the respective sequences SEQ ID NOs: 55 to 92 that correspond to variants of the sequences as set forth in SEQ ID NOs: 2 to 39, wherein SEQ ID NOs: 55 to 92 start with the respective amino acid D17 of SEQ ID NOs: 2 to 39, as mentioned herein, and Table D provides the respective variant of sequence SEQ ID NO: 40, i.e. SEQ ID NO: 131, wherein the variant sequence starts with amino acid D17, as mentioned herein.

In one example, the H5 protein according to the disclosure thus preferably consists of or comprises an amino acid sequence which is at least 95%, preferably 96%, more preferably 97%, still more preferably 98%, yet more preferably 99% or in particular preferably 100% homologous with the polypeptide sequence DQICIGYHANNSTEQVDTIMEKNVTVTHAQDILEKTHNGKLCNLDGVKPLILRDCSVAGWLLGNPMCDEFLNVPEWSYIVEKINPTNDLCYPGNFNDYEELKHLLSRINHFEKIQIIPKNSWSDHEASGVSSACPYQGRSSFFRNVVWLTKKNNAYPTIKKSYNNTNQEDLLVLWGIHHPNDAAEQTRLYQNPTTYISVGTSTLNQRLVPKIATRSKVNGQSGRMEFFWTILKSNDAINFESNGNFIAPENAYKIVKKGDSTIMKSELEYGDCNTKCQTPIGAINSSMPFHNIHPLTIGECPKYVKSNRLVLATGLRNSPQGERRRKKRGLFGAIAGFIEGGWQGMVDGWYGYHHSNEQGSGYAADKESTQKAIDGVTNKVNSIIDKMNTQFEAVGREFNNLERRIENLNKKMEDGFLDVWTYNAELLVLMENERTLDFHDSNVKNLYDKVRLQLRDNAKELGNGCFEFYHRCDNECMESVRNGTYDYPQYSEEARLKREEISGVKLESIGTYQILSIYSTVASSLALAIMV (SEQ ID NO:45), wherein this sequence corresponds to a variant of SEQ ID NO:8, wherein the variant starts with amino acid D17 of SEQ ID NO: 8, wherein the numbering of the N-terminal amino acid position (D17) refers to the amino acid position 17 as exemplarily given in SEQ ID NO 1.

In another example, the H5 protein according to the disclosure thus preferably consists of or comprises an amino acid sequence which is at least 95%, preferably 96%, more preferably 97%, still more preferably 98%, yet more preferably 99% or in particular preferably 100% homologous with the polypeptide sequence wherein the sequence corresponds to SEQ ID NO:13 starting with amino acid D17, and wherein the numbering of the first N-terminal amino acid position (D17) refers to the amino acid position 17 as exemplarily given in SEQ ID NO: 1.

It is thus understood, that the term "variants of SEQ ID NOs: 2 to 40" is preferably also directed to the variant sequences that start with amino acid D17 of SEQ ID NOs: 2 to 40, and wherein the variant sequences of SEQ ID NOs 2 to 39 starting with amino acid D17 are given in Table B and correspond to SEQ ID NOs: 55 to 92, and wherein the sequence of SEQ ID NO: 40 starting with amino acid D17 has the sequence

In a further preferred aspect the H5 protein according to the disclosure comprises a full length C-terminal sequence, wherein the full length C-terminal sequence preferably corresponds to amino acids 527 to 568, wherein the numbering of said amino acid positions refers to the amino acid positions 527 to 568 as exemplarily given in SEQ ID NO 40. The full length C-terminal sequence is thus preferably the sequence IGTYQILSIYSTVASSLALAIMVAGLFLWMCSNGSLQCRICI (SEQ ID NO: 48), wherein in a further preferred aspect the Phenyl alanine residue (F) is a Serine (S) residue or wherein in another preferred aspect the sequence IMVA (SEQ ID NO: 49) is subsituted by the sequence IMMA (SEQ ID NO: 50). Hence the full length C-terminal
sequence is thus preferably the sequence
IGTYQILSIYSTVASSLALAIMVAGLFLWMCSNGSLQCRICI (SEQ ID NO: 51) or the sequence IGTYQILSIYSTVASSLALAIMVAGLSLWMCSNGSLQCRICI (SEQ ID NO: 52) or the sequence IGTYQILSIYSTVASSLALAIMMAGLSLWMCSNGSLQCRICI (SEQ ID NO: 53).

In yet a further preferred aspect the H5 protein according to the disclosure thus consists of or comprises an amino acid sequence which is at least 95%, preferably 96%, more preferably 97%, still more preferably 98%, yet more preferably 99% or in particular preferably 100% homologous with any one of the sequences as set forth in SEQ ID NOs: 2 to 40, sequences as set forth in SEQ ID NOs: 47, 93-130 that correspond to variants of SEQ ID NOs: 2 to 40 that start with amino acid D17 and additionally comprise a full length C-terminal sequence, wherein the numbering of the first N-terminal amino acid position (D17) refers to the amino acid position 17 as exemplarily given in SEQ ID NO 1, and wherein the full length C-terminal sequence corresponds to amino acids 527 to 568, wherein the numbering of these amino acid positions refers to the amino acid positions 527 to 568 as exemplarily given in SEQ ID NO 40.

Table C provides the respective sequences SEQ ID NOs 93 to 130, wherein the sequences start with amino acid D17 and comprise a full-length C-terminal sequence, as described herein, and Table D provides the respective sequence SEQ ID NO: 131, wherein the sequence starts with amino acid D17 of SEQ ID NO: 40 and comprises a full-length C-terminal sequence, as described herein.

In one example, the H5 protein according to the disclosure thus preferably consists of or comprises an amino acid sequence which is at least 95%, preferably 96%, more preferably 97%, still more preferably 98%, yet more preferably 99% or in particular preferably 100% homologous with the polypeptide sequenceDQICIGYHANNSTEQVDTIMEKNVTVTHAQDILEKTHNGKLCNLDGVKPLILRDCSVAGWLLGNPMCDEFLNVPEWSYIVEKINPTNDLCYPGNFNDYEELKHLLSRINHFEKIQIIPKNSWSDHEASGVSSACPYQGRSSFFRNVVWLTKKNNAYPTIKKSYNNTNQEDLLVLWGIHHPNDAAEQTRLYQNPTTYISVGTSTLNQRLVPKIATRSKVNGQSGRMEFFWTILKSNDAINFESNGNFIAPENAYKIVKKGDSTIMKSELEYGDCNTKCQTPIGAINSSMPFHNIHPLTIGECPKYVKSNRLVLATGLRNSPQGERRRKKRGLFGAIAGFIEGGWQGMVDGWYGYHHSNEQGSGYAADKESTQKAIDGVTNKVNSIIDKMNTQFEAVGREFNNLERRIENLNKKMEDGFLDVWTYNAELLVLMENERTLDFHDSNVKNLYDKVRLQLRDNAKELGNGCFEFYHRCDNECMESVRNGTYDYPQYSEEARLKREEISGVKLESIGTYQILSIYSTVASSLALAIMVAGLFLWMCSNGSLQCRICI (SEQ ID NO:45), wherein said sequence corresponds to SEQ ID NO:8 starting with amino acid D17, and additionally comprising a full length C-terminal sequence, wherein the numbering of said first N-terminal amino acid position (D17) refers to the amino acid position 17 as exemplarily given in SEQ ID NO 1, and wherein the full length C-terminal sequence corresponds to amino acids 527 to 568, more particular to amino acids 534 to 568, wherein the numbering of said amino acid positions refers to the amino acid positions 527 to 568, or more particular to amino acid positions 534 to 568, as exemplarily given in SEQ ID NO 40. Herein, this sequence, SEQ ID NO: 45, and sequences having different C-terminal sequences may be referred to as a "variant of SEQ ID NO: 8".

It is thus further understood, that the term "variants of SEQ ID NOs 2 to 40" is preferably also directed to the sequences SEQ ID NOs: 2 to 40 starting with amino acid D17 and having the full length C-terminal sequence, and wherein said sequences SEQ ID No 2 to 39 starting with amino acid D17 and having the full length C-terminal sequence are given in Table C, and said sequence SEQ ID NO: 40 starting with amino acid D17 and having the full length C-terminal sequence is given in Table D.

The terms "variant sequences as set forth in SEQ ID NOs: 2 to 40" or "variant sequences of SEQ ID NOs: 2 to 40", as used herein, preferably also relate to the sequences as set forth in Table B or Table C, respectively, and, additionally the sequence as set forth in Table D, respectively.

It is in particular understood that the term "100% homologous with" as mentioned herein is equivalent with the term "identical to".

Preferably, such H5 protein and any further H5 protein is an isolated H5 protein. It has been found, that H5 proteins, having the modifications described above, are highly antigenic as compared to H5 proteins that do not have the corresponding amino acids.

The term "hemagglutinin 5 (H5)" or "H5 of avian influenza virus" or H5 protein" as used herein means, but is not limited to any naturally occurring H5 protein and any modified forms of H5 protein, including any deletion, substitution and/or insertion mutant of H5 protein, wherein those H5 proteins have
(a) the amino acids 113D, 126H, 145(-), 156R, 160F, 167T, and 181N, wherein the term "145(-)" means that the amino acid position 145 of H5 is deleted, or
(b) the amino acids 87P, 145L, 172T, 201E, 206I, 208K, 254T, 341G and 421K, or
(c) the amino acids 145L, 172T, 254V,
   or, more preferably, have
(d) the amino acids 87L, 113D, 126H, 145(-), 156R, 160F, 167T, and 181N, or
(e) the amino acids 87P, 113N, 126R, 145L, 160Y, 172T, 181H, 201E, 206I, 208K, 254T, 341G and 421K, or
(f) the amino acids 87L, 113N, 126R, 145L, 156G, 160Y, 172T, 181H, 254V.

The numbering of the amino acid positions of the H5 protein as used herein refers to the amino acid position as exemplarily given in SEQ ID NO:1. SEQ ID NO:1 represents the amino sequence of the hemagglutinin of strain 1709-6, which is an old official Egyptian prototype strain for the evaluation of vaccines. In other words, if reference is made to the amino acid at position 113 (amino acid 113), the amino acid residue is meant which corresponds to amino acid 113 of SEQ ID NO:1. However, this reference does not mean that the H5 proteins according to the disclosure have the identical amino acid sequence with SEQ ID NO:1. It only says that the corresponding amino acids of the H5 proteins according to the disclosure code for the amino acid residue as explicitly mentioned. In the current case, amino acid 113 would be Asparagine (N). The term "113D" exemplarily means that the amino acid at positions 113-numbering according to the amino acid positions of SEQ ID NO:1-shall code for the amino acid Aspartic Acid (D). In other words, if reference is made to "H5 protein having the amino acid 113D", a H5 amino acid molecule that normally codes for Asparagine at amino acid position 113-numbering according to the amino acid positions of SEQ ID NO:1-that amino acid shall be substituted by an Aspartic Acid (D). The term "145(-)" or "modification 145(-)" means, that at amino acid position 145 of H5 protein-numbering according to the amino acid positions of SEQ ID NO:1-the amino acid at said position, which is a Serine residue in SEQ ID NO:1, is deleted or missing, respectively. However, most of the known H5 sequences code at amino acid positions 145 for Serine. In any such cases, the term 145(-) modification means, that the residues usually flanking said position (e.g. Serine144 and Glycine146 in SEQ ID NO.1) are directly linked (e.g. to Serine144 and Glycine145). For example, using SEQ ID NO:1, the modified sequence would comprise the sequence Alanine-Serine-Glycine-Valine (ASGV) (SEQ ID NO: 54).

Furthermore, the term "hemagglutinin 5 (H5)" or "H5 of avian influenza virus" or "H5 protein" as used herein means, but is not limited to any naturally occurring H5 protein and any modified forms of H5 protein, including any deletion, substitution and/or insertion mutant of H5 protein, wherein those H5 proteins consist of or comprise an amino acid sequence which is at least 95%, preferably 96%, more preferably 97%, still more preferably 98%, yet more preferably 99% or in particular preferrably 100% homologous with any one of the sequences as set forth in SEQ ID NOs: 2 to 40. Preferably, the H5 protein consists of or comprises an amino acid sequence which is at least 95%, preferably 96%, more preferably 97%, still more preferably 98%, yet more preferably 99% or in particular preferably 100% homologous with any one of the sequences as set forth in SEQ ID NOs: 8, 13 or 40, and wherein H5 proteins that comprise or consist of the amino acid sequence set forth in SEQ ID NO: 13 are in particular most preferred.

Thus, the present disclosure relates to H5 protein and any modified forms of H5 protein, including any deletion, substitution and/or insertion mutant of H5 protein, wherein those H5 proteins have
(a) the amino acids 113D, 126H, 145(-), 156R, 160F, 167T, and 181N, wherein the term "145(-)" means that the amino acid position 145 of H5 is deleted, or
(b) the amino acids 87P, 145L, 172T, 201E, 206I, 208K, 254T, 341G and 421K, or
(c) the amino acids 145L, 172T, 254V,
   wherein the numbering of the amino acid positions of the H5 protein refers to the amino acid position as exemplarily given in SEQ ID NO:1; or
(d) wherein those H5 proteins consist of or comprise an amino acid sequence which is at least 95%, preferably 96%, more preferably 97%, still more preferably 98%, yet more preferably 99% or in particular preferably 100% homologous with any one of the sequences as set forth in SEQ ID NOs: 2 to 40.

Preferably, the H5 protein consists of or comprises an amino acid sequence which is at least 95%, preferably 96%, more preferably 97%, still more preferably 98%, yet more preferably 99% or in particular preferably 100% homologous with any one of the sequences as set forth in SEQ ID NOs: 8, 13 or 40, and most preferably wherein such H5 proteins comprise or consist of the amino acid sequence set forth in SEQ ID NO: 13.

It is self-explanatory, that any of the H5 proteins as provided herewith are antigenic, which mean they show antigenic properties in a standard hemagglutinin inhibition assay for influenza viruses.

According to a further aspect, the present disclosure also relates to any part of the H5 protein, which means any peptide-fragment which shows antigenic properties in a standard hemagglutinin inhibition assay, having at least
(a) the amino acids 113D, 126H, 145(-), 156R, 160F, 167T, and 181N, wherein the term "145(-)" means that the amino acid position 145 of H5 is deleted, or
(b) the amino acids 87P, 145L, 172T, 201E, 206I, 208K, 254T, 341G and 421K, or
(c) the amino acids 145L, 172T, 254V,
wherein the numbering of the amino acid positions of the H5 protein refers to the amino acid position as exemplarily given in SEQ ID NO:1.

A H5 protein shows antigenic properties if it inhibits hemagglutination in a standard hemagglutinin inhibition assay. Normally said antigenic part of H5 protein comprises 200, 180, 160, 150, 140, 130, 120, 110 or most preferably 105 contiguous amino acids of the amino acid sequence that codes for the H5 protein as mentioned above, modified or non-modified, which shows antigenic properties in an standard hemagglutinin inhibition assay. A standard hemagglutinin inhibition assay for example is also described in Stephenson et al., Virus Research vol. 103, pp. 91-95 (2004) with further references. However, the HI assay as described below shall be understood to be the relevant reference assay in connection with all aspects of the disclosure as described herein.

Briefly, HI assay is performed to detect the presence of HA-specific antibodies. A heterologous H5N1 is used at a concentration of four hemagglutinating units [4 HA units] in the HI assay. In U-bottomed microtiter plates serial two-fold serum dilutions in PBS are subsequently mixed with equal volumes (25 µL) containing 4 HA units of virus, and incubated at room temperature (about 25°C) for 30 minutes (min). Chicken red blood cells, at a concentration of 0.5% in PBS, are added to the serum-virus containing wells and incubated for 40 min at room temperature. The HI titers are determined as reciprocals of the highest serum dilutions in which inhibition of hemagglutination was observed.

Of note, *Haesebrouck and Pensaert (1986)* found "that there may exist a correlation between the HI titers against the challenge virus and protection from challenge". *Haesebrouck and Pensaert (1986)* also determined that pigs with HI titers of ≥40 were "completely resistant to challenge and no replication of the virus occurred in the respiratory tract at challenge". Thus, the development of HI titers ≥40 in the vaccinated swine would correlate to protection. (See F. Haesebrouck and M.B. Pensaert, 1986). Effect of intratracheal challenge of fattening pigs previously immunized with an inactivated influenza H1N1 vaccine (Veterinary Microbiology, 11 (1986) 239-249*.*) It is expected that equivalent or at least nearly equivalent H5 HI titers will also result in a complete immune protection of swine against avian influenza virus. Lower titers at least result in a seroconversion of the vaccinated animals and result in partial immune protection of those animals, which also can dramatically reduce the risk of a pandemic.

Moreover, an antigenic part of the H5 protein according to the disclosure includes, but is not limited to, deletion mutants of H5 protein which comprise:
i. any one of the amino acid sequences of SEQ ID NOs:2 to 40;
ii. any peptide that has at least 85%, preferably 95%, even more preferably 96%, even more preferably 97%, even more preferably 98%, even more preferably 99%, most preferably 100% sequence homology to the polypeptide of i) and that comprises hemagglutinin inhibition in a standard hemagglutinin inhibition assay; or
iii. any part of the polypeptides of i) or ii) comprising at least 334 contiguous amino acids of any of such peptides of i) or ii) and wherein any of such peptide comprises hemagglutinin inhibition in a standard hemagglutinin inhibition assay.

Preferably, the antigenic part of the H5 protein according to the disclosureconsists of or comprises an amino acid sequence which is at least 95%, preferably 96%, more preferably 97%, still more preferably 98%, yet more preferably 99% or in particular preferably 100% homologous with any one of the sequences as set forth in SEQ ID NOs: 8, 13, or 40, and most particularly wherein such H5 proteins comprise or consist of the amino acid sequence set forth in SEQ ID NO: 13.

The term "modification" in the context of the disclosure, in particular also refers to amino acid substitutions. For instance, the term "113D" in the context of SEQ ID NO.1 refers to the amino acid substition N113D, i.e. the amino acid asparagine (N) at position 113 has been subsituted by the amino acid aspartic acid (D).

"Sequence homology", as used herein, refers to a method of determining the relatedness of two sequences. To determine sequence homology, two or more sequences are optimally aligned, and gaps are introduced if necessary. In contrast to sequence identity, conservative amino acid substitutions are counted as a match when determining sequence homology. In other words, to obtain a polypeptide or polynucleotide having 95% sequence homology with a reference sequence, 85%, preferably 90%, even more preferably 95% of the amino acid residues or nucleotides in the reference sequence must match or comprise a conservative substitution with another amino acid or nucleotide, or a number of amino acids or nucleotides up to 15%, preferably up to 10%, even more preferably up to 5% of the total amino acid residues or nucleotides, not including conservative substitutions, in the reference sequence may be inserted into the reference sequence. Preferably the homolog sequence comprises at least a stretch of 50, even more preferred of 100, even more preferred of 250, even more preferred of 500 nucleotides. Upon such alignment, sequence homology is ascertained on a position-by-position basis, e.g., the sequences are "homolog" at a particular position if at that position, the nucleotides or amino acid residues are identical. The total number of such position identities is then divided by the total number of nucleotides or amino acid residues in the reference sequence to give % sequence homology. Sequence homology can be readily calculated by known methods, including but not limited to, those described in Computational Molecular Biology, Lesk, A. N., ed., Oxford University Press, New York (1988), Biocomputing: Informatics and Genome Projects, Smith, D.W., ed., Academic Press, New York (1993); Computer Analysis of Sequence Data, Part I, Griffin, A.M., and Griffin, H. G., eds., Humana Press, New Jersey (1994); Sequence Analysis in Molecular Biology, von Heinge, G., Academic Press (1987); Sequence Analysis Primer, Gribskov, M. and Devereux, J., eds., M. Stockton Press, New York (1991); and Carillo, H., and Lipman, D., SIAM J. Applied Math., 48: 1073 (1988). Preferred methods to determine the sequence homology are designed to give the largest match between the sequences tested. Methods to determine sequence homology are codified in publicly available computer programs which determine sequence identity between given sequences. Examples of such programs include, but are not limited to, the GCG program package (Devereux, J., et al., Nucleic Acids Research, 12(1):387 (1984)), BLASTP, BLASTN and FASTA (Altschul, S. F. et al., J. Molec. Biol., 215:403-410 (1990). The BLASTX program is publicly available from NCBI and other sources (BLAST Manual, Altschul, S. et al., NCVI NLM NIH Bethesda, MD 20894, Altschul, S. F. et al., J. Molec. Biol., 215:403-410 (1990)). These programs optimally align sequences using default gap weights in order to produce the highest level of sequence homology between the given and reference sequences.

Another preferable aspect according to the disclosure is the use of a H5 protein having the amino acids 113D, 126H, 145(-), 156R, 160F, 167T, and 181N, or preferably having the amino acids 87L, 113D, 126H, 145(-), 156R, 160F, 167T, and 181N, in a method of treating or preventing infections with a Subclade A H5N1 virus of North African origin, wherein the modification 145(-) means that amino acid position 145 of H5 is deleted and wherein the numbering of the amino acid positions of the H5 protein refers to the amino acid position as exemplarily given in SEQ ID NO:1, and wherein said H5 protein is administered to a subject in need thereof.

In this regard, namely for particularly treating or preventing infections with a Subclade A H5N1 virus of North African origin, it is preferred if the H5 protein used consists of or comprises a contiguous amino acid sequence which is at least 95%, preferably at least 96%, more preferably at least 97%, still more preferably at least 98%, yet more preferably at least 99%, or in particular preferably 100% homologous or identical with any one of the sequences as set forth in SEQ ID NOs: 2 to 12, or 35 or 36.

Likewise, a nucleic acid molecule coding for any of such H5 proteins, e.g. the sequence set forth in SEQ ID NO:41, or a vector comprising said nucleic acid molecule, as mentioned below, are used for a method of treating or preventing infections with a subclade A virus by administering said molecule or vector to a subject in need thereof.

In the context of the disclosure, the term "Subclade A H5N1 virus of North African origin" or "subclade A virus", respectively, in particular relates to any H5N1 influenza virus comprising a H5 protein having the amino acids 113D, 126H, 145(-), 156R, 160F, 167T, and 181N, or preferably having the amino acids 87L, 113D, 126H, 145(-), 156R, 160F, 167T, and 181N, wherein the modification 145(-) means that amino acid position 145 of H5 is deleted and wherein the numbering of the amino acid positions of the H5 protein refers to the amino acid position as exemplarily given in SEQ ID NO:1.

More particular, the term "Subclade A H5N1 virus of North African origin" or "subclade A virus", respectively, relates to any H5N1 influenza virus comprising a H5 protein with at least 95%, preferably at least 96%, more preferably at least 97%, still more preferably at least 98%, yet more preferably at least 99%, or in particular preferably 100% sequence identity with any one of the sequences as set forth in the SEQ ID NOs: 2 to 12, or 35 or 36.

A further preferable aspect according to the disclosure is the use of a H5 protein having the amino acids 87P, 145L, 172T, 201E, 206I, 208K, 254T, 341G and 421K, or preferably having the amino acids 87P, 113N, 126R, 145L, 160Y, 172T, 181H, 201E, 206I, 208K, 254T, 341G, or the use of a H5 protein having the amino acids 145L, 172T, 254V or preferably having the the amino acids 87L, 113N, 126R, 145L, 156G, 160Y, 172T, 181H, 254V, in a method of treating or preventing infections with a Subclade B H5N1 virus of North African origin, wherein the numbering of the amino acid positions of the H5 protein refers to the amino acid position as exemplarily given in SEQ ID NO:1, and wherein said H5 protein is administered to a subject in need thereof.

In this regard, namely for particularly treating or preventing infections with a Subclade B H5N1 virus of North African origin, it is preferred if the H5 protein used consists of or comprises a contiguous amino acid sequence which is at least 95%, preferably at least 96%, more preferably at least 97%, still more preferably at least 98%, yet more preferably at least 99%, or in particular preferably 100% homologous or identical with any one of the sequences as set forth in SEQ ID NOs: 13 to 34, or 37 to 39.

Likewise, in this regard, a nucleic acid molecule coding for any of such H5 proteins, e.g. the sequence set forth in SEQ ID NO:42, or a vector comprising said nucleic acid molecule, as mentioned below, are used for a method of treating or preventing infections with a subclade B virus by administering said molecule or vector to a subject in need thereof.

In the context of the disclosure, the term "Subclade B H5N1 virus of North African origin" or "subclade B virus", respectively, in particular relates to any H5N1 influenza virus comprising a H5 protein having the amino acids 87P, 145L, 172T, 201E, 206I, 208K, 254T, 341G and 421K, or preferably having the amino acids 87P, 113N, 126R, 145L, 160Y, 172T, 181H, 201E, 206I, 208K, 254T, and 341G, or comprising a H5 protein having the amino acids 145L, 172T, 254V or preferably having the the amino acids 87L, 113N, 126R, 145L, 156G, 160Y, 172T, 181H, and 254V, wherein the numbering of the amino acid positions of the H5 protein refers to the amino acid position as exemplarily given in SEQ ID NO:1.

More particularly, the term "Subclade B H5N1 virus of North African origin" or "subclade B virus", respectively, relates to any H5N1 influenza virus comprising a H5 protein with at least 95%, preferably at least 96%, more preferably at least 97%, still more preferably at least 98%, yet more preferably at least 99%, or in particular preferably 100% sequence identity with any one of the sequences as set forth in the SEQ ID NOs: 13 to 34, or 37 to 39.

A particular preferred aspect according to the disclosure is the use of a H5 protein comprising or consisting of a contiguous amino acid sequence which is at least 95%, preferably at least 96%, more preferably at least 97%, still more preferably at least 98%, yet more preferably at least 99%, or in particular preferably 100% homologous or identical with the sequence set forth in SEQ ID NO: 40 in a method of treating or preventing infections with a subclade A H5N1 virus of North African origin and/or with a subclade B H5N1 virus of North African origin, and wherein said H5 protein is administered to a subject in need thereof.

Likewise, in this regard, a nucleic acid molecule coding for any of such H5 proteins, such as the sequence set forth in SEQ ID NO:43, or a vector comprising said nucleic acid molecule, as mentioned below, are used for a method of treating or preventing infections with a subclade A virus and/or a subclade B virus by administering said molecule or vector to a subject in need thereof.

According to a further aspect, the present disclosure also relates to nucleic acid molecules, which code for any of the H5 proteins as described *supra.* Preferably, those nucleic acid molecules are RNA, DNA or copy (c)DNA molecules. Thus, the present disclosure relates to a nucleic acid molecule, preferably a cDNA molecule coding for a H5 protein or any modified forms of H5 protein, including any deletion, substitution and/or insertion mutant of H5 protein, wherein those H5 proteins have
(a) the amino acids 113D, 126H, 145(-), 156R, 160F, 167T, and 181N, wherein the term "145(-)" means that the amino acid position 145 of H5 is deleted, or
(b) the amino acids 87P, 145L, 172T, 201E, 206I, 208K, 254T, 341G and 421K, or
(c) the amino acids 145L, 172T, 254V,
   or, more preferably, have
(d) the amino acids 87L, 113D, 126H, 145(-), 156R, 160F, 167T, and 181N, or
(e) the amino acids 87P, 113N, 126R, 145L, 160Y, 172T, 181H, 201E, 206I, 208K, 254T, 341G and 421K, or
(f) the amino acids 87L, 113N, 126R, 145L, 156G, 160Y, 172T, 181H, 254V,
and wherein the numbering of the amino acid positions of the H5 protein refers to the amino acid position as exemplarily given in SEQ ID NO:1.

Furthermore, the present disclosure relates to a nucleic acid molecule, preferably a cDNA molecule coding for a H5 protein or any modified forms of H5 protein, including any deletion, substitution and/or insertion mutant of H5 protein,wherein the H5 protein consists of or comprises an amino acid sequence which is at least 95%, preferably 96%, more preferably 97%, still more preferably 98%, yet more preferably 99% or in particular preferred 100% homolog with any one of the sequences as set forth in SEQ ID NOs: 2 to 40. Preferably, the H5 protein consists of or comprises an amino acid sequence which is at least 95%, preferably 96%, more preferably 97%, still more preferably 98%, yet more preferably 99% or in particular preferred 100% homolog, with any one of the sequences as set forth in SEQ ID NOs: 8, 13 or 40, and wherein such H5 proteins comprise or consist of the amino acid sequence set forth in SEQ ID NO: 13 are in particular more preferred.

According to a further aspect, the present disclosure also relates to a nucleic acid molecule, preferably a cDNA molecule coding for any part of the H5 protein, which means encoding for any peptide-fragment which shows antigenic properties in a standard hemagglutinin inhibition assay as described *supra,* and having
(a) the amino acids 113D, 126H, 145(-), 156R, 160F, 167T, and 181N, wherein the term "145(-)" means that the amino acid position 145 of H5 is deleted, or
(b) the amino acids 87P, 145L, 172T, 201E, 206I, 208K, 254T, 341G and 421K, or
(c) the amino acids 145L, 172T, 254V,
   or, more preferably, having
(d) the amino acids 87L, 113D, 126H, 145(-), 156R, 160F, 167T, and 181N, or
(e) the amino acids 87P, 113N, 126R, 145L, 160Y, 172T, 181H, 201E, 206I, 208K, 254T, 341G and 421K, or
(f) the amino acids 87L, 113N, 126R, 145L, 156G, 160Y, 172T, 181H, 254V,
and wherein the numbering of the amino acid positions of the H5 protein refers to the amino acid position as exemplarily given in SEQ ID NO:1. Normally such nucleic acid molecules, which code for an antigenic part of H5 protein, comprise 600, 540, 480, 450, 420, 390, 360, 330 or most preferably 315 contiguous nucleotides of the nucleotide sequence that codes for the H5 protein as mentioned above, modified or non-modified, and which shows antigenic properties in a standard hemagglutinin inhibition assay as described herein.

According to a further aspect, the present disclosure also relates to a nucleic acid molecule, preferably a cDNA molecule coding for any part of the H5 protein, which means encoding for any peptide-fragment which shows antigenic properties in a standard hemagglutinin inhibition assay as described *supra,* wherein the H5 protein consists of or comprises an amino acid sequence which is at least 95%, preferably 96%, more preferably 97%, still more preferably 98%, yet more preferably 99% or in particular preferably 100% homologous with any one of the sequences as set forth in SEQ ID NOs: 2 to 40. Preferably, the H5 protein consists of or comprises an amino acid sequence which is at least 95%, preferably 96%, more preferably 97%, still more preferably 98%, yet more preferably 99% or in particular preferably 100% homologous with any one of the sequences as set forth in SEQ ID NOs: 8, 13 or 40, and wherein such H5 proteins comprise or consist of the amino acid sequence set forth in SEQ ID NO: 13 are in particular more preferred.

Further aspects of antigenic parts of the H5 protein are described *supra.* It is in the common knowledge of a person skilled in the art to construct any such nucleic acid molecules, preferably cDNA molecules which codes for the antigenic part of the H5 protein as described *supra.* This knowledge also includes but is not limited to the construction of nucleic acid molecules, preferably of cDNA molecules, which code for antigenic parts of the H5 protein as mentioned above.

Preferably and exemplarily, the aforementioned nucleic acid molecule consists of or comprises any one of the nucleic acid sequence as set forth in SEQ ID NOs: 41 to 43.

Methods, of how to introduce any of the above-mentioned modifications within the nucleotide sequence, including the encoding sequence of the H5 protein of an influenza virus, are well known in the art. The genomic sequence of the entire influenza virus can be modified according to the disclosure, for example according to the methods described in US 6,951,754, with further references.

Furthermore, there may be employed conventional molecular biology, microbiology, and recombinant DNA techniques within the skill of the art to modify a nucleic acid sequence coding for an antigen as described herein. Such techniques are explained fully in the literature. See, e.g., Sambrook et al., Molecular Cloning: A Laboratory Manual, Second Edition (1989) Cold Spring Harbor Laboratory Press, Cold Spring Harbor, N. Y*.;* DNA Cloning: A Practical Approach, Volumes I and II (D. N. Glover ed. 1985*);* Oligonucleotide Synthesis (M. J. Gait ed. 1984*);* Nucleic Acid Hybridization [B. D. Hames & S. J. Higgins eds.(1985*)];* Transcription And Translation [B. D. Hames & S. J. Higgins, eds. (1984*)];* Animal Cell Culture [R. I. Freshney, ed. (1986*)]*; Immobilized Cells And Enzymes [IRL Press, (1986*)]*; B. Perbal, A Practical Guide To Molecular Cloning (1984*);* F. M. Ausubel et al. (eds.), Current Protocols in Molecular Biology, John Wiley & Sons, Inc. 1994*).*

According to a further aspect, the present disclosure also relates to a vector that comprises any of such nucleic acid molecules as described *supra.* In other words, the present disclosure relates to a vector, that includes the coding sequence of any such H5 protein, or part thereof as described *supra.* Preferably, said vector is an expression vector, which allows the expression of any such H5 protein or part thereof as described *supra.* Vectors according to the disclosure are those which are suitable for the transfection or infection of bacterial, yeast or animal cells, *in vitro* or *in vivo.*

Vectors and methods for making and/or using vectors (or recombinants) for expression can be by or analogous to the methods disclosed in: U.S. Patent Nos. 4,603,112, 4,769,330, 5,174,993, 5,505,941, 5,338,683, 5,494,807, 4,722,848, 5,942,235, 5,364,773, 5,762,938, 5,770,212, 5,942,235, 382,425, PCT publications WO 94/16716, WO 96/39491, WO 95/30018, Paoletti, "Applications of pox virus vectors to vaccination: An update, "PNAS USA 93: 11349-11353, October 1996, Moss, "Genetically engineered poxviruses for recombinant gene expression, vaccination, and safety," PNAS USA 93: 11341-11348, October 1996, Smith et al., U. S. Patent No. 4,745,051, (recombinant baculovirus), Richardson, C.D. (Editor), Methods in Molecular Biology 39, "Baculovirus Expression Protocols" (1995 Humana Press Inc.), Smith et al., "Production of Human Beta Interferon in Insect Cells Infected with a Baculovirus Expression Vector", Molecular and Cellular Biology, Dec., 1983, Vol. 3, No. 12, p. 2156-2165; Pennock et al., "Strong and Regulated Expression of Escherichia coli B-Galactosidase in Infect Cells with a Baculovirus vector, "Molecular and Cellular Biology Mar. 1984, Vol. 4, No. 3, p. 399-406; EPA0 370 573, U. S. application No. 920,197, filed October 16,1986, EP Patent publication No. 265785, U. S. Patent No. 4,769,331 (recombinant herpesvirus), Roizman, "The function of herpes simplex virus genes: A primer for genetic engineering of novel vectors," PNAS USA 93:11307-11312, October 1996, Andreansky et al., "The application of genetically engineered herpes simplex viruses to the treatment of experimental brain tumors," PNAS USA 93: 11313-11318, October 1996, Robertson et al. "Epstein-Barr virus vectors for gene delivery to B lymphocytes", PNAS USA 93: 11334-11340, October 1996, Frolov et al., "Alphavirus-based expression vectors: Strategies and applications," PNAS USA 93: 11371-11377, October 1996, Kitson et al., J. Virol. 65,3068-3075,1991; U. S. Patent Nos. 5,591,439, 5,552,143, WO 98/00166, allowed U. S. applications Serial Nos. 08/675,556, and 08/675,566 both filed July 3,1996 (recombinant adenovirus), Grunhaus et al., 1992,"Adenovirus as cloning vectors," Seminars in Virology (Vol. 3) p. 237-52, 1993, Ballay et al. EMBO Journal, vol. 4, p. 3861-65,Graham, Tibtech 8,85-87, April, 1990, Prevec et al., J. Gen Virol. 70,42434, PCT WO 91/11525, Felgner et al. (1994), J. Biol. Chem. 269,2550-2561, Science, 259: 1745-49,1993 and McClements et al., "Immunization with DNA vaccines encoding glycoprotein D or glycoprotein B, alone or in combination, induces protective immunity in animal models of herpes simplex virus-2 disease", PNAS USA 93: 11414-11420, October 1996, and U. S. Patent Nos. 5,591,639, 5,589,466, and 5,580,859, as well as WO 90/11092, WO93/19183, WO94/21797, WO95/11307, WO95/20660, Tang et al., Nature and Furth et al. Analytical Biochemistry, relating to DNA expression vectors, inter alia. See also WO 98/33510; Ju et al., Diabetologia, 41: 736-739,1998 (lentiviral expression system); Sanford et al., U. S. Patent No. 4,945,050; Fischbachet al. (Intracel), WO 90/01543; Robinson et al., seminars in Immunology vol. 9, pp. 271-283 (1997), (DNA vector systems); Szoka et al., U. S. Patent No. (method of inserting DNA into living cells); McCormick et al., U. S. Patent No. 5,677,178 (use of cytopathic viruses); and U. S. Patent No. 5,928,913 (vectors for gene delivery), as well as other documents cited herein.

A viral vector, for instance, selected from pig herpes viruses, such as Aujeszky's disease virus, porcine adenovirus, poxviruses, especially vaccinia virus, avipox virus, canarypox virus, and swinepox virus, as well as DNA vectors (DNA plasmids) are advantageously employed in the practice of the disclosure.

### Methods of producing the H5 proteins according to the presentdisclosure

According to another aspect, the present disclosure provides methods of producing and/or recovering high amounts of recombinant H5 protein: i) by permitting infection of susceptible cells in culture with a recombinant viral vector containing H5 DNA coding sequences, wherein H5 protein is expressed by the recombinant viral vector, and ii) thereafter recovering the H5 protein from cell culture. High amounts of H5 protein means, but are not limited to, more than about 20 µg/mL cell culture, preferably more than about 25 µg/mL, even more preferably more than about 30 µg/mL, even more preferably more than about 40 µg/mL, even more preferably more than about 50 µg/mL, even more preferably more than about 60 µg/mL, even more preferably more than about 80 µg/mL, even more preferably more than about 100 µg/mL, even more preferably than about 150 µg/mL, and most preferably more than about 190 µg/mL.

According to a preferred aspect, the H5 protein is recovered by harvesting the whole (i.e. intact) SF+ cells expressing the H5 protein.

Preferred cells are those susceptible for infection with an appropriate recombinant viral vector, containing a H5 DNA and expressing the H5 protein. Preferably the cells are insect cells, and more preferably, they include the insect cells sold under the trademark SF+ insect cells (Protein Sciences Corporation, Meriden, CT). Preferred cell cultures have a cell count between about 0.3 - 2.0 x 10⁶ cells/mL, more preferably from about 0.35 - 1.9 x 10⁶ cells/mL, still more preferably from about 0.4 - 1.8 x 10⁶ cells/mL, even more preferably from about 0.45 - 1.7 x 10⁶ cells/mL, and most preferably from about 0.5 - 1.5 x 10⁶ cells/mL.

Preferred viral vectors include baculovirus such as BaculoGold (BD Biosciences Pharmingen, San Diego, CA), in particular provided that the production cells are insect cells. Although the baculovirus expression system is preferred, it is understood by those of skill in the art that other expression systems will work for purposes of the present disclosure, namely the expression of H5 into the supernatant of a cell culture. Such other expression systems may require the use of a signal sequence in order to cause H5 expression into the media.

Appropriate growth media will also be determinable by those of skill in the art with a preferred growth media being serum-free insect cell media such as Excell 420 (JRH Biosciences, Inc., Lenexa, KS) and the like.

The recombinant viral vector containing the H5 DNA sequences has a preferred multiplicity of infection (MOI) of between about 0.03 - 1.5, more preferably from about 0.05 - 1.3, still more preferably from about 0.09 - 1.1, and most preferably from about 0.1 - 1.0, when used for the infection of the susceptible cells. Preferably the MOIs mentioned above relates to one mL of cell culture fluid. Preferably, the method described herein comprises the infection of 0.35 - 1.9 x 10⁶ cells/mL, still more preferably of about 0.4 - 1.8 x 10⁶ cells/mL, even more preferably of about 0.45 - 1.7 x 10⁶ cells/mL, and most preferably of about 0.5-1.5 x 10⁶ cells/mL with a recombinant viral vector containing a H5 DNA and expressing the H5 protein having a MOI (multiplicity of infection) of between about 0.03 - 1.5, more preferably from about 0.05 - 1.3, still more preferably from about 0.09 - 1.1, and most preferably from about 0.1 - 1.0.

The infected cells are then incubated over a period of up to ten days, more preferably from about two days to about ten days, still more preferably from about four days to about nine days, and most preferably from about five days to about eight days. Preferred incubation conditions include a temperature between about 22 - 32°C, more preferably from about 24 - 30°C, still more preferably from about 25 - 29°C, even more preferably from about 26 - 28°C, and most preferably about 27°C. Preferably, the SF+ cells are observed following inoculation for characteristic baculovirus-induced changes. Such observation may include monitoring cell density trends and the decrease in viability during the post-infection period. It was found that peak viral titer is observed 3-5 days after infection and peak H5 protein expression in the cells is obtained between days 5 and 8, and/or when cell viability decreases to less than 10%.

Thus, one aspect of the present disclosure provides a method of producing and/or recovering recombinant H5 protein, preferably in amounts described above, by **i)** permitting infection of a number of susceptible cells (see above) in culture with a recombinant viral vector with a MOI as defined above, **ii)** expressing H5 protein by the recombinant viral vector, and **iii)** thereafter recovering the H5 protein from the cells obtained between days 5 and 8 after infection and/or cell viability decreases to less then 10%. Preferably, the recombinant viral vector is a recombinant baculovirus containing H5 DNA coding sequences and the cells are SF+ cells. Additionally, it is preferred that the culture be periodically examined for macroscopic and microscopic evidence of contamination or for atypical changes in cell morphology during the post-infection period. Any culture exhibiting any contamination should be discarded.

For recovery of H5 protein that will be used in an immunogenic or immunological composition such as a vaccine, the inclusion of an inactivation step is preferred in order to inactivate the viral vector.

An "immunogenic or immunological composition" refers to a composition of matter that comprises at least one antigen which elicits an immunological response in the host of a cellular and/ or antibody-mediated immune response to the composition or vaccine of interest. Usually, an "immunological response" includes but is not limited to one or more of the following effects: the production or activation of antibodies, B cells, helper T cells, suppressor T cells, and/or cytotoxic T cells and/or gamma-delta T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

Thus, the present disclosure also relates to a method of producing and/or recovering recombinant H5 protein, preferably in amounts described above, by **i)** permitting infection of a number of susceptible cells (see above) in culture with a recombinant viral vector with a MOI as defined above, **ii)** expressing H5 protein by the recombinant viral vector, **iii)** recovering the H5 expressed in cells obtained between days 5 and 8 after infection and/or cell viability decreases to less then 10%, and **iv)** inactivating the recombinant viral vector.

Preferably, this inactivation is done either just before or just after the filtration step, with after the filtration step being the preferred time for inactivation. Any conventional inactivation method can be used for purposes of the present disclosure. Thus, inactivation can be performed by chemical and/or physical treatments. In preferred forms, the volume of harvest fluids is determined and the temperature is brought to between about 32 - 42°C, more preferably between about 34 - 40°C, and most preferably between about 35 - 39°C. Preferred inactivation methods include the addition of cyclized binary ethylenimine (BEI), preferably in a concentration of about 1 to about 20 mM, preferably of about 2 to about 10 mM, still more preferably of about 2 to about 8 mM, still more preferably of about 3 to about 7 mM, most preferably of about 5 mM. For example the inactivation includes the addition of a solution of 2-bromoethyleneamine hydrobromide , preferably of about 0.4M, which has been cyclized to 0.2M binary ethylenimine (BEI) in 0.3N NaOH, to the fluids to give a final concentration of about 5mM BEI. Preferably, the fluids are then stirred continuously for 72 - 96 hours and the inactivated harvest fluids can be stored frozen at -40°C or below or between about 1 - 7°C. After inactivation is completed a sodium thiosulfate solution, preferably at 1.0M is added to neutralize any residual BEI. Preferably, the sodium thiosulfate is added in equivalent amount as compared to the BEI added prior to for inactivation. For example, in the event BEI is added to a final concentration of 5mM, a 1.0M sodium thiosulfate solution is added to give a final minimum concentration of 5 mM to neutralize any residual BEI.

Thus, one further aspect of the present disclosure relates to a method of producing recombinant H5 protein, preferably in amounts described above, by **i)** permitting infection of a number of susceptible cells (see above) in culture with a recombinant viral vector with a MOI as defined above, **ii)** expressing H5 protein by the recombinant viral vector, **iii)** recovering the H5 expressed in the cells obtained between days 5 and 8 after infection and/or cell viability decreases to less then 10%, and **iv)** inactivating the recombinant viral vector. Preferably, the recombinant viral vector is a baculovirus containing H5 DNA coding sequences and the cells are SF+ cells. Preferred inactivation steps are those described above. Preferably, inactivation is performed between about 35 - 39°C and in the presence of 2 to 8 mM BEI, still more preferred in the presence of about 5 mM BEI.

According to one further aspect of the present disclosure, the method described above also includes a neutralization step after step **iv).** This step **v)** comprises adding of an equivalent amount of an agent that neutralizes the inactivation agent within the solution. Preferably, if the inactivation agent is BEI, addition of sodium thiosulfate to an equivalent amount is preferred. Thus, according to a further aspect, step **v)** comprises adding of a sodium thiosulfate solution to a final concentration of about 1 to about 20 mM, preferably of about 2 to about 10 mM, still more preferably of about 2 to about 8 mM, still more preferably of about 3 to about 7 mM most preferably of about 5 mM, when the inactivation agent is BEI.

In preferred forms and especially in forms that will use the recombinant H5 protein in an immunogenic composition such as a vaccine, each lot of harvested H5 protein will be tested for inactivation by passage in the anchorage dependent, baculovirus susceptible insect cells, such as Sf9 cells. In a preferred form of this testing, 150 cm² of appropriate cell culture monolayer is inoculated with 1.0 mL of inactivated H5 fluids and maintained at 25 - 29°C for 14 days with at least two passages. At the end of the maintenance period, the cell monolayers are examined for cytopathogenic effect (CPE) typical of H5 baculovirus. Preferably, positive virus controls are also used. Such controls can consist of one culture of Sf9 cells inoculated with a non-inactivated reference H5 baculovirus and one flask of Sf9 cells that remain non-inoculated. After incubation and passage, the absence of virus-infected cells in the BEI treated viral fluids would constitute a satisfactory inactivation test. The control cells inoculated with the reference virus should exhibit CPE typical of H5 baculovirus and the non-inoculated flask should not exhibit any evidence of H5 baculovirus CPE. Alternatively, at the end of the maintenance period, the supernatant samples could be collected and inoculated onto a Sf9 96 well plate, which has been loaded with Sf9 cells, and then maintained at 25 - 29°C for 5 - 6 days. The plate is then fixed and stained with anti-H5 antibody conjugated to FITC or any labeled antibody directed to baculovirus specific proteins (i.e. gp64). The absence of CPE, H5 expression, or expression of baculovirus specific proteins (i.e. gp64) in the BEI treated viral fluids constitutes a satisfactory inactivation test. The control cells inoculated with the reference virus should exhibit CPE and IFA activity and the non-inoculated flask should not exhibit any evidence of H5 baculovirus CPE and contain no IFA activity.

Thus a further aspect described herein, relates to an inactivation test for determining the effectiveness of the inactivation of the recombination viral vector expressing H5 protein, comprises the steps: **i)** contacting at least a portion of the culture fluid containing the recombinant viral vector with an inactivating agent, preferably as described above, **ii)** adding a neutralization agent to neutralize the inactivation agent, preferably as described above, and **iii)** determining the residual infectivity by the assays as described above.

After inactivation, the relative amount of recombinant H5 protein in a sample can be determined in a number of ways. Preferred methods of quantitation include SDS-PAGE densitometry, ELISA, and animal vaccination studies that correlate known quantities of vaccine with clinical outcomes (serology, etc.). When SDS-PAGE is utilized for quantitation, the sample material containing an unknown amount of recombinant H5 protein is run on a gel, together with samples that contain different known amounts of recombinant H5 protein. A standard curve can then be produced based on the known samples and the amount of recombinant H5 in the unknown sample can be determined by comparison with this standard curve. Because ELISAs are generally recognized as the industry standard for antigen quantitation, they are preferred for quantitation.

### Vaccines comprising H5 proteins or nucleic acid molecules or vectors coding for those

According to a further aspect, the present disclosure relates to vaccines or pharmaceutical compositions in general, that comprises,
one or more of the H5 proteins as described herein;
i. one or more of the nucleic acid molecules as described herein, coding for any such H5 proteins ; and/or
ii. one or more of the vectors as described herein, including any such nucleic acid molecules and coding for any such H5 proteins as described herein; and
iii. a pharmaceutical acceptable carrier and/or excipient.

The term "pharmaceutical composition" "Pharmaceutical/vaccine composition" as described herein, includes but is not limited to, vaccines for the reduction or prevention of an infection or to a composition of matter for the treatment and lessening of an infection.

The preparation of nucleic acid based vaccines, preferably cDNA vaccines, coding for influenza hemagglutinin are described for example in Deck et al, Vaccine 1997; 15(1):71-78*;* Ulmer et al., Science 1993; 259:1745-1749*;* Ulmer et al., Vaccine 1994;12(16):1541-1544*.* Any of those methods can be used for the production of nucleic acid based vaccines, preferably cDNA vaccines, coding for an influenza H5 protein as described herein.

Moreover, a vaccine, which comprises H5 protein or parts thereof as described herein, can be produced by conventional approaches, e.g. by recombinant expression techniques or by biochemical purification and separation techniques. Recombinant expression techniques, including the expression in insect cells are well known in the art, and described for example in Sambrook et al., Molecular Cloning: A Laboratory Manual, Second Edition (1989) Cold Spring Harbor Laboratory Press, Cold Spring Harbor, N.Y*.;* DNA Cloning: A Practical Approach, Volumes I and II (D. N. Glover ed. 1985*);* Oligonucleotide Synthesis (M. J. Gait ed. 1984*);* Nucleic Acid Hybridization [B. D. Hames & S. J. Higgins eds.(1985*)];* Transcription And Translation [B. D. Hames & S. J. Higgins, eds. (1984*)];* Animal Cell Culture [R. I. Freshney, ed. (1986*)]*; Immobilized Cells And Enzymes [IRL Press, (1986*)]*; B. Perbal, A Practical Guide To Molecular Cloning (1984*);* F. M. Ausubel et al. (eds.), Current Protocols in Molecular Biology, John Wiley & Sons, Inc. 1994*).* Further examples of well established recombinant expression systems are bacterial expression systems such as *E. coli* or *B. subtilis,* yeast-based expression systems such as *S. cerevisiae* or *S. pombe,* or mammalian cell expression systems such as the BHK-, CHO- and/or NS0-based expression systems. Such systems are well known in the art and generally available, e.g. commercially through Clontech Laboratories, Inc. 4030 Fabian Way, Palo Alto, California 94303-4607, USA. Further expression strategies are for example described in Lüschow et al., Vaccine no. 19 (2001), pp. 4249-4259, or Veit et al., PNAS vol. 103 (2006), pp. 8197-8202*.* Furthermore, recombinant adeno-associated virus systems are well established and for example described in US 5,436,146 or WO200203872 with further references. Moreover, vaccinia (pox) virus based expression systems, for example as described in US 6,265,183 with further references, are also well established and suitable to produce recombinant antigen(s), antigenic composition(s) as used according to the disclosure. Further suitable expression systems make use of recombinant popova viruses, such as SV40, fowl pox virus, pseudorabies viruses and retroviruses.

The relevant pharmaceutical/vaccine compositions as described herein, can also comprise inactivated virus which comprises H5 protein as described herein, an apathogenic version of a live virus comprising H5 protein as described herein, preparation and/or fragments of a virus, wherein said preparation and/or fragment comprise the H5 protein as described herein.

The skilled person knows additional components which may be comprised in said compositions/vaccines together with antigen (see for example, Remington's Pharmaceutical Sciences. (1990). 18th ed. Mack Publ., East*on*). The expert may use known injectable, physiologically acceptable sterile solutions. For preparing a ready-to-use solution, aqueous isotonic solutions, such as e.g. saline or corresponding plasma protein solutions are readily available. The pharmaceutical composition/vaccine may be present as lyophylisates or dry preparations, which can be reconstituted with a known injectable solution directly before use under sterile conditions, e.g. as a kit of parts.

In addition the pharmaceutical/vaccine compositions of the present disclosure can include one or more veterinary-acceptable carriers. As used herein, "a veterinary-acceptable carrier" includes but is not limited to any and all solvents, dispersion media, coatings, adjuvants, stabilizing agents, diluents, preservatives, antibacterial and antifungal agents, isotonic agents, adsorption delaying agents, and the like.

Diluents can include water, saline, dextrose, ethanol, glycerol, and the like. Isotonic agents can include sodium chloride, dextrose, mannitol, sorbitol, and lactose, among others. Stabilizers include albumin and alkali salts of ethylendiamintetracetic acid, among others.

A preservative as used herein, refers to an anti-microbiological active agent, such as for example Gentamycin, Merthiolate, and the like. In particular adding of a preservative is most preferred for the preparation of a multi-dose composition. Those anti-microbiological active agents are added in concentrations effective to prevent the composition of interest for any microbiological contamination or for inhibition of any microbiological growth within the composition of interest.

"Adjuvants" as used herein, can include aluminum hydroxide and aluminum phosphate, saponins e.g., Quil A, QS-21 (Cambridge Biotech Inc., Cambridge MA), GPI-0100 (Galenica Pharmaceuticals, Inc., Birmingham, AL), water-in-oil emulsion, oil-in-water emulsion, water-in-oil-in-water emulsion, wherein an Emulsigen - based adjuvants is particularly preferred according to the disclosure.

The emulsion can be based in particular on light liquid paraffin oil (European Pharmacopoeia type); isoprenoid oil such as squalane or squalene ; oil resulting from the oligomerization of alkenes, in particular of isobutene or decene; esters of acids or of alcohols containing a linear alkyl group, more particularly plant oils, ethyl oleate, propylene glycol di-(caprylate/caprate), glyceryl tri-(caprylate/caprate) or propylene glycol dioleate; esters of branched fatty acids or alcohols, in particular isostearic acid esters. The oil is used in combination with emulsifiers to form the emulsion. The emulsifiers are preferably nonionic surfactants, in particular esters of sorbitan, of mannide (e.g. anhydromannitol oleate), of glycol, of polyglycerol, of propylene glycol and of oleic, isostearic, ricinoleic or hydroxystearic acid, which are optionally ethoxylated, and polyoxypropylene-polyoxyethylene copolymer blocks, in particular the Pluronic products, especially L121. See Hunter et al., The Theory and Practical Application of Adjuvants (Ed.Stewart-Tull, D. E. S.). John Wiley and Sons, NY, pp51-94 (1995) and Todd et al., Vaccine 15:564-570 (1997). Examples for suitable oil-in water emulsions are Emulsigen-based adjuvants, such as EMULSIGEN®, EMULSIGEN-D®, EMULSIGEN-P®, EMULSIGEN-75® (MVP Laboratories, Inc. Omaha, NE, USA). It has been found, that pharmaceutical/vaccine compositions that comprise H5 protein, preferably recombinant H5 protein as described herein, have been effectively adjuvanted with oil-in water emulsions, preferably with such Emulsigen-based adjuvants, more preferably with EMULSIGEN® and EMULSIGEN-D®.

Moreover, it is possible to use the SPT emulsion described on page 147 of "Vaccine Design, The Subunit and Adjuvant Approach" edited by M. Powell and M. Newman, Plenum Press, 1995, and the emulsion MF59 described on page 183 of this same book.

A further instance of an adjuvant is a compound chosen from the polymers of acrylic or methacrylic acid and the copolymers of maleic anhydride and alkenyl derivative. Advantageous adjuvant compounds are the polymers of acrylic or methacrylic acid which are cross-linked, especially with polyalkenyl ethers of sugars orpolyalcohols. These compounds are known by the term carbomer (Phameuropa Vol. 8, No. 2, June 1996). Persons skilled in the art can also refer to U. S. Patent No. 2,909,462 which describes such acrylic polymers cross-linked with a polyhydroxylated compound having at least 3 hydroxyl groups, preferably not more than 8, the hydrogen atoms of at least three hydroxyls being replaced by unsaturated aliphatic radicals having at least 2 carbon atoms. The preferred radicals are those containing from 2 to 4 carbon atoms, e.g. vinyls, allyls and other ethylenically unsaturated groups. The unsaturated radicals may themselves contain other substituents, such as methyl. The products sold under the name Carbopol ; (BF Goodrich, Ohio, USA) are particularly appropriate. They are cross-linked with an allyl sucrose or with allyl pentaerythritol. Among then, there may be mentioned Carbopol 974P, 934P and 971P. Most preferred is the use of Carbopol 971P. Among the copolymers of maleic anhydride and alkenyl derivative, the copolymers EMA (Monsanto) which are copolymers of maleic anhydride and ethylene. The dissolution of these polymers in water leads to an acid solution that will be neutralized, preferably to physiological pH, in order to give the adjuvant solution into which the immunogenic, immunological or vaccine composition itself will be incorporated.

Further suitable adjuvants include, but are not limited to, the RIBI adjuvant system (Ribi Inc.), Block co-polymer (CytRx, Atlanta GA), SAF-M (Chiron, Emeryville CA), monophosphoryl lipid A, Avridine lipid-amine adjuvant, heat-labile enterotoxin from E. coli (recombinant or otherwise), cholera toxin, or muramyl dipeptide among many others.

Preferably, the adjuvant is added in an amount of about 100 µg to about 10 mg per dose. Even more preferred the adjuvant is added in an amount of about 100 µg to about 10 mg per dose. Even more preferred the adjuvant is added in an amount of about 500 µg to about 5 mg per dose. Even more preferred the adjuvant is added in an amount of about 750 µg to about 2,5 mg per dose. Most preferred the adjuvant is added in an amount of about 1 mg per dose.

The pharmaceutical/vaccine compositions, can further include one or more other immunomodulatory agents such as, e. g., interleukins, interferons, or other cytokines. The pharmaceutical/vaccine compositions can also include Gentamicin and Merthiolate. While the amounts and concentrations of adjuvants and additives useful in the context of the present disclosure can readily be determined by the skilled artisan, the present disclosure contemplates compositions comprising from about 50 µg to about 2000 µg of adjuvant and preferably about 250 µg/ ml dose of the vaccine composition. In another preferred aspect, the present disclosure contemplates vaccine compositions comprising from about 1ug/ml to about 60 µg/ml of antibiotics, and more preferably less than about 30 µg/ml of antibiotics.

Thus, according to a further aspect, the present disclosure also relates to a pharmaceutical/vaccine composition comprising
I) a therapeutically effective amount of any one of the H5 proteins of influenza virus as described *supra,*
II) and a pharmaceutically acceptable adjuvants as described above.

Preferably, the adjuvant is selected from the group consisting of:
a) EMULSIGEN®, a oil-in-water emulsion (o/w);
b) EMULSIGEN-D®, a oil-in-water (o/w) with dimethyldioctadecylammonum bromide (DDA);
c) a Polygen, a copolymer;
d) EMULSIGEN-P®, a oil-in-water (o/w) with a proprietary immunostimulant;
e) Carbigen is a cross-linked polymer;
f) EMULSIGEN-75®, a double adjuvants comprise of a oil-in-water (o/w) with a cross-linked polymer; and
g) ISA 70 is a water-in-oil (w/o).

Most preferably, the adjuvants is a oil-in-water emulsion such as an emulsigen-based adjuvant selected from the group consisting of EMULSIGEN®, EMULSIGEN-D®, EMULSIGEN-P®, EMULSIGEN-75®, EMULSIGEN® and EMULSIGEN-P®. Most preferably EMULSIGEN® and EMULSIGEN-P® are used in the formulation of the current disclosure.

According to a further aspect, the pharmaceutical/vaccine compositions as provided herewith, comprise one or more antigen. Preferably, that further antigen is an antigen of a poultry or mammalian pathogen. According to a further aspect, that additional antigen is an further influenza antigen such as hemagglutinin H3, H7, H9, or any other hemagglutinin of influenza virus. The additional antigen(s) can be added in a purified form, as part of an antigenic preparation, in form of a killed microorganism or in form of a modified live microorganism.

The term "antigen", as used herein means, but is not limited to, peptides, polypeptides, glycopeptides, or polysaccharides which are capable of specifically interacting with an antigen recognition molecule of the immune system, such as an immunoglobulin (antibody) or T cell antigen receptor in order to elicit, activate or stimulate an immune response directed to said antigen in a host to which said antigen is administered. The term "antigen" also refers to nucleic acid molecules, preferably DNA- or RNA-molecules, each of which codes for and express a peptide, polypeptide, or glycopeptide that is capable of specifically interacting with an antigen recognition molecule of the immune system, such as an immunoglobulin (antibody) or T cell antigen receptor in order to elicit, activate or stimulate an immune response against the antigen that is coded by the nucleic acid molecule. The antigen used for the preparation of the pharmaceutical composition which is used according to the disclosure is a microorganism or an antigenic part and/or preparation of said microorganism. In this connection, the term "immunization", as used herein, means but is not limited to, any cause or enhancement of an immune response. The term "immune response" is already described *supra.*

Administration strategies for influenza vaccines are well known in the art. Mucosal vaccination strategies for inactivated and attenuated virus vaccines are contemplated. While the mucosa can be targeted by local delivery of a vaccine, various strategies have been employed to deliver immunogenic proteins to the mucosa.

In a specific aspect, the vaccine can be administered in an admixture with, or as a conjugate or chimeric fusion protein with, cholera toxin, such as cholera toxin B or a cholera toxin A/B chimera (Hajishengallis , J Immunol., 154:4322-32, 1995*;* Jobling and Holmes, Infect Immun., 60:4915-24, 1992). Mucosal vaccines based on use of the cholera toxin B subunit have been described (Lebens and Holmgren, Dev Biol Stand 82:215-27, 1994). In another aspect, an admixture with heat labile enterotoxin (LT) can be prepared for mucosal vaccination.

Other mucosal immunization strategies include encapsulating the virus in microcapsules (US 5,075,109, US 5,820,883, and US 5,853,763) and using an immunopotentiating membranous carrier (WO 98/0558). Immunogenicity of orally administered immunogens can be enhanced by using red blood cells (rbc) or rbc ghosts (US 5,643,577), or by using blue tongue antigen (US 5,690,938).

According to another aspect, the present disclosure relates to a method for preparing a pharmaceutical /vaccine composition as described above, preferably a method for producing a vaccine which comprises a recombinant, baculovirus expressed H5 protein as described *supra.* Generally, this method includes the steps of transfecting a construct into a virus, wherein the construct comprises **i)** recombinant H5 cDNA as described herein, **ii)** infecting cells in growth media with the transfected virus, **iii)** causing the virus to express the recombinant H5 protein as described herein **iv)** recovering the expressed H5 protein from the culture **v)** and preparing the composition by blending the expressed H5 protein with a suitable adjuvant and/or other pharmaceutically acceptable carrier.

Preferred adjuvants are those described above. Thus according to a further aspect, the method for preparing an antigenic composition, such as for example a vaccine, for invoking an immune response against influenza infections comprises i) preparing and recovering H5 protein, and **ii)** admixing this with a suitable adjuvants.

In addition, the vaccine composition of the present disclosure can also include diluents, isotonic agents, stabilizers, an/or preservatives. Diluents can include water, saline, dextrose, ethanol, glycerol, and the like. Isotonic agents can include anorganic or organic salts, e.g. sodium chloride, dextrose, mannitol, sorbitol, and lactose, saccharides, trehalose, mannitol, saccharose among others. Stabilizers include albumin and alkali salts of ethylendiamintetracetic acid, among others. Suitable adjuvants, are those described above.

### Medicinal use of any of such H5 proteins, nucleic acid molecules, vectors and vaccines

The H5 proteins as provided herewith, the nucleic acid molecules coding for any such H5 proteins, the vectors comprising any such nucleic acid molecules coding for any such H5 proteins as described herein, and any pharmaceutical/vaccine composition comprising any of such H5 protein, nucleic acid molecule or vector can be used as a medicine, preferably for the treatment and prophylaxis of infections, caused by influenza virus, most preferably by influenza A virus. The H5 proteins as provided herewith, the nucleic acid molecules encoding for any such H5 proteins, the vectors comprising any such nucleic acid molecules encoding for any such H5 proteins as described herein, and any pharmaceutical/vaccine composition comprising any of such H5 protein, nucleic acid molecule or vector, as described herein, can be used for the treatment or prophylaxis of human beings as well as in veterinary medicine. When used in veterinary medicine, the treatment of poultry, preferably bird, chicken, duck, turkey and the like as well as mammals, preferably pigs, cattle, horses, seals, camels, dogs, cats, hamsters, mice and the like, is preferred.

Thus, according to another aspect the present disclosure relates to the use of H5 proteins as provided herewith, the nucleic acid molecules encoding for any such H5 proteins, the vectors comprising any such nucleic acid molecules encoding for any such H5 proteins as described herein and any pharmaceutical/vaccine compositions comprising any of such H5 protein, nucleic acid molecule or vector as described herein, can be used as a medicine, preferably as a medicine for human beings and/or as veterinary medicine.

Moreover, H5 proteins as provided herewith, the nucleic acid molecules coding for any such H5 proteins, the vectors comprising any such nucleic acid molecules coding for any such H5 protein, as described herein, can be used for the preparation of a pharmaceutical composition, as described herein, for the prophylaxis or treatment of infections caused by viral influenza. As mentioned above, those pharmaceutical compositions/vaccine compositions can be used for the treatment and/or prophylaxis of human beings as well as for the treatment and/or prophylaxis of animals, such as poultry, preferably bird, chicken, duck, turkey and the like as well as mammals, preferably pigs, cattle, horses, seals, camels, dogs, cats, hamsters, mice and the like.

According to the disclosure, the use of the H5 protein as described herein for the preparation of a pharmaceutical composition for the prophylaxis or treatment of infections caused by viral influenza is preferred, preferably due to infections with H5N1 of North African origin, in particular due to infections with a virus comprising a H5 protein with at least 95%, preferably 96%, more preferably 97%, still more preferably 98%, yet more preferably 99% or in particular preferably 100% sequence identity, with any one of the sequences as set forth in SEQ ID NOs: 2 to 40, preferably with the sequences as set forth in SEQ ID NOs: 8, 13 or 40. The use of the H5 proteins encoded by the sequences as set forth in the SEQ ID NOs: 8, 13 or 40 are particularly preferred.

It is thus understood that the term "H5N1 of North African origin" according to the disclosure is in particular directed to a H5N1 virus comprising H5 protein with at least 95%, preferably 96%, more preferably 97%, still more preferably 98%, yet more preferably 99% or in particuclar preferably 100% sequence identity with any one of the sequences as set forth in the SEQ ID NOs:2 to 40. Preferably the term "H5N1 of North African origin" according to the disclosure is in particular directed to a H5N1 virus comprising H5 protein with at least 95%, preferably 96%, more preferably 97%, still more preferably 98%, yet more preferably 99% or in particuclar preferred 100% sequence identity with any one of the sequences as set forth in the SEQ ID NOs:8, 13 or 40. According to further preferred aspect, the term "H5N1 of North African origin" according to the disclosure is in particular directed to a H5N1 virus comprising H5 protein with at least 95%, preferably 96%, more preferably 97%, still more preferably 98%, yet more preferably 99% or in particuclar preferred 100% sequence identity with the sequence as set forth in the SEQ ID NO:13. According to further preferred aspect, the term "H5N1 of North African origin" according to the disclosure is in particular directed to a H5N1 virus comprising H5 protein with at least 95%, preferably 96%, more preferably 97%, still more preferably 98%, yet more preferably 99% or in particuclar preferably 100% sequence identity with the sequence as set forth in the SEQ ID NO:8.

Accordingly, the phrase "infection(s) with H5N1 of North African origin" as described herein, in particular refers to an infection with such a virus.

The disclosure is further directed to the use of the nucleic acid molecule according to the disclosure, such as a nucleic acid molecule consisting of or comprising any one of the nucleic acid sequences as set forth in SEQ ID NOs: 41 to 43, for the preparation of a pharmaceutical composition for the prophylaxis or treatment of infections caused by viral influenza, preferably caused by H5N1 of North African origin, in particular caused by a virus comprising H5 protein with at least 95%, preferably 96%, more preferably 97%, still more preferably 98%, yet more preferably 99% or in parituclar preferred 100% sequence identity, with any one of the sequences as set forth in SEQ ID NOs: 2 to 40, wherein the sequences as set forth in the SEQ ID NOs: 8, 13 and 40 are particularly preferred.

Likewise, the disclosure comprises the use of the vector according to the disclosure for the preparation of a pharmaceutical composition for the prophylaxis or treatment of infections caused by viral influenza, preferably caused by H5N1 of North African origin. In particular the vector codes for any H5 protein as described.

H5 proteins as provided herewith, the nucleic acid molecules coding for any such H5 proteins, the vectors comprising any such nucleic acid molecules coding for any such H5 proteins, as described herein, can be used for the preparation of a pharmaceutical composition, as described herein, are suitable for the treatment and prophylaxis of viral influenza infection, which preferably are caused by avian, swine or human influenza virus or any combination or hybrid thereof.

According to a further aspect, the present disclosure also relates to a method for the treatment or prophylaxis of influenza virus infections, wherein the method comprising administration of a therapeutically effective amount of the H5 protein as describe herein, to a subject in need of such a treatment. Moreover, the present disclosure also relates to a method for the treatment or prophylaxis of influenza virus infections, wherein the method comprising administration of a therapeutically effective amount of any H5 nucleic acid molecule or vector as described herein, that codes for any H5 protein as described herein, to a subject in need of such a treatment. Furthermore, the present disclosure also relates to a method for the treatment or prophylaxis of influenza virus infections, wherein the method comprising administration of a therapeutically effective amount of the vaccine comprising any such H5 protein, nucleic acid molecule or vector, as described herein, to a subject in need of such a treatment. The subject in need thereof can be a human being as well as an animal, preferably poultry, even more preferably bird, chicken, duck, turkey or a mammal, preferably pig, cattle, horse, seal, camel, dog, cat, hamster, mouse and the like.

Preferably, when chicken are vaccinated, the H5 protein as described herein can be used for vaccination at day 1 of age or later, e.g. at day 10, or at day 1 to 10, or at day 10 or later.

Preferably the influenza infection that can be treated by the administration of any H5 protein, the nucleic acid molecule or vector encoding for any such H5 protein, or any pharmaceutical / vaccine compositions as described herein, is caused by avian, swine or human influenza virus or any combination or hybrid thereof.

According to another aspect, the present disclosure relates to a kit of parts, that comprises **i)** any of such H5 protein as described herein, the nucleic acid molecule or vector encoding for any such H5 protein, or any pharmaceutical / vaccine composition comprising any of such H5 protein, nucleic acid molecule or vector as described herein, and **ii)** a package leaflet indicating the use of such H5 protein, nucleic acid molecule, vector or vaccine for the treatment or prophylaxis of infections caused by influenza virus. When chicken are vaccinated, the H5 protein as described herein can be used for vaccination at day 1 on age or later.

According to a further aspect, that kit in parts comprises at least one further antigen of a poultry or mammalian pathogen and the information indication the medicinal, human or veterinary use of that additional antigen.

### EXAMPLES

The following examples set forth preferred materials and procedures in accordance with the present invention. It is to be understood, however, that these examples are provided by way of illustration only, and nothing therein should be deemed a limitation upon the overall scope of the invention.

### EXAMPLE 1

### A. Molecular information about Egyptian isolates

Information is based on the classification scheme proposed by Dr. G. Cattoli. Subclade 2.2.1 of HP AIV H5N1 that has been previously reported for the North Africa area. Currently, genetic variation has given rise to sublineages A and B, which were circulating in Egypt during 2010. The above mentioned classification follows internatonal guidelines as available at the WHO website
"www.who.int./csr/disease/avian_influenza/guidelines/nomenclature/en/".

### a) Homology between the North African isolates.

Considering the sequences from the H5 hemagglutinin gene in positive samples from 2010, pairwise nucleotide distances within every sublineage are in the range 0.5 to 1.4 , which is less than 1.5 % (sequences are above 98.5 % similar).

Above values are under the criteria defined to classify new isolates as possible sublineages, according to WHO/FAO/OIE guidelines.

The percentage of similarity between sequences from sublineage A and sublineage B, from subclade 2.2.1 circulating in North Africa in 2010, is between 96-98 %.

### b) Differences between Egyptian isolates H5N1 vs. other H5N1.

In the following table the percentage of similarity of representative sequences from sublineages A and B from 2010 Highly pathogenic Avian Influenza (HPAI) H5N1, currently circulating in North Africa, in comparison with the corresponding sequences from prototype H5N1 strains are found.

| Representative strain | Sublineage A | Sublineage B |
|---|---|---|
| Subclade 1 (Vietnam-1304-2004) | 94.5 | 93.3 |
| Subclade 2.3.4 (Anhui-1-2005) | 94.3 | 92.8 |
| Subclade 2.1 (Indonesia-5-2005) | 94.8 | 94 |

### c) Amino Acid Sequences

The 38 sequences provided by IZSVe (Istituto Zooprofilattico Sperimentale delle Venezie) are found below and in the sequence listing.

### B. Identification criteria for the selection of vaccine strains, relevant motives in the sequence

### Criterium 1.

Genetic distances as related with phylogenetic analysis. The separation of taxa in a phylogenetic tree is the support to infer the most distant related isolates according to the H5 HA sequence. The most distant isolates were selected.

### Criterium 2.

Frequency of each sublineage in the sampling from 2010. This frequency is not related to how much the samples represent the field circulation of HPAI H5N1 virus. The prevalence of every sublineage in the whole 2010 data set was the basis to pick isolates and proposed them as new challenge strains.

In addition, sequence-based analysis was used to confirm phylogenetic findings. Changes in the H5 HA sequence inside or around the Receptor-Binding Site (RBS) were considered important to define new vaccine strains as well. In Tables 1 and 2, below, the antigen sequence variation is shown.

The consensus sequence is expected to combine properties from all the sequences available. This is, in the event that the consensus sequence does not exist in nature for unknown reasons, the expression platform utilized for the vaccine, and offers the opportunity to express a protein which could provide unique antigenic features.

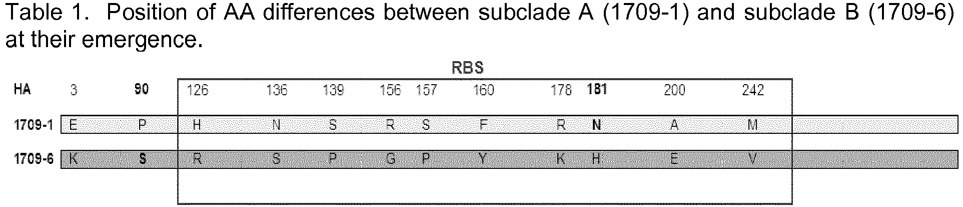

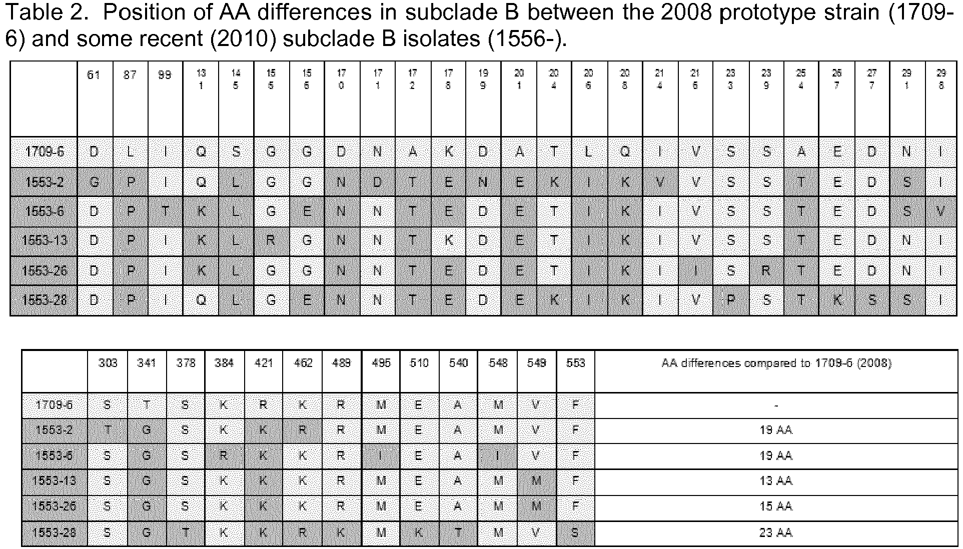

### C. Identification criteria for the selection of challenge strains

The criteria for the selection of the challenge strains were essentially the same criteria of the vaccine strains as mentioned above.

### Criterium 1.

Genetic distances as related with phylogenetic analysis were examined. The separation of taxa in a phylogenetic tree is the support to infer the most distant related isolates according to the H5 HA sequence. The most distant isolates were selected. Criterium 2.

Frequency of each sublineage in the sampling from 2010 was examined. This frequency is not related with how much the samples represents the field circulation of HPAI H5N1 virus. The prevalence of every sublineage in the whole 2010 data set was the basis to pick up isolates and proposed them as new challenge strains.

In addition, sequence-based analysis was used to confirm phylogenetic findings. Changes in the H5 HA sequence inside or around the Receptor-Binding Site (RBS) were considered important to define new vaccines or challenge strains as well. In the picture below antigen sequence variation is shown.

Table 1 depicts the position of AA (amino acid) differences between subclade A (1709-1) and subclade B (1709-6) at their emergence, and Table 2 depicts the position of AA differences in subclade B between the 2008 prototype strain (1709-6) and some recent (2010) subclade B isolates (1553).

### D. Construction of a recombinant baculoviruses coding for and expressing HA H5 antigens

The recombinant baculovirus containing the H5 HA antigen is generated as follows: the coding sequences of the H5 HA (SEQ ID NO:8, SEQ ID NO:13, SEQ ID NO:40) are chemically synthesized and subcloned into the transfer vector pVL1392 (BD Biosciences Pharmingen, San Diego, CA). The pVL1392 plasmids containing the genes coding for each H5 HA antigen are then co-transfected with DiamondBac® (Sigma) baculovirus DNA into Sf9 insect cells (BD Biosciences Pharmingen) to generate the recombinant baculovirus containing the genes H5 HA. The recombinant baculoviruses containing the genes coding for H5 HA are plaque-purified and Master Seed Viruses (MSVs) are propagated on the SF+ cell line, aliquoted, and stored at -70°C. Insect cells are infected with H5 HA baculoviruses as described above to generate MSV or Working Seed. Viruses express H5 HA antigen as detected by polyclonal serum or monoclonal antibodies in an indirect fluorescent antibody assay or Western blot.

After being seeded with the appropriate amounts of recombinant baculoviruses (H5 HA for each antigen sequence, respectively), spinner flasks containing SF+ cells (Protein Sciences, Inc., Meriden, CT) are then incubated at 27 ± 2°C for 7 days and with stirring at 100rpm during that time. The flasks used ventilated caps to allow for air flow. The crude whole cell culture containing baculovirus infected SF+ cells and the cell culture supernatants of each culture are harvested.

### E. Data for the HI, serological response of the Egyptian isolates vs. BEST H5 (current construct) vaccine

In the following table, values of HI titers and corresponding GMT's (Geometric Mean titers) are shown, for groups of chickens vaccinated with the BEST Al + ND KV (Baculovirus Expressions System Technology and Newcastle Disease, Killed Virus) vaccine at 3 weeks of age. The same experimental groups were challenged with the mentioned Egyptian HPAI H5N1 strains and the level of protection is included to confirm that the level of antibodies correlated with a high degree of protection.

The following table depicts: Vaccine prototypes including the H5 HA antigen that triggers a good immune response. HI titers, as a measurement of antibodies levels, from chickens vaccinated at 10 days of age exhibited values above 4 logs base2. In the table, raw data are shown from each vaccinated animal and corresponding GMT per group. The level of antibodies for the two groups was confirmed that conferred protection before challenge using the strains shown at the bottom. The strains included a recent 2010 field-isolate of HP H5N1 AIV ("1553-2"), and the official strain ("1709-6") that the Egyptian government uses to evaluate vaccine batches.

| **Group 1A** | | **Group 1C** | |
|---|---|---|---|
| **Antigen** | **H5BEST** | **Antigen** | **H5Best** |
| **Animal ID** | **HI titre Log2** | **Animal ID** | **HI titre Log2** |
| **1** | 7 | **14** | 7 |
| **5** | 8 | **15** | 6 |
| **6** | 7 | **16** | 7 |
| **7** | 7 | **17** | 6 |
| **8** | 7 | **23** | 7 |
| **9** | 8 | **24** | 7 |
| **10** | 6 | **26** | 7 |
| **11** | 7 | **28** | 9 |
| **12** | 7 | **29** | 7 |
| **13** | 8 | **30** | 7 |
| **GMT** | 7.2 | **GMT** | 7 |
| | | | |

| **GROUP** | **Survival rate** | **Challenge Virus** | |
|---|---|---|---|
| **1A** | **90%** | A/chicken/Egypt/1553-2/2010 | |
| **1C** | **90%** | A/chicken/Egypt/1709-6/2008 | |

### EXAMPLE 2

### Preparation of pharmaceutical compositions (vaccines) comprising different HA H5 antigens

The crude whole cell H5 HA protein expressed in insect cells by baculovirus-based expression system is harvested. Baculoviruses are inactivated in the presence of 10 mM cyclized binary ethylenimine (BEI) (final concentration) between about 32 and 39°C for 72 to 96 hours. After inactivation is completed, a 0.3 M sodium thiosulfate solution is added to a final concentration of 10mM to neutralize any residual BEI. After neutralization various adjuvants are added and the following vaccine/ pharmaceutical compositions are generated.

**VACCINES**

| **Generic product name** | 501 |
|---|---|
| **Antigen** | Crude whole-cell H5 HA Clade A protein expressed in insect cells by a baculovirus-based expression system. |
| **Formulation** | An experimental vaccine comprised of cultured insect cells and supernatant expressing recombinant H5 HA. The vaccine is adjuvanted with Emulsigen. |
| | |

| **Generic product name** | 502 |
|---|---|
| **Antigen** | Crude whole-cell H5 HA Clade A protein expressed in insect cells by a baculovirus-based expression system. |
| **Formulation** | An experimental vaccine comprised of cultured insect cells and supernatant expressing recombinant H5 HA. The vaccine is adjuvanted with Emulsigen-D. |

| **Generic product name** | 503 |
|---|---|
| **Antigen** | Crude whole-cell H5 HA Clade A protein expressed in insect cells by a baculovirus-based expression system. |
| **Formulation** | An experimental vaccine comprised of cultured insect cells and supernatant expressing recombinant H5 HA. The vaccine is adjuvanted with Polygen. |
| | |

| **Generic product name** | 504 |
|---|---|
| **Antigen** | Crude whole-cell H5 HA Clade A protein expressed in insect cells by a baculovirus-based expression system. |
| **Formulation** | An experimental vaccine comprised of cultured insect cells and supernatant expressing recombinant H5 HA. The vaccine is adjuvanted with Emulsigen-P. |
| | |

| **Generic product name** | 505 |
|---|---|
| **Antigen** | Crude whole-cell H5 HA Clade A protein expressed in insect cells by a baculovirus-based expression system. |
| **Formulation** | An experimental vaccine comprised of cultured insect cells and supernatant expressing recombinant H5 HA. The vaccine is adjuvanted with Carbigen. |
| | |

| **Generic product name** | 506 |
|---|---|
| **Antigen** | Crude whole-cell H5 HA Clade A protein expressed in insect cells by a baculovirus-based expression system. |
| **Formulation** | An experimental vaccine comprised of cultured insect cells and supernatant expressing recombinant H5 HA. The vaccine is adjuvanted with Emulsigen-75. |
| | |

| **Generic product name** | 507 |
|---|---|
| **Antigen** | Crude whole-cell H5 HA Clade A protein expressed in insect cells by a baculovirus-based expression system. |
| **Formulation** | An experimental vaccine comprised of cultured insect cells and supernatant expressing recombinant H5 HA. The vaccine is adjuvanted with ISA 70. |

Series 501 to 507 corresponds to the same antigen H5 HA Clade A formulated using seven different adjuvants. Series 508-514 is the corresponding series with antigen H5 HA Clade B. Series 515-521 is the corresponding series with antigen H5 HA Clade consensus. Series 522-528 is the corresponding series with antigen H5 HA Clade WHO.

### EXAMPLE 3

### Vaccination of birds against avian influenza

### INTRODUCTION

The purpose of this study is to determine the ability of experimental vaccines containing a crude extract of recombinant H5 hemagglutinin antigen to induce hemagglutination inhibition (HI) titers in chicken and to provide protection from challenge with Highly Pathogenic (HP) H5N1 Avian Influenza Virus (AIV). In addition, a conventional inactivated vaccine Volvac® AI (Boehringer Ingelheim Vetmedica, Mexico) is used for control.

### STUDY DESIGN:

SPF birds (15-25) are vaccinated independently with different experimental vaccines at 1 or 10 days of age by subcutaneous route with 0.2 or 0.5 ml in the back of the neck; all the birds are maintained in isolators during the experiment. Feed and water are provided *ad libitum.* Challenge is conducted 21 days post vaccination with a H5N1 highly pathogenic avian Influenza strain.

Serum samples are obtained by bleeding birds from the jugular vein at 7, 14, and 21 days post-vaccination. The serums obtained are stored to 4°C until running the Hemagglutination Inhibition (HI) test, as described above, to obtain the antibodies titers.

### VACCINES and CHALLENGE VIRUS:

Four different formulations are evaluated independently:
i. H5 HA Clade A: H5 HA antigen is formulated in oil emulsion based on Boehringer Ingelheim Vetmedica procedures.
ii. H5 HA Clade B: H5 HA antigen is formulated in oil emulsion based on Boehringer Ingelheim Vetmedica procedures..
iii. H5 HA Clade consensus: H5 HA antigen is formulated in oil emulsion based on Boehringer Ingelheim Vetmedica procedures.
iv. H5 HA Clade WHO: H5 HA antigen is formulated in oil emulsion based on Boehringer Ingelheim Vetmedica procedures.

An Avian Influenza Boehringer Ingelheim Vetmedica oil emulsion vaccine is used as control Volvac® AI (Boehringer Ingelheim Vetmedica, Mexico).

Challenge are conducted in vaccinated and non-vaccinated chickens by inoculation by intra-nasal route with 0.2 ml containing 10^{6.0} EID-50/ml per bird of the H5N1 challenge virus. After the challenge, signs and mortality are recorded. Ten days post-inoculation all the survivors chickens are euthanized according to animal laboratory procedures.

### Example of the experimental design of the prototypes, formulation and clinical studies

Water/oil emulsion including 500 HAU's / dose (HAU=Haemmagglutination Units) is prepared for each BEST (Baculovirus Expression System Technology)-Egypt construct. Three constructs are foreseen, based on recent discussions. SPF (Specific Pathogen Free) chickens are vaccinated once at day 10 of age using 0.5 ml of every vaccine prototype per bird, by the sub-cutaneous route in the back of the neck.

The positive control for protection is vaccinated administering 0.5 ml of the VOLVAC® AI + ND KV (Boehringer Ingelheim Vetmedica, Mexico) by the subcutaneous route in the back of the neck.

| **Vaccine prototype** | **Vaccination age** | **Challenge age** | **Cloacal and Tracheal swabs** |
|---|---|---|---|
| BEST-Egypt Subclade A | 1-10 | 31 | 3,5,7, 10, postinfection |
| BEST-Egypt Subclade B | 1-10 | 31 | 3,5,7, 10, postinfection |
| BEST-Egypt Consensus | 1-10 | 31 | 3,5,7, 10, postinfection |
| BEST-Egypt WHO | 1-10 | 31 | 3,5,7, 10, postinfection |
| VOLVAC® AI + ND KV | 1-10 | 31 | 3, 5, 7, 10, postinfection |
| Control (no vaccinated) | - | 31 | 3,5,7, 10, postinfection |

Each bird is challenged with 10 to 6 EID50/0.1ml viral dose oronasally. The experimental infection uses challenge strain for Egypt (A/chicken/Egypt/1063/2010).

### RESULTS:

Positive serum titer is considered log₂ 4 according to OIE standards. Based on this criterion, serological results are posittive. The best serological titers are observed in the vaccine formulated with the H5 HA antigens, both in birds vaccinated at 1 day of age or 10 days of age. The lowest serological titers are observed with the Volvac® vaccine. In the challenge study it is observed, that all vaccines prototypes confer full protection. The lowest protection in the challenge study is observed with the Volvac® vaccine, when administered, both at 1 or 10 days of age.

### In the sequence listing (SEQ ID NOs: 1 to 43):

SEQ ID NO: 1 corresponds to H5 sequence of H5N1 "1709-6",
SEQ ID NO: 2 corresponds to H5 sequence of H5N1 "1553-1/A1",
SEQ ID NO: 3 corresponds to H5 sequence of H5N1 "1553-15/A1",
SEQ ID NO: 4 corresponds to H5 sequence of H5N1 "2095-50/A1",
SEQ ID NO: 5 corresponds to H5 sequence of H5N1 "3982-2/A1",
SEQ ID NO: 6 corresponds to H5 sequence of H5N1 "3982-5/A1",
SEQ ID NO: 7 corresponds to H5 sequence of H5N1 "3982-7/A1",
SEQ ID NO: 8 corresponds to H5 sequence of H5N1 "3982-8/A1",
SEQ ID NO: 9 corresponds to H5 sequence of H5N1 "3982-9/A1",
SEQ ID NO: 10 corresponds to H5 sequence of H5N1 "3982-12/A1",
SEQ ID NO: 11 corresponds to H5 sequence of H5N1 "3982-20/A1",
SEQ ID NO: 12 corresponds to H5 sequence of H5N1 "3982-44/A1",
SEQ ID NO: 13 corresponds to H5 sequence of H5N1 "1553-2/B1",
SEQ ID NO: 14 corresponds to H5 sequence of H5N1 "1553-6/B1",
SEQ ID NO: 15 corresponds to H5 sequence of H5N1 "1553-13/B2",
SEQ ID NO: 16 corresponds to H5 sequence of H5N1 "1553-26/B2",
SEQ ID NO: 17 corresponds to H5 sequence of H5N1 "1553-28/B1",
SEQ ID NO: 18 corresponds to H5 sequence of H5N1 "2095-39/B2",
SEQ ID NO: 19 corresponds to H5 sequence of H5N1 "2095-46/B1",
SEQ ID NO: 20 corresponds to H5 sequence of H5N1 "2095-49/B1",
SEQ ID NO: 21 corresponds to H5 sequence of H5N1 "2095-65/B1",
SEQ ID NO: 22 corresponds to H5 sequence of H5N1 "2095-68/B2",
SEQ ID NO: 23 corresponds to H5 sequence of H5N1 "2095-70/B2",
SEQ ID NO: 24 corresponds to H5 sequence of H5N1 "2095-73/B2",
SEQ ID NO: 25 corresponds to H5 sequence of H5N1 "2095-75/B2",
SEQ ID NO: 26 corresponds to H5 sequence of H5N1 "3982-3/B1",
SEQ ID NO: 27 corresponds to H5 sequence of H5N1 "3982-4/B1",
SEQ ID NO: 28 corresponds to H5 sequence of H5N1 "3982-13/B1",
SEQ ID NO: 29 corresponds to H5 sequence of H5N1 "3982-14/B2",
SEQ ID NO: 30 corresponds to H5 sequence of H5N1 "3982-19/B3",
SEQ ID NO: 31 corresponds to H5 sequence of H5N1 "3982-21/B2",
SEQ ID NO: 32 corresponds to H5 sequence of H5N1 "3982-43/B1",
SEQ ID NO: 33 corresponds to H5 sequence of H5N1 "3982-50/B1",
SEQ ID NO: 34 corresponds to H5 sequence of H5N1 "3982-52/B1",
SEQ ID NO: 35 corresponds to H5 sequence of H5N1 "3982-55/A1",
SEQ ID NO: 36 corresponds to H5 sequence of H5N1 "3982-56/A1",
SEQ ID NO: 37 corresponds to H5 sequence of H5N1 "3982-78/B2",
SEQ ID NO: 38 corresponds to H5 sequence of H5N1 "4794-17/B ",
SEQ ID NO: 39 corresponds to H5 sequence of H5N1 "4794-18/B",
SEQ ID NO: 40 corresponds to H5 sequence translated from SEQ ID NO: 43,
SEQ ID NO: 41 codes for a H5 sequence of H5N1 "3982-8/A1" (SEQ ID NO: 8),
SEQ ID NO: 42 codes for a H5 sequence of H5N1 "1553-2/B1" (SEQ ID NO: 13), and
SEQ ID NO: 43 corresponds to the consensus sequence obtained after analysis of the 38 H5 HA gene sequences coding for SEQ ID NOs: 2 to 39.

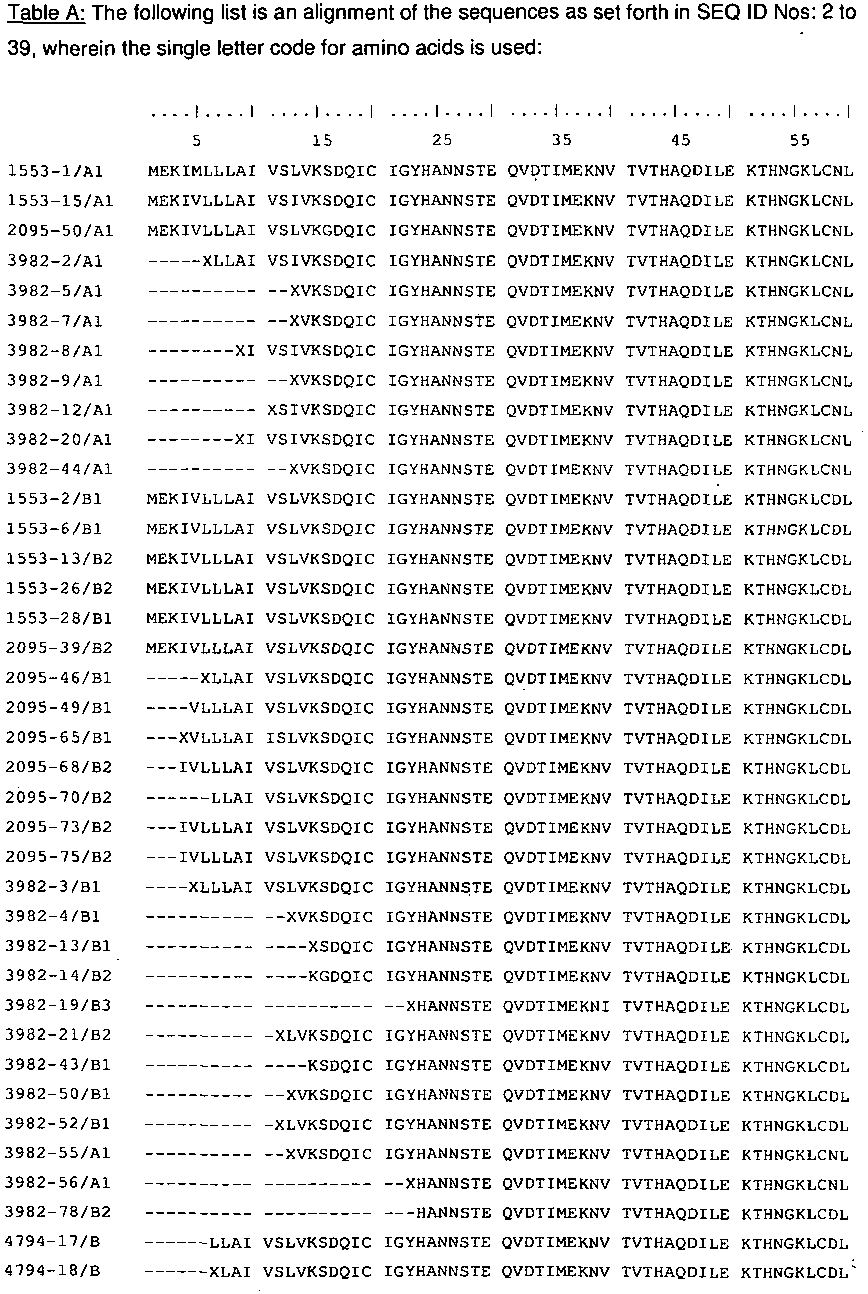

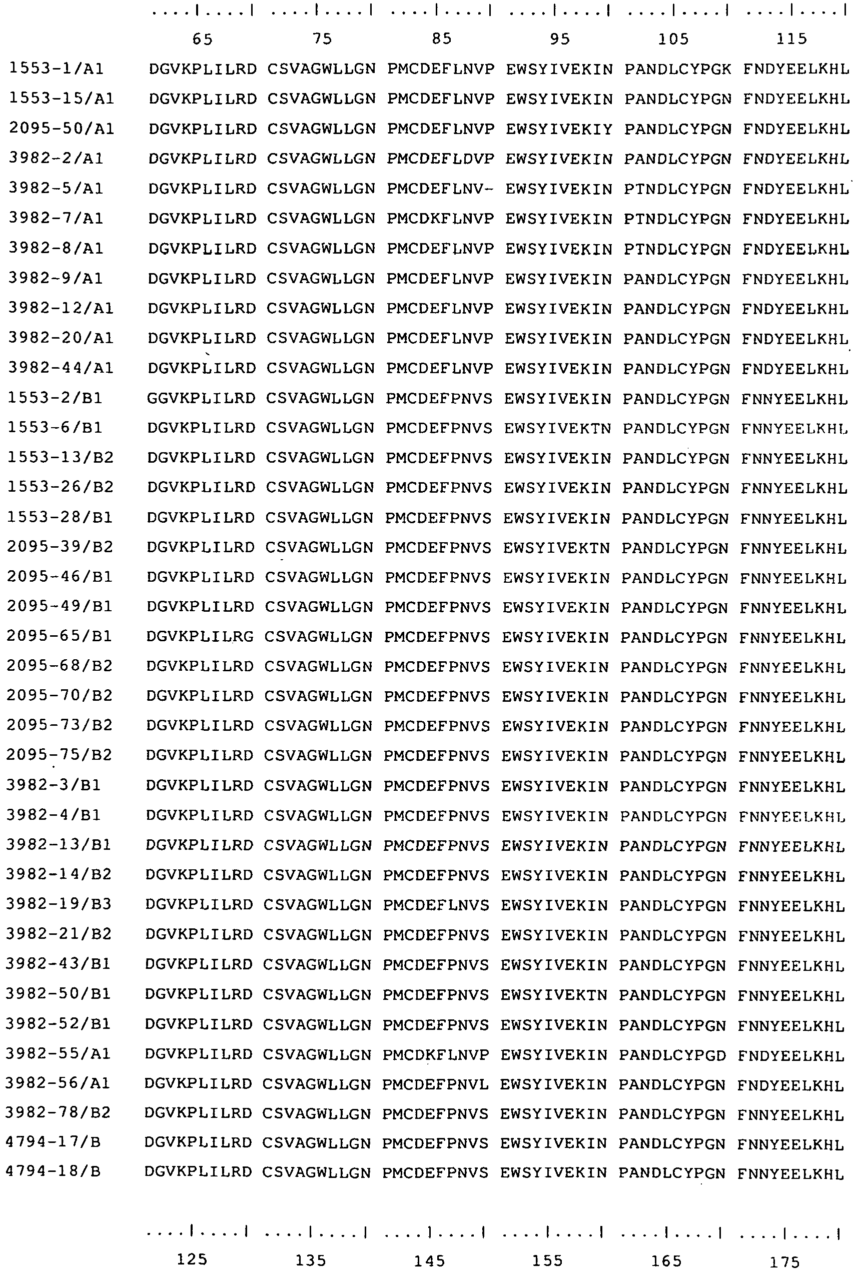

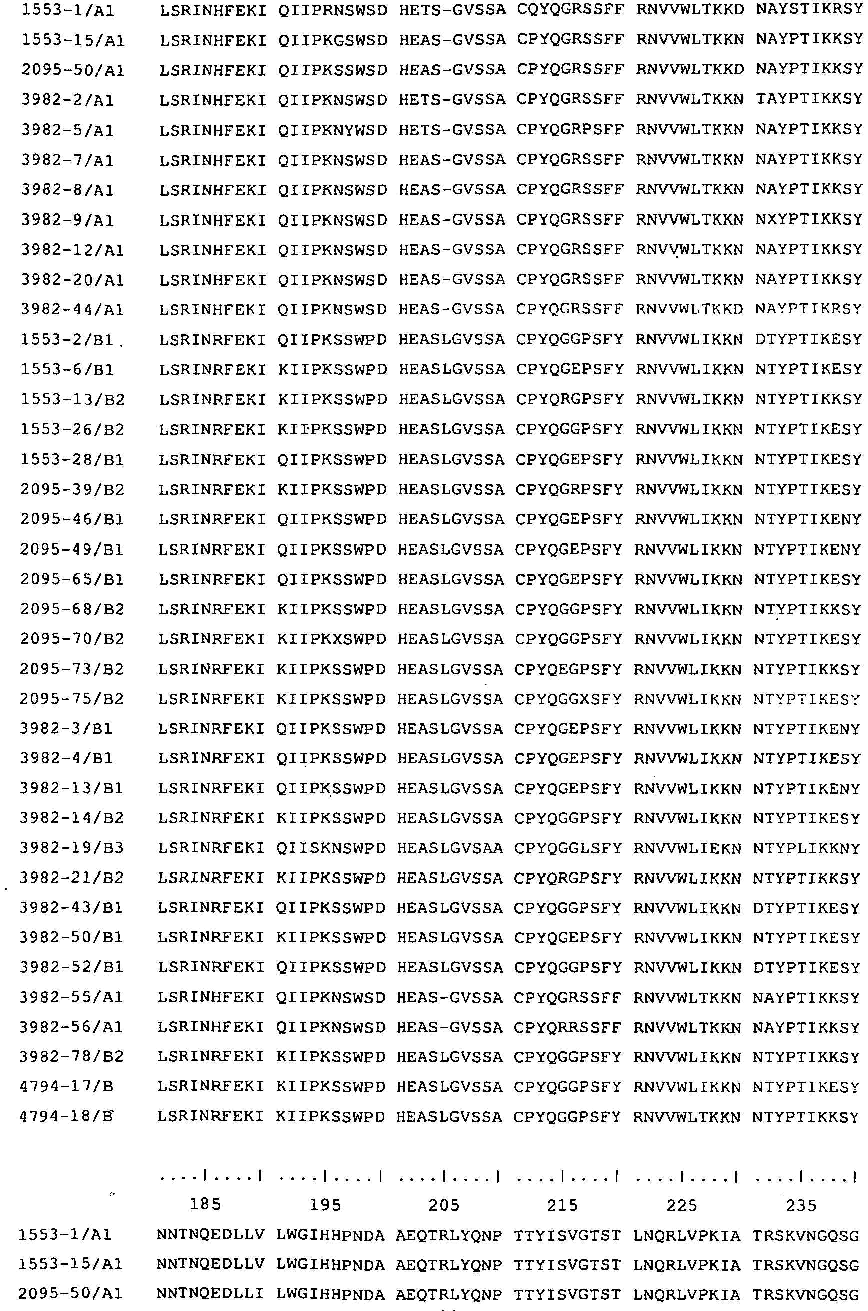

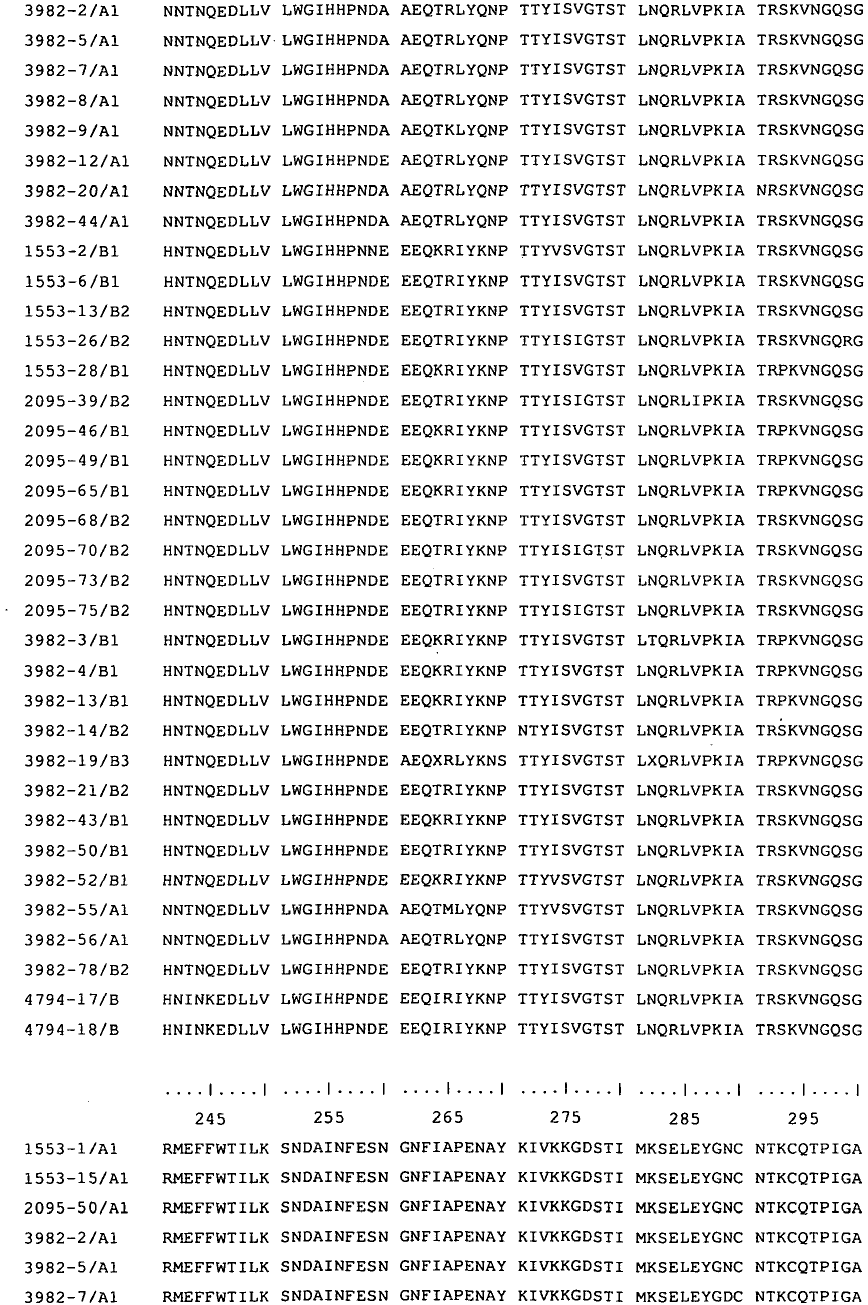

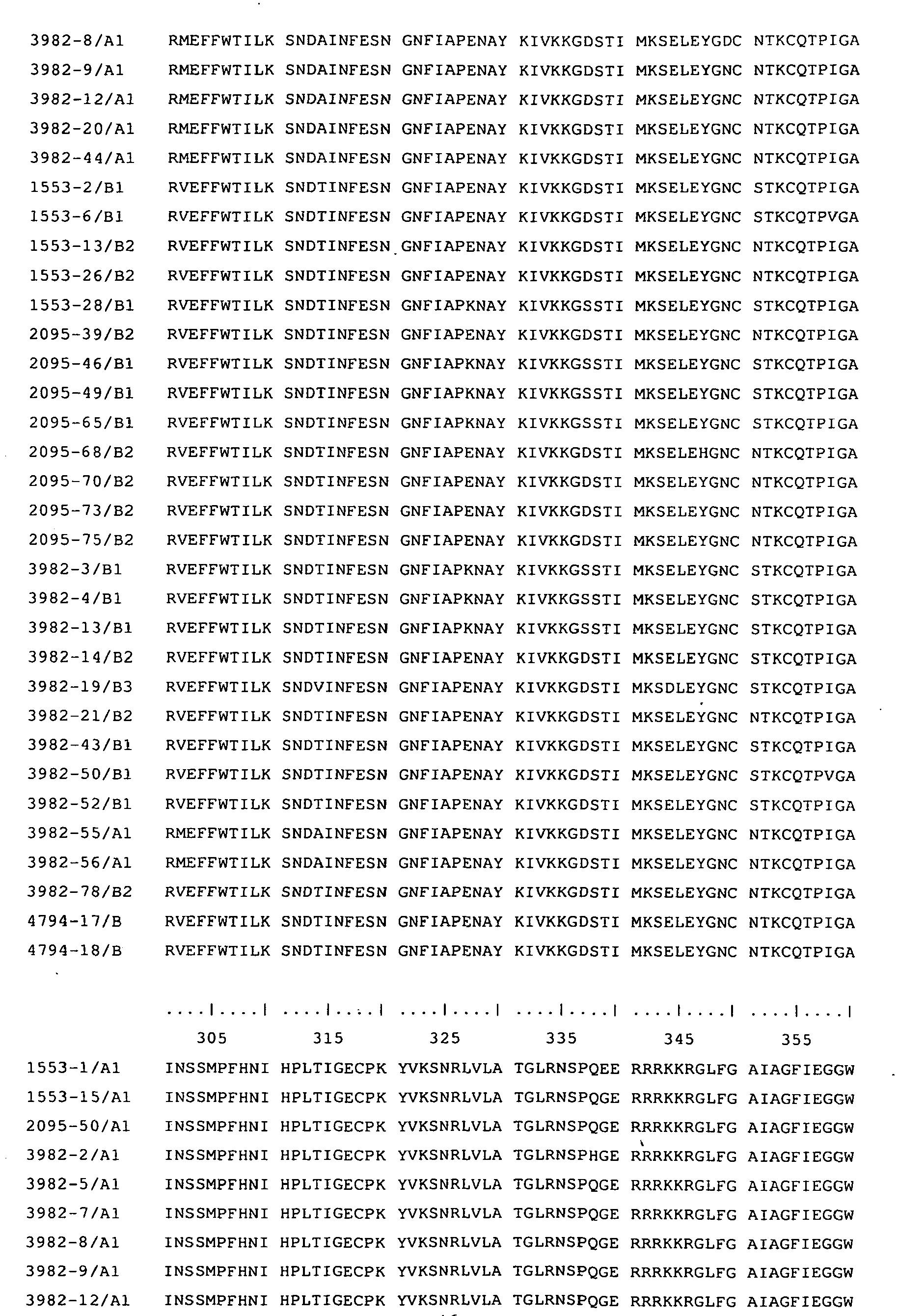

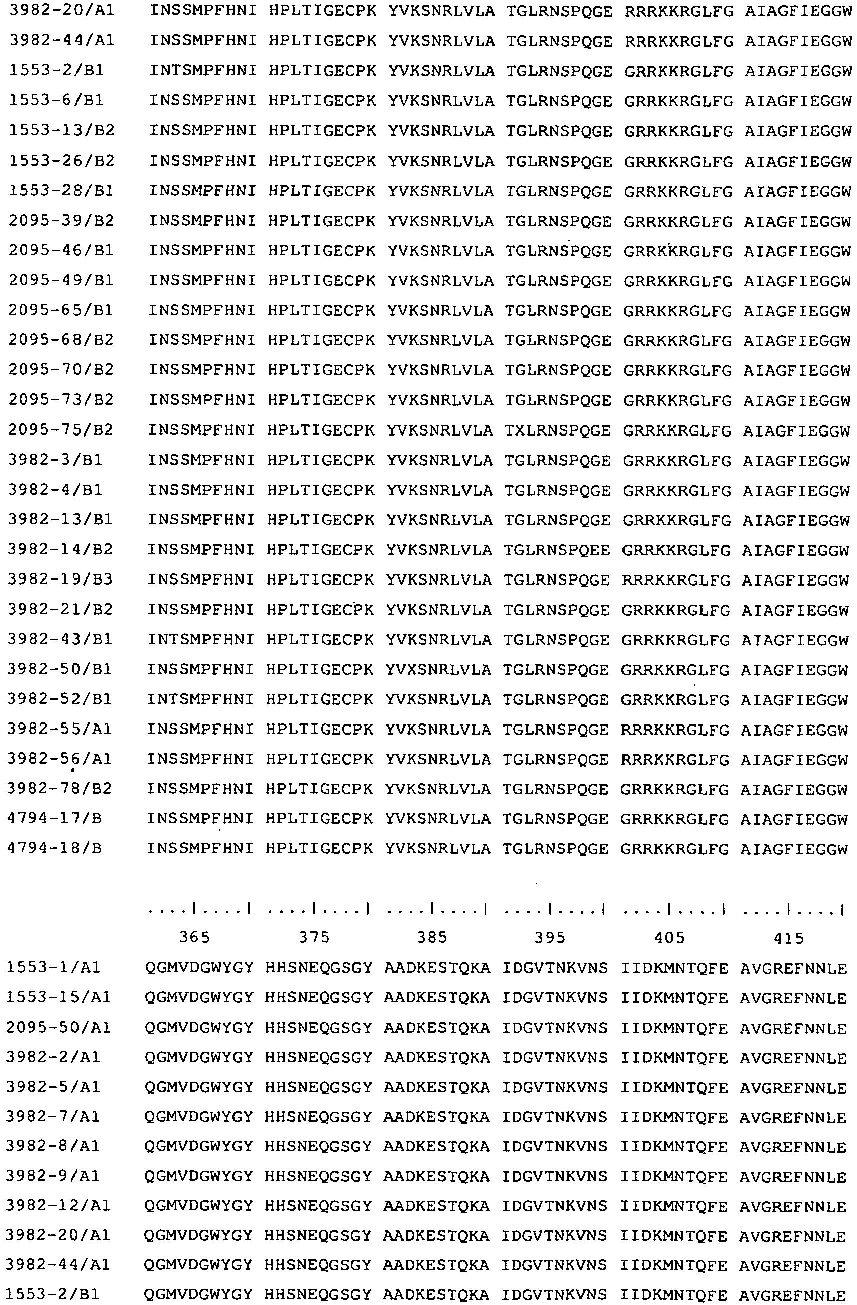

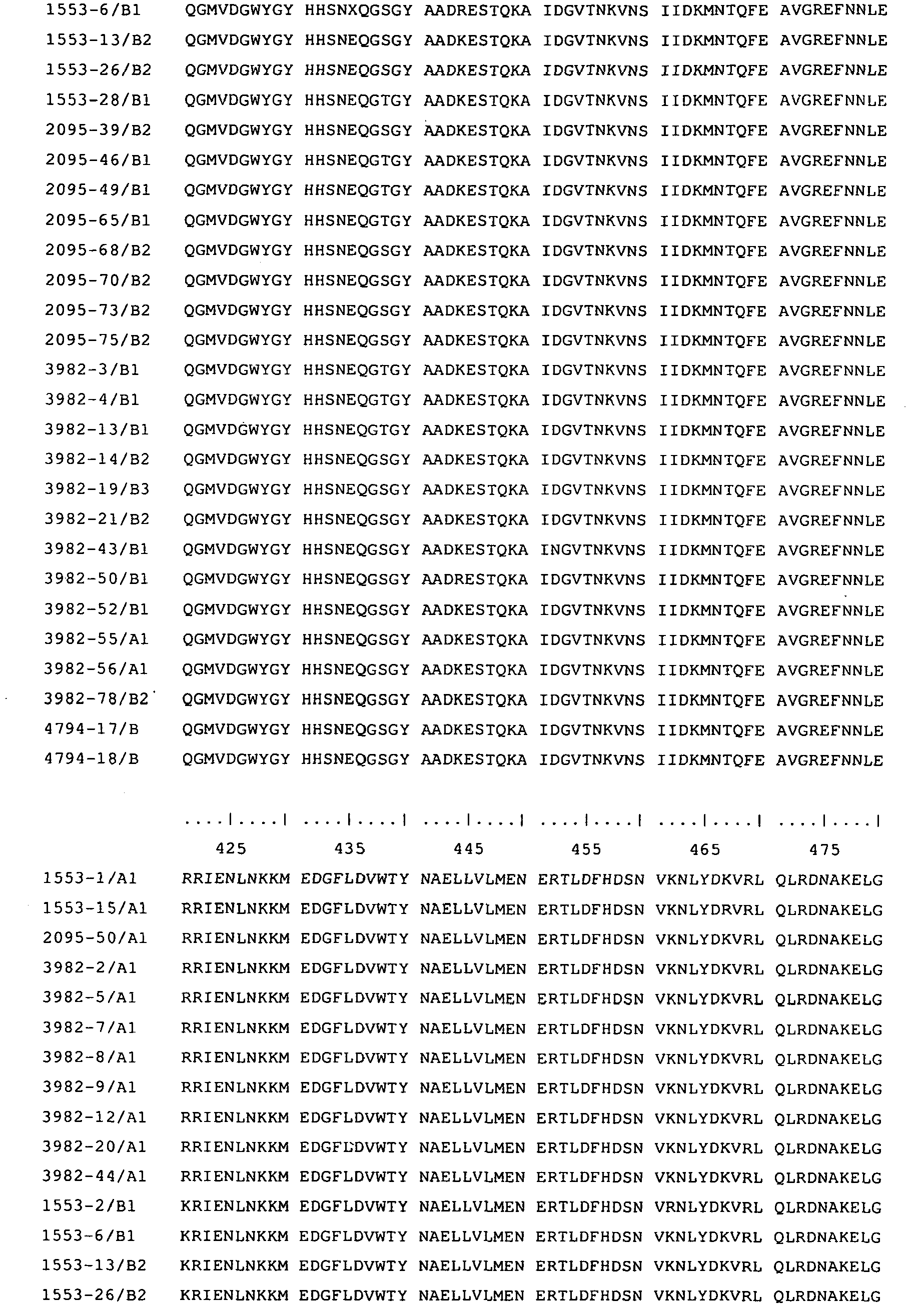

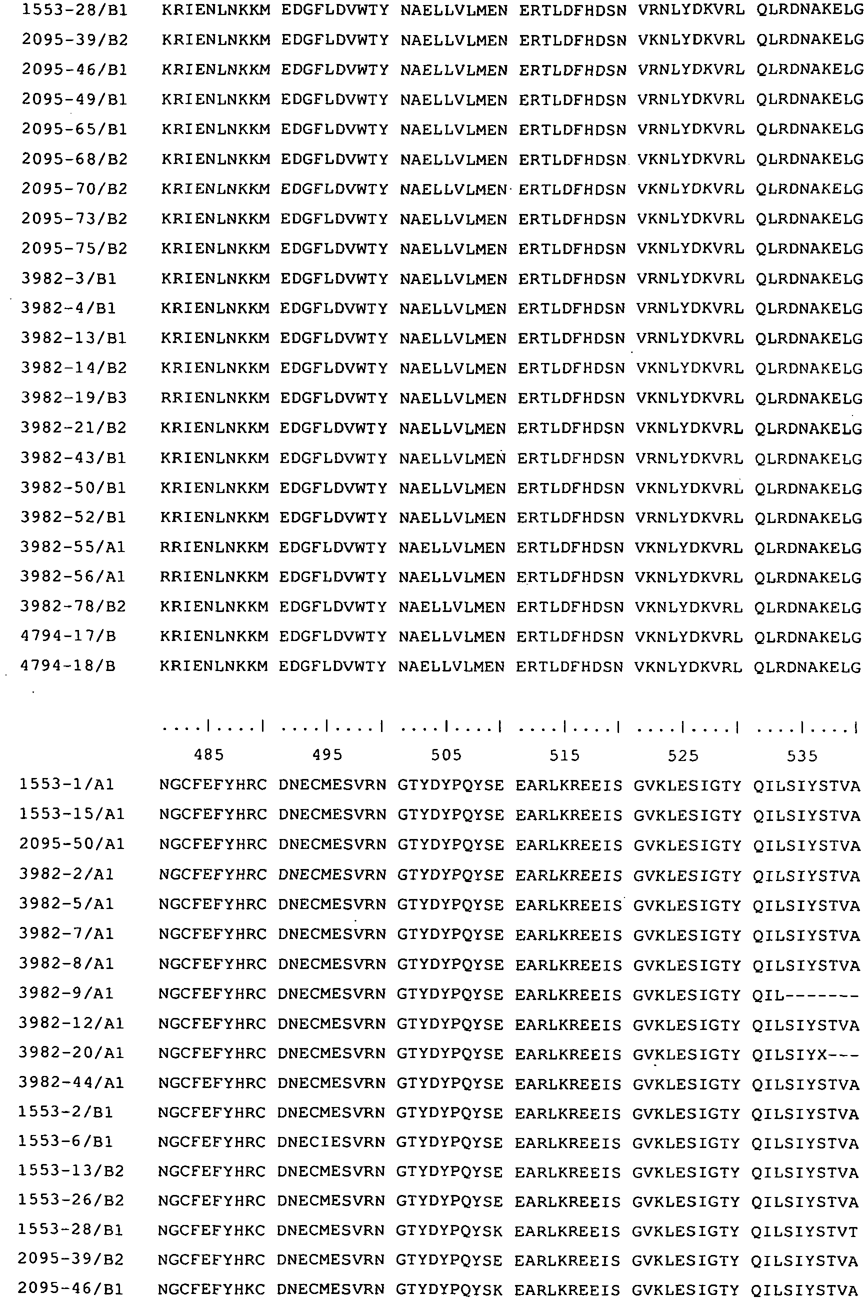

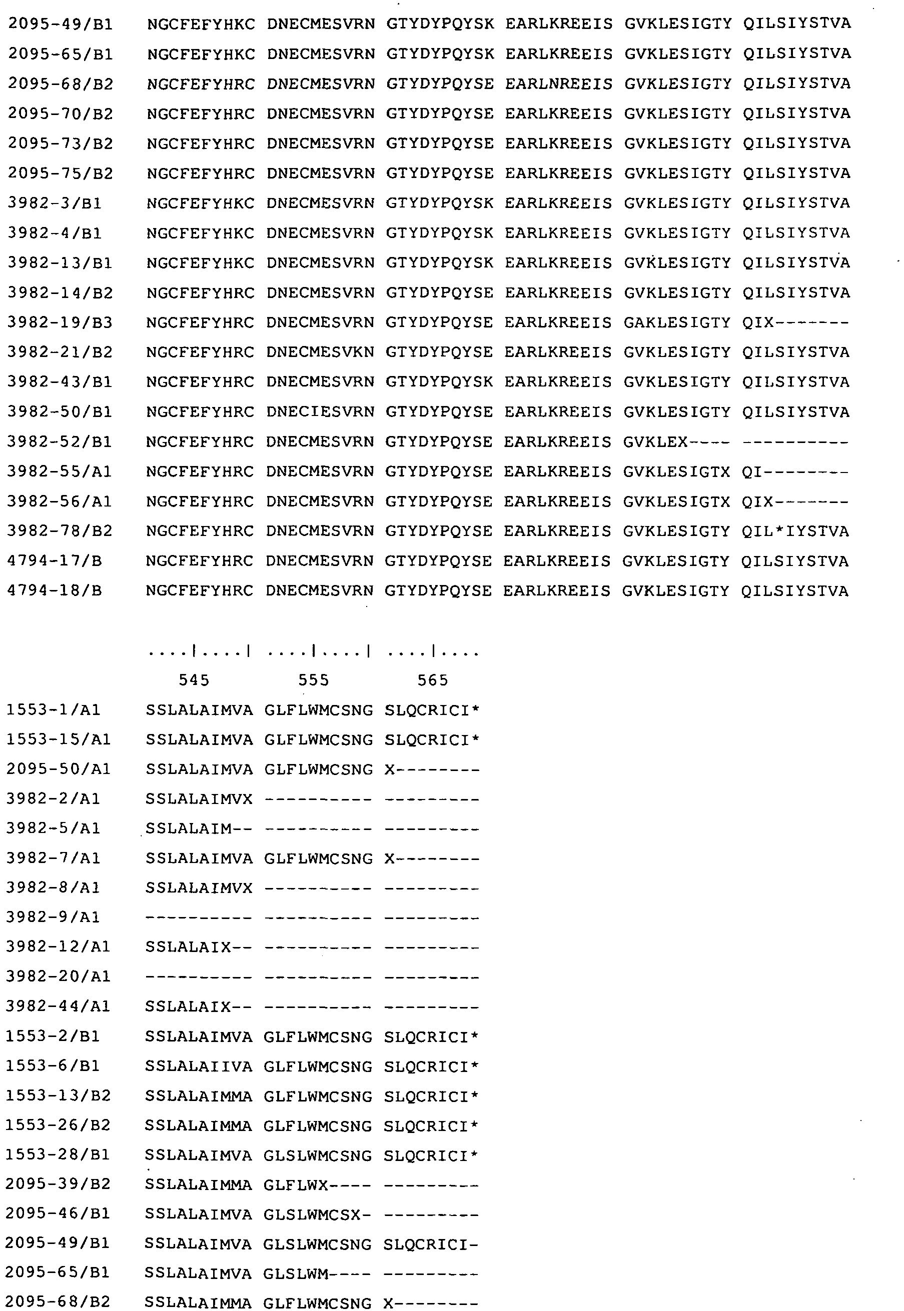

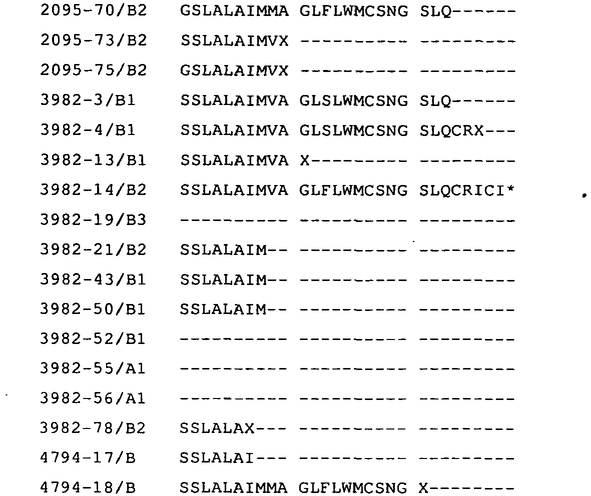

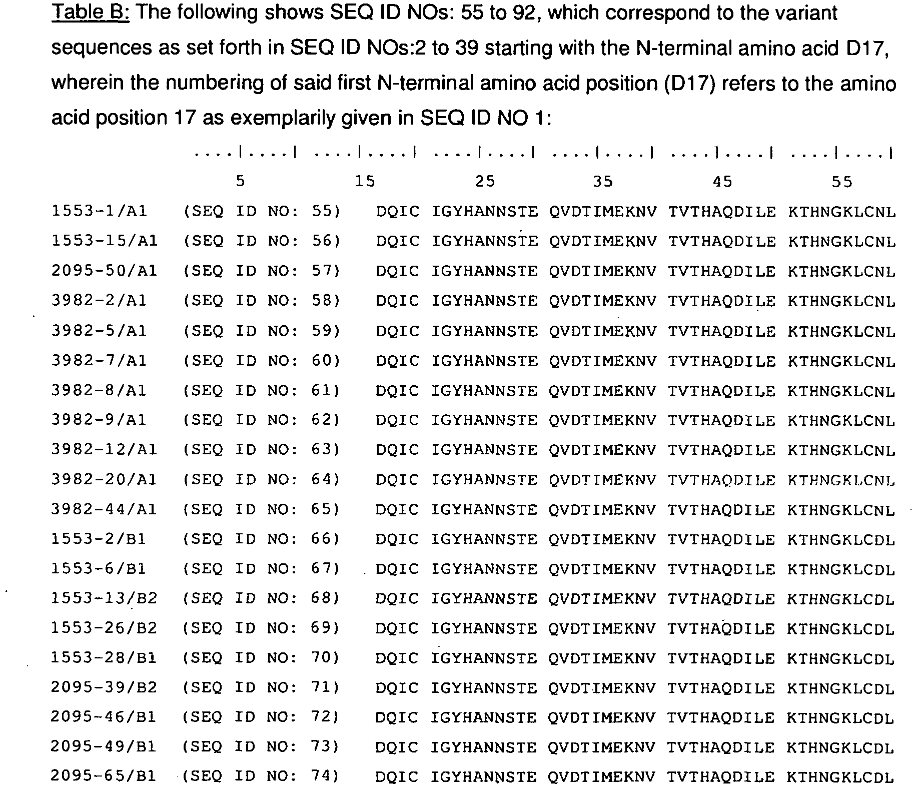

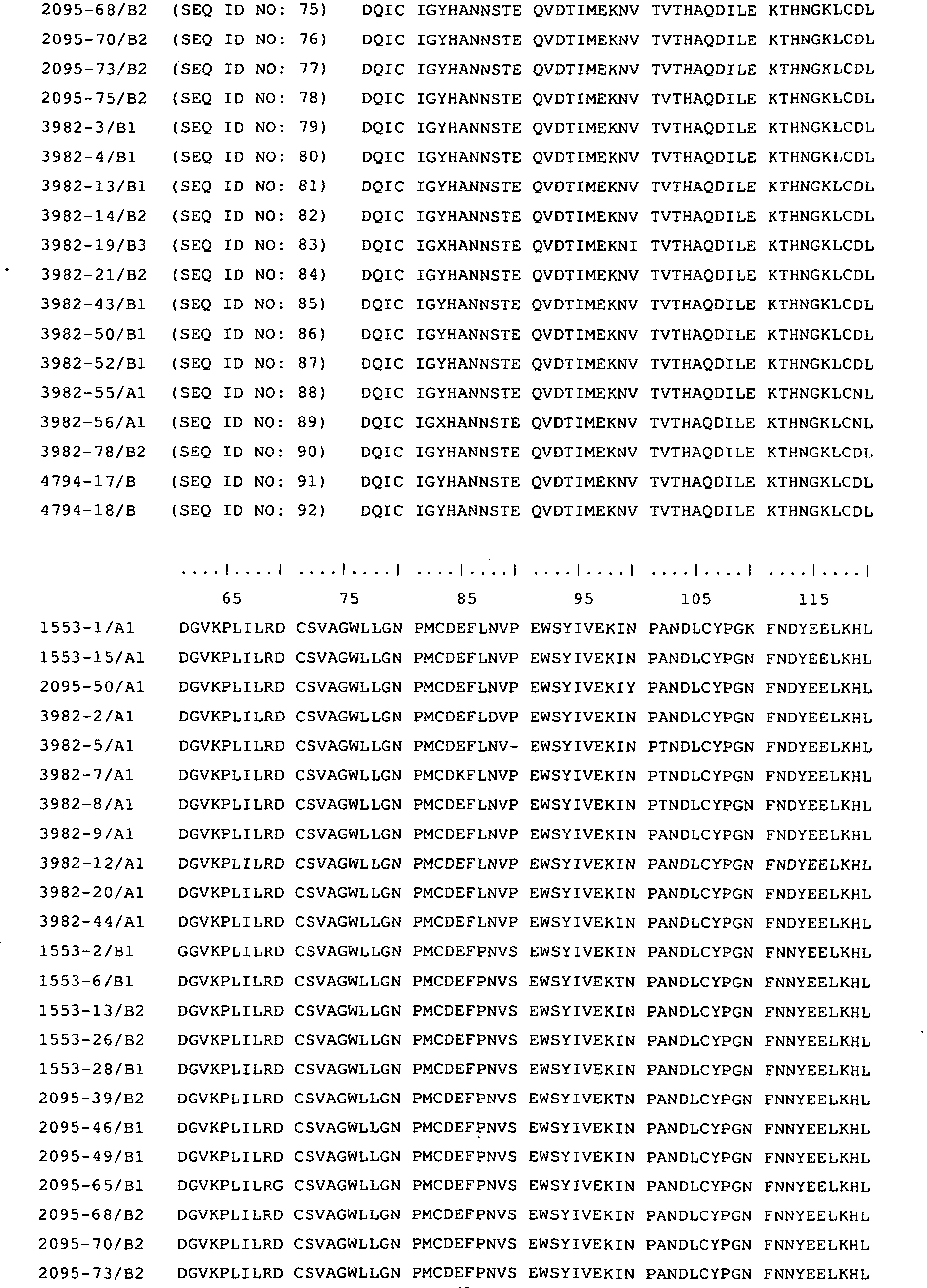

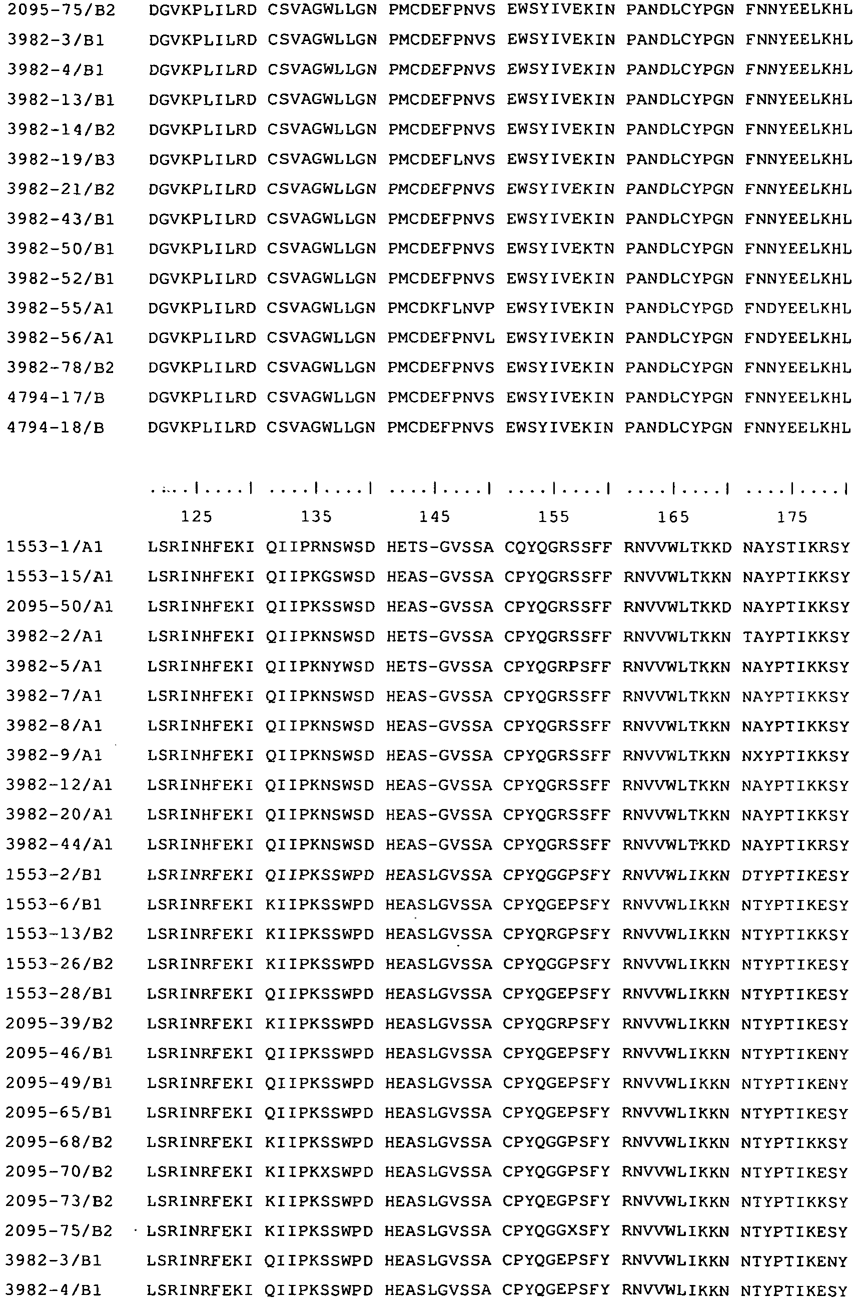

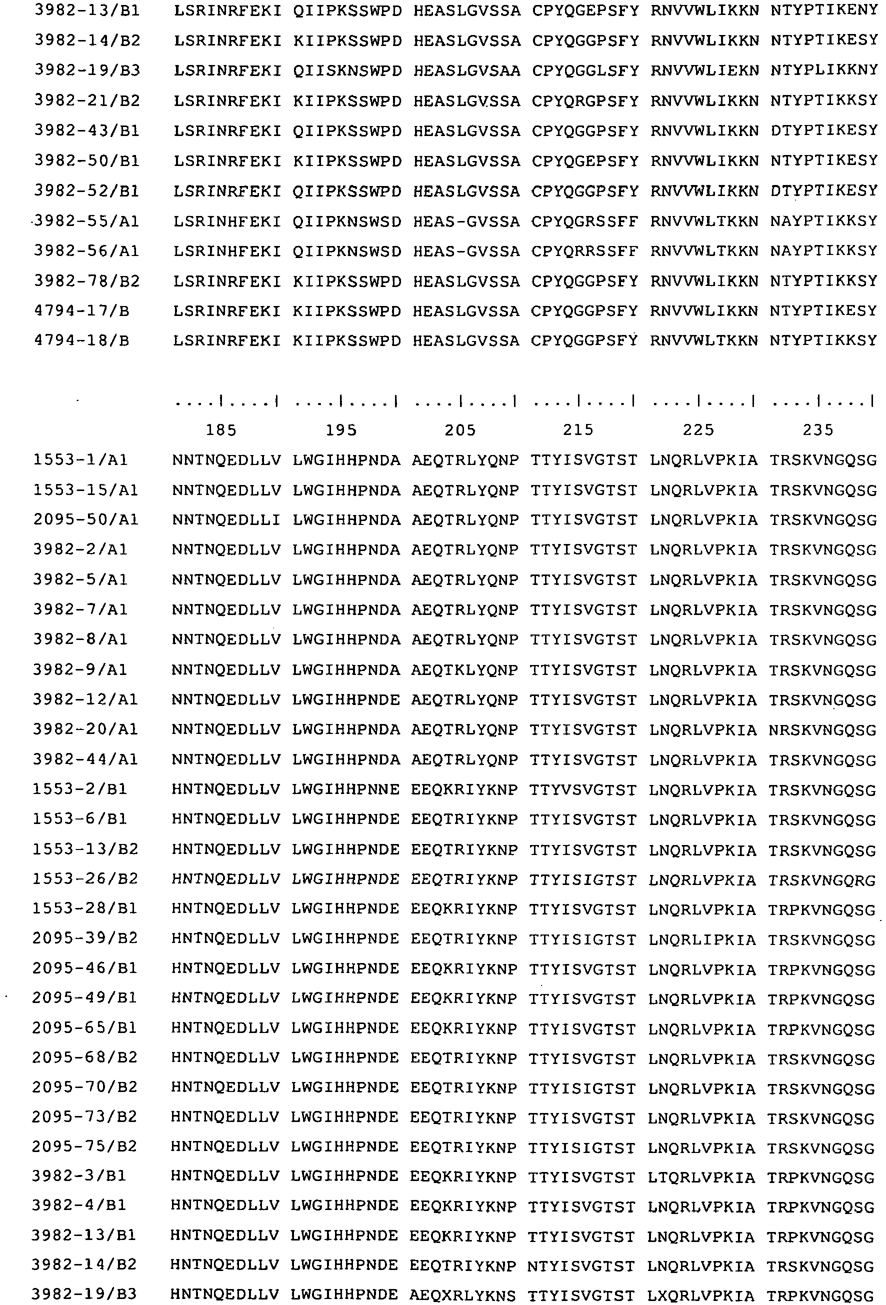

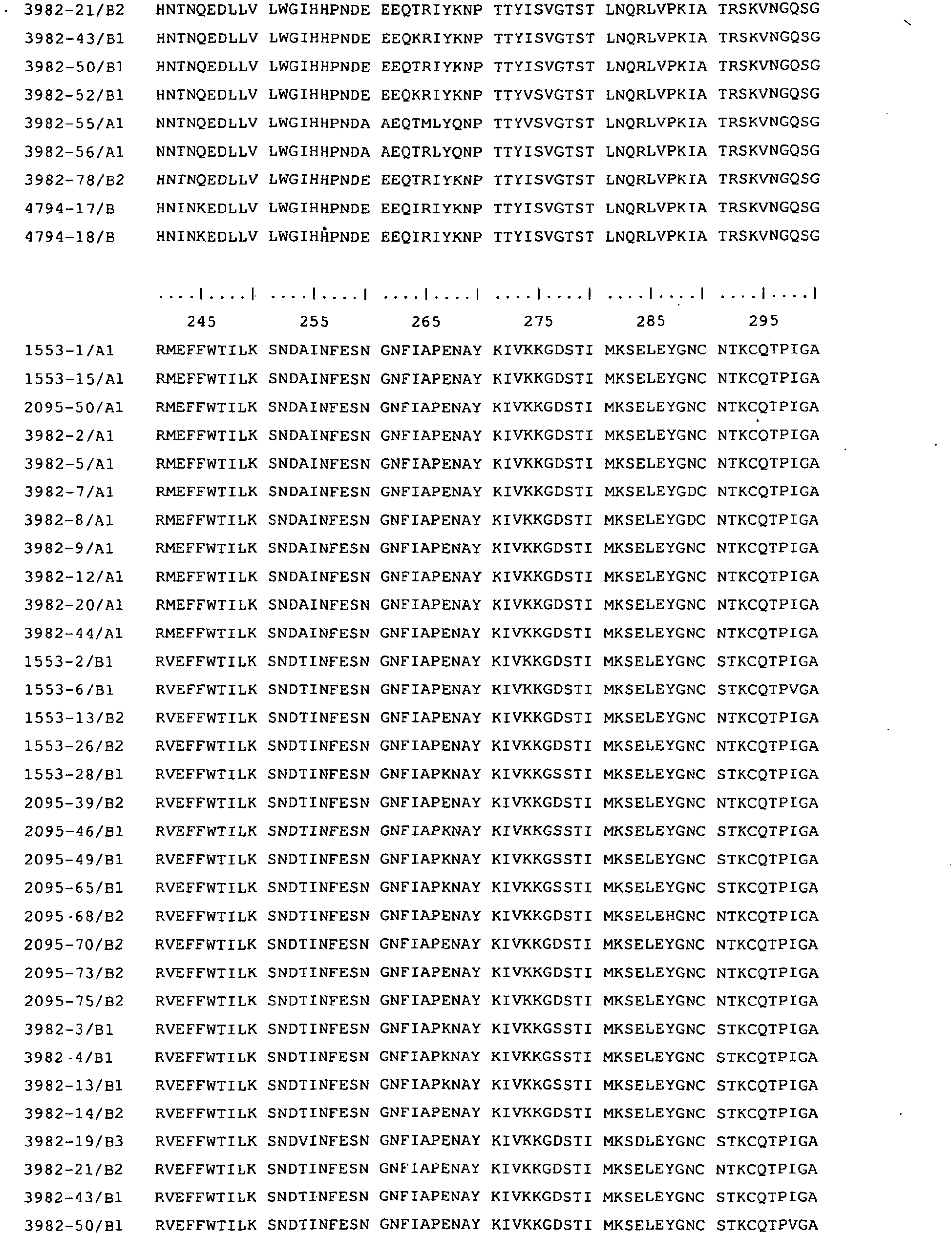

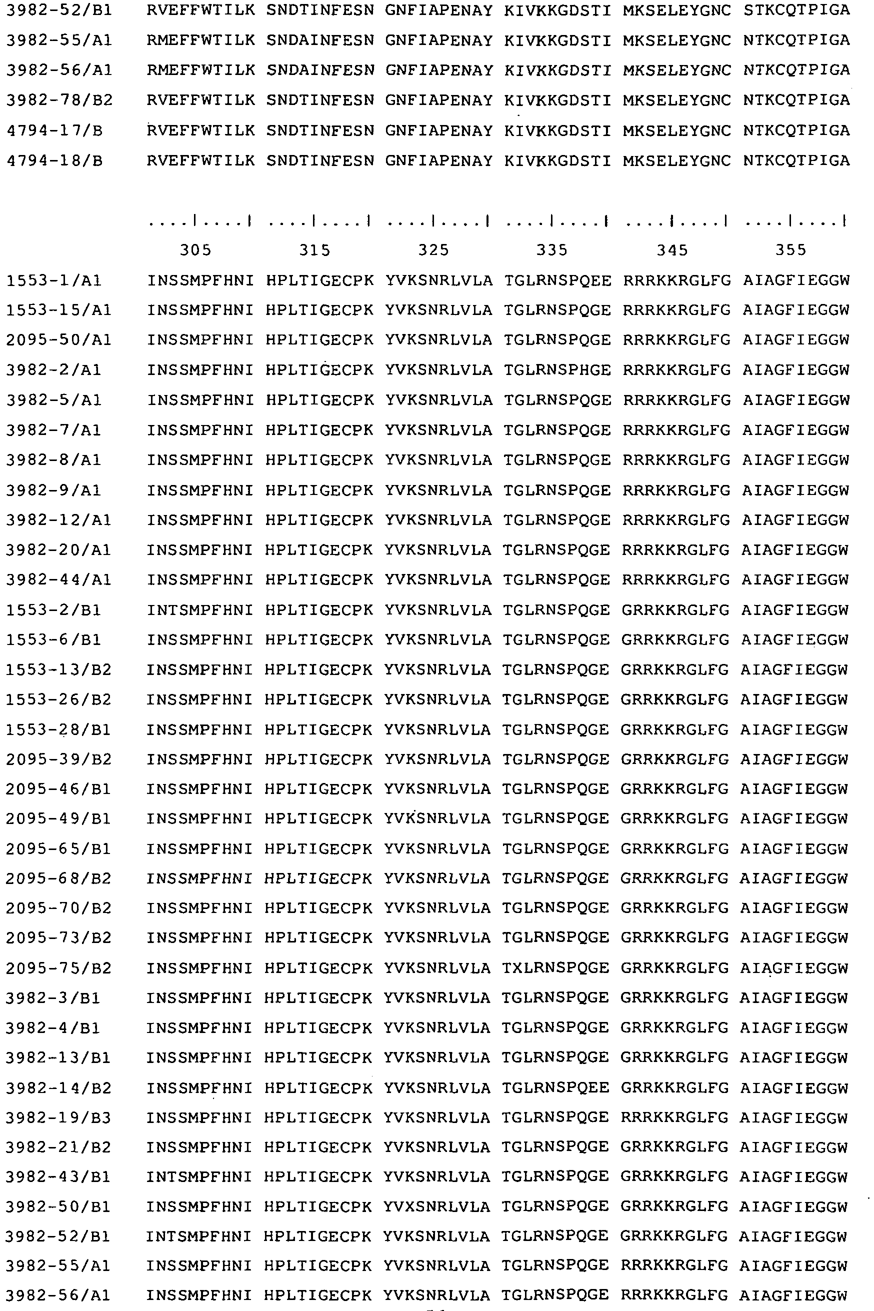

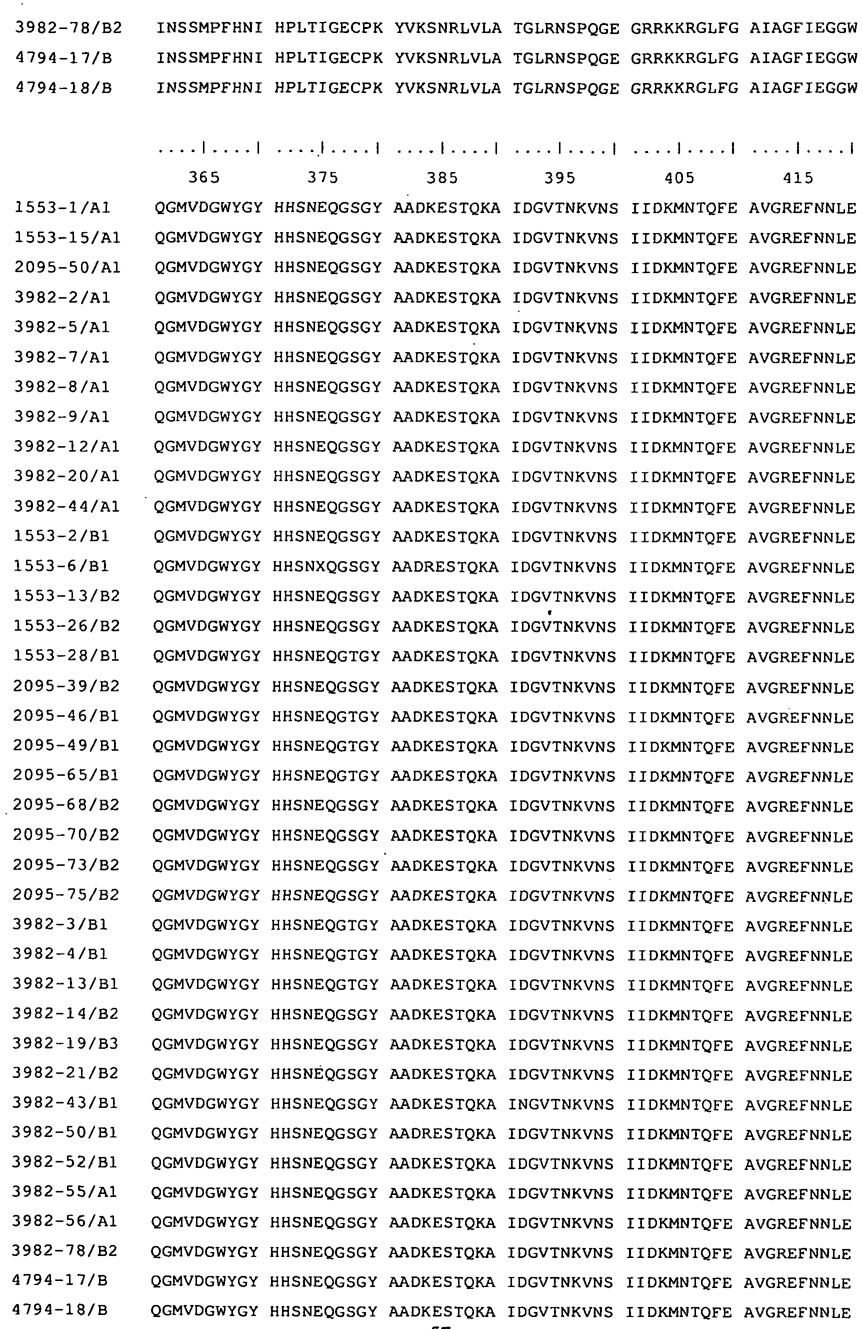

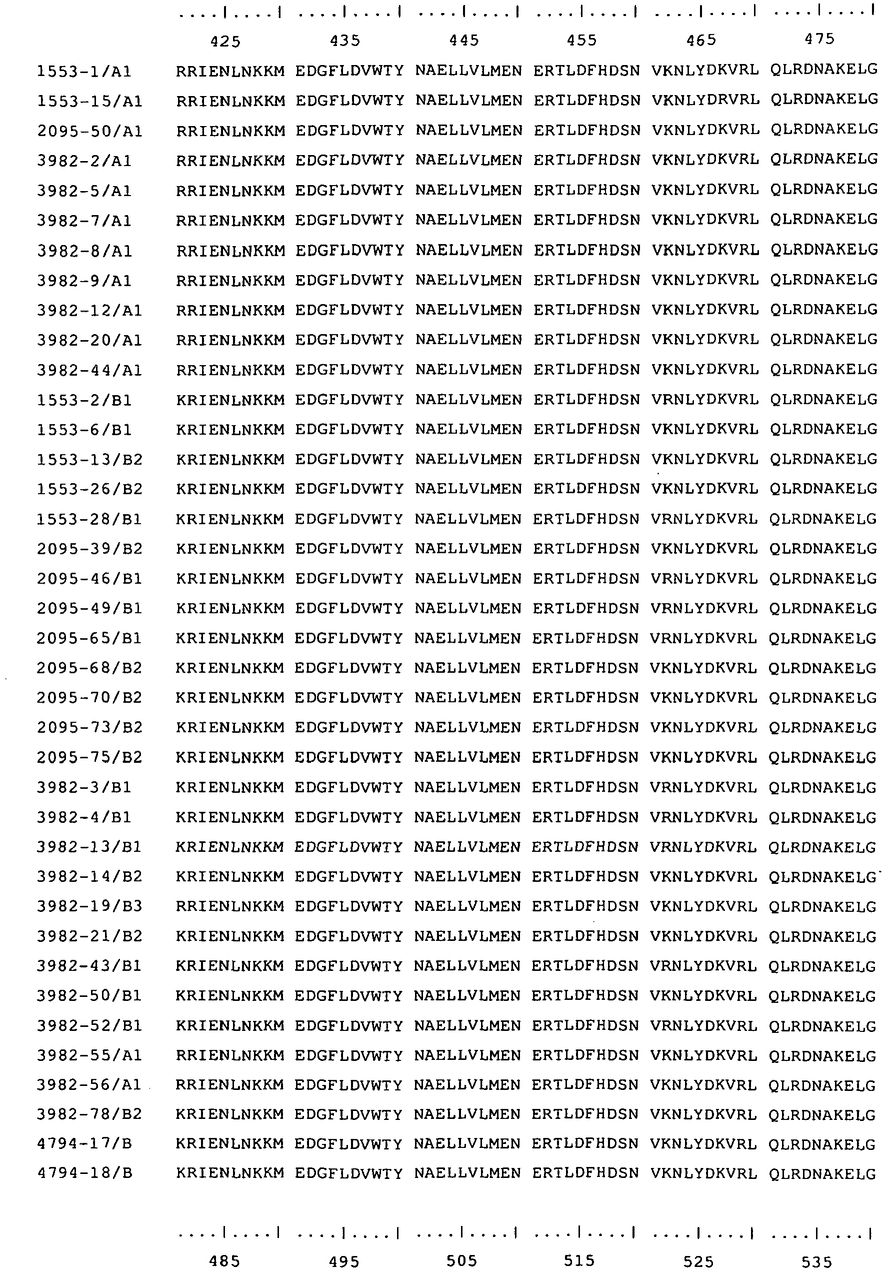

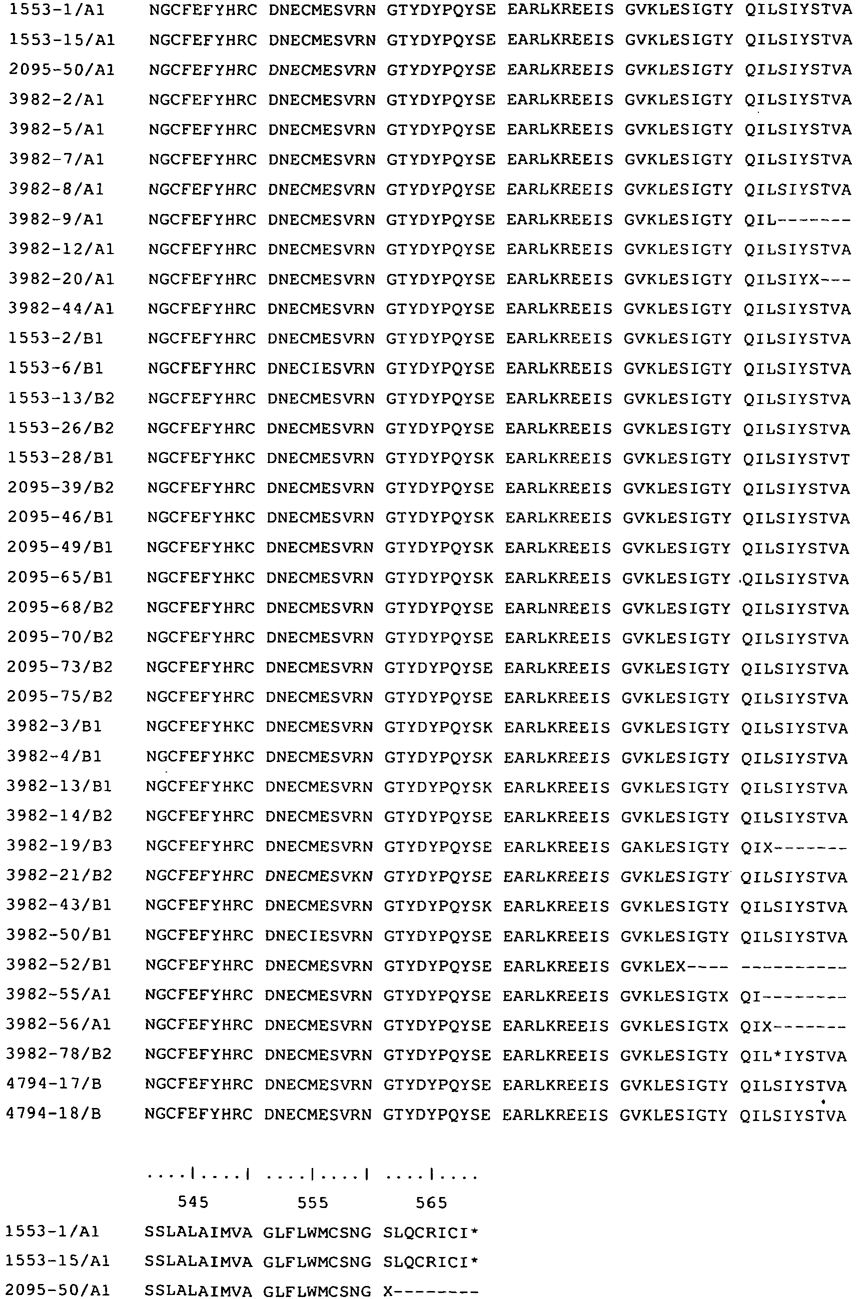

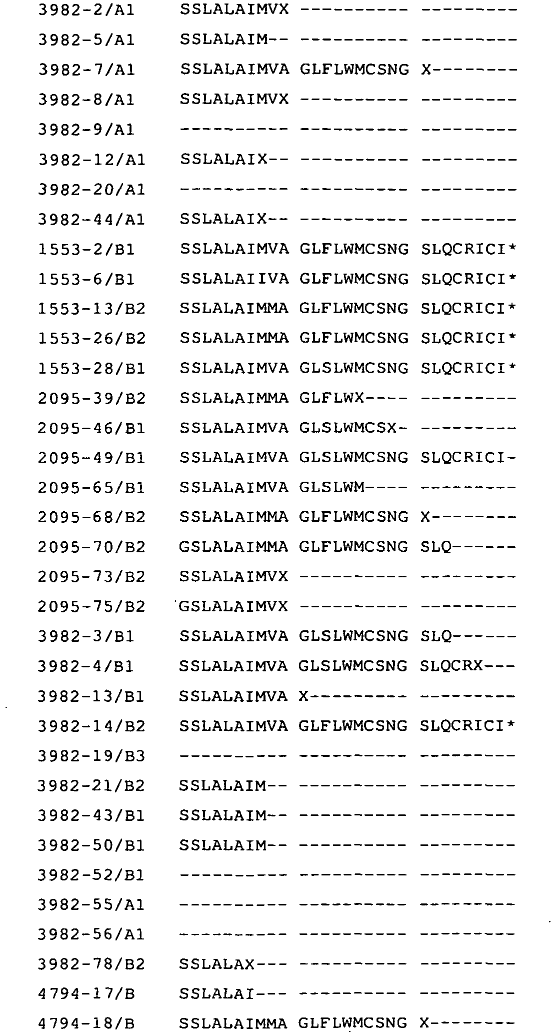

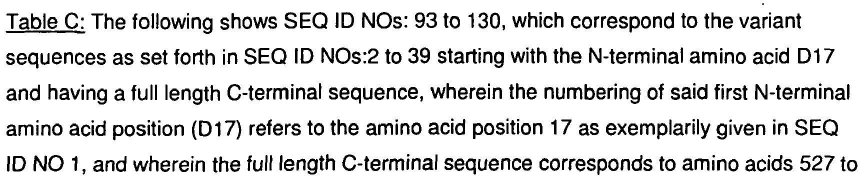

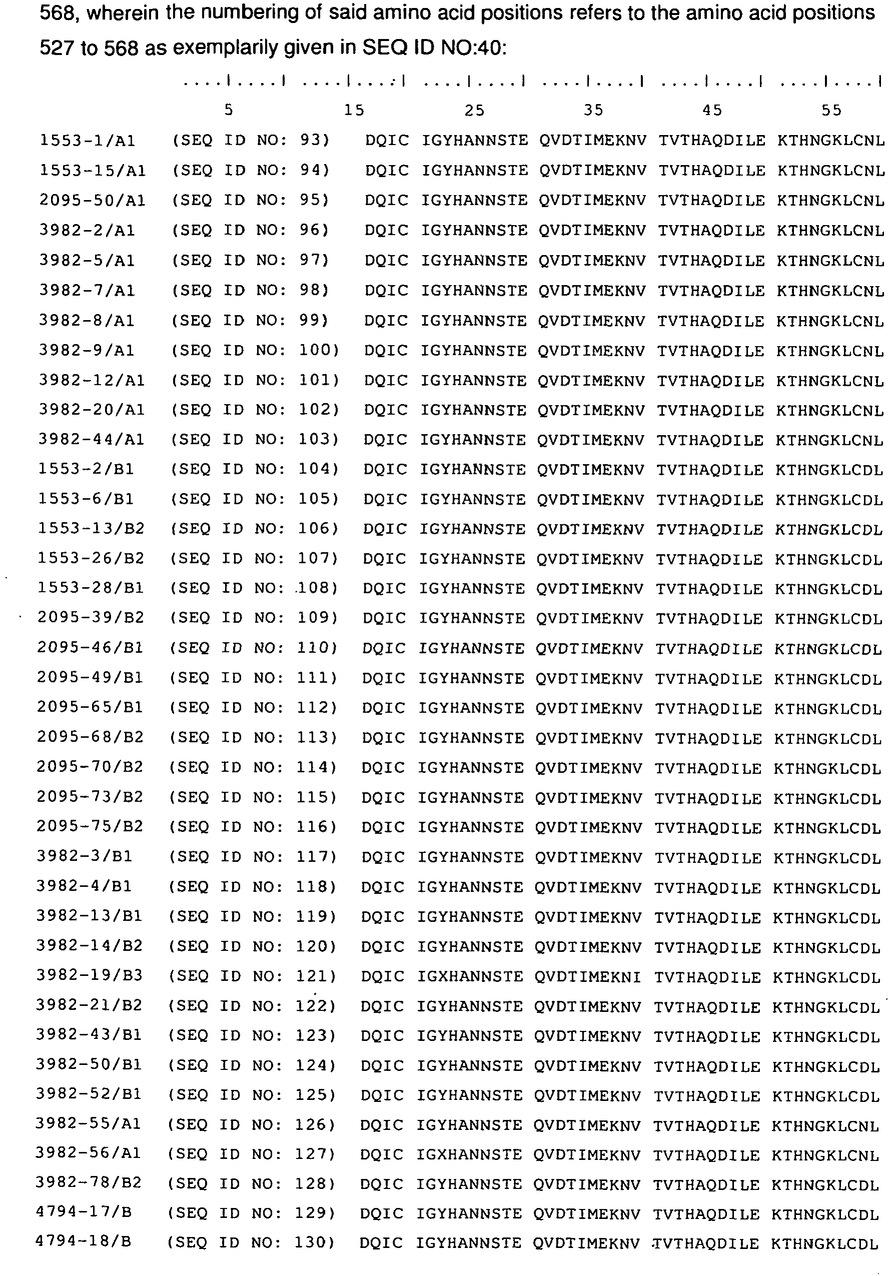

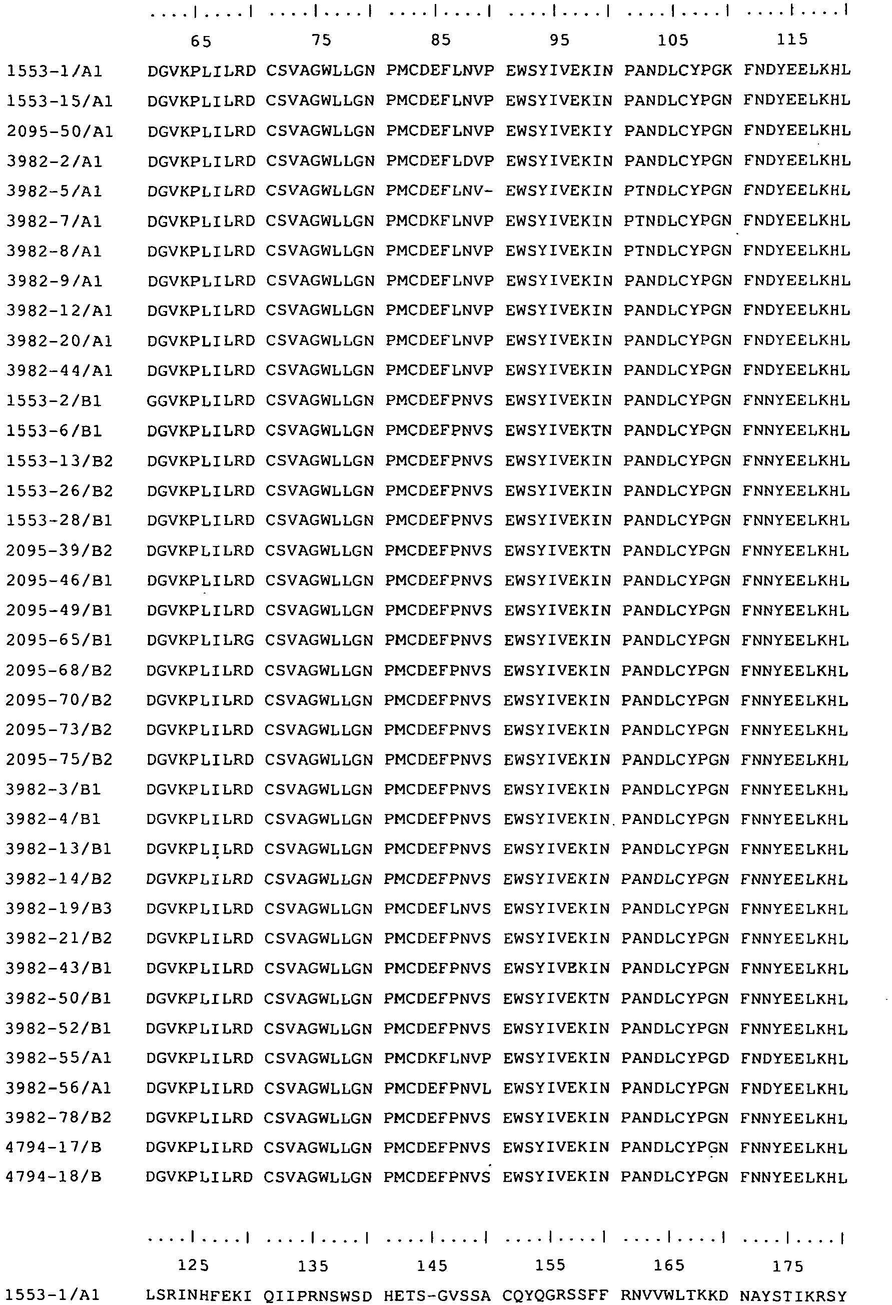

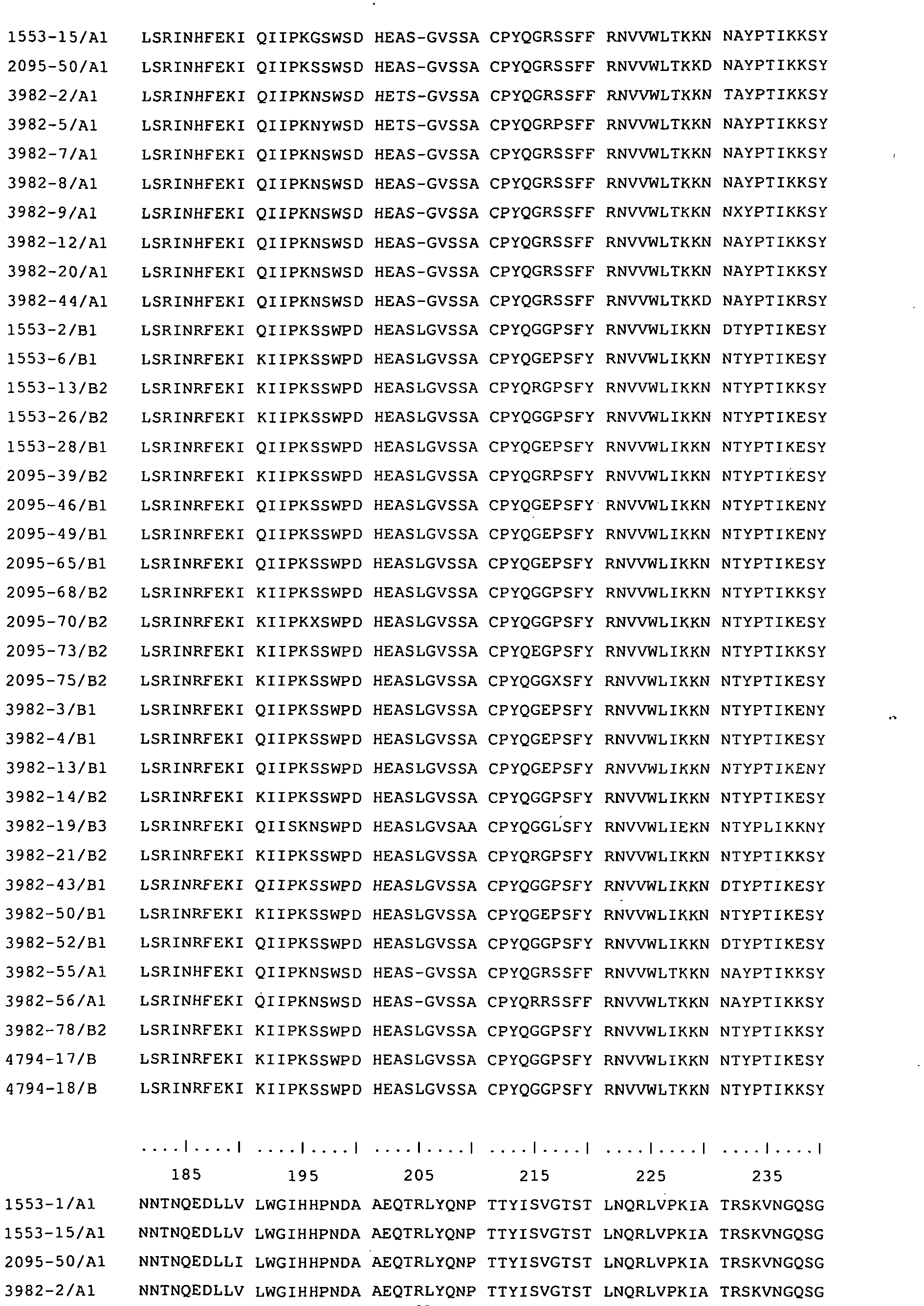

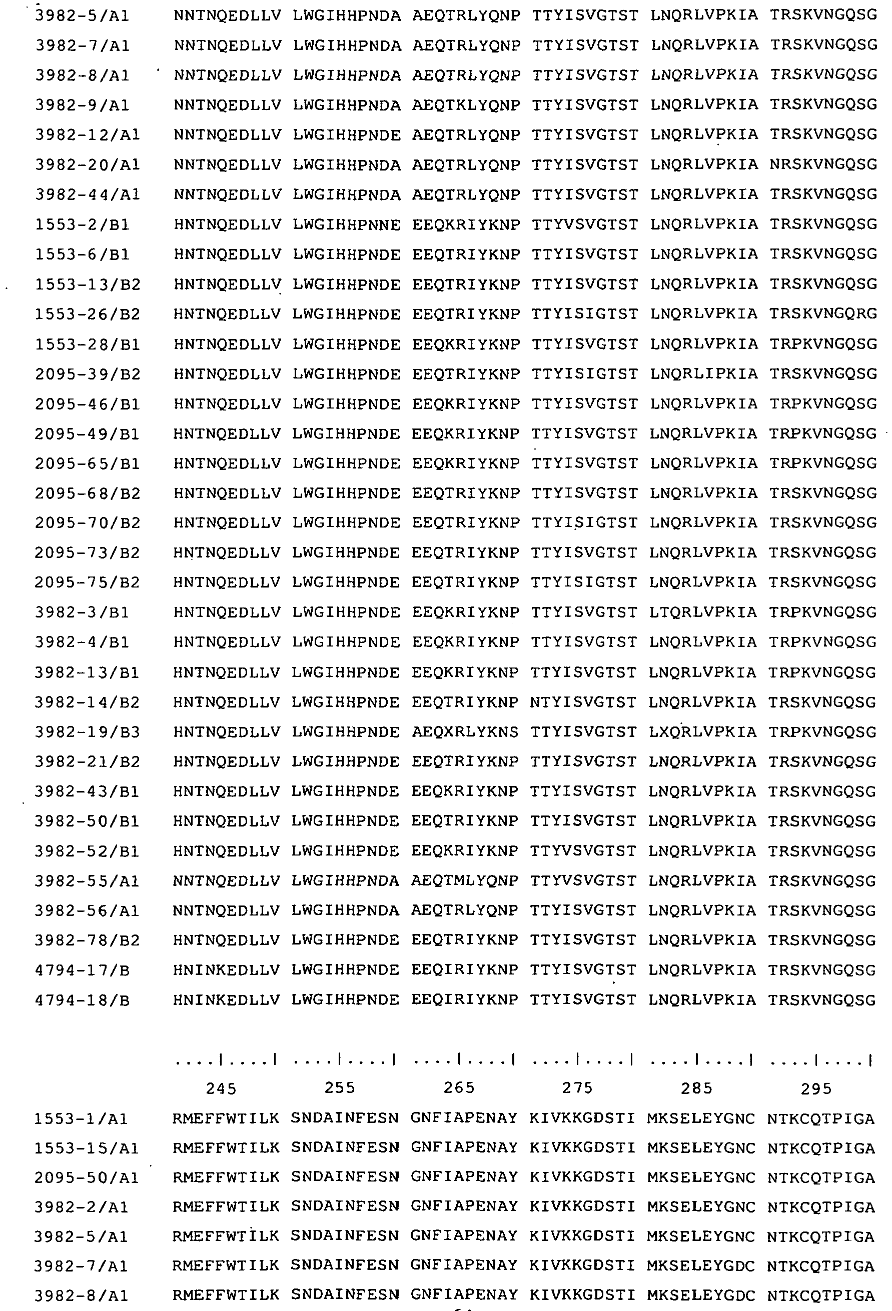

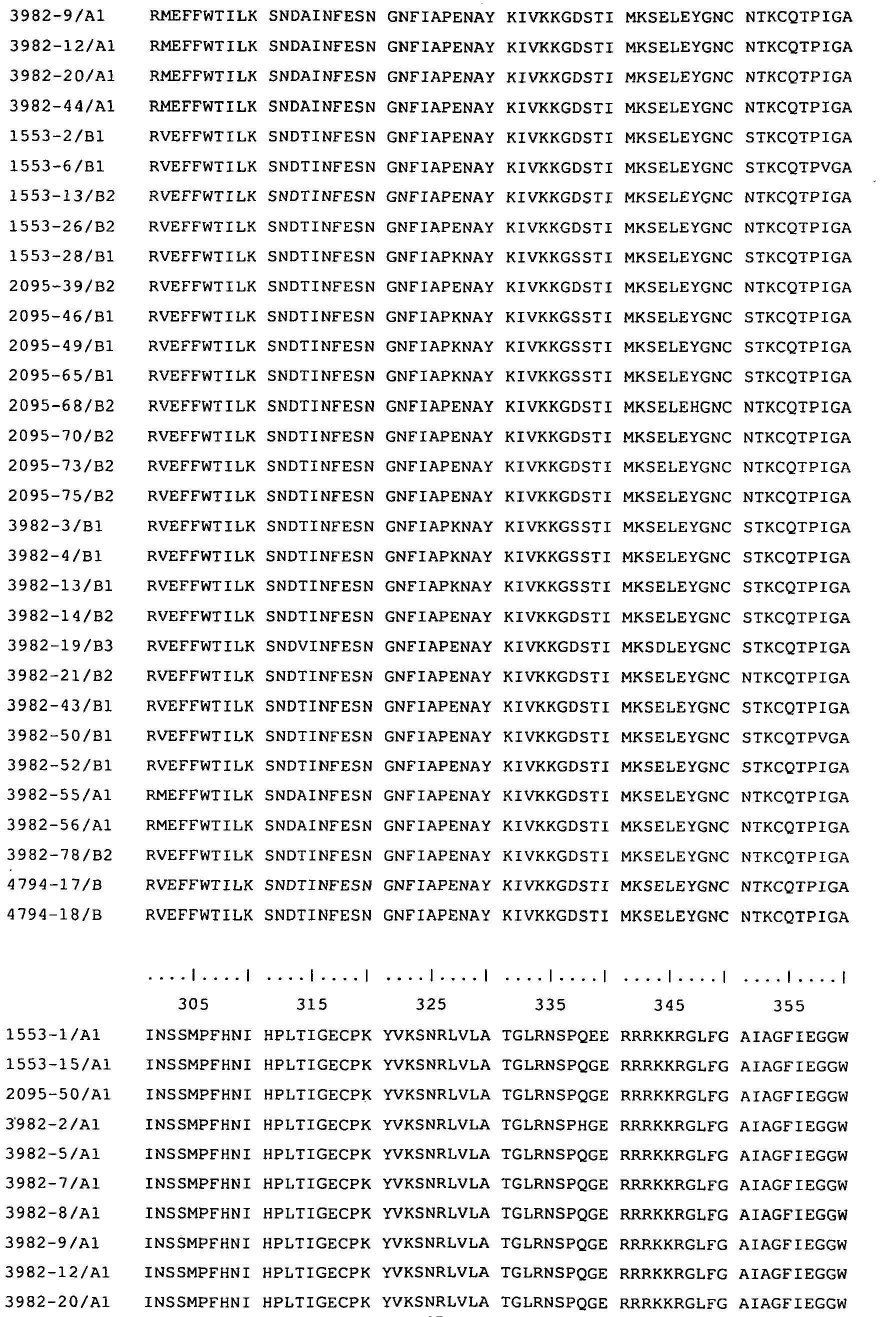

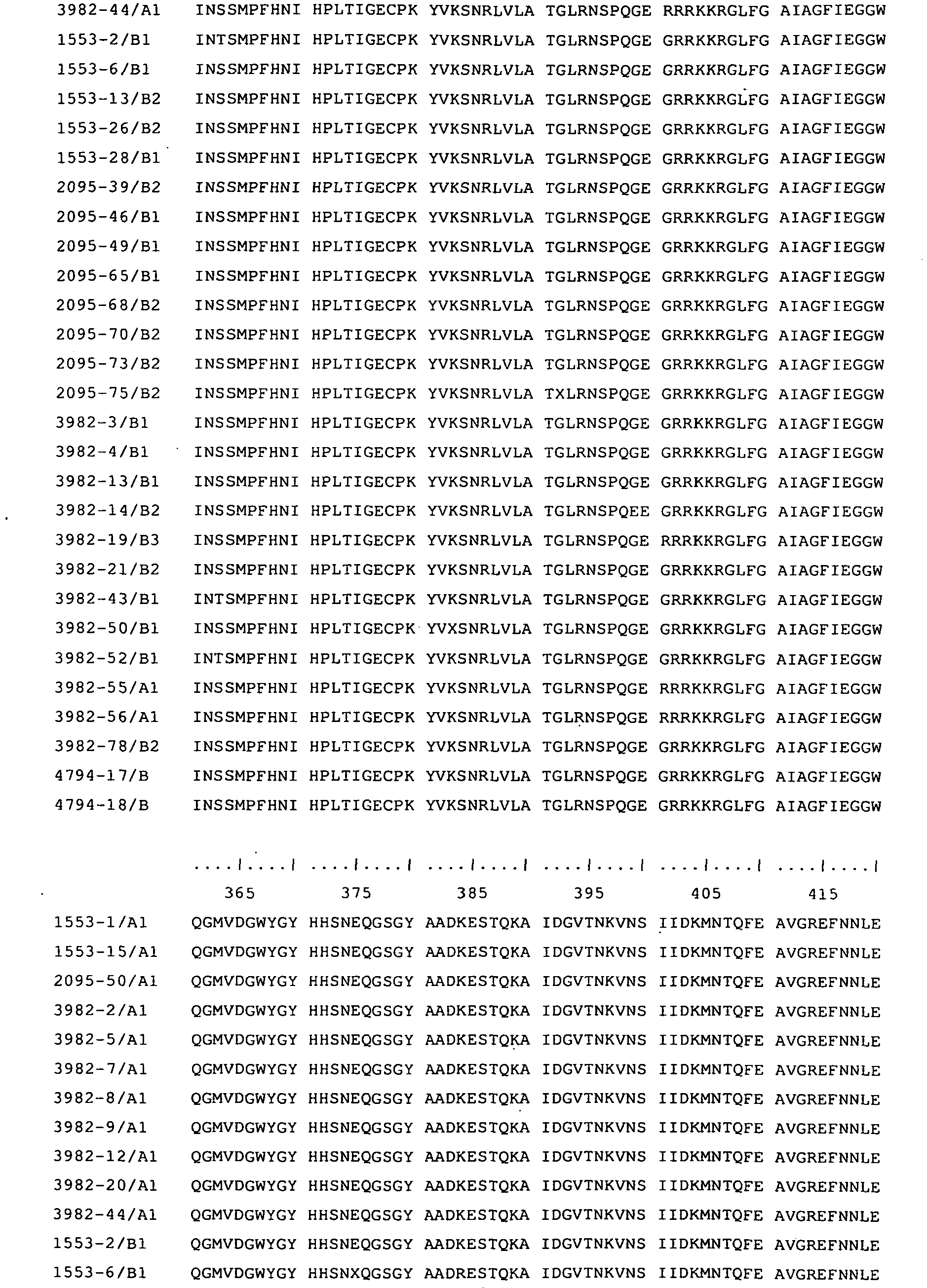

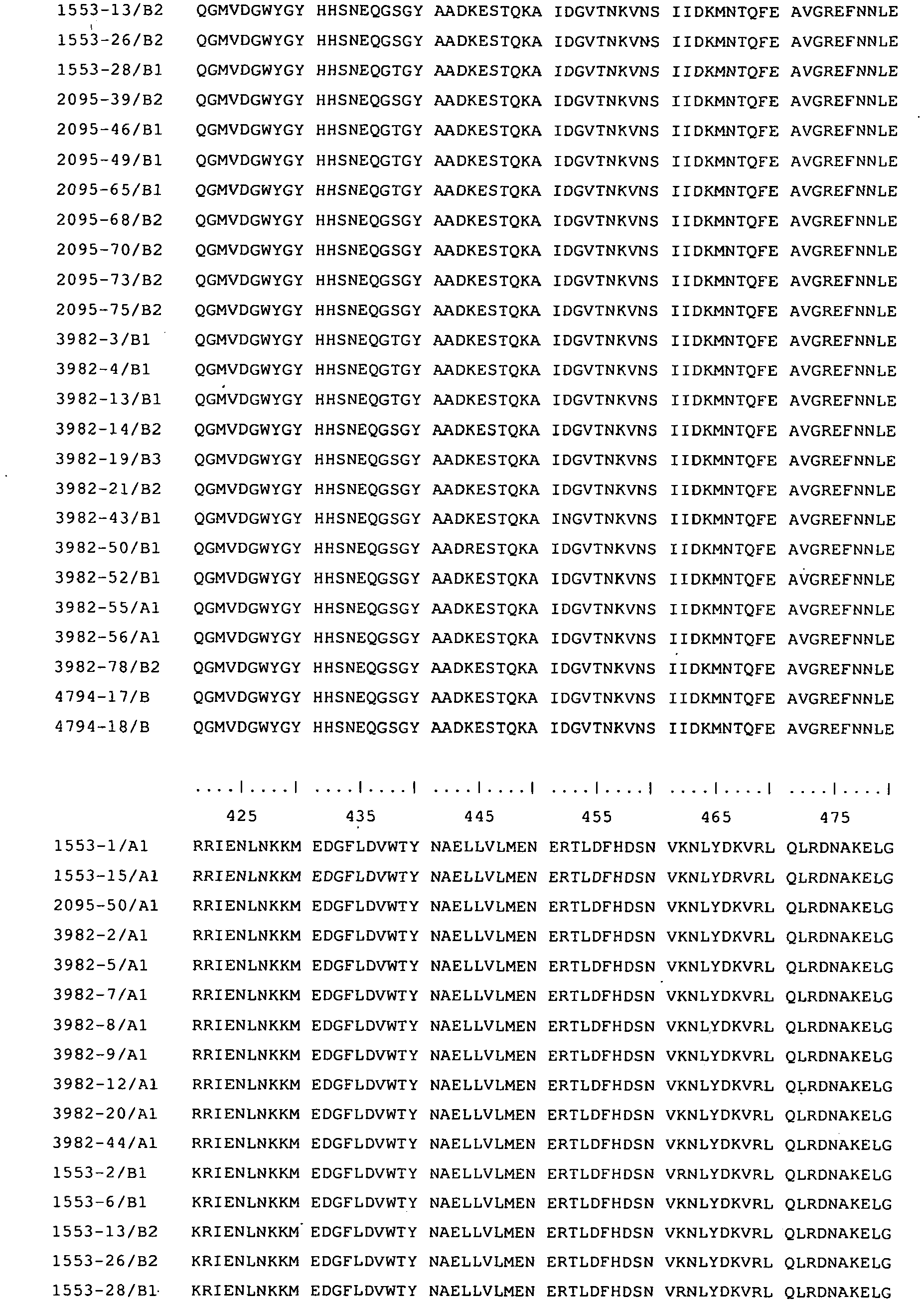

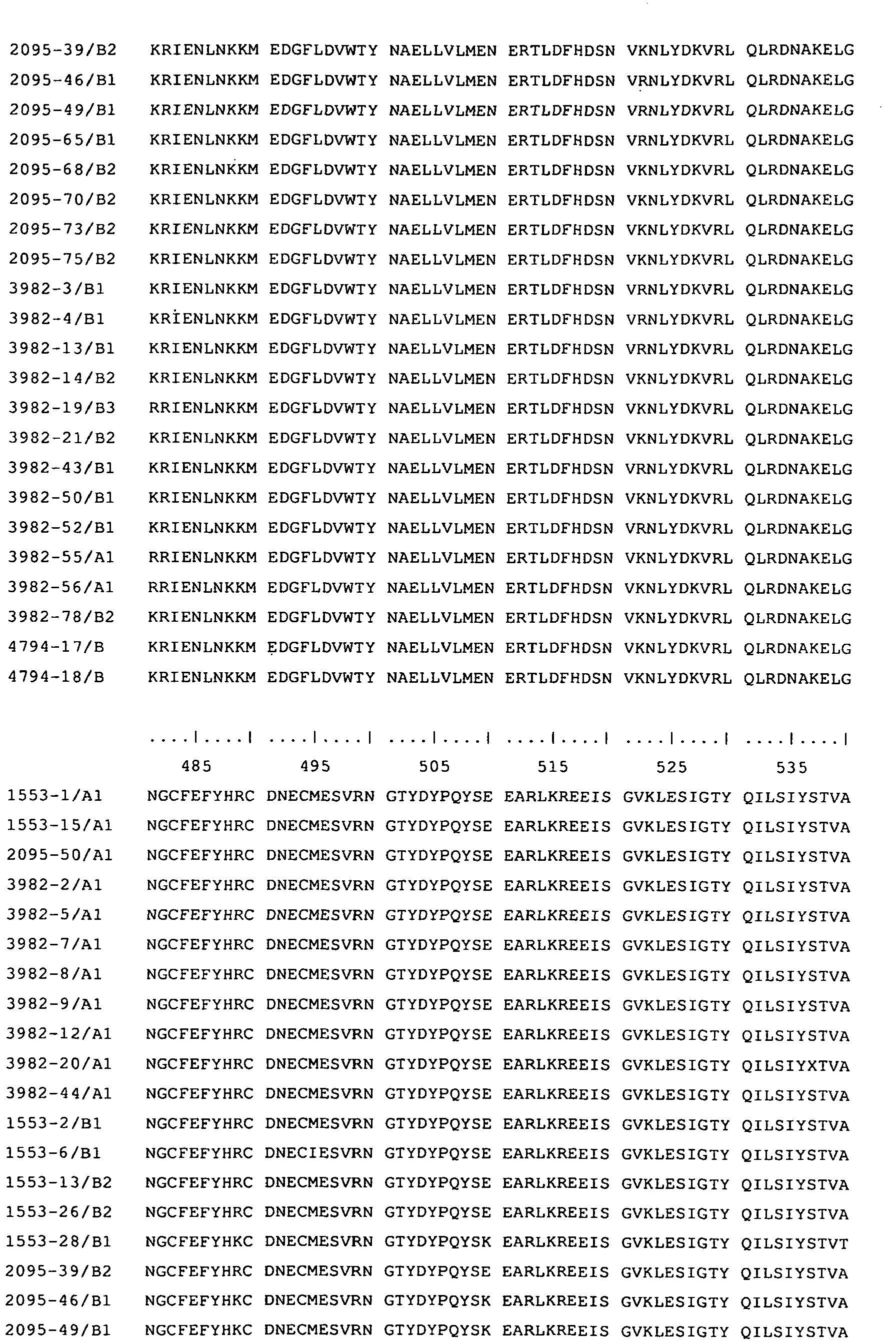

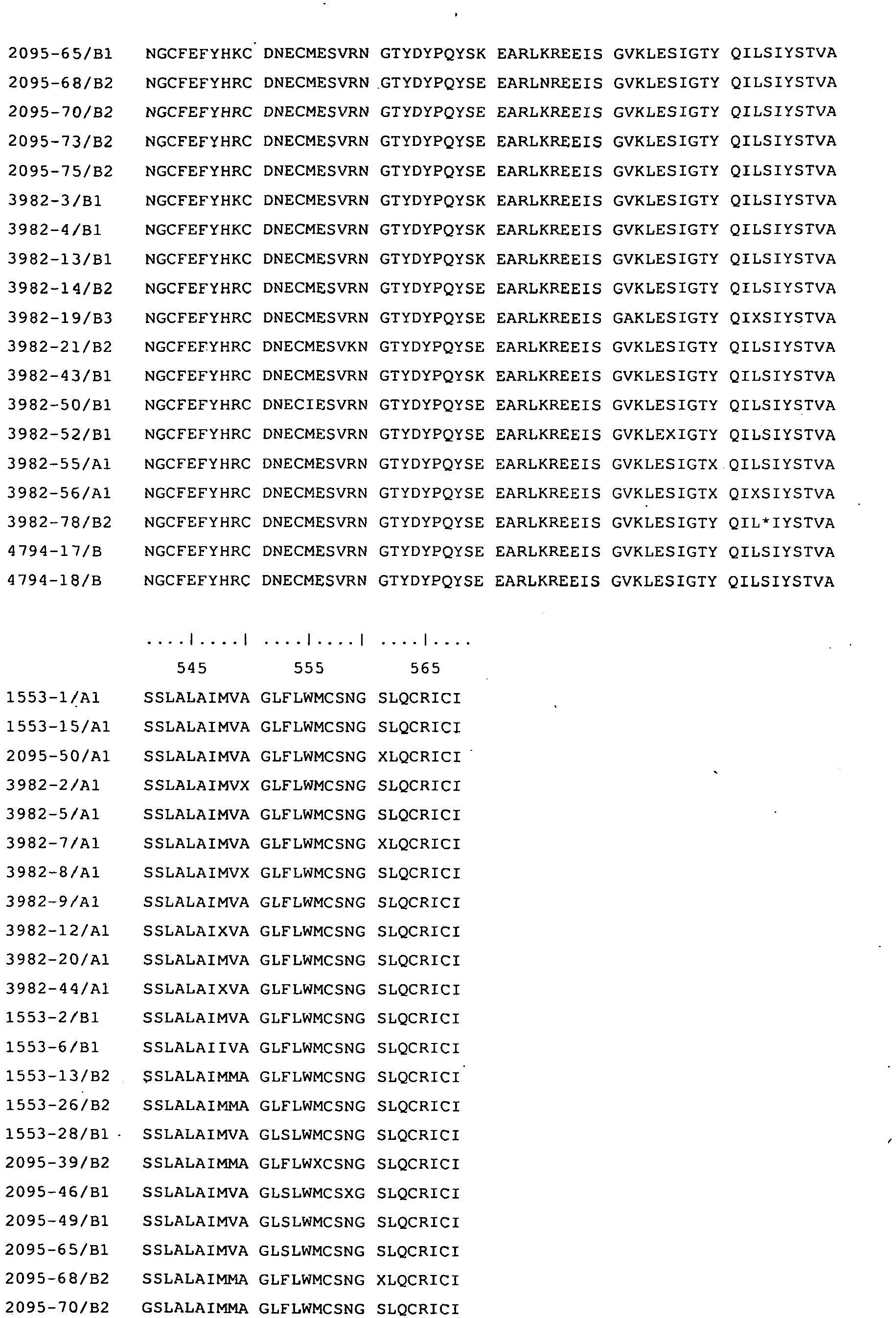

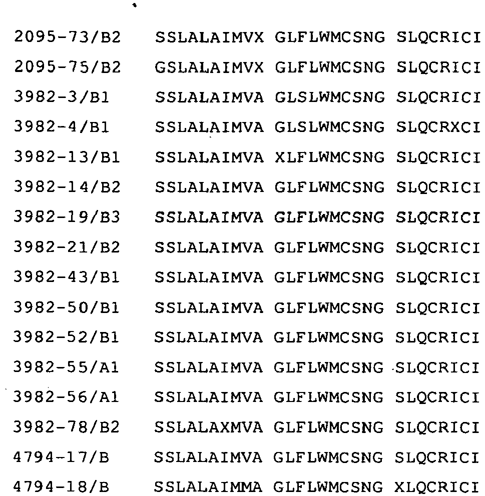

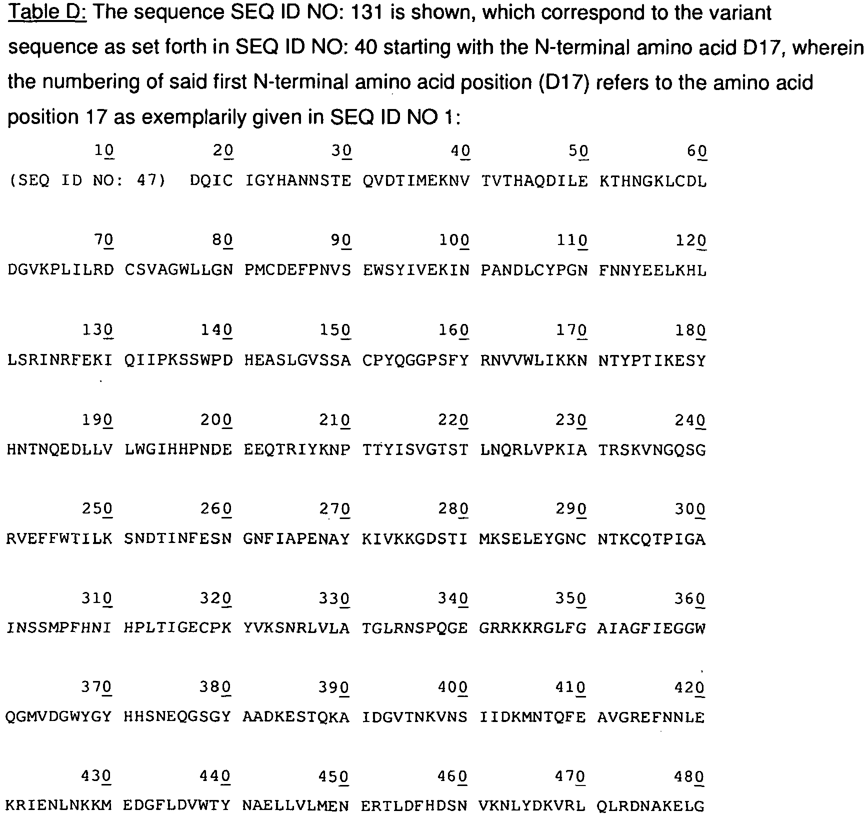

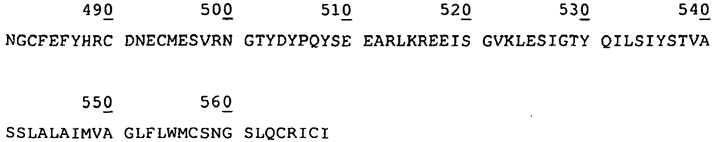

It is understood that the sequence provided in Table D (SEQ ID NO: 131) is a variatn of the sequence SEQ ID NO: 40 starting with amino acid D17 and having the full length C-terminal sequence, as mentioned herein.

## Claims

1. A vaccine comprising
a) H5 protein of influenza virus, wherein the H5 protein consists of or comprises any one of the sequences as set forth in SEQ ID NOs: 2 to 12, or 35 or 36 or 40;
b) a pharmaceutical acceptable carrier and/or excipient.

2. The vaccine according to claim 1, wherein the H5 protein consists of or comprises the sequence as set forth in SEQ ID NOs: 8 or SEQ ID NOs: 40.

3. The vaccine according to claim 1 or 2, wherein the H5 protein does not comprise the influenza A H5N1 hemagglutinin signal sequence.

4. The vaccine according to any one of claims 1 to 3, wherein the sequence of said H5 protein starts with the N-terminal amino acid D17, wherein said numbering of D17 corresponds to SEQ ID NO: 1.

5. The vaccine according to any one of claims 1 to 4, wherein the H5 protein has the amino acid 239N.

6. The vaccine according to any one of claims 1 to 5, wherein the excipient is one or more adjuvants, and wherein the adjuvant is an Emulsigen- based adjuvant.

7. A method for producing the vaccine comprising a H5 protein as defined in any one of claims 1 to 5, wherein said method comprises the steps of:
a. isolating or amplifying the nucleic acid that codes for such H5 protein;
b. cloning the H5 encoding nucleic acid in an expression vector; and
c. expressing the H5 protein,
and wherein the expression vector is in particular a recombinant baculovirus and/or wherein the H5 protein is in particular expressed in insect cells.

8. A method for producing the vaccine comprising a H5 protein as defined in any one of claims 1 to 5, wherein said method comprises the steps of:
a. obtaining a H5 protein as defined in any one of claims 1 to 5; and
b. admixing the H5 protein of step a) with a pharmaceutical acceptable carrier and/or excipient.

9. The vaccine according to any one of claims 1 to 6 for use in a method of treating or preventing infections with Subclade A H5N1 virus of North African origin.

10. A kit of parts, that comprises
a. the vaccine according to any one of claims 1 to 6; and
b. package leaflet indicating the use of such H5 protein, vector or vaccine of a) for the treatment or prophylaxis of infections caused by influenza virus,
and wherein such kit preferably comprises at least one further antigen of poultry or mammalian pathogen.

## Patentansprüche

1. Vakzine, umfassend
a) H5-Protein des Influenzavirus, wobei das H5-Protein aus irgendeiner der in SEQ ID NOs: 2 bis 12 oder 35 oder 36 oder 40 dargelegten Sequenzen besteht oder diese umfasst;
b) einen pharmazeutisch verträglichen Träger und/oder Exzipienten.

2. Vakzine nach Anspruch 1, wobei das H5-Protein aus der in SEQ ID NOs: 8 oder SEQ ID NOs: 40 dargelegten Sequenz besteht oder diese umfasst.

3. Vakzine nach Anspruch 1 oder 2, wobei das H5-Protein nicht die Influenza A H5N1-Hämagglutinin-Signalsequenz umfasst.

4. Vakzine nach irgendeinem der Ansprüche 1 bis 3, wobei die Sequenz des H5-Proteins mit der N-terminalen Aminosäure D17 beginnt, wobei die Nummerierung von D17 der SEQ ID NO: 1 entspricht.

5. Vakzine nach irgendeinem der Ansprüche 1 bis 4, wobei das H5-Protein die Aminosäure 239N aufweist.

6. Vakzine nach irgendeinem der Ansprüche 1 bis 5, wobei der Exzipient ein oder mehrere Adjuvantien ist und wobei das Adjuvans ein Adjuvans auf Emulsigenbasis ist.

7. Verfahren zur Herstellung der Vakzine, umfassend ein H5-Protein wie in irgendeinem der Ansprüche 1 bis 5 definiert, wobei das Verfahren die folgenden Schritte umfasst:
a. Isolieren oder Amplifizieren der Nucleinsäure, die ein solches H5-Protein kodiert;
b. Klonieren der H5-kodierenden Nucleinsäure in einem Expressionsvektor; und
c. Expression des H5-Proteins,
und wobei der Expressionsvektor insbesondere ein rekombinantes Baculovirus ist und/oder wobei das H5-Protein insbesondere in Insektenzellen exprimiert wird.

8. Verfahren zur Herstellung der Vakzine, umfassend ein H5-Protein wie in irgendeinem der Ansprüche 1 bis 5 definiert, wobei das Verfahren folgende Schritte umfasst:
a. Erhalten eines H5-Proteins wie in irgendeinem der Ansprüche 1 bis 5 definiert; und
b. Mischen des H5-Proteins von Schritt a) mit einem pharmazeutisch verträglichen Träger und/oder Exzipienten.

9. Vakzine nach irgendeinem der Ansprüche 1 bis 6 zur Verwendung in einem Verfahren zur Behandlung oder Prävention von Infektionen mit Subklade A H5N1-Virus nordafrikanischen Ursprungs.

10. Kit von Teilen, das umfasst
a. die Vakzine nach irgendeinem der Ansprüche 1 bis 6; und
b. Beipackzettel, der die Verwendung eines solchen H5-Proteins, Vektors oder einer solchen Vakzine von a) für die Behandlung oder Prophylaxe von durch Influenzavirus verursachten Infektionen angibt,
und wobei ein solches Kit vorzugsweise mindestens ein weiteres Antigen von Geflügel- oder Säugetierpathogen umfasst.

## Revendications

1. Vaccin comprenant
a) une protéine H5 de virus de la grippe, dans lequel la protéine H5 consiste en ou comprend l'une quelconque des séquences représentées par les SEQ ID NO : 2 à 12, ou 35 ou 36 ou 40 ;
b) un support et/ou excipient pharmaceutiquement acceptable.

2. Vaccin selon la revendication 1, dans lequel la protéine H5 consiste en ou comprend la séquence représentée par SEQ ID NO : 8 ou SEQ ID NO : 40.

3. Vaccin selon la revendication 1 ou 2, dans lequel la protéine H5 ne comprend pas la séquence signal de l'hémagglutinine de la grippe A H5N1.

4. Vaccin selon l'une quelconque des revendications 1 à 3, dans lequel la séquence de ladite protéine H5 commence avec l'acide aminé N-terminal D17, dans lequel ladite numérotation de D17 correspond à SEQ ID NO : 1.

5. Vaccin selon l'une quelconque des revendications 1 à 4, dans lequel la protéine H5 contient l'acide aminé 239N.

6. Vaccin selon l'une quelconque des revendications 1 à 5, dans lequel l'excipient est un ou plusieurs adjuvants, et dans lequel l'adjuvant est un adjuvant à base d'Emulsigen.

7. Méthode de production du vaccin comprenant une protéine H5 telle que définie dans l'une quelconque des revendications 1 à 5, dans laquelle ladite méthode comprend les étapes :
a. de l'isolement ou l'amplification de l'acide nucléique qui code pour une telle protéine H5 ;
b. du clonage de l'acide nucléique codant pour H5 dans un vecteur d'expression ; et
c. de l'expression de la protéine H5,
et dans laquelle le vecteur d'expression est en particulier un baculovirus recombinant et/ou dans laquelle la protéine H5 est exprimée en particulier dans des cellules d'insecte.

8. Méthode de production du vaccin comprenant une protéine H5 telle que définie dans l'une quelconque des revendications 1 à 5, dans laquelle ladite méthode comprend les étapes :
a. de l'obtention d'une protéine H5 telle que définie dans l'une quelconque des revendications 1 à 5 ; et
b. du mélange de la protéine H5 de l'étape a) avec un support et/ou un excipient pharmaceutiquement acceptable.

9. Vaccin selon l'une quelconque des revendications 1 à 6 pour une utilisation dans une méthode de traitement ou de prévention d'infections avec le virus H5N1 de sous-clade A d'origine nord-africaine.

10. Kit de parties, qui comprend
a. le vaccin selon l'une quelconque des revendications 1 à 6 ; et
b. une notice indiquant l'utilisation d'une telle protéine H5, d'un tel vecteur ou d'un tel vaccin de a) pour le traitement ou la prophylaxie des infections provoquées par un virus de la grippe,
et dans lequel ce kit comprend de préférence au moins un antigène supplémentaire de pathogène de volaille ou de mammifère.


### SEQUENCE LISTING

<110> Boehringer Ingelheim Vetmedica S.A. de C.V., Mexico Boehringer Ingelheim Vetmedica GmbH Gonzalez-Hernandez, Paulino C Leon Kempis, Maria del Rocio Realpe-Quintero, Mauricio A Stadler, Konrad Vaughn, Eric M
<120> NOVEL HEMAGGLUTININ 5 (H5) PROTEINS FOR THE TREATMENT AND PREVENTION OF INFLUENZA INFECTIONS
<130> Case 1-2674
<160> 131
<170> PatentIn version 3.5
<210> 1
   <211> 562
   <212> PRT
   <213> H5N1
<400> 1
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Leu Asn Val Ser Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Gln Ile Ile Pro Lys Ser Ser Trp Pro Asp His Glu Ala Ser
       130                 135                 140
   Ser Gly Val Ser Ser Ala Cys Pro Tyr Gln Gly Gly Pro Ser Phe Tyr
   145 150 155 160
   Arg Asn Val Val Trp Leu Ile Lys Lys Asp Asn Ala Tyr Pro Thr Ile
   165 170 175
   Lys Lys Ser Tyr His Asn Thr Asn Gln Glu Asp Leu Leu Val Leu Trp
   180 185 190
   Gly Ile His His Pro Asn Asp Glu Ala Glu Gln Thr Arg Leu Tyr Gln
   195 200 205
   Asn Pro Thr Thr Tyr Ile Ser Val Gly Thr Ser Thr Leu Asn Gln Arg
   210 215 220
   Leu Val Pro Lys Ile Ala Thr Arg Ser Lys Val Asn Gly Gln Ser Gly
   225 230 235 240
   Arg Val Glu Phe Phe Trp Thr Ile Leu Lys Ser Asn Asp Ala Ile Asn
   245 250 255
   Phe Glu Ser Asn Gly Asn Phe Ile Ala Pro Glu Asn Ala Tyr Lys Ile
   260 265 270
   Val Lys Lys Gly Asp Ser Thr Ile Met Lys Ser Glu Leu Glu Tyr Gly
   275 280 285
   Asn Cys Asn Thr Lys Cys Gln Thr Pro Ile Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Gly Glu Arg Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
                   405                 410                 415
   Asn Asn Leu Glu Arg Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Arg Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Met Val Ala Gly Leu Phe Leu Trp Met Cys Ser Asn Gly
   545                 550                 555                 560
   Ser Leu
<210> 2
   <211> 567
   <212> PRT
   <213> H5N1
<400> 2
   Met Glu Lys Ile Met Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asn Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Leu Asn Val Pro Glu Trp Ser Tyr Ile Val
                   85                  90                  95
   Glu Lys Ile Asn Pro Ala Asn Asp Leu Cys Tyr Pro Gly Lys Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Arg Asn Ser Trp Ser Asp His Glu Thr Ser
       130                 135                 140
   Gly Val Ser Ser Ala Cys Gln Tyr Gln Gly Arg Ser Ser Phe Phe Arg
   145                 150                 155                 160
   Asn Val Val Trp Leu Thr Lys Lys Asp Asn Ala Tyr Ser Thr Ile Lys
                   165                 170                 175
   Arg Ser Tyr Asn Asn Thr Asn Gln Glu Asp Leu Leu Val Leu Trp Gly
               180                 185                 190
   Ile His His Pro Asn Asp Ala Ala Glu Gln Thr Arg Leu Tyr Gln Asn
           195                 200                 205
   Pro Thr Thr Tyr Ile Ser Val Gly Thr Ser Thr Leu Asn Gln Arg Leu
       210                 215                 220
   Val Pro Lys Ile Ala Thr Arg Ser Lys Val Asn Gly Gln Ser Gly Arg
   225                 230                 235                 240
   Met Glu Phe Phe Trp Thr Ile Leu Lys Ser Asn Asp Ala Ile Asn Phe
                   245                 250                 255
   Glu Ser Asn Gly Asn Phe Ile Ala Pro Glu Asn Ala Tyr Lys Ile Val
               260                 265                 270
   Lys Lys Gly Asp Ser Thr Ile Met Lys Ser Glu Leu Glu Tyr Gly Asn
           275                 280                 285
   Cys Asn Thr Lys Cys Gln Thr Pro Ile Gly Ala Ile Asn Ser Ser Met
       290                 295                 300
   Pro Phe His Asn Ile His Pro Leu Thr Ile Gly Glu Cys Pro Lys Tyr
   305                 310                 315                 320
   Val Lys Ser Asn Arg Leu Val Leu Ala Thr Gly Leu Arg Asn Ser Pro
                   325                 330                 335
   Gln Glu Glu Arg Arg Arg Lys Lys Arg Gly Leu Phe Gly Ala Ile Ala
               340                 345                 350
   Gly Phe Ile Glu Gly Gly Trp Gln Gly Met Val Asp Gly Trp Tyr Gly
           355                 360                 365
   Tyr His His Ser Asn Glu Gln Gly Ser Gly Tyr Ala Ala Asp Lys Glu
       370                 375                 380
   Ser Thr Gln Lys Ala Ile Asp Gly Val Thr Asn Lys Val Asn Ser Ile
   385                 390                 395                 400
   Ile Asp Lys Met Asn Thr Gln Phe Glu Ala Val Gly Arg Glu Phe Asn
                   405                 410                 415
   Asn Leu Glu Arg Arg Ile Glu Asn Leu Asn Lys Lys Met Glu Asp Gly
               420                 425                 430
   Phe Leu Asp Val Trp Thr Tyr Asn Ala Glu Leu Leu Val Leu Met Glu
           435                 440                 445
   Asn Glu Arg Thr Leu Asp Phe His Asp Ser Asn Val Lys Asn Leu Tyr
       450                 455                 460
   Asp Lys Val Arg Leu Gln Leu Arg Asp Asn Ala Lys Glu Leu Gly Asn
   465                 470                 475                 480
   Gly Cys Phe Glu Phe Tyr His Arg Cys Asp Asn Glu Cys Met Glu Ser
                   485                 490                 495
   Val Arg Asn Gly Thr Tyr Asp Tyr Pro Gln Tyr Ser Glu Glu Ala Arg
               500                 505                 510
   Leu Lys Arg Glu Glu Ile Ser Gly Val Lys Leu Glu Ser Ile Gly Thr
           515                 520                 525
   Tyr Gln Ile Leu Ser Ile Tyr Ser Thr Val Ala Ser Ser Leu Ala Leu
       530                 535                 540
   Ala Ile Met Val Ala Gly Leu Phe Leu Trp Met Cys Ser Asn Gly Ser
   545                 550                 555                 560
   Leu Gln Cys Arg Ile Cys Ile
   565
<210> 3
   <211> 567
   <212> PRT
   <213> H5N1
<400> 3
   Met Glu Lys Ile Val Leu Leu Leu Ala Ile Val Ser Ile Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asn Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Leu Asn Val Pro Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Gly Ser Trp Ser Asp His Glu Ala Ser
       130                 135                 140
   Gly Val Ser Ser Ala Cys Pro Tyr Gln Gly Arg Ser Ser Phe Phe Arg
   145                 150                 155                 160
   Asn Val Val Trp Leu Thr Lys Lys Asn Asn Ala Tyr Pro Thr Ile Lys
                   165                 170                 175
   Lys Ser Tyr Asn Asn Thr Asn Gln Glu Asp Leu Leu Val Leu Trp Gly
               180                 185                 190
   Ile His His Pro Asn Asp Ala Ala Glu Gln Thr Arg Leu Tyr Gln Asn
           195                 200                 205
   Pro Thr Thr Tyr Ile Ser Val Gly Thr Ser Thr Leu Asn Gln Arg Leu
       210                 215                 220
   Val Pro Lys Ile Ala Thr Arg Ser Lys Val Asn Gly Gln Ser Gly Arg
   225                 230                 235                 240
   Met Glu Phe Phe Trp Thr Ile Leu Lys Ser Asn Asp Ala Ile Asn Phe
                   245                 250                 255
   Glu Ser Asn Gly Asn Phe Ile Ala Pro Glu Asn Ala Tyr Lys Ile Val
               260                 265                 270
   Lys Lys Gly Asp Ser Thr Ile Met Lys Ser Glu Leu Glu Tyr Gly Asn
           275                 280                 285
   Cys Asn Thr Lys Cys Gln Thr Pro Ile Gly Ala Ile Asn Ser Ser Met
       290                 295                 300
   Pro Phe His Asn Ile His Pro Leu Thr Ile Gly Glu Cys Pro Lys Tyr
   305                 310                 315                 320
   Val Lys Ser Asn Arg Leu Val Leu Ala Thr Gly Leu Arg Asn Ser Pro
                   325                 330                 335
   Gln Gly Glu Arg Arg Arg Lys Lys Arg Gly Leu Phe Gly Ala Ile Ala
               340                 345                 350
   Gly Phe Ile Glu Gly Gly Trp Gln Gly Met Val Asp Gly Trp Tyr Gly
           355                 360                 365
   Tyr His His Ser Asn Glu Gln Gly Ser Gly Tyr Ala Ala Asp Lys Glu
       370                 375                 380
   Ser Thr Gln Lys Ala Ile Asp Gly Val Thr Asn Lys Val Asn Ser Ile
   385                 390                 395                 400
   Ile Asp Lys Met Asn Thr Gln Phe Glu Ala Val Gly Arg Glu Phe Asn
                   405                 410                 415
   Asn Leu Glu Arg Arg Ile Glu Asn Leu Asn Lys Lys Met Glu Asp Gly
               420                 425                 430
   Phe Leu Asp Val Trp Thr Tyr Asn Ala Glu Leu Leu Val Leu Met Glu
           435                 440                 445
   Asn Glu Arg Thr Leu Asp Phe His Asp Ser Asn Val Lys Asn Leu Tyr
   450 455 460
   Asp Arg Val Arg Leu Gln Leu Arg Asp Asn Ala Lys Glu Leu Gly Asn
   465 470 475 480
   Gly Cys Phe Glu Phe Tyr His Arg Cys Asp Asn Glu Cys Met Glu Ser
   485 490 495
   Val Arg Asn Gly Thr Tyr Asp Tyr Pro Gln Tyr Ser Glu Glu Ala Arg
   500 505 510
   Leu Lys Arg Glu Glu Ile Ser Gly Val Lys Leu Glu Ser Ile Gly Thr
   515 520 525
   Tyr Gln Ile Leu Ser Ile Tyr Ser Thr Val Ala Ser Ser Leu Ala Leu
   530 535 540
   Ala Ile Met Val Ala Gly Leu Phe Leu Trp Met Cys Ser Asn Gly Ser
   545 550 555 560
   Leu Gln Cys Arg Ile Cys Ile
   565
<210> 4
   <211> 560
   <212> PRT
   <213> H5N1
<220>
   <221> misc_feature
   <222> (560)..(560)
   <223> Xaa can be any naturally occurring amino acid
<400> 4
   Met Glu Lys Ile Val Leu Leu Leu Ala Ile Val Ser Leu Val Lys Gly
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asn Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Leu Asn Val Pro Glu Trp Ser Tyr Ile Val
                   85                  90                  95
   Glu Lys Ile Tyr Pro Ala Asn Asp Leu Cys Tyr Pro Gly Asn Phe Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Ser Ser Trp Ser Asp His Glu Ala Ser
       130                 135                 140
   Gly Val Ser Ser Ala Cys Pro Tyr Gln Gly Arg Ser Ser Phe Phe Arg
   145                 150                 155                 160
   Asn Val Val Trp Leu Thr Lys Lys Asp Asn Ala Tyr Pro Thr Ile Lys
                   165                 170                 175
   Lys Ser Tyr Asn Asn Thr Asn Gln Glu Asp Leu Leu Ile Leu Trp Gly
               180                 185                 190
   Ile His His Pro Asn Asp Ala Ala Glu Gln Thr Arg Leu Tyr Gln Asn
           195                 200                 205
   Pro Thr Thr Tyr Ile Ser Val Gly Thr Ser Thr Leu Asn Gln Arg Leu
       210                 215                 220
   Val Pro Lys Ile Ala Thr Arg Ser Lys Val Asn Gly Gln Ser Gly Arg
   225                 230                 235                 240
   Met Glu Phe Phe Trp Thr Ile Leu Lys Ser Asn Asp Ala Ile Asn Phe
                   245                 250                 255
   Glu Ser Asn Gly Asn Phe Ile Ala Pro Glu Asn Ala Tyr Lys Ile Val
               260                 265                 270
   Lys Lys Gly Asp Ser Thr Ile Met Lys Ser Glu Leu Glu Tyr Gly Asn
           275                 280                 285
   Cys Asn Thr Lys Cys Gln Thr Pro Ile Gly Ala Ile Asn Ser Ser Met
       290                 295                 300
   Pro Phe His Asn Ile His Pro Leu Thr Ile Gly Glu Cys Pro Lys Tyr
   305                 310                 315                 320
   Val Lys Ser Asn Arg Leu Val Leu Ala Thr Gly Leu Arg Asn Ser Pro
                   325                 330                 335
   Gln Gly Glu Arg Arg Arg Lys Lys Arg Gly Leu Phe Gly Ala Ile Ala
               340                 345                 350
   Gly Phe Ile Glu Gly Gly Trp Gln Gly Met Val Asp Gly Trp Tyr Gly
           355                 360                 365
   Tyr His His Ser Asn Glu Gln Gly Ser Gly Tyr Ala Ala Asp Lys Glu
       370                 375                 380
   Ser Thr Gln Lys Ala Ile Asp Gly Val Thr Asn Lys Val Asn Ser Ile
   385                 390                 395                 400
   Ile Asp Lys Met Asn Thr Gln Phe Glu Ala Val Gly Arg Glu Phe Asn
                   405                 410                 415
   Asn Leu Glu Arg Arg Ile Glu Asn Leu Asn Lys Lys Met Glu Asp Gly
               420                 425                 430
   Phe Leu Asp Val Trp Thr Tyr Asn Ala Glu Leu Leu Val Leu Met Glu
           435                 440                 445
   Asn Glu Arg Thr Leu Asp Phe His Asp Ser Asn Val Lys Asn Leu Tyr
       450                 455                 460
   Asp Lys Val Arg Leu Gln Leu Arg Asp Asn Ala Lys Glu Leu Gly Asn
   465                 470                 475                 480
   Gly Cys Phe Glu Phe Tyr His Arg Cys Asp Asn Glu Cys Met Glu Ser
                   485                 490                 495
   Val Arg Asn Gly Thr Tyr Asp Tyr Pro Gln Tyr Ser Glu Glu Ala Arg
               500                 505                 510
   Leu Lys Arg Glu Glu Ile Ser Gly Val Lys Leu Glu Ser Ile Gly Thr
           515                 520                 525
   Tyr Gln Ile Leu Ser Ile Tyr Ser Thr Val Ala Ser Ser Leu Ala Leu
       530                 535                 540
   Ala Ile Met Val Ala Gly Leu Phe Leu Trp Met Cys Ser Asn Gly Xaa
   545                 550                 555                 560
<210> 5
   <211> 544
   <212> PRT
   <213> H5N1
<220>
   <221> misc_feature
   <222> (1)..(1)
   <223> Xaa can be any naturally occurring amino acid
<220>
   <221> misc_feature
   <222> (544)..(544)
   <223> Xaa can be any naturally occurring amino acid
<400> 5
   Xaa Leu Leu Ala Ile Val Ser Ile Val Lys Ser Asp Gln Ile Cys Ile
   1               5                   10                  15
   Gly Tyr His Ala Asn Asn Ser Thr Glu Gln Val Asp Thr Ile Met Glu
               20                  25                  30
   Lys Asn Val Thr Val Thr His Ala Gln Asp Ile Leu Glu Lys Thr His
           35                  40                  45
   Asn Gly Lys Leu Cys Asn Leu Asp Gly Val Lys Pro Leu Ile Leu Arg
       50                  55                  60
   Asp Cys Ser Val Ala Gly Trp Leu Leu Gly Asn Pro Met Cys Asp Glu
   65                  70                  75                  80
   Phe Leu Asp Val Pro Glu Trp Ser Tyr Ile Val Glu Lys Ile Asn Pro
                   85                  90                  95
   Ala Asn Asp Leu Cys Tyr Pro Gly Asn Phe Asn Asp Tyr Glu Glu Leu
               100                 105                 110
   Lys His Leu Leu Ser Arg Ile Asn His Phe Glu Lys Ile Gln Ile Ile
           115                 120                 125
   Pro Lys Asn Ser Trp Ser Asp His Glu Thr Ser Gly Val Ser Ser Ala
       130                 135                 140
   Cys Pro Tyr Gln Gly Arg Ser Ser Phe Phe Arg Asn Val Val Trp Leu
   145                 150                 155                 160
   Thr Lys Lys Asn Thr Ala Tyr Pro Thr Ile Lys Lys Ser Tyr Asn Asn
                   165                 170                 175
   Thr Asn Gln Glu Asp Leu Leu Val Leu Trp Gly Ile His His Pro Asn
               180                 185                 190
   Asp Ala Ala Glu Gln Thr Arg Leu Tyr Gln Asn Pro Thr Thr Tyr Ile
           195                 200                 205
   Ser Val Gly Thr Ser Thr Leu Asn Gln Arg Leu Val Pro Lys Ile Ala
       210                 215                 220
   Thr Arg Ser Lys Val Asn Gly Gln Ser Gly Arg Met Glu Phe Phe Trp
   225                 230                 235                 240
   Thr Ile Leu Lys Ser Asn Asp Ala Ile Asn Phe Glu Ser Asn Gly Asn
                   245                 250                 255
   Phe Ile Ala Pro Glu Asn Ala Tyr Lys Ile Val Lys Lys Gly Asp Ser
               260                 265                 270
   Thr Ile Met Lys Ser Glu Leu Glu Tyr Gly Asn Cys Asn Thr Lys Cys
           275                 280                 285
   Gln Thr Pro Ile Gly Ala Ile Asn Ser Ser Met Pro Phe His Asn Ile
       290                 295                 300
   His Pro Leu Thr Ile Gly Glu Cys Pro Lys Tyr Val Lys Ser Asn Arg
   305                 310                 315                 320
   Leu Val Leu Ala Thr Gly Leu Arg Asn Ser Pro His Gly Glu Arg Arg
                   325                 330                 335
   Arg Lys Lys Arg Gly Leu Phe Gly Ala Ile Ala Gly Phe Ile Glu Gly
               340                 345                 350
   Gly Trp Gln Gly Met Val Asp Gly Trp Tyr Gly Tyr His His Ser Asn
           355                 360                 365
   Glu Gln Gly Ser Gly Tyr Ala Ala Asp Lys Glu Ser Thr Gln Lys Ala
       370                 375                 380
   Ile Asp Gly Val Thr Asn Lys Val Asn Ser Ile Ile Asp Lys Met Asn
   385                 390                 395                 400
   Thr Gln Phe Glu Ala Val Gly Arg Glu Phe Asn Asn Leu Glu Arg Arg
                   405                 410                 415
   Ile Glu Asn Leu Asn Lys Lys Met Glu Asp Gly Phe Leu Asp Val Trp
               420                 425                 430
   Thr Tyr Asn Ala Glu Leu Leu Val Leu Met Glu Asn Glu Arg Thr Leu
           435                 440                 445
   Asp Phe His Asp Ser Asn Val Lys Asn Leu Tyr Asp Lys Val Arg Leu
   450 455 460
   Gln Leu Arg Asp Asn Ala Lys Glu Leu Gly Asn Gly Cys Phe Glu Phe
   465 470 475 480
   Tyr His Arg Cys Asp Asn Glu Cys Met Glu Ser Val Arg Asn Gly Thr
   485 490 495
   Tyr Asp Tyr Pro Gln Tyr Ser Glu Glu Ala Arg Leu Lys Arg Glu Glu
   500 505 510
   Ile Ser Gly Val Lys Leu Glu Ser Ile Gly Thr Tyr Gln Ile Leu Ser
   515 520 525
   Ile Tyr Ser Thr Val Ala Ser Ser Leu Ala Leu Ala Ile Met Val Xaa
   530 535 540
<210> 6
   <211> 534
   <212> PRT
   <213> H5N1
<220>
   <221> misc_feature
   <222> (1)..(1)
   <223> Xaa can be any naturally occurring amino acid
<400> 6
   Xaa Val Lys Ser Asp Gln Ile Cys Ile Gly Tyr His Ala Asn Asn Ser
   1 5 10 15
   Thr Glu Gln Val Asp Thr Ile Met Glu Lys Asn Val Thr Val Thr His
   20 25 30
   Ala Gln Asp Ile Leu Glu Lys Thr His Asn Gly Lys Leu Cys Asn Leu
   35 40 45
   Asp Gly Val Lys Pro Leu Ile Leu Arg Asp Cys Ser Val Ala Gly Trp
   50 55 60
   Leu Leu Gly Asn Pro Met Cys Asp Glu Phe Leu Asn Val Glu Trp Ser
   65 70 75 80
   Tyr Ile Val Glu Lys Ile Asn Pro Thr Asn Asp Leu Cys Tyr Pro Gly
   85 90 95
   Asn Phe Asn Asp Tyr Glu Glu Leu Lys His Leu Leu Ser Arg Ile Asn
   100 105 110
   His Phe Glu Lys Ile Gln Ile Ile Pro Lys Asn Tyr Trp Ser Asp His
   115 120 125
   Glu Thr Ser Gly Val Ser Ser Ala Cys Pro Tyr Gln Gly Arg Pro Ser
   130 135 140
   Phe Phe Arg Asn Val Val Trp Leu Thr Lys Lys Asn Asn Ala Tyr Pro
   145 150 155 160
   Thr Ile Lys Lys Ser Tyr Asn Asn Thr Asn Gln Glu Asp Leu Leu Val
   165 170 175
   Leu Trp Gly Ile His His Pro Asn Asp Ala Ala Glu Gln Thr Arg Leu
   180 185 190
   Tyr Gln Asn Pro Thr Thr Tyr Ile Ser Val Gly Thr Ser Thr Leu Asn
   195 200 205
   Gln Arg Leu Val Pro Lys Ile Ala Thr Arg Ser Lys Val Asn Gly Gln
   210 215 220
   Ser Gly Arg Met Glu Phe Phe Trp Thr Ile Leu Lys Ser Asn Asp Ala
   225 230 235 240
   Ile Asn Phe Glu Ser Asn Gly Asn Phe Ile Ala Pro Glu Asn Ala Tyr
   245 250 255
   Lys Ile Val Lys Lys Gly Asp Ser Thr Ile Met Lys Ser Glu Leu Glu
   260 265 270
   Tyr Gly Asn Cys Asn Thr Lys Cys Gln Thr Pro Ile Gly Ala Ile Asn
   275 280 285
   Ser Ser Met Pro Phe His Asn Ile His Pro Leu Thr Ile Gly Glu Cys
   290 295 300
   Pro Lys Tyr Val Lys Ser Asn Arg Leu Val Leu Ala Thr Gly Leu Arg
   305 310 315 320
   Asn Ser Pro Gln Gly Glu Arg Arg Arg Lys Lys Arg Gly Leu Phe Gly
   325 330 335
   Ala Ile Ala Gly Phe Ile Glu Gly Gly Trp Gln Gly Met Val Asp Gly
   340 345 350
   Trp Tyr Gly Tyr His His Ser Asn Glu Gln Gly Ser Gly Tyr Ala Ala
   355 360 365
   Asp Lys Glu Ser Thr Gln Lys Ala Ile Asp Gly Val Thr Asn Lys Val
   370 375 380
   Asn Ser Ile Ile Asp Lys Met Asn Thr Gln Phe Glu Ala Val Gly Arg
   385 390 395 400
   Glu Phe Asn Asn Leu Glu Arg Arg Ile Glu Asn Leu Asn Lys Lys Met
   405 410 415
   Glu Asp Gly Phe Leu Asp Val Trp Thr Tyr Asn Ala Glu Leu Leu Val
   420 425 430
   Leu Met Glu Asn Glu Arg Thr Leu Asp Phe His Asp Ser Asn Val Lys
   435 440 445
   Asn Leu Tyr Asp Lys Val Arg Leu Gln Leu Arg Asp Asn Ala Lys Glu
   450 455 460
   Leu Gly Asn Gly Cys Phe Glu Phe Tyr His Arg Cys Asp Asn Glu Cys
   465 470 475 480
   Met Glu Ser Val Arg Asn Gly Thr Tyr Asp Tyr Pro Gln Tyr Ser Glu
   485 490 495
   Glu Ala Arg Leu Lys Arg Glu Glu Ile Ser Gly Val Lys Leu Glu Ser
   500 505 510
   Ile Gly Thr Tyr Gln Ile Leu Ser Ile Tyr Ser Thr Val Ala Ser Ser
   515 520 525
   Leu Ala Leu Ala Ile Met
   530
<210> 7
   <211> 548
   <212> PRT
   <213> H5N1
<220>
   <221> misc_feature
   <222> (1)..(1)
   <223> Xaa can be any naturally occurring amino acid
<220>
   <221> misc_feature
   <222> (548)..(548)
   <223> Xaa can be any naturally occurring amino acid
<400> 7
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
               20                  25                  30
   Ala Gln Asp Ile Leu Glu Lys Thr His Asn Gly Lys Leu Cys Asn Leu
           35                  40                  45
   Asp Gly Val Lys Pro Leu Ile Leu Arg Asp Cys Ser Val Ala Gly Trp
       50                  55                  60
   Leu Leu Gly Asn Pro Met Cys Asp Lys Phe Leu Asn Val Pro Glu Trp
   65                  70                  75                  80
   Ser Tyr Ile Val Glu Lys Ile Asn Pro Thr Asn Asp Leu Cys Tyr Pro
                   85                  90                  95
   Gly Asn Phe Asn Asp Tyr Glu Glu Leu Lys His Leu Leu Ser Arg Ile
               100                 105                 110
   Asn His Phe Glu Lys Ile Gln Ile Ile Pro Lys Asn Ser Trp Ser Asp
           115                 120                 125
   His Glu Ala Ser Gly Val Ser Ser Ala Cys Pro Tyr Gln Gly Arg Ser
       130                 135                 140
   Ser Phe Phe Arg Asn Val Val Trp Leu Thr Lys Lys Asn Asn Ala Tyr
   145                 150                 155                 160
   Pro Thr Ile Lys Lys Ser Tyr Asn Asn Thr Asn Gln Glu Asp Leu Leu
                   165                 170                 175
   Val Leu Trp Gly Ile His His Pro Asn Asp Ala Ala Glu Gln Thr Arg
               180                 185                 190
   Leu Tyr Gln Asn Pro Thr Thr Tyr Ile Ser Val Gly Thr Ser Thr Leu
           195                 200                 205
   Asn Gln Arg Leu Val Pro Lys Ile Ala Thr Arg Ser Lys Val Asn Gly
       210                 215                 220
   Gln Ser Gly Arg Met Glu Phe Phe Trp Thr Ile Leu Lys Ser Asn Asp
   225                 230                 235                 240
   Ala Ile Asn Phe Glu Ser Asn Gly Asn Phe Ile Ala Pro Glu Asn Ala
                   245                 250                 255
   Tyr Lys Ile Val Lys Lys Gly Asp Ser Thr Ile Met Lys Ser Glu Leu
               260                 265                 270
   Glu Tyr Gly Asp Cys Asn Thr Lys Cys Gln Thr Pro Ile Gly Ala Ile
           275                 280                 285
   Asn Ser Ser Met Pro Phe His Asn Ile His Pro Leu Thr Ile Gly Glu
       290                 295                 300
   Cys Pro Lys Tyr Val Lys Ser Asn Arg Leu Val Leu Ala Thr Gly Leu
   305                 310                 315                 320
   Arg Asn Ser Pro Gln Gly Glu Arg Arg Arg Lys Lys Arg Gly Leu Phe
                   325                 330                 335
   Gly Ala Ile Ala Gly Phe Ile Glu Gly Gly Trp Gln Gly Met Val Asp
               340                 345                 350
   Gly Trp Tyr Gly Tyr His His Ser Asn Glu Gln Gly Ser Gly Tyr Ala
           355                 360                 365
   Ala Asp Lys Glu Ser Thr Gln Lys Ala Ile Asp Gly Val Thr Asn Lys
       370                 375                 380
   Val Asn Ser Ile Ile Asp Lys Met Asn Thr Gln Phe Glu Ala Val Gly
   385                 390                 395                 400
   Arg Glu Phe Asn Asn Leu Glu Arg Arg Ile Glu Asn Leu Asn Lys Lys
                   405                 410                 415
   Met Glu Asp Gly Phe Leu Asp Val Trp Thr Tyr Asn Ala Glu Leu Leu
               420                 425                 430
   Val Leu Met Glu Asn Glu Arg Thr Leu Asp Phe His Asp Ser Asn Val
           435                 440                 445
   Lys Asn Leu Tyr Asp Lys Val Arg Leu Gln Leu Arg Asp Asn Ala Lys
       450                 455                 460
   Glu Leu Gly Asn Gly Cys Phe Glu Phe Tyr His Arg Cys Asp Asn Glu
   465                 470                 475                 480
   Cys Met Glu Ser Val Arg Asn Gly Thr Tyr Asp Tyr Pro Gln Tyr Ser
                   485                 490                 495
   Glu Glu Ala Arg Leu Lys Arg Glu Glu Ile Ser Gly Val Lys Leu Glu
   500 505 510
   Ser Ile Gly Thr Tyr Gln Ile Leu Ser Ile Tyr Ser Thr Val Ala Ser
   515 520 525
   Ser Leu Ala Leu Ala Ile Met Val Ala Gly Leu Phe Leu Trp Met Cys
   530 535 540
   Ser Asn Gly Xaa
   545
<210> 8
   <211> 541
   <212> PRT
   <213> H5N1
<220>
   <221> misc_feature
   <222> (1)..(1)
   <223> Xaa can be any naturally occurring amino acid
<220>
   <221> misc_feature
   <222> (541)..(541)
   <223> Xaa can be any naturally occurring amino acid
<400> 8
   Xaa Ile Val Ser Ile Val Lys Ser Asp Gln Ile Cys Ile Gly Tyr His
   1               5                   10                  15
   Ala Asn Asn Ser Thr Glu Gln Val Asp Thr Ile Met Glu Lys Asn Val
               20                  25                  30
   Thr Val Thr His Ala Gln Asp Ile Leu Glu Lys Thr His Asn Gly Lys
           35                  40                  45
   Leu Cys Asn Leu Asp Gly Val Lys Pro Leu Ile Leu Arg Asp Cys Ser
       50                  55                  60
   Val Ala Gly Trp Leu Leu Gly Asn Pro Met Cys Asp Glu Phe Leu Asn
   65                  70                  75                  80
   Val Pro Glu Trp Ser Tyr Ile Val Glu Lys Ile Asn Pro Thr Asn Asp
                   85                  90                  95
   Leu Cys Tyr Pro Gly Asn Phe Asn Asp Tyr Glu Glu Leu Lys His Leu
               100                 105                 110
   Leu Ser Arg Ile Asn His Phe Glu Lys Ile Gln Ile Ile Pro Lys Asn
           115                 120                 125
   Ser Trp Ser Asp His Glu Ala Ser Gly Val Ser Ser Ala Cys Pro Tyr
       130                 135                 140
   Gln Gly Arg Ser Ser Phe Phe Arg Asn Val Val Trp Leu Thr Lys Lys
   145                 150                 155                 160
   Asn Asn Ala Tyr Pro Thr Ile Lys Lys Ser Tyr Asn Asn Thr Asn Gln
                   165                 170                 175
   Glu Asp Leu Leu Val Leu Trp Gly Ile His His Pro Asn Asp Ala Ala
               180                 185                 190
   Glu Gln Thr Arg Leu Tyr Gln Asn Pro Thr Thr Tyr Ile Ser Val Gly
           195                 200                 205
   Thr Ser Thr Leu Asn Gln Arg Leu Val Pro Lys Ile Ala Thr Arg Ser
       210                 215                 220
   Lys Val Asn Gly Gln Ser Gly Arg Met Glu Phe Phe Trp Thr Ile Leu
   225                 230                 235                 240
   Lys Ser Asn Asp Ala Ile Asn Phe Glu Ser Asn Gly Asn Phe Ile Ala
                   245                 250                 255
   Pro Glu Asn Ala Tyr Lys Ile Val Lys Lys Gly Asp Ser Thr Ile Met
               260                 265                 270
   Lys Ser Glu Leu Glu Tyr Gly Asp Cys Asn Thr Lys Cys Gln Thr Pro
           275                 280                 285
   Ile Gly Ala Ile Asn Ser Ser Met Pro Phe His Asn Ile His Pro Leu
       290                 295                 300
   Thr Ile Gly Glu Cys Pro Lys Tyr Val Lys Ser Asn Arg Leu Val Leu
   305                 310                 315                 320
   Ala Thr Gly Leu Arg Asn Ser Pro Gln Gly Glu Arg Arg Arg Lys Lys
                   325                 330                 335
   Arg Gly Leu Phe Gly Ala Ile Ala Gly Phe Ile Glu Gly Gly Trp Gln
               340                 345                 350
   Gly Met Val Asp Gly Trp Tyr Gly Tyr His His Ser Asn Glu Gln Gly
           355                 360                 365
   Ser Gly Tyr Ala Ala Asp Lys Glu Ser Thr Gln Lys Ala Ile Asp Gly
   370 375 380
   Val Thr Asn Lys Val Asn Ser Ile Ile Asp Lys Met Asn Thr Gln Phe
   385 390 395 400
   Glu Ala Val Gly Arg Glu Phe Asn Asn Leu Glu Arg Arg Ile Glu Asn
   405 410 415
   Leu Asn Lys Lys Met Glu Asp Gly Phe Leu Asp Val Trp Thr Tyr Asn
   420 425 430
   Ala Glu Leu Leu Val Leu Met Glu Asn Glu Arg Thr Leu Asp Phe His
   435 440 445
   Asp Ser Asn Val Lys Asn Leu Tyr Asp Lys Val Arg Leu Gln Leu Arg
   450 455 460
   Asp Asn Ala Lys Glu Leu Gly Asn Gly Cys Phe Glu Phe Tyr His Arg
   465 470 475 480
   Cys Asp Asn Glu Cys Met Glu Ser Val Arg Asn Gly Thr Tyr Asp Tyr
   485 490 495
   Pro Gln Tyr Ser Glu Glu Ala Arg Leu Lys Arg Glu Glu Ile Ser Gly
   500 505 510
   Val Lys Leu Glu Ser Ile Gly Thr Tyr Gln Ile Leu Ser Ile Tyr Ser
   515 520 525
   Thr Val Ala Ser Ser Leu Ala Leu Ala Ile Met Val Xaa
   530 535 540
<210> 9
   <211> 520
   <212> PRT
   <213> H5N1
<220>
   <221> misc_feature
   <222> (1)..(1)
   <223> Xaa can be any naturally occurring amino acid
<220>
   <221> misc_feature
   <222> (159)..(159)
   <223> Xaa can be any naturally occurring amino acid
<400> 9
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
   20 25 30
   Ala Gln Asp Ile Leu Glu Lys Thr His Asn Gly Lys Leu Cys Asn Leu
   35 40 45
   Asp Gly Val Lys Pro Leu Ile Leu Arg Asp Cys Ser Val Ala Gly Trp
   50 55 60
   Leu Leu Gly Asn Pro Met Cys Asp Glu Phe Leu Asn Val Pro Glu Trp
   65 70 75 80
   Ser Tyr Ile Val Glu Lys Ile Asn Pro Ala Asn Asp Leu Cys Tyr Pro
   85 90 95
   Gly Asn Phe Asn Asp Tyr Glu Glu Leu Lys His Leu Leu Ser Arg Ile
   100 105 110
   Asn His Phe Glu Lys Ile Gln Ile Ile Pro Lys Asn Ser Trp Ser Asp
   115 120 125
   His Glu Ala Ser Gly Val Ser Ser Ala Cys Pro Tyr Gln Gly Arg Ser
   130 135 140
   Ser Phe Phe Arg Asn Val Val Trp Leu Thr Lys Lys Asn Asn Xaa Tyr
   145 150 155 160
   Pro Thr Ile Lys Lys Ser Tyr Asn Asn Thr Asn Gln Glu Asp Leu Leu
   165 170 175
   Val Leu Trp Gly Ile His His Pro Asn Asp Ala Ala Glu Gln Thr Lys
   180 185 190
   Leu Tyr Gln Asn Pro Thr Thr Tyr Ile Ser Val Gly Thr Ser Thr Leu
   195 200 205
   Asn Gln Arg Leu Val Pro Lys Ile Ala Thr Arg Ser Lys Val Asn Gly
   210 215 220
   Gln Ser Gly Arg Met Glu Phe Phe Trp Thr Ile Leu Lys Ser Asn Asp
   225 230 235 240
   Ala Ile Asn Phe Glu Ser Asn Gly Asn Phe Ile Ala Pro Glu Asn Ala
   245 250 255
   Tyr Lys Ile Val Lys Lys Gly Asp Ser Thr Ile Met Lys Ser Glu Leu
   260 265 270
   Glu Tyr Gly Asn Cys Asn Thr Lys Cys Gln Thr Pro Ile Gly Ala Ile
   275 280 285
   Asn Ser Ser Met Pro Phe His Asn Ile His Pro Leu Thr Ile Gly Glu
   290 295 300
   Cys Pro Lys Tyr Val Lys Ser Asn Arg Leu Val Leu Ala Thr Gly Leu
   305 310 315 320
   Arg Asn Ser Pro Gln Gly Glu Arg Arg Arg Lys Lys Arg Gly Leu Phe
   325 330 335
   Gly Ala Ile Ala Gly Phe Ile Glu Gly Gly Trp Gln Gly Met Val Asp
   340 345 350
   Gly Trp Tyr Gly Tyr His His Ser Asn Glu Gln Gly Ser Gly Tyr Ala
   355 360 365
   Ala Asp Lys Glu Ser Thr Gln Lys Ala Ile Asp Gly Val Thr Asn Lys
   370 375 380
   Val Asn Ser Ile Ile Asp Lys Met Asn Thr Gln Phe Glu Ala Val Gly
   385 390 395 400
   Arg Glu Phe Asn Asn Leu Glu Arg Arg Ile Glu Asn Leu Asn Lys Lys
   405 410 415
   Met Glu Asp Gly Phe Leu Asp Val Trp Thr Tyr Asn Ala Glu Leu Leu
   420 425 430
   Val Leu Met Glu Asn Glu Arg Thr Leu Asp Phe His Asp Ser Asn Val
   435 440 445
   Lys Asn Leu Tyr Asp Lys Val Arg Leu Gln Leu Arg Asp Asn Ala Lys
   450 455 460
   Glu Leu Gly Asn Gly Cys Phe Glu Phe Tyr His Arg Cys Asp Asn Glu
   465 470 475 480
   Cys Met Glu Ser Val Arg Asn Gly Thr Tyr Asp Tyr Pro Gln Tyr Ser
   485 490 495
   Glu Glu Ala Arg Leu Lys Arg Glu Glu Ile Ser Gly Val Lys Leu Glu
   500 505 510
   Ser Ile Gly Thr Tyr Gln Ile Leu
   515 520
<210> 10
   <211> 537
   <212> PRT
   <213> H5N1
<220>
   <221> misc_feature
   <222> (1)..(1)
   <223> Xaa can be any naturally occurring amino acid
<220>
   <221> misc_feature
   <222> (537)..(537)
   <223> Xaa can be any naturally occurring amino acid
<400> 10
   Xaa Ser Ile Val Lys Ser Asp Gln Ile Cys Ile Gly Tyr His Ala Asn
   1               5                   10                  15
   Asn Ser Thr Glu Gln Val Asp Thr Ile Met Glu Lys Asn Val Thr Val
               20                  25                  30
   Thr His Ala Gln Asp Ile Leu Glu Lys Thr His Asn Gly Lys Leu Cys
           35                  40                  45
   Asn Leu Asp Gly Val Lys Pro Leu Ile Leu Arg Asp Cys Ser Val Ala
       50                  55                  60
   Gly Trp Leu Leu Gly Asn Pro Met Cys Asp Glu Phe Leu Asn Val Pro
   65                  70                  75                  80
   Glu Trp Ser Tyr Ile Val Glu Lys Ile Asn Pro Ala Asn Asp Leu Cys
                   85                  90                  95
   Tyr Pro Gly Asn Phe Asn Asp Tyr Glu Glu Leu Lys His Leu Leu Ser
               100                 105                 110
   Arg Ile Asn His Phe Glu Lys Ile Gln Ile Ile Pro Lys Asn Ser Trp
           115                 120                 125
   Ser Asp His Glu Ala Ser Gly Val Ser Ser Ala Cys Pro Tyr Gln Gly
       130                 135                 140
   Arg Ser Ser Phe Phe Arg Asn Val Val Trp Leu Thr Lys Lys Asn Asn
   145                 150                 155                 160
   Ala Tyr Pro Thr Ile Lys Lys Ser Tyr Asn Asn Thr Asn Gln Glu Asp
                   165                 170                 175
   Leu Leu Val Leu Trp Gly Ile His His Pro Asn Asp Glu Ala Glu Gln
               180                 185                 190
   Thr Arg Leu Tyr Gln Asn Pro Thr Thr Tyr Ile Ser Val Gly Thr Ser
           195                 200                 205
   Thr Leu Asn Gln Arg Leu Val Pro Lys Ile Ala Thr Arg Ser Lys Val
       210                 215                 220
   Asn Gly Gln Ser Gly Arg Met Glu Phe Phe Trp Thr Ile Leu Lys Ser
   225                 230                 235                 240
   Asn Asp Ala Ile Asn Phe Glu Ser Asn Gly Asn Phe Ile Ala Pro Glu
                   245                 250                 255
   Asn Ala Tyr Lys Ile Val Lys Lys Gly Asp Ser Thr Ile Met Lys Ser
               260                 265                 270
   Glu Leu Glu Tyr Gly Asn Cys Asn Thr Lys Cys Gln Thr Pro Ile Gly
           275                 280                 285
   Ala Ile Asn Ser Ser Met Pro Phe His Asn Ile His Pro Leu Thr Ile
       290                 295                 300
   Gly Glu Cys Pro Lys Tyr Val Lys Ser Asn Arg Leu Val Leu Ala Thr
   305                 310                 315                 320
   Gly Leu Arg Asn Ser Pro Gln Gly Glu Arg Arg Arg Lys Lys Arg Gly
                   325                 330                 335
   Leu Phe Gly Ala Ile Ala Gly Phe Ile Glu Gly Gly Trp Gln Gly Met
               340                 345                 350
   Val Asp Gly Trp Tyr Gly Tyr His His Ser Asn Glu Gln Gly Ser Gly
           355                 360                 365
   Tyr Ala Ala Asp Lys Glu Ser Thr Gln Lys Ala Ile Asp Gly Val Thr
       370                 375                 380
   Asn Lys Val Asn Ser Ile Ile Asp Lys Met Asn Thr Gln Phe Glu Ala
   385                 390                 395                 400
   Val Gly Arg Glu Phe Asn Asn Leu Glu Arg Arg Ile Glu Asn Leu Asn
                   405                 410                 415
   Lys Lys Met Glu Asp Gly Phe Leu Asp Val Trp Thr Tyr Asn Ala Glu
   420 425 430
   Leu Leu Val Leu Met Glu Asn Glu Arg Thr Leu Asp Phe His Asp Ser
   435 440 445
   Asn Val Lys Asn Leu Tyr Asp Lys Val Arg Leu Gln Leu Arg Asp Asn
   450 455 460
   Ala Lys Glu Leu Gly Asn Gly Cys Phe Glu Phe Tyr His Arg Cys Asp
   465 470 475 480
   Asn Glu Cys Met Glu Ser Val Arg Asn Gly Thr Tyr Asp Tyr Pro Gln
   485 490 495
   Tyr Ser Glu Glu Ala Arg Leu Lys Arg Glu Glu Ile Ser Gly Val Lys
   500 505 510
   Leu Glu Ser Ile Gly Thr Tyr Gln Ile Leu Ser Ile Tyr Ser Thr Val
   515 520 525
   Ala Ser Ser Leu Ala Leu Ala Ile Xaa
   530 535
<210> 11
   <211> 528
   <212> PRT
   <213> H5N1
<220>
   <221> misc_feature
   <222> (1)..(1)
   <223> Xaa can be any naturally occurring amino acid
<220>
   <221> misc_feature
   <222> (528)..(528)
   <223> Xaa can be any naturally occurring amino acid
<400> 11
   Xaa Ile Val Ser Ile Val Lys Ser Asp Gln Ile Cys Ile Gly Tyr His
   1               5                   10                  15
   Ala Asn Asn Ser Thr Glu Gln Val Asp Thr Ile Met Glu Lys Asn Val
               20                  25                  30
   Thr Val Thr His Ala Gln Asp Ile Leu Glu Lys Thr His Asn Gly Lys
           35                  40                  45
   Leu Cys Asn Leu Asp Gly Val Lys Pro Leu Ile Leu Arg Asp Cys Ser
       50                  55                  60
   Val Ala Gly Trp Leu Leu Gly Asn Pro Met Cys Asp Glu Phe Leu Asn
   65                  70                  75                  80
   Val Pro Glu Trp Ser Tyr Ile Val Glu Lys Ile Asn Pro Ala Asn Asp
                   85                  90                  95
   Leu Cys Tyr Pro Gly Asn Phe Asn Asp Tyr Glu Glu Leu Lys His Leu
               100                 105                 110
   Leu Ser Arg Ile Asn His Phe Glu Lys Ile Gln Ile Ile Pro Lys Asn
           115                 120                 125
   Ser Trp Ser Asp His Glu Ala Ser Gly Val Ser Ser Ala Cys Pro Tyr
       130                 135                 140
   Gln Gly Arg Ser Ser Phe Phe Arg Asn Val Val Trp Leu Thr Lys Lys
   145                 150                 155                 160
   Asn Asn Ala Tyr Pro Thr Ile Lys Lys Ser Tyr Asn Asn Thr Asn Gln
                   165                 170                 175
   Glu Asp Leu Leu Val Leu Trp Gly Ile His His Pro Asn Asp Ala Ala
               180                 185                 190
   Glu Gln Thr Arg Leu Tyr Gln Asn Pro Thr Thr Tyr Ile Ser Val Gly
           195                 200                 205
   Thr Ser Thr Leu Asn Gln Arg Leu Val Pro Lys Ile Ala Asn Arg Ser
       210                 215                 220
   Lys Val Asn Gly Gln Ser Gly Arg Met Glu Phe Phe Trp Thr Ile Leu
   225                 230                 235                 240
   Lys Ser Asn Asp Ala Ile Asn Phe Glu Ser Asn Gly Asn Phe Ile Ala
                   245                 250                 255
   Pro Glu Asn Ala Tyr Lys Ile Val Lys Lys Gly Asp Ser Thr Ile Met
               260                 265                 270
   Lys Ser Glu Leu Glu Tyr Gly Asn Cys Asn Thr Lys Cys Gln Thr Pro
           275                 280                 285
   Ile Gly Ala Ile Asn Ser Ser Met Pro Phe His Asn Ile His Pro Leu
       290                 295                 300
   Thr Ile Gly Glu Cys Pro Lys Tyr Val Lys Ser Asn Arg Leu Val Leu
   305                 310                 315                 320
   Ala Thr Gly Leu Arg Asn Ser Pro Gln Gly Glu Arg Arg Arg Lys Lys
                   325                 330                 335
   Arg Gly Leu Phe Gly Ala Ile Ala Gly Phe Ile Glu Gly Gly Trp Gln
               340                 345                 350
   Gly Met Val Asp Gly Trp Tyr Gly Tyr His His Ser Asn Glu Gln Gly
           355                 360                 365
   Ser Gly Tyr Ala Ala Asp Lys Glu Ser Thr Gln Lys Ala Ile Asp Gly
       370                 375                 380
   Val Thr Asn Lys Val Asn Ser Ile Ile Asp Lys Met Asn Thr Gln Phe
   385                 390                 395                 400
   Glu Ala Val Gly Arg Glu Phe Asn Asn Leu Glu Arg Arg Ile Glu Asn
                   405                 410                 415
   Leu Asn Lys Lys Met Glu Asp Gly Phe Leu Asp Val Trp Thr Tyr Asn
               420                 425                 430
   Ala Glu Leu Leu Val Leu Met Glu Asn Glu Arg Thr Leu Asp Phe His
           435                 440                 445
   Asp Ser Asn Val Lys Asn Leu Tyr Asp Lys Val Arg Leu Gln Leu Arg
       450                 455                 460
   Asp Asn Ala Lys Glu Leu Gly Asn Gly Cys Phe Glu Phe Tyr His Arg
   465                 470                 475                 480
   Cys Asp Asn Glu Cys Met Glu Ser Val Arg Asn Gly Thr Tyr Asp Tyr
                   485                 490                 495
   Pro Gln Tyr Ser Glu Glu Ala Arg Leu Lys Arg Glu Glu Ile Ser Gly
               500                 505                 510
   Val Lys Leu Glu Ser Ile Gly Thr Tyr Gln Ile Leu Ser Ile Tyr Xaa
           515                 520                 525
<210> 12
   <211> 535
   <212> PRT
   <213> H5N1
<220>
   <221> misc_feature
   <222> (1)..(1)
   <223> Xaa can be any naturally occurring amino acid
<220>
   <221> misc_feature
   <222> (535)..(535)
   <223> Xaa can be any naturally occurring amino acid
<400> 12
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
               20                  25                  30
   Ala Gln Asp Ile Leu Glu Lys Thr His Asn Gly Lys Leu Cys Asn Leu
           35                  40                  45
   Asp Gly Val Lys Pro Leu Ile Leu Arg Asp Cys Ser Val Ala Gly Trp
       50                  55                  60
   Leu Leu Gly Asn Pro Met Cys Asp Glu Phe Leu Asn Val Pro Glu Trp
   65                  70                  75                  80
   Ser Tyr Ile Val Glu Lys Ile Asn Pro Ala Asn Asp Leu Cys Tyr Pro
                   85                  90                  95
   Gly Asn Phe Asn Asp Tyr Glu Glu Leu Lys His Leu Leu Ser Arg Ile
               100                 105                 110
   Asn His Phe Glu Lys Ile Gln Ile Ile Pro Lys Asn Ser Trp Ser Asp
           115                 120                 125
   His Glu Ala Ser Gly Val Ser Ser Ala Cys Pro Tyr Gln Gly Arg Ser
       130                 135                 140
   Ser Phe Phe Arg Asn Val Val Trp Leu Thr Lys Lys Asp Asn Ala Tyr
   145                 150                 155                 160
   Pro Thr Ile Lys Arg Ser Tyr Asn Asn Thr Asn Gln Glu Asp Leu Leu
                   165                 170                 175
   Val Leu Trp Gly Ile His His Pro Asn Asp Ala Ala Glu Gln Thr Arg
               180                 185                 190
   Leu Tyr Gln Asn Pro Thr Thr Tyr Ile Ser Val Gly Thr Ser Thr Leu
           195                 200                 205
   Asn Gln Arg Leu Val Pro Lys Ile Ala Thr Arg Ser Lys Val Asn Gly
       210                 215                 220
   Gln Ser Gly Arg Met Glu Phe Phe Trp Thr Ile Leu Lys Ser Asn Asp
   225                 230                 235                 240
   Ala Ile Asn Phe Glu Ser Asn Gly Asn Phe Ile Ala Pro Glu Asn Ala
                   245                 250                 255
   Tyr Lys Ile Val Lys Lys Gly Asp Ser Thr Ile Met Lys Ser Glu Leu
               260                 265                 270
   Glu Tyr Gly Asn Cys Asn Thr Lys Cys Gln Thr Pro Ile Gly Ala Ile
           275                 280                 285
   Asn Ser Ser Met Pro Phe His Asn Ile His Pro Leu Thr Ile Gly Glu
       290                 295                 300
   Cys Pro Lys Tyr Val Lys Ser Asn Arg Leu Val Leu Ala Thr Gly Leu
   305                 310                 315                 320
   Arg Asn Ser Pro Gln Gly Glu Arg Arg Arg Lys Lys Arg Gly Leu Phe
                   325                 330                 335
   Gly Ala Ile Ala Gly Phe Ile Glu Gly Gly Trp Gln Gly Met Val Asp
               340                 345                 350
   Gly Trp Tyr Gly Tyr His His Ser Asn Glu Gln Gly Ser Gly Tyr Ala
           355                 360                 365
   Ala Asp Lys Glu Ser Thr Gln Lys Ala Ile Asp Gly Val Thr Asn Lys
       370                 375                 380
   Val Asn Ser Ile Ile Asp Lys Met Asn Thr Gln Phe Glu Ala Val Gly
   385                 390                 395                 400
   Arg Glu Phe Asn Asn Leu Glu Arg Arg Ile Glu Asn Leu Asn Lys Lys
                   405                 410                 415
   Met Glu Asp Gly Phe Leu Asp Val Trp Thr Tyr Asn Ala Glu Leu Leu
               420                 425                 430
   Val Leu Met Glu Asn Glu Arg Thr Leu Asp Phe His Asp Ser Asn Val
           435                 440                 445
   Lys Asn Leu Tyr Asp Lys Val Arg Leu Gln Leu Arg Asp Asn Ala Lys
       450                 455                 460
   Glu Leu Gly Asn Gly Cys Phe Glu Phe Tyr His Arg Cys Asp Asn Glu
   465                 470                 475                 480
   Cys Met Glu Ser Val Arg Asn Gly Thr Tyr Asp Tyr Pro Gln Tyr Ser
                   485                 490                 495
   Glu Glu Ala Arg Leu Lys Arg Glu Glu Ile Ser Gly Val Lys Leu Glu
               500                 505                 510
   Ser Ile Gly Thr Tyr Gln Ile Leu Ser Ile Tyr Ser Thr Val Ala Ser
           515                 520                 525
   Ser Leu Ala Leu Ala Ile Xaa
       530                 535
<210> 13
   <211> 568
   <212> PRT
   <213> H5N1
<400> 13
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Gly Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Pro Asn Val Ser Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Gln Ile Ile Pro Lys Ser Ser Trp Pro Asp His Glu Ala Ser
       130                 135                 140
   Leu Gly Val Ser Ser Ala Cys Pro Tyr Gln Gly Gly Pro Ser Phe Tyr
   145                 150                 155                 160
   Arg Asn Val Val Trp Leu Ile Lys Lys Asn Asp Thr Tyr Pro Thr Ile
                   165                 170                 175
   Lys Glu Ser Tyr His Asn Thr Asn Gln Glu Asp Leu Leu Val Leu Trp
               180                 185                 190
   Gly Ile His His Pro Asn Asn Glu Glu Glu Gln Lys Arg Ile Tyr Lys
           195                 200                 205
   Asn Pro Thr Thr Tyr Val Ser Val Gly Thr Ser Thr Leu Asn Gln Arg
       210                 215                 220
   Leu Val Pro Lys Ile Ala Thr Arg Ser Lys Val Asn Gly Gln Ser Gly
   225                 230                 235                 240
   Arg Val Glu Phe Phe Trp Thr Ile Leu Lys Ser Asn Asp Thr Ile Asn
                   245                 250                 255
   Phe Glu Ser Asn Gly Asn Phe Ile Ala Pro Glu Asn Ala Tyr Lys Ile
               260                 265                 270
   Val Lys Lys Gly Asp Ser Thr Ile Met Lys Ser Glu Leu Glu Tyr Gly
           275                 280                 285
   Asn Cys Ser Thr Lys Cys Gln Thr Pro Ile Gly Ala Ile Asn Thr Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Gly Glu Gly Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Lys Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Arg Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Arg Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Met Val Ala Gly Leu Phe Leu Trp Met Cys Ser Asn Gly
   545                 550                 555                 560
   Ser Leu Gln Cys Arg Ile Cys Ile
                   565
<210> 14
   <211> 568
   <212> PRT
   <213> H5N1
<220>
   <221> misc_feature
   <222> (375)..(375)
   <223> Xaa can be any naturally occurring amino acid
<400> 14
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Pro Asn Val Ser Glu Trp Ser Tyr Ile Val
                   85                  90                  95
   Glu Lys Thr Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Lys Ile Ile Pro Lys Ser Ser Trp Pro Asp His Glu Ala Ser
       130                 135                 140
   Leu Gly Val Ser Ser Ala Cys Pro Tyr Gln Gly Glu Pro Ser Phe Tyr
   145                 150                 155                 160
   Arg Asn Val Val Trp Leu Ile Lys Lys Asn Asn Thr Tyr Pro Thr Ile
                   165                 170                 175
   Lys Glu Ser Tyr His Asn Thr Asn Gln Glu Asp Leu Leu Val Leu Trp
               180                 185                 190
   Gly Ile His His Pro Asn Asp Glu Glu Glu Gln Thr Arg Ile Tyr Lys
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Ser Lys Val Asn Gly Gln Ser Gly
   225                 230                 235                 240
   Arg Val Glu Phe Phe Trp Thr Ile Leu Lys Ser Asn Asp Thr Ile Asn
                   245                 250                 255
   Phe Glu Ser Asn Gly Asn Phe Ile Ala Pro Glu Asn Ala Tyr Lys Ile
               260                 265                 270
   Val Lys Lys Gly Asp Ser Thr Ile Met Lys Ser Glu Leu Glu Tyr Gly
   275 280 285
   Asn Cys Ser Thr Lys Cys Gln Thr Pro Val Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Gly Glu Gly Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Xaa Gln Gly Ser Gly Tyr Ala Ala Asp Arg
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Lys Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Arg Cys Asp Asn Glu Cys Ile Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Ala Ser Ser Leu Ala
   530 535 540
   Leu Ala Ile Ile Val Ala Gly Leu Phe Leu Trp Met Cys Ser Asn Gly
   545 550 555 560
   Ser Leu Gln Cys Arg Ile Cys Ile
   565
<210> 15
   <211> 568
   <212> PRT
   <213> H5N1
<400> 15
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Pro Asn Val Ser Glu Trp Ser Tyr Ile Val
   85 90 95
   Glu Lys Ile Asn Pro Ala Asn Asp Leu Cys Tyr Pro Gly Asn Phe Asn
   100 105 110
   Asn Tyr Glu Glu Leu Lys His Leu Leu Ser Arg Ile Asn Arg Phe Glu
   115 120 125
   Lys Ile Lys Ile Ile Pro Lys Ser Ser Trp Pro Asp His Glu Ala Ser
   130 135 140
   Leu Gly Val Ser Ser Ala Cys Pro Tyr Gln Arg Gly Pro Ser Phe Tyr
   145 150 155 160
   Arg Asn Val Val Trp Leu Ile Lys Lys Asn Asn Thr Tyr Pro Thr Ile
   165 170 175
   Lys Lys Ser Tyr His Asn Thr Asn Gln Glu Asp Leu Leu Val Leu Trp
   180 185 190
   Gly Ile His His Pro Asn Asp Glu Glu Glu Gln Thr Arg Ile Tyr Lys
   195 200 205
   Asn Pro Thr Thr Tyr Ile Ser Val Gly Thr Ser Thr Leu Asn Gln Arg
   210 215 220
   Leu Val Pro Lys Ile Ala Thr Arg Ser Lys Val Asn Gly Gln Ser Gly
   225 230 235 240
   Arg Val Glu Phe Phe Trp Thr Ile Leu Lys Ser Asn Asp Thr Ile Asn
   245 250 255
   Phe Glu Ser Asn Gly Asn Phe Ile Ala Pro Glu Asn Ala Tyr Lys Ile
   260 265 270
   Val Lys Lys Gly Asp Ser Thr Ile Met Lys Ser Glu Leu Glu Tyr Gly
   275 280 285
   Asn Cys Asn Thr Lys Cys Gln Thr Pro Ile Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Gly Glu Gly Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Lys Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Arg Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Ala Ser Ser Leu Ala
   530 535 540
   Leu Ala Ile Met Met Ala Gly Leu Phe Leu Trp Met Cys Ser Asn Gly
   545 550 555 560
   Ser Leu Gln Cys Arg Ile Cys Ile
   565
<210> 16
   <211> 568
   <212> PRT
   <213> H5N1
<400> 16
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Pro Asn Val Ser Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Lys Ile Ile Pro Lys Ser Ser Trp Pro Asp His Glu Ala Ser
       130                 135                 140
   Leu Gly Val Ser Ser Ala Cys Pro Tyr Gln Gly Gly Pro Ser Phe Tyr
   145                 150                 155                 160
   Arg Asn Val Val Trp Leu Ile Lys Lys Asn Asn Thr Tyr Pro Thr Ile
                   165                 170                 175
   Lys Glu Ser Tyr His Asn Thr Asn Gln Glu Asp Leu Leu Val Leu Trp
               180                 185                 190
   Gly Ile His His Pro Asn Asp Glu Glu Glu Gln Thr Arg Ile Tyr Lys
           195                 200                 205
   Asn Pro Thr Thr Tyr Ile Ser Ile Gly Thr Ser Thr Leu Asn Gln Arg
       210                 215                 220
   Leu Val Pro Lys Ile Ala Thr Arg Ser Lys Val Asn Gly Gln Arg Gly
   225                 230                 235                 240
   Arg Val Glu Phe Phe Trp Thr Ile Leu Lys Ser Asn Asp Thr Ile Asn
                   245                 250                 255
   Phe Glu Ser Asn Gly Asn Phe Ile Ala Pro Glu Asn Ala Tyr Lys Ile
               260                 265                 270
   Val Lys Lys Gly Asp Ser Thr Ile Met Lys Ser Glu Leu Glu Tyr Gly
           275                 280                 285
   Asn Cys Asn Thr Lys Cys Gln Thr Pro Ile Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
   325 330 335
   Pro Gln Gly Glu Gly Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Lys Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Arg Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Ala Ser Ser Leu Ala
   530 535 540
   Leu Ala Ile Met Met Ala Gly Leu Phe Leu Trp Met Cys Ser Asn Gly
   545 550 555 560
   Ser Leu Gln Cys Arg Ile Cys Ile
   565
<210> 17
   <211> 568
   <212> PRT
   <213> H5N1
<400> 17
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Pro Asn Val Ser Glu Trp Ser Tyr Ile Val
                   85                  90                  95
   Glu Lys Ile Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Gln Ile Ile Pro Lys Ser Ser Trp Pro Asp His Glu Ala Ser
       130                 135                 140
   Leu Gly Val Ser Ser Ala Cys Pro Tyr Gln Gly Glu Pro Ser Phe Tyr
   145                 150                 155                 160
   Arg Asn Val Val Trp Leu Ile Lys Lys Asn Asn Thr Tyr Pro Thr Ile
                   165                 170                 175
   Lys Glu Ser Tyr His Asn Thr Asn Gln Glu Asp Leu Leu Val Leu Trp
               180                 185                 190
   Gly Ile His His Pro Asn Asp Glu Glu Glu Gln Lys Arg Ile Tyr Lys
           195                 200                 205
   Asn Pro Thr Thr Tyr Ile Ser Val Gly Thr Ser Thr Leu Asn Gln Arg
       210                 215                 220
   Leu Val Pro Lys Ile Ala Thr Arg Pro Lys Val Asn Gly Gln Ser Gly
   225 230 235 240
   Arg Val Glu Phe Phe Trp Thr Ile Leu Lys Ser Asn Asp Thr Ile Asn
   245 250 255
   Phe Glu Ser Asn Gly Asn Phe Ile Ala Pro Lys Asn Ala Tyr Lys Ile
   260 265 270
   Val Lys Lys Gly Ser Ser Thr Ile Met Lys Ser Glu Leu Glu Tyr Gly
   275 280 285
   Asn Cys Ser Thr Lys Cys Gln Thr Pro Ile Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Gly Glu Gly Arg Arg Lys Lys Arg Gly Leu Phe Gly Ala Ile
   340 345 350
   Ala Gly Phe Ile Glu Gly Gly Trp Gln Gly Met Val Asp Gly Trp Tyr
   355 360 365
   Gly Tyr His His Ser Asn Glu Gln Gly Thr Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Lys Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Arg Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Lys Cys Asp Asn Glu Cys Met Glu
   485 490 495
   Ser Val Arg Asn Gly Thr Tyr Asp Tyr Pro Gln Tyr Ser Lys Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Thr Ser Ser Leu Ala
   530 535 540
   Leu Ala Ile Met Val Ala Gly Leu Ser Leu Trp Met Cys Ser Asn Gly
   545 550 555 560
   Ser Leu Gln Cys Arg Ile Cys Ile
   565
<210> 18
   <211> 556
   <212> PRT
   <213> H5N1
<220>
   <221> misc_feature
   <222> (556)..(556)
   <223> Xaa can be any naturally occurring amino acid
<400> 18
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Pro Asn Val Ser Glu Trp Ser Tyr Ile Val
   85 90 95
   Glu Lys Thr Asn Pro Ala Asn Asp Leu Cys Tyr Pro Gly Asn Phe Asn
               100                 105                 110
   Asn Tyr Glu Glu Leu Lys His Leu Leu Ser Arg Ile Asn Arg Phe Glu
           115                 120                 125
   Lys Ile Lys Ile Ile Pro Lys Ser Ser Trp Pro Asp His Glu Ala Ser
       130                 135                 140
   Leu Gly Val Ser Ser Ala Cys Pro Tyr Gln Gly Arg Pro Ser Phe Tyr
   145                 150                 155                 160
   Arg Asn Val Val Trp Leu Ile Lys Lys Asn Asn Thr Tyr Pro Thr Ile
                   165                 170                 175
   Lys Glu Ser Tyr His Asn Thr Asn Gln Glu Asp Leu Leu Val Leu Trp
               180                 185                 190
   Gly Ile His His Pro Asn Asp Glu Glu Glu Gln Thr Arg Ile Tyr Lys
           195                 200                 205
   Asn Pro Thr Thr Tyr Ile Ser Ile Gly Thr Ser Thr Leu Asn Gln Arg
       210                 215                 220
   Leu Ile Pro Lys Ile Ala Thr Arg Ser Lys Val Asn Gly Gln Ser Gly
   225                 230                 235                 240
   Arg Val Glu Phe Phe Trp Thr Ile Leu Lys Ser Asn Asp Thr Ile Asn
                   245                 250                 255
   Phe Glu Ser Asn Gly Asn Phe Ile Ala Pro Glu Asn Ala Tyr Lys Ile
               260                 265                 270
   Val Lys Lys Gly Asp Ser Thr Ile Met Lys Ser Glu Leu Glu Tyr Gly
           275                 280                 285
   Asn Cys Asn Thr Lys Cys Gln Thr Pro Ile Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Gly Glu Gly Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
   355 360 365
   Gly Tyr His His Ser Asn Glu Gln Gly Ser Gly Tyr Ala Ala Asp Lys
   370 375 380
   Glu Ser Thr Gln Lys Ala Ile Asp Gly Val Thr Asn Lys Val Asn Ser
   385 390 395 400
   Ile Ile Asp Lys Met Asn Thr Gln Phe Glu Ala Val Gly Arg Glu Phe
   405 410 415
   Asn Asn Leu Glu Lys Arg Ile Glu Asn Leu Asn Lys Lys Met Glu Asp
   420 425 430
   Gly Phe Leu Asp Val Trp Thr Tyr Asn Ala Glu Leu Leu Val Leu Met
   435 440 445
   Glu Asn Glu Arg Thr Leu Asp Phe His Asp Ser Asn Val Lys Asn Leu
   450 455 460
   Tyr Asp Lys Val Arg Leu Gln Leu Arg Asp Asn Ala Lys Glu Leu Gly
   465 470 475 480
   Asn Gly Cys Phe Glu Phe Tyr His Arg Cys Asp Asn Glu Cys Met Glu
   485 490 495
   Ser Val Arg Asn Gly Thr Tyr Asp Tyr Pro Gln Tyr Ser Glu Glu Ala
   500 505 510
   Arg Leu Lys Arg Glu Glu Ile Ser Gly Val Lys Leu Glu Ser Ile Gly
   515 520 525
   Thr Tyr Gln Ile Leu Ser Ile Tyr Ser Thr Val Ala Ser Ser Leu Ala
   530 535 540
   Leu Ala Ile Met Met Ala Gly Leu Phe Leu Trp Xaa
   545 550 555
<210> 19
   <211> 554
   <212> PRT
   <213> H5N1
<220>
   <221> misc_feature
   <222> (1)..(1)
   <223> Xaa can be any naturally occurring amino acid
<220>
   <221> misc_feature
   <222> (554)..(554)
   <223> Xaa can be any naturally occurring amino acid
<400> 19
   Xaa Leu Leu Ala Ile Val Ser Leu Val Lys Ser Asp Gln Ile Cys Ile
   1               5                   10                  15
   Gly Tyr His Ala Asn Asn Ser Thr Glu Gln Val Asp Thr Ile Met Glu
               20                  25                  30
   Lys Asn Val Thr Val Thr His Ala Gln Asp Ile Leu Glu Lys Thr His
           35                  40                  45
   Asn Gly Lys Leu Cys Asp Leu Asp Gly Val Lys Pro Leu Ile Leu Arg
       50                  55                  60
   Asp Cys Ser Val Ala Gly Trp Leu Leu Gly Asn Pro Met Cys Asp Glu
   65                  70                  75                  80
   Phe Pro Asn Val Ser Glu Trp Ser Tyr Ile Val Glu Lys Ile Asn Pro
                   85                  90                  95
   Ala Asn Asp Leu Cys Tyr Pro Gly Asn Phe Asn Asn Tyr Glu Glu Leu
               100                 105                 110
   Lys His Leu Leu Ser Arg Ile Asn Arg Phe Glu Lys Ile Gln Ile Ile
           115                 120                 125
   Pro Lys Ser Ser Trp Pro Asp His Glu Ala Ser Leu Gly Val Ser Ser
       130                 135                 140
   Ala Cys Pro Tyr Gln Gly Glu Pro Ser Phe Tyr Arg Asn Val Val Trp
   145                 150                 155                 160
   Leu Ile Lys Lys Asn Asn Thr Tyr Pro Thr Ile Lys Glu Asn Tyr His
                   165                 170                 175
   Asn Thr Asn Gln Glu Asp Leu Leu Val Leu Trp Gly Ile His His Pro
               180                 185                 190
   Asn Asp Glu Glu Glu Gln Lys Arg Ile Tyr Lys Asn Pro Thr Thr Tyr
           195                 200                 205
   Ile Ser Val Gly Thr Ser Thr Leu Asn Gln Arg Leu Val Pro Lys Ile
       210                 215                 220
   Ala Thr Arg Pro Lys Val Asn Gly Gln Ser Gly Arg Val Glu Phe Phe
   225                 230                 235                 240
   Trp Thr Ile Leu Lys Ser Asn Asp Thr Ile Asn Phe Glu Ser Asn Gly
                   245                 250                 255
   Asn Phe Ile Ala Pro Lys Asn Ala Tyr Lys Ile Val Lys Lys Gly Ser
               260                 265                 270
   Ser Thr Ile Met Lys Ser Glu Leu Glu Tyr Gly Asn Cys Ser Thr Lys
           275                 280                 285
   Cys Gln Thr Pro Ile Gly Ala Ile Asn Ser Ser Met Pro Phe His Asn
       290                 295                 300
   Ile His Pro Leu Thr Ile Gly Glu Cys Pro Lys Tyr Val Lys Ser Asn
   305                 310                 315                 320
   Arg Leu Val Leu Ala Thr Gly Leu Arg Asn Ser Pro Gln Gly Glu Gly
                   325                 330                 335
   Arg Arg Lys Lys Arg Gly Leu Phe Gly Ala Ile Ala Gly Phe Ile Glu
               340                 345                 350
   Gly Gly Trp Gln Gly Met Val Asp Gly Trp Tyr Gly Tyr His His Ser
           355                 360                 365
   Asn Glu Gln Gly Thr Gly Tyr Ala Ala Asp Lys Glu Ser Thr Gln Lys
       370                 375                 380
   Ala Ile Asp Gly Val Thr Asn Lys Val Asn Ser Ile Ile Asp Lys Met
   385                 390                 395                 400
   Asn Thr Gln Phe Glu Ala Val Gly Arg Glu Phe Asn Asn Leu Glu Lys
                   405                 410                 415
   Arg Ile Glu Asn Leu Asn Lys Lys Met Glu Asp Gly Phe Leu Asp Val
               420                 425                 430
   Trp Thr Tyr Asn Ala Glu Leu Leu Val Leu Met Glu Asn Glu Arg Thr
           435                 440                 445
   Leu Asp Phe His Asp Ser Asn Val Arg Asn Leu Tyr Asp Lys Val Arg
       450                 455                 460
   Leu Gln Leu Arg Asp Asn Ala Lys Glu Leu Gly Asn Gly Cys Phe Glu
   465                 470                 475                 480
   Phe Tyr His Lys Cys Asp Asn Glu Cys Met Glu Ser Val Arg Asn Gly
                   485                 490                 495
   Thr Tyr Asp Tyr Pro Gln Tyr Ser Lys Glu Ala Arg Leu Lys Arg Glu
               500                 505                 510
   Glu Ile Ser Gly Val Lys Leu Glu Ser Ile Gly Thr Tyr Gln Ile Leu
           515                 520                 525
   Ser Ile Tyr Ser Thr Val Ala Ser Ser Leu Ala Leu Ala Ile Met Val
       530                 535                 540
   Ala Gly Leu Ser Leu Trp Met Cys Ser Xaa
   545                 550
<210> 20
   <211> 564
   <212> PRT
   <213> H5N1
<400> 20
   Val Leu Leu Leu Ala Ile Val Ser Leu Val Lys Ser Asp Gln Ile Cys
   1               5                   10                  15
   Ile Gly Tyr His Ala Asn Asn Ser Thr Glu Gln Val Asp Thr Ile Met
               20                  25                  30
   Glu Lys Asn Val Thr Val Thr His Ala Gln Asp Ile Leu Glu Lys Thr
           35                  40                  45
   His Asn Gly Lys Leu Cys Asp Leu Asp Gly Val Lys Pro Leu Ile Leu
       50                  55                  60
   Arg Asp Cys Ser Val Ala Gly Trp Leu Leu Gly Asn Pro Met Cys Asp
   65                  70                  75                  80
   Glu Phe Pro Asn Val Ser Glu Trp Ser Tyr Ile Val Glu Lys Ile Asn
                   85                  90                  95
   Pro Ala Asn Asp Leu Cys Tyr Pro Gly Asn Phe Asn Asn Tyr Glu Glu
               100                 105                 110
   Leu Lys His Leu Leu Ser Arg Ile Asn Arg Phe Glu Lys Ile Gln Ile
           115                 120                 125
   Ile Pro Lys Ser Ser Trp Pro Asp His Glu Ala Ser Leu Gly Val Ser
       130                 135                 140
   Ser Ala Cys Pro Tyr Gln Gly Glu Pro Ser Phe Tyr Arg Asn Val Val
   145 150 155 160
   Trp Leu Ile Lys Lys Asn Asn Thr Tyr Pro Thr Ile Lys Glu Asn Tyr
   165 170 175
   His Asn Thr Asn Gln Glu Asp Leu Leu Val Leu Trp Gly Ile His His
   180 185 190
   Pro Asn Asp Glu Glu Glu Gln Lys Arg Ile Tyr Lys Asn Pro Thr Thr
   195 200 205
   Tyr Ile Ser Val Gly Thr Ser Thr Leu Asn Gln Arg Leu Val Pro Lys
   210 215 220
   Ile Ala Thr Arg Pro Lys Val Asn Gly Gln Ser Gly Arg Val Glu Phe
   225 230 235 240
   Phe Trp Thr Ile Leu Lys Ser Asn Asp Thr Ile Asn Phe Glu Ser Asn
   245 250 255
   Gly Asn Phe Ile Ala Pro Lys Asn Ala Tyr Lys Ile Val Lys Lys Gly
   260 265 270
   Ser Ser Thr Ile Met Lys Ser Glu Leu Glu Tyr Gly Asn Cys Ser Thr
   275 280 285
   Lys Cys Gln Thr Pro Ile Gly Ala Ile Asn Ser Ser Met Pro Phe His
   290 295 300
   Asn Ile His Pro Leu Thr Ile Gly Glu Cys Pro Lys Tyr Val Lys Ser
   305 310 315 320
   Asn Arg Leu Val Leu Ala Thr Gly Leu Arg Asn Ser Pro Gln Gly Glu
   325 330 335
   Gly Arg Arg Lys Lys Arg Gly Leu Phe Gly Ala Ile Ala Gly Phe Ile
   340 345 350
   Glu Gly Gly Trp Gln Gly Met Val Asp Gly Trp Tyr Gly Tyr His His
   355 360 365
   Ser Asn Glu Gln Gly Thr Gly Tyr Ala Ala Asp Lys Glu Ser Thr Gln
   370 375 380
   Lys Ala Ile Asp Gly Val Thr Asn Lys Val Asn Ser Ile Ile Asp Lys
   385 390 395 400
   Met Asn Thr Gln Phe Glu Ala Val Gly Arg Glu Phe Asn Asn Leu Glu
   405 410 415
   Lys Arg Ile Glu Asn Leu Asn Lys Lys Met Glu Asp Gly Phe Leu Asp
   420 425 430
   Val Trp Thr Tyr Asn Ala Glu Leu Leu Val Leu Met Glu Asn Glu Arg
   435 440 445
   Thr Leu Asp Phe His Asp Ser Asn Val Arg Asn Leu Tyr Asp Lys Val
   450 455 460
   Arg Leu Gln Leu Arg Asp Asn Ala Lys Glu Leu Gly Asn Gly Cys Phe
   465 470 475 480
   Glu Phe Tyr His Lys Cys Asp Asn Glu Cys Met Glu Ser Val Arg Asn
   485 490 495
   Gly Thr Tyr Asp Tyr Pro Gln Tyr Ser Lys Glu Ala Arg Leu Lys Arg
   500 505 510
   Glu Glu Ile Ser Gly Val Lys Leu Glu Ser Ile Gly Thr Tyr Gln Ile
   515 520 525
   Leu Ser Ile Tyr Ser Thr Val Ala Ser Ser Leu Ala Leu Ala Ile Met
   530 535 540
   Val Ala Gly Leu Ser Leu Trp Met Cys Ser Asn Gly Ser Leu Gln Cys
   545 550 555 560
   Arg Ile Cys Ile
<210> 21
   <211> 553
   <212> PRT
   <213> H5N1
<220>
   <221> misc_feature
   <222> (1)..(1)
   <223> Xaa can be any naturally occurring amino acid
<400> 21
   Xaa Val Leu Leu Leu Ala Ile Ile Ser Leu Val Lys Ser Asp Gln Ile
   1 5 10 15
   Cys Ile Gly Tyr His Ala Asn Asn Ser Thr Glu Gln Val Asp Thr Ile
               20                  25                  30
   Met Glu Lys Asn Val Thr Val Thr His Ala Gln Asp Ile Leu Glu Lys
           35                  40                  45
   Thr His Asn Gly Lys Leu Cys Asp Leu Asp Gly Val Lys Pro Leu Ile
       50                  55                  60
   Leu Arg Gly Cys Ser Val Ala Gly Trp Leu Leu Gly Asn Pro Met Cys
   65                  70                  75                  80
   Asp Glu Phe Pro Asn Val Ser Glu Trp Ser Tyr Ile Val Glu Lys Ile
                   85                  90                  95
   Asn Pro Ala Asn Asp Leu Cys Tyr Pro Gly Asn Phe Asn Asn Tyr Glu
               100                 105                 110
   Glu Leu Lys His Leu Leu Ser Arg Ile Asn Arg Phe Glu Lys Ile Gln
           115                 120                 125
   Ile Ile Pro Lys Ser Ser Trp Pro Asp His Glu Ala Ser Leu Gly Val
       130                 135                 140
   Ser Ser Ala Cys Pro Tyr Gln Gly Glu Pro Ser Phe Tyr Arg Asn Val
   145                 150                 155                 160
   Val Trp Leu Ile Lys Lys Asn Asn Thr Tyr Pro Thr Ile Lys Glu Ser
                   165                 170                 175
   Tyr His Asn Thr Asn Gln Glu Asp Leu Leu Val Leu Trp Gly Ile His
               180                 185                 190
   His Pro Asn Asp Glu Glu Glu Gln Lys Arg Ile Tyr Lys Asn Pro Thr
           195                 200                 205
   Thr Tyr Ile Ser Val Gly Thr Ser Thr Leu Asn Gln Arg Leu Val Pro
       210                 215                 220
   Lys Ile Ala Thr Arg Pro Lys Val Asn Gly Gln Ser Gly Arg Val Glu
   225                 230                 235                 240
   Phe Phe Trp Thr Ile Leu Lys Ser Asn Asp Thr Ile Asn Phe Glu Ser
                   245                 250                 255
   Asn Gly Asn Phe Ile Ala Pro Lys Asn Ala Tyr Lys Ile Val Lys Lys
               260                 265                 270
   Gly Ser Ser Thr Ile Met Lys Ser Glu Leu Glu Tyr Gly Asn Cys Ser
   275 280 285
   Thr Lys Cys Gln Thr Pro Ile Gly Ala Ile Asn Ser Ser Met Pro Phe
   290 295 300
   His Asn Ile His Pro Leu Thr Ile Gly Glu Cys Pro Lys Tyr Val Lys
   305 310 315 320
   Ser Asn Arg Leu Val Leu Ala Thr Gly Leu Arg Asn Ser Pro Gln Gly
   325 330 335
   Glu Gly Arg Arg Lys Lys Arg Gly Leu Phe Gly Ala Ile Ala Gly Phe
   340 345 350
   Ile Glu Gly Gly Trp Gln Gly Met Val Asp Gly Trp Tyr Gly Tyr His
   355 360 365
   His Ser Asn Glu Gln Gly Thr Gly Tyr Ala Ala Asp Lys Glu Ser Thr
   370 375 380
   Gln Lys Ala Ile Asp Gly Val Thr Asn Lys Val Asn Ser Ile Ile Asp
   385 390 395 400
   Lys Met Asn Thr Gln Phe Glu Ala Val Gly Arg Glu Phe Asn Asn Leu
   405 410 415
   Glu Lys Arg Ile Glu Asn Leu Asn Lys Lys Met Glu Asp Gly Phe Leu
   420 425 430
   Asp Val Trp Thr Tyr Asn Ala Glu Leu Leu Val Leu Met Glu Asn Glu
   435 440 445
   Arg Thr Leu Asp Phe His Asp Ser Asn Val Arg Asn Leu Tyr Asp Lys
   450 455 460
   Val Arg Leu Gln Leu Arg Asp Asn Ala Lys Glu Leu Gly Asn Gly Cys
   465 470 475 480
   Phe Glu Phe Tyr His Lys Cys Asp Asn Glu Cys Met Glu Ser Val Arg
   485 490 495
   Asn Gly Thr Tyr Asp Tyr Pro Gln Tyr Ser Lys Glu Ala Arg Leu Lys
   500 505 510
   Arg Glu Glu Ile Ser Gly Val Lys Leu Glu Ser Ile Gly Thr Tyr Gln
   515 520 525
   Ile Leu Ser Ile Tyr Ser Thr Val Ala Ser Ser Leu Ala Leu Ala Ile
   530 535 540
   Met Val Ala Gly Leu Ser Leu Trp Met
   545 550
<210> 22
   <211> 558
   <212> PRT
   <213> H5N1
<220>
   <221> misc_feature
   <222> (558)..(558)
   <223> Xaa can be any naturally occurring amino acid
<400> 22
   Ile Val Leu Leu Leu Ala Ile Val Ser Leu Val Lys Ser Asp Gln Ile
   1 5 10 15
   Cys Ile Gly Tyr His Ala Asn Asn Ser Thr Glu Gln Val Asp Thr Ile
   20 25 30
   Met Glu Lys Asn Val Thr Val Thr His Ala Gln Asp Ile Leu Glu Lys
   35 40 45
   Thr His Asn Gly Lys Leu Cys Asp Leu Asp Gly Val Lys Pro Leu Ile
   50 55 60
   Leu Arg Asp Cys Ser Val Ala Gly Trp Leu Leu Gly Asn Pro Met Cys
   65 70 75 80
   Asp Glu Phe Pro Asn Val Ser Glu Trp Ser Tyr Ile Val Glu Lys Ile
   85 90 95
   Asn Pro Ala Asn Asp Leu Cys Tyr Pro Gly Asn Phe Asn Asn Tyr Glu
   100 105 110
   Glu Leu Lys His Leu Leu Ser Arg Ile Asn Arg Phe Glu Lys Ile Lys
   115 120 125
   Ile Ile Pro Lys Ser Ser Trp Pro Asp His Glu Ala Ser Leu Gly Val
   130 135 140
   Ser Ser Ala Cys Pro Tyr Gln Gly Gly Pro Ser Phe Tyr Arg Asn Val
   145 150 155 160
   Val Trp Leu Ile Lys Lys Asn Asn Thr Tyr Pro Thr Ile Lys Lys Ser
   165 170 175
   Tyr His Asn Thr Asn Gln Glu Asp Leu Leu Val Leu Trp Gly Ile His
   180 185 190
   His Pro Asn Asp Glu Glu Glu Gln Thr Arg Ile Tyr Lys Asn Pro Thr
   195 200 205
   Thr Tyr Ile Ser Val Gly Thr Ser Thr Leu Asn Gln Arg Leu Val Pro
   210 215 220
   Lys Ile Ala Thr Arg Ser Lys Val Asn Gly Gln Ser Gly Arg Val Glu
   225 230 235 240
   Phe Phe Trp Thr Ile Leu Lys Ser Asn Asp Thr Ile Asn Phe Glu Ser
   245 250 255
   Asn Gly Asn Phe Ile Ala Pro Glu Asn Ala Tyr Lys Ile Val Lys Lys
   260 265 270
   Gly Asp Ser Thr Ile Met Lys Ser Glu Leu Glu His Gly Asn Cys Asn
   275 280 285
   Thr Lys Cys Gln Thr Pro Ile Gly Ala Ile Asn Ser Ser Met Pro Phe
   290 295 300
   His Asn Ile His Pro Leu Thr Ile Gly Glu Cys Pro Lys Tyr Val Lys
   305 310 315 320
   Ser Asn Arg Leu Val Leu Ala Thr Gly Leu Arg Asn Ser Pro Gln Gly
   325 330 335
   Glu Gly Arg Arg Lys Lys Arg Gly Leu Phe Gly Ala Ile Ala Gly Phe
   340 345 350
   Ile Glu Gly Gly Trp Gln Gly Met Val Asp Gly Trp Tyr Gly Tyr His
   355 360 365
   His Ser Asn Glu Gln Gly Ser Gly Tyr Ala Ala Asp Lys Glu Ser Thr
   370 375 380
   Gln Lys Ala Ile Asp Gly Val Thr Asn Lys Val Asn Ser Ile Ile Asp
   385 390 395 400
   Lys Met Asn Thr Gln Phe Glu Ala Val Gly Arg Glu Phe Asn Asn Leu
   405 410 415
   Glu Lys Arg Ile Glu Asn Leu Asn Lys Lys Met Glu Asp Gly Phe Leu
                   420                 425                 430
   Asp Val Trp Thr Tyr Asn Ala Glu Leu Leu Val Leu Met Glu Asn Glu
           435                 440                 445
   Arg Thr Leu Asp Phe His Asp Ser Asn Val Lys Asn Leu Tyr Asp Lys
       450                 455                 460
   Val Arg Leu Gln Leu Arg Asp Asn Ala Lys Glu Leu Gly Asn Gly Cys
   465                 470                 475                 480
   Phe Glu Phe Tyr His Arg Cys Asp Asn Glu Cys Met Glu Ser Val Arg
                   485                 490                 495
   Asn Gly Thr Tyr Asp Tyr Pro Gln Tyr Ser Glu Glu Ala Arg Leu Asn
               500                 505                 510
   Arg Glu Glu Ile Ser Gly Val Lys Leu Glu Ser Ile Gly Thr Tyr Gln
           515                 520                 525
   Ile Leu Ser Ile Tyr Ser Thr Val Ala Ser Ser Leu Ala Leu Ala Ile
       530                 535                 540
   Met Met Ala Gly Leu Phe Leu Trp Met Cys Ser Asn Gly Xaa
   545                 550                 555
<210> 23
   <211> 557
   <212> PRT
   <213> H5N1
<220>
   <221> misc_feature
   <222> (130)..(130)
   <223> Xaa can be any naturally occurring amino acid
<400> 23
   Leu Leu Ala Ile Val Ser Leu Val Lys Ser Asp Gln Ile Cys Ile Gly
   1               5                   10                  15
   Tyr His Ala Asn Asn Ser Thr Glu Gln Val Asp Thr Ile Met Glu Lys
               20                  25                  30
   Asn Val Thr Val Thr His Ala Gln Asp Ile Leu Glu Lys Thr His Asn
           35                  40                  45
   Gly Lys Leu Cys Asp Leu Asp Gly Val Lys Pro Leu Ile Leu Arg Asp
       50                  55                  60
   Cys Ser Val Ala Gly Trp Leu Leu Gly Asn Pro Met Cys Asp Glu Phe
   65                  70                  75                  80
   Pro Asn Val Ser Glu Trp Ser Tyr Ile Val Glu Lys Ile Asn Pro Ala
                   85                  90                  95
   Asn Asp Leu Cys Tyr Pro Gly Asn Phe Asn Asn Tyr Glu Glu Leu Lys
               100                 105                 110
   His Leu Leu Ser Arg Ile Asn Arg Phe Glu Lys Ile Lys Ile Ile Pro
           115                 120                 125
   Lys Xaa Ser Trp Pro Asp His Glu Ala Ser Leu Gly Val Ser Ser Ala
       130                 135                 140
   Cys Pro Tyr Gln Gly Gly Pro Ser Phe Tyr Arg Asn Val Val Trp Leu
   145                 150                 155                 160
   Ile Lys Lys Asn Asn Thr Tyr Pro Thr Ile Lys Glu Ser Tyr His Asn
                   165                 170                 175
   Thr Asn Gln Glu Asp Leu Leu Val Leu Trp Gly Ile His His Pro Asn
               180                 185                 190
   Asp Glu Glu Glu Gln Thr Arg Ile Tyr Lys Asn Pro Thr Thr Tyr Ile
           195                 200                 205
   Ser Ile Gly Thr Ser Thr Leu Asn Gln Arg Leu Val Pro Lys Ile Ala
       210                 215                 220
   Thr Arg Ser Lys Val Asn Gly Gln Ser Gly Arg Val Glu Phe Phe Trp
   225                 230                 235                 240
   Thr Ile Leu Lys Ser Asn Asp Thr Ile Asn Phe Glu Ser Asn Gly Asn
                   245                 250                 255
   Phe Ile Ala Pro Glu Asn Ala Tyr Lys Ile Val Lys Lys Gly Asp Ser
               260                 265                 270
   Thr Ile Met Lys Ser Glu Leu Glu Tyr Gly Asn Cys Asn Thr Lys Cys
           275                 280                 285
   Gln Thr Pro Ile Gly Ala Ile Asn Ser Ser Met Pro Phe His Asn Ile
       290                 295                 300
   His Pro Leu Thr Ile Gly Glu Cys Pro Lys Tyr Val Lys Ser Asn Arg
   305                 310                 315                 320
   Leu Val Leu Ala Thr Gly Leu Arg Asn Ser Pro Gln Gly Glu Gly Arg
                   325                 330                 335
   Arg Lys Lys Arg Gly Leu Phe Gly Ala Ile Ala Gly Phe Ile Glu Gly
               340                 345                 350
   Gly Trp Gln Gly Met Val Asp Gly Trp Tyr Gly Tyr His His Ser Asn
           355                 360                 365
   Glu Gln Gly Ser Gly Tyr Ala Ala Asp Lys Glu Ser Thr Gln Lys Ala
       370                 375                 380
   Ile Asp Gly Val Thr Asn Lys Val Asn Ser Ile Ile Asp Lys Met Asn
   385                 390                 395                 400
   Thr Gln Phe Glu Ala Val Gly Arg Glu Phe Asn Asn Leu Glu Lys Arg
                   405                 410                 415
   Ile Glu Asn Leu Asn Lys Lys Met Glu Asp Gly Phe Leu Asp Val Trp
               420                 425                 430
   Thr Tyr Asn Ala Glu Leu Leu Val Leu Met Glu Asn Glu Arg Thr Leu
           435                 440                 445
   Asp Phe His Asp Ser Asn Val Lys Asn Leu Tyr Asp Lys Val Arg Leu
       450                 455                 460
   Gln Leu Arg Asp Asn Ala Lys Glu Leu Gly Asn Gly Cys Phe Glu Phe
   465                 470                 475                 480
   Tyr His Arg Cys Asp Asn Glu Cys Met Glu Ser Val Arg Asn Gly Thr
                   485                 490                 495
   Tyr Asp Tyr Pro Gln Tyr Ser Glu Glu Ala Arg Leu Lys Arg Glu Glu
               500                 505                 510
   Ile Ser Gly Val Lys Leu Glu Ser Ile Gly Thr Tyr Gln Ile Leu Ser
           515                 520                 525
   Ile Tyr Ser Thr Val Ala Gly Ser Leu Ala Leu Ala Ile Met Met Ala
       530                 535                 540
   Gly Leu Phe Leu Trp Met Cys Ser Asn Gly Ser Leu Gln
   545                 550                 555
<210> 24
   <211> 547
   <212> PRT
   <213> H5N1
<220>
   <221> misc_feature
   <222> (547)..(547)
   <223> Xaa can be any naturally occurring amino acid
<400> 24
   Ile Val Leu Leu Leu Ala Ile Val Ser Leu Val Lys Ser Asp Gln Ile
   1               5                   10                  15
   Cys Ile Gly Tyr His Ala Asn Asn Ser Thr Glu Gln Val Asp Thr Ile
               20                  25                  30
   Met Glu Lys Asn Val Thr Val Thr His Ala Gln Asp Ile Leu Glu Lys
           35                  40                  45
   Thr His Asn Gly Lys Leu Cys Asp Leu Asp Gly Val Lys Pro Leu Ile
       50                  55                  60
   Leu Arg Asp Cys Ser Val Ala Gly Trp Leu Leu Gly Asn Pro Met Cys
   65                  70                  75                  80
   Asp Glu Phe Pro Asn Val Ser Glu Trp Ser Tyr Ile Val Glu Lys Ile
                   85                  90                  95
   Asn Pro Ala Asn Asp Leu Cys Tyr Pro Gly Asn Phe Asn Asn Tyr Glu
               100                 105                 110
   Glu Leu Lys His Leu Leu Ser Arg Ile Asn Arg Phe Glu Lys Ile Lys
           115                 120                 125
   Ile Ile Pro Lys Ser Ser Trp Pro Asp His Glu Ala Ser Leu Gly Val
       130                 135                 140
   Ser Ser Ala Cys Pro Tyr Gln Glu Gly Pro Ser Phe Tyr Arg Asn Val
   145                 150                 155                 160
   Val Trp Leu Ile Lys Lys Asn Asn Thr Tyr Pro Thr Ile Lys Lys Ser
                   165                 170                 175
   Tyr His Asn Thr Asn Gln Glu Asp Leu Leu Val Leu Trp Gly Ile His
               180                 185                 190
   His Pro Asn Asp Glu Glu Glu Gln Thr Arg Ile Tyr Lys Asn Pro Thr
           195                 200                 205
   Thr Tyr Ile Ser Val Gly Thr Ser Thr Leu Asn Gln Arg Leu Val Pro
       210                 215                 220
   Lys Ile Ala Thr Arg Ser Lys Val Asn Gly Gln Ser Gly Arg Val Glu
   225                 230                 235                 240
   Phe Phe Trp Thr Ile Leu Lys Ser Asn Asp Thr Ile Asn Phe Glu Ser
                   245                 250                 255
   Asn Gly Asn Phe Ile Ala Pro Glu Asn Ala Tyr Lys Ile Val Lys Lys
               260                 265                 270
   Gly Asp Ser Thr Ile Met Lys Ser Glu Leu Glu Tyr Gly Asn Cys Asn
           275                 280                 285
   Thr Lys Cys Gln Thr Pro Ile Gly Ala Ile Asn Ser Ser Met Pro Phe
       290                 295                 300
   His Asn Ile His Pro Leu Thr Ile Gly Glu Cys Pro Lys Tyr Val Lys
   305                 310                 315                 320
   Ser Asn Arg Leu Val Leu Ala Thr Gly Leu Arg Asn Ser Pro Gln Gly
                   325                 330                 335
   Glu Gly Arg Arg Lys Lys Arg Gly Leu Phe Gly Ala Ile Ala Gly Phe
               340                 345                 350
   Ile Glu Gly Gly Trp Gln Gly Met Val Asp Gly Trp Tyr Gly Tyr His
           355                 360                 365
   His Ser Asn Glu Gln Gly Ser Gly Tyr Ala Ala Asp Lys Glu Ser Thr
       370                 375                 380
   Gln Lys Ala Ile Asp Gly Val Thr Asn Lys Val Asn Ser Ile Ile Asp
   385                 390                 395                 400
   Lys Met Asn Thr Gln Phe Glu Ala Val Gly Arg Glu Phe Asn Asn Leu
                   405                 410                 415
   Glu Lys Arg Ile Glu Asn Leu Asn Lys Lys Met Glu Asp Gly Phe Leu
               420                 425                 430
   Asp Val Trp Thr Tyr Asn Ala Glu Leu Leu Val Leu Met Glu Asn Glu
           435                 440                 445
   Arg Thr Leu Asp Phe His Asp Ser Asn Val Lys Asn Leu Tyr Asp Lys
   450 455 460
   Val Arg Leu Gln Leu Arg Asp Asn Ala Lys Glu Leu Gly Asn Gly Cys
   465 470 475 480
   Phe Glu Phe Tyr His Arg Cys Asp Asn Glu Cys Met Glu Ser Val Arg
   485 490 495
   Asn Gly Thr Tyr Asp Tyr Pro Gln Tyr Ser Glu Glu Ala Arg Leu Lys
   500 505 510
   Arg Glu Glu Ile Ser Gly Val Lys Leu Glu Ser Ile Gly Thr Tyr Gln
   515 520 525
   Ile Leu Ser Ile Tyr Ser Thr Val Ala Ser Ser Leu Ala Leu Ala Ile
   530 535 540
   Met Val Xaa
   545
<210> 25
   <211> 547
   <212> PRT
   <213> H5N1
<220>
   <221> misc_feature
   <222> (154)..(154)
   <223> Xaa can be any naturally occurring amino acid
<220>
   <221> misc_feature
   <222> (329)..(329)
   <223> Xaa can be any naturally occurring amino acid
<220>
   <221> misc_feature
   <222> (547)..(547)
   <223> Xaa can be any naturally occurring amino acid
<400> 25
   Ile Val Leu Leu Leu Ala Ile Val Ser Leu Val Lys Ser Asp Gln Ile
   1 5 10 15
   Cys Ile Gly Tyr His Ala Asn Asn Ser Thr Glu Gln Val Asp Thr Ile
   20 25 30
   Met Glu Lys Asn Val Thr Val Thr His Ala Gln Asp Ile Leu Glu Lys
   35 40 45
   Thr His Asn Gly Lys Leu Cys Asp Leu Asp Gly Val Lys Pro Leu Ile
       50                  55                  60
   Leu Arg Asp Cys Ser Val Ala Gly Trp Leu Leu Gly Asn Pro Met Cys
   65                  70                  75                  80
   Asp Glu Phe Pro Asn Val Ser Glu Trp Ser Tyr Ile Val Glu Lys Ile
                   85                  90                  95
   Asn Pro Ala Asn Asp Leu Cys Tyr Pro Gly Asn Phe Asn Asn Tyr Glu
               100                 105                 110
   Glu Leu Lys His Leu Leu Ser Arg Ile Asn Arg Phe Glu Lys Ile Lys
           115                 120                 125
   Ile Ile Pro Lys Ser Ser Trp Pro Asp His Glu Ala Ser Leu Gly Val
       130                 135                 140
   Ser Ser Ala Cys Pro Tyr Gln Gly Gly Xaa Ser Phe Tyr Arg Asn Val
   145                 150                 155                 160
   Val Trp Leu Ile Lys Lys Asn Asn Thr Tyr Pro Thr Ile Lys Glu Ser
                   165                 170                 175
   Tyr His Asn Thr Asn Gln Glu Asp Leu Leu Val Leu Trp Gly Ile His
               180                 185                 190
   His Pro Asn Asp Glu Glu Glu Gln Thr Arg Ile Tyr Lys Asn Pro Thr
           195                 200                 205
   Thr Tyr Ile Ser Ile Gly Thr Ser Thr Leu Asn Gln Arg Leu Val Pro
       210                 215                 220
   Lys Ile Ala Thr Arg Ser Lys Val Asn Gly Gln Ser Gly Arg Val Glu
   225                 230                 235                 240
   Phe Phe Trp Thr Ile Leu Lys Ser Asn Asp Thr Ile Asn Phe Glu Ser
                   245                 250                 255
   Asn Gly Asn Phe Ile Ala Pro Glu Asn Ala Tyr Lys Ile Val Lys Lys
               260                 265                 270
   Gly Asp Ser Thr Ile Met Lys Ser Glu Leu Glu Tyr Gly Asn Cys Asn
           275                 280                 285
   Thr Lys Cys Gln Thr Pro Ile Gly Ala Ile Asn Ser Ser Met Pro Phe
       290                 295                 300
   His Asn Ile His Pro Leu Thr Ile Gly Glu Cys Pro Lys Tyr Val Lys
   305                 310                 315                 320
   Ser Asn Arg Leu Val Leu Ala Thr Xaa Leu Arg Asn Ser Pro Gln Gly
                   325                 330                 335
   Glu Gly Arg Arg Lys Lys Arg Gly Leu Phe Gly Ala Ile Ala Gly Phe
               340                 345                 350
   Ile Glu Gly Gly Trp Gln Gly Met Val Asp Gly Trp Tyr Gly Tyr His
           355                 360                 365
   His Ser Asn Glu Gln Gly Ser Gly Tyr Ala Ala Asp Lys Glu Ser Thr
       370                 375                 380
   Gln Lys Ala Ile Asp Gly Val Thr Asn Lys Val Asn Ser Ile Ile Asp
   385                 390                 395                 400
   Lys Met Asn Thr Gln Phe Glu Ala Val Gly Arg Glu Phe Asn Asn Leu
                   405                 410                 415
   Glu Lys Arg Ile Glu Asn Leu Asn Lys Lys Met Glu Asp Gly Phe Leu
               420                 425                 430
   Asp Val Trp Thr Tyr Asn Ala Glu Leu Leu Val Leu Met Glu Asn Glu
           435                 440                 445
   Arg Thr Leu Asp Phe His Asp Ser Asn Val Lys Asn Leu Tyr Asp Lys
       450                 455                 460
   Val Arg Leu Gln Leu Arg Asp Asn Ala Lys Glu Leu Gly Asn Gly Cys
   465                 470                 475                 480
   Phe Glu Phe Tyr His Arg Cys Asp Asn Glu Cys Met Glu Ser Val Arg
                   485                 490                 495
   Asn Gly Thr Tyr Asp Tyr Pro Gln Tyr Ser Glu Glu Ala Arg Leu Lys
               500                 505                 510
   Arg Glu Glu Ile Ser Gly Val Lys Leu Glu Ser Ile Gly Thr Tyr Gln
           515                 520                 525
   Ile Leu Ser Ile Tyr Ser Thr Val Ala Gly Ser Leu Ala Leu Ala Ile
       530                 535                 540
   Met Val Xaa
   545
<210> 26
   <211> 559
   <212> PRT
   <213> H5N1
<220>
   <221> misc_feature
   <222> (1)..(1)
   <223> Xaa can be any naturally occurring amino acid
<400> 26
   Xaa Leu Leu Leu Ala Ile Val Ser Leu Val Lys Ser Asp Gln Ile Cys
   1               5                   10                  15
   Ile Gly Tyr His Ala Asn Asn Ser Thr Glu Gln Val Asp Thr Ile Met
               20                  25                  30
   Glu Lys Asn Val Thr Val Thr His Ala Gln Asp Ile Leu Glu Lys Thr
           35                  40                  45
   His Asn Gly Lys Leu Cys Asp Leu Asp Gly Val Lys Pro Leu Ile Leu
       50                  55                  60
   Arg Asp Cys Ser Val Ala Gly Trp Leu Leu Gly Asn Pro Met Cys Asp
   65                  70                  75                  80
   Glu Phe Pro Asn Val Ser Glu Trp Ser Tyr Ile Val Glu Lys Ile Asn
                   85                  90                  95
   Pro Ala Asn Asp Leu Cys Tyr Pro Gly Asn Phe Asn Asn Tyr Glu Glu
               100                 105                 110
   Leu Lys His Leu Leu Ser Arg Ile Asn Arg Phe Glu Lys Ile Gln Ile
           115                 120                 125
   Ile Pro Lys Ser Ser Trp Pro Asp His Glu Ala Ser Leu Gly Val Ser
       130                 135                 140
   Ser Ala Cys Pro Tyr Gln Gly Glu Pro Ser Phe Tyr Arg Asn Val Val
   145                 150                 155                 160
   Trp Leu Ile Lys Lys Asn Asn Thr Tyr Pro Thr Ile Lys Glu Asn Tyr
                   165                 170                 175
   His Asn Thr Asn Gln Glu Asp Leu Leu Val Leu Trp Gly Ile His His
               180                 185                 190
   Pro Asn Asp Glu Glu Glu Gln Lys Arg Ile Tyr Lys Asn Pro Thr Thr
           195                 200                 205
   Tyr Ile Ser Val Gly Thr Ser Thr Leu Thr Gln Arg Leu Val Pro Lys
       210                 215                 220
   Ile Ala Thr Arg Pro Lys Val Asn Gly Gln Ser Gly Arg Val Glu Phe
   225                 230                 235                 240
   Phe Trp Thr Ile Leu Lys Ser Asn Asp Thr Ile Asn Phe Glu Ser Asn
                   245                 250                 255
   Gly Asn Phe Ile Ala Pro Lys Asn Ala Tyr Lys Ile Val Lys Lys Gly
               260                 265                 270
   Ser Ser Thr Ile Met Lys Ser Glu Leu Glu Tyr Gly Asn Cys Ser Thr
           275                 280                 285
   Lys Cys Gln Thr Pro Ile Gly Ala Ile Asn Ser Ser Met Pro Phe His
       290                 295                 300
   Asn Ile His Pro Leu Thr Ile Gly Glu Cys Pro Lys Tyr Val Lys Ser
   305                 310                 315                 320
   Asn Arg Leu Val Leu Ala Thr Gly Leu Arg Asn Ser Pro Gln Gly Glu
                   325                 330                 335
   Gly Arg Arg Lys Lys Arg Gly Leu Phe Gly Ala Ile Ala Gly Phe Ile
               340                 345                 350
   Glu Gly Gly Trp Gln Gly Met Val Asp Gly Trp Tyr Gly Tyr His His
           355                 360                 365
   Ser Asn Glu Gln Gly Thr Gly Tyr Ala Ala Asp Lys Glu Ser Thr Gln
       370                 375                 380
   Lys Ala Ile Asp Gly Val Thr Asn Lys Val Asn Ser Ile Ile Asp Lys
   385                 390                 395                 400
   Met Asn Thr Gln Phe Glu Ala Val Gly Arg Glu Phe Asn Asn Leu Glu
                   405                 410                 415
   Lys Arg Ile Glu Asn Leu Asn Lys Lys Met Glu Asp Gly Phe Leu Asp
               420                 425                 430
   Val Trp Thr Tyr Asn Ala Glu Leu Leu Val Leu Met Glu Asn Glu Arg
           435                 440                 445
   Thr Leu Asp Phe His Asp Ser Asn Val Arg Asn Leu Tyr Asp Lys Val
   450 455 460
   Arg Leu Gln Leu Arg Asp Asn Ala Lys Glu Leu Gly Asn Gly Cys Phe
   465 470 475 480
   Glu Phe Tyr His Lys Cys Asp Asn Glu Cys Met Glu Ser Val Arg Asn
   485 490 495
   Gly Thr Tyr Asp Tyr Pro Gln Tyr Ser Lys Glu Ala Arg Leu Lys Arg
   500 505 510
   Glu Glu Ile Ser Gly Val Lys Leu Glu Ser Ile Gly Thr Tyr Gln Ile
   515 520 525
   Leu Ser Ile Tyr Ser Thr Val Ala Ser Ser Leu Ala Leu Ala Ile Met
   530 535 540
   Val Ala Gly Leu Ser Leu Trp Met Cys Ser Asn Gly Ser Leu Gln
   545 550 555
<210> 27
   <211> 554
   <212> PRT
   <213> H5N1
<220>
   <221> misc_feature
   <222> (1)..(1)
   <223> Xaa can be any naturally occurring amino acid
<220>
   <221> misc_feature
   <222> (554)..(554)
   <223> Xaa can be any naturally occurring amino acid
<400> 27
   Xaa Val Lys Ser Asp Gln Ile Cys Ile Gly Tyr His Ala Asn Asn Ser
   1 5 10 15
   Thr Glu Gln Val Asp Thr Ile Met Glu Lys Asn Val Thr Val Thr His
   20 25 30
   Ala Gln Asp Ile Leu Glu Lys Thr His Asn Gly Lys Leu Cys Asp Leu
   35 40 45
   Asp Gly Val Lys Pro Leu Ile Leu Arg Asp Cys Ser Val Ala Gly Trp
   50 55 60
   Leu Leu Gly Asn Pro Met Cys Asp Glu Phe Pro Asn Val Ser Glu Trp
   65                  70                  75                  80
   Ser Tyr Ile Val Glu Lys Ile Asn Pro Ala Asn Asp Leu Cys Tyr Pro
                   85                  90                  95
   Gly Asn Phe Asn Asn Tyr Glu Glu Leu Lys His Leu Leu Ser Arg Ile
               100                 105                 110
   Asn Arg Phe Glu Lys Ile Gln Ile Ile Pro Lys Ser Ser Trp Pro Asp
           115                 120                 125
   His Glu Ala Ser Leu Gly Val Ser Ser Ala Cys Pro Tyr Gln Gly Glu
       130                 135                 140
   Pro Ser Phe Tyr Arg Asn Val Val Trp Leu Ile Lys Lys Asn Asn Thr
   145                 150                 155                 160
   Tyr Pro Thr Ile Lys Glu Ser Tyr His Asn Thr Asn Gln Glu Asp Leu
                   165                 170                 175
   Leu Val Leu Trp Gly Ile His His Pro Asn Asp Glu Glu Glu Gln Lys
               180                 185                 190
   Arg Ile Tyr Lys Asn Pro Thr Thr Tyr Ile Ser Val Gly Thr Ser Thr
           195                 200                 205
   Leu Asn Gln Arg Leu Val Pro Lys Ile Ala Thr Arg Pro Lys Val Asn
       210                 215                 220
   Gly Gln Ser Gly Arg Val Glu Phe Phe Trp Thr Ile Leu Lys Ser Asn
   225                 230                 235                 240
   Asp Thr Ile Asn Phe Glu Ser Asn Gly Asn Phe Ile Ala Pro Lys Asn
                   245                 250                 255
   Ala Tyr Lys Ile Val Lys Lys Gly Ser Ser Thr Ile Met Lys Ser Glu
               260                 265                 270
   Leu Glu Tyr Gly Asn Cys Ser Thr Lys Cys Gln Thr Pro Ile Gly Ala
           275                 280                 285
   Ile Asn Ser Ser Met Pro Phe His Asn Ile His Pro Leu Thr Ile Gly
       290                 295                 300
   Glu Cys Pro Lys Tyr Val Lys Ser Asn Arg Leu Val Leu Ala Thr Gly
   305                 310                 315                 320
   Leu Arg Asn Ser Pro Gln Gly Glu Gly Arg Arg Lys Lys Arg Gly Leu
                   325                 330                 335
   Phe Gly Ala Ile Ala Gly Phe Ile Glu Gly Gly Trp Gln Gly Met Val
               340                 345                 350
   Asp Gly Trp Tyr Gly Tyr His His Ser Asn Glu Gln Gly Thr Gly Tyr
           355                 360                 365
   Ala Ala Asp Lys Glu Ser Thr Gln Lys Ala Ile Asp Gly Val Thr Asn
       370                 375                 380
   Lys Val Asn Ser Ile Ile Asp Lys Met Asn Thr Gln Phe Glu Ala Val
   385                 390                 395                 400
   Gly Arg Glu Phe Asn Asn Leu Glu Lys Arg Ile Glu Asn Leu Asn Lys
                   405                 410                 415
   Lys Met Glu Asp Gly Phe Leu Asp Val Trp Thr Tyr Asn Ala Glu Leu
               420                 425                 430
   Leu Val Leu Met Glu Asn Glu Arg Thr Leu Asp Phe His Asp Ser Asn
           435                 440                 445
   Val Arg Asn Leu Tyr Asp Lys Val Arg Leu Gln Leu Arg Asp Asn Ala
       450                 455                 460
   Lys Glu Leu Gly Asn Gly Cys Phe Glu Phe Tyr His Lys Cys Asp Asn
   465                 470                 475                 480
   Glu Cys Met Glu Ser Val Arg Asn Gly Thr Tyr Asp Tyr Pro Gln Tyr
                   485                 490                 495
   Ser Lys Glu Ala Arg Leu Lys Arg Glu Glu Ile Ser Gly Val Lys Leu
               500                 505                 510
   Glu Ser Ile Gly Thr Tyr Gln Ile Leu Ser Ile Tyr Ser Thr Val Ala
           515                 520                 525
   Ser Ser Leu Ala Leu Ala Ile Met Val Ala Gly Leu Ser Leu Trp Met
       530                 535                 540
   Cys Ser Asn Gly Ser Leu Gln Cys Arg Xaa
   545                 550
<210> 28
   <211> 537
   <212> PRT
   <213> H5N1
<220>
   <221> misc_feature
   <222> (1)..(1)
   <223> Xaa can be any naturally occurring amino acid
<220>
   <221> misc_feature
   <222> (537)..(537)
   <223> Xaa can be any naturally occurring amino acid
<400> 28
   Xaa Ser Asp Gln Ile Cys Ile Gly Tyr His Ala Asn Asn Ser Thr Glu
   1               5                   10                  15
   Gln Val Asp Thr Ile Met Glu Lys Asn Val Thr Val Thr His Ala Gln
               20                  25                  30
   Asp Ile Leu Glu Lys Thr His Asn Gly Lys Leu Cys Asp Leu Asp Gly
           35                  40                  45
   Val Lys Pro Leu Ile Leu Arg Asp Cys Ser Val Ala Gly Trp Leu Leu
       50                  55                  60
   Gly Asn Pro Met Cys Asp Glu Phe Pro Asn Val Ser Glu Trp Ser Tyr
   65                  70                  75                  80
   Ile Val Glu Lys Ile Asn Pro Ala Asn Asp Leu Cys Tyr Pro Gly Asn
                   85                  90                  95
   Phe Asn Asn Tyr Glu Glu Leu Lys His Leu Leu Ser Arg Ile Asn Arg
               100                 105                 110
   Phe Glu Lys Ile Gln Ile Ile Pro Lys Ser Ser Trp Pro Asp His Glu
           115                 120                 125
   Ala Ser Leu Gly Val Ser Ser Ala Cys Pro Tyr Gln Gly Glu Pro Ser
       130                 135                 140
   Phe Tyr Arg Asn Val Val Trp Leu Ile Lys Lys Asn Asn Thr Tyr Pro
   145                 150                 155                 160
   Thr Ile Lys Glu Asn Tyr His Asn Thr Asn Gln Glu Asp Leu Leu Val
                   165                 170                 175
   Leu Trp Gly Ile His His Pro Asn Asp Glu Glu Glu Gln Lys Arg Ile
               180                 185                 190
   Tyr Lys Asn Pro Thr Thr Tyr Ile Ser Val Gly Thr Ser Thr Leu Asn
   195 200 205
   Gln Arg Leu Val Pro Lys Ile Ala Thr Arg Pro Lys Val Asn Gly Gln
   210 215 220
   Ser Gly Arg Val Glu Phe Phe Trp Thr Ile Leu Lys Ser Asn Asp Thr
   225 230 235 240
   Ile Asn Phe Glu Ser Asn Gly Asn Phe Ile Ala Pro Lys Asn Ala Tyr
   245 250 255
   Lys Ile Val Lys Lys Gly Ser Ser Thr Ile Met Lys Ser Glu Leu Glu
   260 265 270
   Tyr Gly Asn Cys Ser Thr Lys Cys Gln Thr Pro Ile Gly Ala Ile Asn
   275 280 285
   Ser Ser Met Pro Phe His Asn Ile His Pro Leu Thr Ile Gly Glu Cys
   290 295 300
   Pro Lys Tyr Val Lys Ser Asn Arg Leu Val Leu Ala Thr Gly Leu Arg
   305 310 315 320
   Asn Ser Pro Gln Gly Glu Gly Arg Arg Lys Lys Arg Gly Leu Phe Gly
   325 330 335
   Ala Ile Ala Gly Phe Ile Glu Gly Gly Trp Gln Gly Met Val Asp Gly
   340 345 350
   Trp Tyr Gly Tyr His His Ser Asn Glu Gln Gly Thr Gly Tyr Ala Ala
   355 360 365
   Asp Lys Glu Ser Thr Gln Lys Ala Ile Asp Gly Val Thr Asn Lys Val
   370 375 380
   Asn Ser Ile Ile Asp Lys Met Asn Thr Gln Phe Glu Ala Val Gly Arg
   385 390 395 400
   Glu Phe Asn Asn Leu Glu Lys Arg Ile Glu Asn Leu Asn Lys Lys Met
   405 410 415
   Glu Asp Gly Phe Leu Asp Val Trp Thr Tyr Asn Ala Glu Leu Leu Val
   420 425 430
   Leu Met Glu Asn Glu Arg Thr Leu Asp Phe His Asp Ser Asn Val Arg
   435 440 445
   Asn Leu Tyr Asp Lys Val Arg Leu Gln Leu Arg Asp Asn Ala Lys Glu
   450 455 460
   Leu Gly Asn Gly Cys Phe Glu Phe Tyr His Lys Cys Asp Asn Glu Cys
   465 470 475 480
   Met Glu Ser Val Arg Asn Gly Thr Tyr Asp Tyr Pro Gln Tyr Ser Lys
   485 490 495
   Glu Ala Arg Leu Lys Arg Glu Glu Ile Ser Gly Val Lys Leu Glu Ser
   500 505 510
   Ile Gly Thr Tyr Gln Ile Leu Ser Ile Tyr Ser Thr Val Ala Ser Ser
   515 520 525
   Leu Ala Leu Ala Ile Met Val Ala Xaa
   530 535
<210> 29
   <211> 554
   <212> PRT
   <213> H5N1
<400> 29
   Lys Gly Asp Gln Ile Cys Ile Gly Tyr His Ala Asn Asn Ser Thr Glu
   1               5                   10                  15
   Gln Val Asp Thr Ile Met Glu Lys Asn Val Thr Val Thr His Ala Gln
               20                  25                  30
   Asp Ile Leu Glu Lys Thr His Asn Gly Lys Leu Cys Asp Leu Asp Gly
           35                  40                  45
   Val Lys Pro Leu Ile Leu Arg Asp Cys Ser Val Ala Gly Trp Leu Leu
       50                  55                  60
   Gly Asn Pro Met Cys Asp Glu Phe Pro Asn Val Ser Glu Trp Ser Tyr
   65                  70                  75                  80
   Ile Val Glu Lys Ile Asn Pro Ala Asn Asp Leu Cys Tyr Pro Gly Asn
                   85                  90                  95
   Phe Asn Asn Tyr Glu Glu Leu Lys His Leu Leu Ser Arg Ile Asn Arg
               100                 105                 110
   Phe Glu Lys Ile Lys Ile Ile Pro Lys Ser Ser Trp Pro Asp His Glu
               115                 120                 125
   Ala Ser Leu Gly Val Ser Ser Ala Cys Pro Tyr Gln Gly Gly Pro Ser
       130                 135                 140
   Phe Tyr Arg Asn Val Val Trp Leu Ile Lys Lys Asn Asn Thr Tyr Pro
   145                 150                 155                 160
   Thr Ile Lys Glu Ser Tyr His Asn Thr Asn Gln Glu Asp Leu Leu Val
                   165                 170                 175
   Leu Trp Gly Ile His His Pro Asn Asp Glu Glu Glu Gln Thr Arg Ile
               180                 185                 190
   Tyr Lys Asn Pro Asn Thr Tyr Ile Ser Val Gly Thr Ser Thr Leu Asn
           195                 200                 205
   Gln Arg Leu Val Pro Lys Ile Ala Thr Arg Ser Lys Val Asn Gly Gln
       210                 215                 220
   Ser Gly Arg Val Glu Phe Phe Trp Thr Ile Leu Lys Ser Asn Asp Thr
   225                 230                 235                 240
   Ile Asn Phe Glu Ser Asn Gly Asn Phe Ile Ala Pro Glu Asn Ala Tyr
                   245                 250                 255
   Lys Ile Val Lys Lys Gly Asp Ser Thr Ile Met Lys Ser Glu Leu Glu
               260                 265                 270
   Tyr Gly Asn Cys Ser Thr Lys Cys Gln Thr Pro Ile Gly Ala Ile Asn
           275                 280                 285
   Ser Ser Met Pro Phe His Asn Ile His Pro Leu Thr Ile Gly Glu Cys
       290                 295                 300
   Pro Lys Tyr Val Lys Ser Asn Arg Leu Val Leu Ala Thr Gly Leu Arg
   305                 310                 315                 320
   Asn Ser Pro Gln Glu Glu Gly Arg Arg Lys Lys Arg Gly Leu Phe Gly
                   325                 330                 335
   Ala Ile Ala Gly Phe Ile Glu Gly Gly Trp Gln Gly Met Val Asp Gly
               340                 345                 350
   Trp Tyr Gly Tyr His His Ser Asn Glu Gln Gly Ser Gly Tyr Ala Ala
           355                 360                 365
   Asp Lys Glu Ser Thr Gln Lys Ala Ile Asp Gly Val Thr Asn Lys Val
       370                 375                 380
   Asn Ser Ile Ile Asp Lys Met Asn Thr Gln Phe Glu Ala Val Gly Arg
   385                 390                 395                 400
   Glu Phe Asn Asn Leu Glu Lys Arg Ile Glu Asn Leu Asn Lys Lys Met
                   405                 410                 415
   Glu Asp Gly Phe Leu Asp Val Trp Thr Tyr Asn Ala Glu Leu Leu Val
               420                 425                 430
   Leu Met Glu Asn Glu Arg Thr Leu Asp Phe His Asp Ser Asn Val Lys
           435                 440                 445
   Asn Leu Tyr Asp Lys Val Arg Leu Gln Leu Arg Asp Asn Ala Lys Glu
       450                 455                 460
   Leu Gly Asn Gly Cys Phe Glu Phe Tyr His Arg Cys Asp Asn Glu Cys
   465                 470                 475                 480
   Met Glu Ser Val Arg Asn Gly Thr Tyr Asp Tyr Pro Gln Tyr Ser Glu
                   485                 490                 495
   Glu Ala Arg Leu Lys Arg Glu Glu Ile Ser Gly Val Lys Leu Glu Ser
               500                 505                 510
   Ile Gly Thr Tyr Gln Ile Leu Ser Ile Tyr Ser Thr Val Ala Ser Ser
           515                 520                 525
   Leu Ala Leu Ala Ile Met Val Ala Gly Leu Phe Leu Trp Met Cys Ser
       530                 535                 540
   Asn Gly Ser Leu Gln Cys Arg Ile Cys Ile
   545                 550
<210> 30
   <211> 511
   <212> PRT
   <213> H5N1
<220>
   <221> misc_feature
   <222> (1)..(1)
   <223> Xaa can be any naturally occurring amino acid
<220>
   <221> misc_feature
   <222> (182)..(182)
   <223> Xaa can be any naturally occurring amino acid
<220>
   <221> misc_feature
   <222> (200)..(200)
   <223> Xaa can be any naturally occurring amino acid
<220>
   <221> misc_feature
   <222> (511)..(511)
   <223> Xaa can be any naturally occurring amino acid
<400> 30
   Xaa His Ala Asn Asn Ser Thr Glu Gln Val Asp Thr Ile Met Glu Lys
   1               5                   10                  15
   Asn Ile Thr Val Thr His Ala Gln Asp Ile Leu Glu Lys Thr His Asn
               20                  25                  30
   Gly Lys Leu Cys Asp Leu Asp Gly Val Lys Pro Leu Ile Leu Arg Asp
           35                  40                  45
   Cys Ser Val Ala Gly Trp Leu Leu Gly Asn Pro Met Cys Asp Glu Phe
       50                  55                  60
   Leu Asn Val Ser Glu Trp Ser Tyr Ile Val Glu Lys Ile Asn Pro Ala
   65                  70                  75                  80
   Asn Asp Leu Cys Tyr Pro Gly Asn Phe Asn Asn Tyr Glu Glu Leu Lys
                   85                  90                  95
   His Leu Leu Ser Arg Ile Asn Arg Phe Glu Lys Ile Gln Ile Ile Ser
               100                 105                 110
   Lys Asn Ser Trp Pro Asp His Glu Ala Ser Leu Gly Val Ser Ala Ala
           115                 120                 125
   Cys Pro Tyr Gln Gly Gly Leu Ser Phe Tyr Arg Asn Val Val Trp Leu
       130                 135                 140
   Ile Glu Lys Asn Asn Thr Tyr Pro Leu Ile Lys Lys Asn Tyr His Asn
   145                 150                 155                 160
   Thr Asn Gln Glu Asp Leu Leu Val Leu Trp Gly Ile His His Pro Asn
                   165                 170                 175
   Asp Glu Ala Glu Gln Xaa Arg Leu Tyr Lys Asn Ser Thr Thr Tyr Ile
               180                 185                 190
   Ser Val Gly Thr Ser Thr Leu Xaa Gln Arg Leu Val Pro Lys Ile Ala
           195                 200                 205
   Thr Arg Pro Lys Val Asn Gly Gln Ser Gly Arg Val Glu Phe Phe Trp
   210 215 220
   Thr Ile Leu Lys Ser Asn Asp Val Ile Asn Phe Glu Ser Asn Gly Asn
   225 230 235 240
   Phe Ile Ala Pro Glu Asn Ala Tyr Lys Ile Val Lys Lys Gly Asp Ser
   245 250 255
   Thr Ile Met Lys Ser Asp Leu Glu Tyr Gly Asn Cys Ser Thr Lys Cys
   260 265 270
   Gln Thr Pro Ile Gly Ala Ile Asn Ser Ser Met Pro Phe His Asn Ile
   275 280 285
   His Pro Leu Thr Ile Gly Glu Cys Pro Lys Tyr Val Lys Ser Asn Arg
   290 295 300
   Leu Val Leu Ala Thr Gly Leu Arg Asn Ser Pro Gln Gly Glu Arg Arg
   305 310 315 320
   Arg Lys Lys Arg Gly Leu Phe Gly Ala Ile Ala Gly Phe Ile Glu Gly
   325 330 335
   Gly Trp Gln Gly Met Val Asp Gly Trp Tyr Gly Tyr His His Ser Asn
   340 345 350
   Glu Gln Gly Ser Gly Tyr Ala Ala Asp Lys Glu Ser Thr Gln Lys Ala
   355 360 365
   Ile Asp Gly Val Thr Asn Lys Val Asn Ser Ile Ile Asp Lys Met Asn
   370 375 380
   Thr Gln Phe Glu Ala Val Gly Arg Glu Phe Asn Asn Leu Glu Arg Arg
   385 390 395 400
   Ile Glu Asn Leu Asn Lys Lys Met Glu Asp Gly Phe Leu Asp Val Trp
   405 410 415
   Thr Tyr Asn Ala Glu Leu Leu Val Leu Met Glu Asn Glu Arg Thr Leu
   420 425 430
   Asp Phe His Asp Ser Asn Val Lys Asn Leu Tyr Asp Lys Val Arg Leu
   435 440 445
   Gln Leu Arg Asp Asn Ala Lys Glu Leu Gly Asn Gly Cys Phe Glu Phe
   450 455 460
   Tyr His Arg Cys Asp Asn Glu Cys Met Glu Ser Val Arg Asn Gly Thr
   465 470 475 480
   Tyr Asp Tyr Pro Gln Tyr Ser Glu Glu Ala Arg Leu Lys Arg Glu Glu
   485 490 495
   Ile Ser Gly Ala Lys Leu Glu Ser Ile Gly Thr Tyr Gln Ile Xaa
   500 505 510
<210> 31
   <211> 537
   <212> PRT
   <213> H5N1
<220>
   <221> misc_feature
   <222> (1)..(1)
   <223> Xaa can be any naturally occurring amino acid
<400> 31
   Xaa Leu Val Lys Ser Asp Gln Ile Cys Ile Gly Tyr His Ala Asn Asn
   1 5 10 15
   Ser Thr Glu Gln Val Asp Thr Ile Met Glu Lys Asn Val Thr Val Thr
   20 25 30
   His Ala Gln Asp Ile Leu Glu Lys Thr His Asn Gly Lys Leu Cys Asp
   35 40 45
   Leu Asp Gly Val Lys Pro Leu Ile Leu Arg Asp Cys Ser Val Ala Gly
   50 55 60
   Trp Leu Leu Gly Asn Pro Met Cys Asp Glu Phe Pro Asn Val Ser Glu
   65 70 75 80
   Trp Ser Tyr Ile Val Glu Lys Ile Asn Pro Ala Asn Asp Leu Cys Tyr
   85 90 95
   Pro Gly Asn Phe Asn Asn Tyr Glu Glu Leu Lys His Leu Leu Ser Arg
   100 105 110
   Ile Asn Arg Phe Glu Lys Ile Lys Ile Ile Pro Lys Ser Ser Trp Pro
   115 120 125
   Asp His Glu Ala Ser Leu Gly Val Ser Ser Ala Cys Pro Tyr Gln Arg
   130 135 140
   Gly Pro Ser Phe Tyr Arg Asn Val Val Trp Leu Ile Lys Lys Asn Asn
   145 150 155 160
   Thr Tyr Pro Thr Ile Lys Lys Ser Tyr His Asn Thr Asn Gln Glu Asp
   165 170 175
   Leu Leu Val Leu Trp Gly Ile His His Pro Asn Asp Glu Glu Glu Gln
   180 185 190
   Thr Arg Ile Tyr Lys Asn Pro Thr Thr Tyr Ile Ser Val Gly Thr Ser
   195 200 205
   Thr Leu Asn Gln Arg Leu Val Pro Lys Ile Ala Thr Arg Ser Lys Val
   210 215 220
   Asn Gly Gln Ser Gly Arg Val Glu Phe Phe Trp Thr Ile Leu Lys Ser
   225 230 235 240
   Asn Asp Thr Ile Asn Phe Glu Ser Asn Gly Asn Phe Ile Ala Pro Glu
   245 250 255
   Asn Ala Tyr Lys Ile Val Lys Lys Gly Asp Ser Thr Ile Met Lys Ser
   260 265 270
   Glu Leu Glu Tyr Gly Asn Cys Asn Thr Lys Cys Gln Thr Pro Ile Gly
   275 280 285
   Ala Ile Asn Ser Ser Met Pro Phe His Asn Ile His Pro Leu Thr Ile
   290 295 300
   Gly Glu Cys Pro Lys Tyr Val Lys Ser Asn Arg Leu Val Leu Ala Thr
   305 310 315 320
   Gly Leu Arg Asn Ser Pro Gln Gly Glu Gly Arg Arg Lys Lys Arg Gly
   325 330 335
   Leu Phe Gly Ala Ile Ala Gly Phe Ile Glu Gly Gly Trp Gln Gly Met
   340 345 350
   Val Asp Gly Trp Tyr Gly Tyr His His Ser Asn Glu Gln Gly Ser Gly
   355 360 365
   Tyr Ala Ala Asp Lys Glu Ser Thr Gln Lys Ala Ile Asp Gly Val Thr
   370 375 380
   Asn Lys Val Asn Ser Ile Ile Asp Lys Met Asn Thr Gln Phe Glu Ala
   385 390 395 400
   Val Gly Arg Glu Phe Asn Asn Leu Glu Lys Arg Ile Glu Asn Leu Asn
   405 410 415
   Lys Lys Met Glu Asp Gly Phe Leu Asp Val Trp Thr Tyr Asn Ala Glu
   420 425 430
   Leu Leu Val Leu Met Glu Asn Glu Arg Thr Leu Asp Phe His Asp Ser
   435 440 445
   Asn Val Lys Asn Leu Tyr Asp Lys Val Arg Leu Gln Leu Arg Asp Asn
   450 455 460
   Ala Lys Glu Leu Gly Asn Gly Cys Phe Glu Phe Tyr His Arg Cys Asp
   465 470 475 480
   Asn Glu Cys Met Glu Ser Val Lys Asn Gly Thr Tyr Asp Tyr Pro Gln
   485 490 495
   Tyr Ser Glu Glu Ala Arg Leu Lys Arg Glu Glu Ile Ser Gly Val Lys
   500 505 510
   Leu Glu Ser Ile Gly Thr Tyr Gln Ile Leu Ser Ile Tyr Ser Thr Val
   515 520 525
   Ala Ser Ser Leu Ala Leu Ala Ile Met
   530 535
<210> 32
   <211> 534
   <212> PRT
   <213> H5N1
<400> 32
   Lys Ser Asp Gln Ile Cys Ile Gly Tyr His Ala Asn Asn Ser Thr Glu
   1 5 10 15
   Gln Val Asp Thr Ile Met Glu Lys Asn Val Thr Val Thr His Ala Gln
   20 25 30
   Asp Ile Leu Glu Lys Thr His Asn Gly Lys Leu Cys Asp Leu Asp Gly
   35 40 45
   Val Lys Pro Leu Ile Leu Arg Asp Cys Ser Val Ala Gly Trp Leu Leu
   50 55 60
   Gly Asn Pro Met Cys Asp Glu Phe Pro Asn Val Ser Glu Trp Ser Tyr
   65 70 75 80
   Ile Val Glu Lys Ile Asn Pro Ala Asn Asp Leu Cys Tyr Pro Gly Asn
                   85                  90                  95
   Phe Asn Asn Tyr Glu Glu Leu Lys His Leu Leu Ser Arg Ile Asn Arg
               100                 105                 110
   Phe Glu Lys Ile Gln Ile Ile Pro Lys Ser Ser Trp Pro Asp His Glu
           115                 120                 125
   Ala Ser Leu Gly Val Ser Ser Ala Cys Pro Tyr Gln Gly Gly Pro Ser
       130                 135                 140
   Phe Tyr Arg Asn Val Val Trp Leu Ile Lys Lys Asn Asp Thr Tyr Pro
   145                 150                 155                 160
   Thr Ile Lys Glu Ser Tyr His Asn Thr Asn Gln Glu Asp Leu Leu Val
                   165                 170                 175
   Leu Trp Gly Ile His His Pro Asn Asp Glu Glu Glu Gln Lys Arg Ile
               180                 185                 190
   Tyr Lys Asn Pro Thr Thr Tyr Ile Ser Val Gly Thr Ser Thr Leu Asn
           195                 200                 205
   Gln Arg Leu Val Pro Lys Ile Ala Thr Arg Ser Lys Val Asn Gly Gln
       210                 215                 220
   Ser Gly Arg Val Glu Phe Phe Trp Thr Ile Leu Lys Ser Asn Asp Thr
   225                 230                 235                 240
   Ile Asn Phe Glu Ser Asn Gly Asn Phe Ile Ala Pro Glu Asn Ala Tyr
                   245                 250                 255
   Lys Ile Val Lys Lys Gly Asp Ser Thr Ile Met Lys Ser Glu Leu Glu
               260                 265                 270
   Tyr Gly Asn Cys Ser Thr Lys Cys Gln Thr Pro Ile Gly Ala Ile Asn
           275                 280                 285
   Thr Ser Met Pro Phe His Asn Ile His Pro Leu Thr Ile Gly Glu Cys
       290                 295                 300
   Pro Lys Tyr Val Lys Ser Asn Arg Leu Val Leu Ala Thr Gly Leu Arg
   305                 310                 315                 320
   Asn Ser Pro Gln Gly Glu Gly Arg Arg Lys Lys Arg Gly Leu Phe Gly
                   325                 330                 335
   Ala Ile Ala Gly Phe Ile Glu Gly Gly Trp Gln Gly Met Val Asp Gly
               340                 345                 350
   Trp Tyr Gly Tyr His His Ser Asn Glu Gln Gly Ser Gly Tyr Ala Ala
           355                 360                 365
   Asp Lys Glu Ser Thr Gln Lys Ala Ile Asn Gly Val Thr Asn Lys Val
       370                 375                 380
   Asn Ser Ile Ile Asp Lys Met Asn Thr Gln Phe Glu Ala Val Gly Arg
   385                 390                 395                 400
   Glu Phe Asn Asn Leu Glu Lys Arg Ile Glu Asn Leu Asn Lys Lys Met
                   405                 410                 415
   Glu Asp Gly Phe Leu Asp Val Trp Thr Tyr Asn Ala Glu Leu Leu Val
               420                 425                 430
   Leu Met Glu Asn Glu Arg Thr Leu Asp Phe His Asp Ser Asn Val Arg
           435                 440                 445
   Asn Leu Tyr Asp Lys Val Arg Leu Gln Leu Arg Asp Asn Ala Lys Glu
       450                 455                 460
   Leu Gly Asn Gly Cys Phe Glu Phe Tyr His Arg Cys Asp Asn Glu Cys
   465                 470                 475                 480
   Met Glu Ser Val Arg Asn Gly Thr Tyr Asp Tyr Pro Gln Tyr Ser Lys
                   485                 490                 495
   Glu Ala Arg Leu Lys Arg Glu Glu Ile Ser Gly Val Lys Leu Glu Ser
               500                 505                 510
   Ile Gly Thr Tyr Gln Ile Leu Ser Ile Tyr Ser Thr Val Ala Ser Ser
           515                 520                 525
   Leu Ala Leu Ala Ile Met
       530
<210> 33
   <211> 536
   <212> PRT
   <213> H5N1
<220>
   <221> misc_feature
   <222> (1)..(1)
   <223> Xaa can be any naturally occurring amino acid
<220>
   <221> misc_feature
   <222> (311)..(311)
   <223> Xaa can be any naturally occurring amino acid
<400> 33
   Xaa Val Lys Ser Asp Gln Ile Cys Ile Gly Tyr His Ala Asn Asn Ser
   1               5                   10                  15
   Thr Glu Gln Val Asp Thr Ile Met Glu Lys Asn Val Thr Val Thr His
               20                  25                  30
   Ala Gln Asp Ile Leu Glu Lys Thr His Asn Gly Lys Leu Cys Asp Leu
           35                  40                  45
   Asp Gly Val Lys Pro Leu Ile Leu Arg Asp Cys Ser Val Ala Gly Trp
       50                  55                  60
   Leu Leu Gly Asn Pro Met Cys Asp Glu Phe Pro Asn Val Ser Glu Trp
   65                  70                  75                  80
   Ser Tyr Ile Val Glu Lys Thr Asn Pro Ala Asn Asp Leu Cys Tyr Pro
                   85                  90                  95
   Gly Asn Phe Asn Asn Tyr Glu Glu Leu Lys His Leu Leu Ser Arg Ile
               100                 105                 110
   Asn Arg Phe Glu Lys Ile Lys Ile Ile Pro Lys Ser Ser Trp Pro Asp
           115                 120                 125
   His Glu Ala Ser Leu Gly Val Ser Ser Ala Cys Pro Tyr Gln Gly Glu
       130                 135                 140
   Pro Ser Phe Tyr Arg Asn Val Val Trp Leu Ile Lys Lys Asn Asn Thr
   145                 150                 155                 160
   Tyr Pro Thr Ile Lys Glu Ser Tyr His Asn Thr Asn Gln Glu Asp Leu
                   165                 170                 175
   Leu Val Leu Trp Gly Ile His His Pro Asn Asp Glu Glu Glu Gln Thr
               180                 185                 190
   Arg Ile Tyr Lys Asn Pro Thr Thr Tyr Ile Ser Val Gly Thr Ser Thr
           195                 200                 205
   Leu Asn Gln Arg Leu Val Pro Lys Ile Ala Thr Arg Ser Lys Val Asn
       210                 215                 220
   Gly Gln Ser Gly Arg Val Glu Phe Phe Trp Thr Ile Leu Lys Ser Asn
   225                 230                 235                 240
   Asp Thr Ile Asn Phe Glu Ser Asn Gly Asn Phe Ile Ala Pro Glu Asn
                   245                 250                 255
   Ala Tyr Lys Ile Val Lys Lys Gly Asp Ser Thr Ile Met Lys Ser Glu
               260                 265                 270
   Leu Glu Tyr Gly Asn Cys Ser Thr Lys Cys Gln Thr Pro Val Gly Ala
           275                 280                 285
   Ile Asn Ser Ser Met Pro Phe His Asn Ile His Pro Leu Thr Ile Gly
       290                 295                 300
   Glu Cys Pro Lys Tyr Val Xaa Ser Asn Arg Leu Val Leu Ala Thr Gly
   305                 310                 315                 320
   Leu Arg Asn Ser Pro Gln Gly Glu Gly Arg Arg Lys Lys Arg Gly Leu
                   325                 330                 335
   Phe Gly Ala Ile Ala Gly Phe Ile Glu Gly Gly Trp Gln Gly Met Val
               340                 345                 350
   Asp Gly Trp Tyr Gly Tyr His His Ser Asn Glu Gln Gly Ser Gly Tyr
           355                 360                 365
   Ala Ala Asp Arg Glu Ser Thr Gln Lys Ala Ile Asp Gly Val Thr Asn
       370                 375                 380
   Lys Val Asn Ser Ile Ile Asp Lys Met Asn Thr Gln Phe Glu Ala Val
   385                 390                 395                 400
   Gly Arg Glu Phe Asn Asn Leu Glu Lys Arg Ile Glu Asn Leu Asn Lys
                   405                 410                 415
   Lys Met Glu Asp Gly Phe Leu Asp Val Trp Thr Tyr Asn Ala Glu Leu
               420                 425                 430
   Leu Val Leu Met Glu Asn Glu Arg Thr Leu Asp Phe His Asp Ser Asn
           435                 440                 445
   Val Lys Asn Leu Tyr Asp Lys Val Arg Leu Gln Leu Arg Asp Asn Ala
       450                 455                 460
   Lys Glu Leu Gly Asn Gly Cys Phe Glu Phe Tyr His Arg Cys Asp Asn
   465                 470                 475                 480
   Glu Cys Ile Glu Ser Val Arg Asn Gly Thr Tyr Asp Tyr Pro Gln Tyr
                   485                 490                 495
   Ser Glu Glu Ala Arg Leu Lys Arg Glu Glu Ile Ser Gly Val Lys Leu
               500                 505                 510
   Glu Ser Ile Gly Thr Tyr Gln Ile Leu Ser Ile Tyr Ser Thr Val Ala
           515                 520                 525
   Ser Ser Leu Ala Leu Ala Ile Met
       530                 535
<210> 34
   <211> 515
   <212> PRT
   <213> H5N1
<220>
   <221> misc_feature
   <222> (1)..(1)
   <223> Xaa can be any naturally occurring amino acid
<220>
   <221> misc_feature
   <222> (515)..(515)
   <223> Xaa can be any naturally occurring amino acid
<400> 34
   Xaa Leu Val Lys Ser Asp Gln Ile Cys Ile Gly Tyr His Ala Asn Asn
   1               5                   10                  15
   Ser Thr Glu Gln Val Asp Thr Ile Met Glu Lys Asn Val Thr Val Thr
               20                  25                  30
   His Ala Gln Asp Ile Leu Glu Lys Thr His Asn Gly Lys Leu Cys Asp
           35                  40                  45
   Leu Asp Gly Val Lys Pro Leu Ile Leu Arg Asp Cys Ser Val Ala Gly
       50                  55                  60
   Trp Leu Leu Gly Asn Pro Met Cys Asp Glu Phe Pro Asn Val Ser Glu
   65                  70                  75                  80
   Trp Ser Tyr Ile Val Glu Lys Ile Asn Pro Ala Asn Asp Leu Cys Tyr
                   85                  90                  95
   Pro Gly Asn Phe Asn Asn Tyr Glu Glu Leu Lys His Leu Leu Ser Arg
               100                 105                 110
   Ile Asn Arg Phe Glu Lys Ile Gln Ile Ile Pro Lys Ser Ser Trp Pro
           115                 120                 125
   Asp His Glu Ala Ser Leu Gly Val Ser Ser Ala Cys Pro Tyr Gln Gly
       130                 135                 140
   Gly Pro Ser Phe Tyr Arg Asn Val Val Trp Leu Ile Lys Lys Asn Asp
   145                 150                 155                 160
   Thr Tyr Pro Thr Ile Lys Glu Ser Tyr His Asn Thr Asn Gln Glu Asp
                   165                 170                 175
   Leu Leu Val Leu Trp Gly Ile His His Pro Asn Asp Glu Glu Glu Gln
               180                 185                 190
   Lys Arg Ile Tyr Lys Asn Pro Thr Thr Tyr Val Ser Val Gly Thr Ser
           195                 200                 205
   Thr Leu Asn Gln Arg Leu Val Pro Lys Ile Ala Thr Arg Ser Lys Val
       210                 215                 220
   Asn Gly Gln Ser Gly Arg Val Glu Phe Phe Trp Thr Ile Leu Lys Ser
   225                 230                 235                 240
   Asn Asp Thr Ile Asn Phe Glu Ser Asn Gly Asn Phe Ile Ala Pro Glu
                   245                 250                 255
   Asn Ala Tyr Lys Ile Val Lys Lys Gly Asp Ser Thr Ile Met Lys Ser
               260                 265                 270
   Glu Leu Glu Tyr Gly Asn Cys Ser Thr Lys Cys Gln Thr Pro Ile Gly
           275                 280                 285
   Ala Ile Asn Thr Ser Met Pro Phe His Asn Ile His Pro Leu Thr Ile
       290                 295                 300
   Gly Glu Cys Pro Lys Tyr Val Lys Ser Asn Arg Leu Val Leu Ala Thr
   305                 310                 315                 320
   Gly Leu Arg Asn Ser Pro Gln Gly Glu Gly Arg Arg Lys Lys Arg Gly
                   325                 330                 335
   Leu Phe Gly Ala Ile Ala Gly Phe Ile Glu Gly Gly Trp Gln Gly Met
               340                 345                 350
   Val Asp Gly Trp Tyr Gly Tyr His His Ser Asn Glu Gln Gly Ser Gly
   355 360 365
   Tyr Ala Ala Asp Lys Glu Ser Thr Gln Lys Ala Ile Asp Gly Val Thr
   370 375 380
   Asn Lys Val Asn Ser Ile Ile Asp Lys Met Asn Thr Gln Phe Glu Ala
   385 390 395 400
   Val Gly Arg Glu Phe Asn Asn Leu Glu Lys Arg Ile Glu Asn Leu Asn
   405 410 415
   Lys Lys Met Glu Asp Gly Phe Leu Asp Val Trp Thr Tyr Asn Ala Glu
   420 425 430
   Leu Leu Val Leu Met Glu Asn Glu Arg Thr Leu Asp Phe His Asp Ser
   435 440 445
   Asn Val Arg Asn Leu Tyr Asp Lys Val Arg Leu Gln Leu Arg Asp Asn
   450 455 460
   Ala Lys Glu Leu Gly Asn Gly Cys Phe Glu Phe Tyr His Arg Cys Asp
   465 470 475 480
   Asn Glu Cys Met Glu Ser Val Arg Asn Gly Thr Tyr Asp Tyr Pro Gln
   485 490 495
   Tyr Ser Glu Glu Ala Arg Leu Lys Arg Glu Glu Ile Ser Gly Val Lys
   500 505 510
   Leu Glu Xaa
   515
<210> 35
   <211> 519
   <212> PRT
   <213> H5N1
<220>
   <221> misc_feature
   <222> (1)..(1)
   <223> Xaa can be any naturally occurring amino acid
<220>
   <221> misc_feature
   <222> (517)..(517)
   <223> Xaa can be any naturally occurring amino acid
<400> 35
   Xaa Val Lys Ser Asp Gln Ile Cys Ile Gly Tyr His Ala Asn Asn Ser
   1 5 10 15
   Thr Glu Gln Val Asp Thr Ile Met Glu Lys Asn Val Thr Val Thr His
   20 25 30
   Ala Gln Asp Ile Leu Glu Lys Thr His Asn Gly Lys Leu Cys Asn Leu
   35 40 45
   Asp Gly Val Lys Pro Leu Ile Leu Arg Asp Cys Ser Val Ala Gly Trp
   50 55 60
   Leu Leu Gly Asn Pro Met Cys Asp Lys Phe Leu Asn Val Pro Glu Trp
   65 70 75 80
   Ser Tyr Ile Val Glu Lys Ile Asn Pro Ala Asn Asp Leu Cys Tyr Pro
   85 90 95
   Gly Asp Phe Asn Asp Tyr Glu Glu Leu Lys His Leu Leu Ser Arg Ile
   100 105 110
   Asn His Phe Glu Lys Ile Gln Ile Ile Pro Lys Asn Ser Trp Ser Asp
   115 120 125
   His Glu Ala Ser Gly Val Ser Ser Ala Cys Pro Tyr Gln Gly Arg Ser
   130 135 140
   Ser Phe Phe Arg Asn Val Val Trp Leu Thr Lys Lys Asn Asn Ala Tyr
   145 150 155 160
   Pro Thr Ile Lys Lys Ser Tyr Asn Asn Thr Asn Gln Glu Asp Leu Leu
   165 170 175
   Val Leu Trp Gly Ile His His Pro Asn Asp Ala Ala Glu Gln Thr Met
   180 185 190
   Leu Tyr Gln Asn Pro Thr Thr Tyr Val Ser Val Gly Thr Ser Thr Leu
   195 200 205
   Asn Gln Arg Leu Val Pro Lys Ile Ala Thr Arg Ser Lys Val Asn Gly
   210 215 220
   Gln Ser Gly Arg Met Glu Phe Phe Trp Thr Ile Leu Lys Ser Asn Asp
   225 230 235 240
   Ala Ile Asn Phe Glu Ser Asn Gly Asn Phe Ile Ala Pro Glu Asn Ala
   245 250 255
   Tyr Lys Ile Val Lys Lys Gly Asp Ser Thr Ile Met Lys Ser Glu Leu
               260                 265                 270
   Glu Tyr Gly Asn Cys Asn Thr Lys Cys Gln Thr Pro Ile Gly Ala Ile
           275                 280                 285
   Asn Ser Ser Met Pro Phe His Asn Ile His Pro Leu Thr Ile Gly Glu
       290                 295                 300
   Cys Pro Lys Tyr Val Lys Ser Asn Arg Leu Val Leu Ala Thr Gly Leu
   305                 310                 315                 320
   Arg Asn Ser Pro Gln Gly Glu Arg Arg Arg Lys Lys Arg Gly Leu Phe
                   325                 330                 335
   Gly Ala Ile Ala Gly Phe Ile Glu Gly Gly Trp Gln Gly Met Val Asp
               340                 345                 350
   Gly Trp Tyr Gly Tyr His His Ser Asn Glu Gln Gly Ser Gly Tyr Ala
           355                 360                 365
   Ala Asp Lys Glu Ser Thr Gln Lys Ala Ile Asp Gly Val Thr Asn Lys
       370                 375                 380
   Val Asn Ser Ile Ile Asp Lys Met Asn Thr Gln Phe Glu Ala Val Gly
   385                 390                 395                 400
   Arg Glu Phe Asn Asn Leu Glu Arg Arg Ile Glu Asn Leu Asn Lys Lys
                   405                 410                 415
   Met Glu Asp Gly Phe Leu Asp Val Trp Thr Tyr Asn Ala Glu Leu Leu
               420                 425                 430
   Val Leu Met Glu Asn Glu Arg Thr Leu Asp Phe His Asp Ser Asn Val
           435                 440                 445
   Lys Asn Leu Tyr Asp Lys Val Arg Leu Gln Leu Arg Asp Asn Ala Lys
       450                 455                 460
   Glu Leu Gly Asn Gly Cys Phe Glu Phe Tyr His Arg Cys Asp Asn Glu
   465                 470                 475                 480
   Cys Met Glu Ser Val Arg Asn Gly Thr Tyr Asp Tyr Pro Gln Tyr Ser
                   485                 490                 495
   Glu Glu Ala Arg Leu Lys Arg Glu Glu Ile Ser Gly Val Lys Leu Glu
               500                 505                 510
   Ser Ile Gly Thr Xaa Gln Ile
   515
<210> 36
   <211> 510
   <212> PRT
   <213> H5N1
<220>
   <221> misc_feature
   <222> (1)..(1)
   <223> Xaa can be any naturally occurring amino acid
<220>
   <221> misc_feature
   <222> (507)..(507)
   <223> Xaa can be any naturally occurring amino acid
<220>
   <221> misc_feature
   <222> (510)..(510)
   <223> Xaa can be any naturally occurring amino acid
<400> 36
   Xaa His Ala Asn Asn Ser Thr Glu Gln Val Asp Thr Ile Met Glu Lys
   1 5 10 15
   Asn Val Thr Val Thr His Ala Gln Asp Ile Leu Glu Lys Thr His Asn
   20 25 30
   Gly Lys Leu Cys Asn Leu Asp Gly Val Lys Pro Leu Ile Leu Arg Asp
   35 40 45
   Cys Ser Val Ala Gly Trp Leu Leu Gly Asn Pro Met Cys Asp Glu Phe
   50 55 60
   Pro Asn Val Leu Glu Trp Ser Tyr Ile Val Glu Lys Ile Asn Pro Ala
   65 70 75 80
   Asn Asp Leu Cys Tyr Pro Gly Asn Phe Asn Asp Tyr Glu Glu Leu Lys
   85 90 95
   His Leu Leu Ser Arg Ile Asn His Phe Glu Lys Ile Gln Ile Ile Pro
   100 105 110
   Lys Asn Ser Trp Ser Asp His Glu Ala Ser Gly Val Ser Ser Ala Cys
   115 120 125
   Pro Tyr Gln Arg Arg Ser Ser Phe Phe Arg Asn Val Val Trp Leu Thr
   130 135 140
   Lys Lys Asn Asn Ala Tyr Pro Thr Ile Lys Lys Ser Tyr Asn Asn Thr
   145                 150                 155                 160
   Asn Gln Glu Asp Leu Leu Val Leu Trp Gly Ile His His Pro Asn Asp
                   165                 170                 175
   Ala Ala Glu Gln Thr Arg Leu Tyr Gln Asn Pro Thr Thr Tyr Ile Ser
               180                 185                 190
   Val Gly Thr Ser Thr Leu Asn Gln Arg Leu Val Pro Lys Ile Ala Thr
           195                 200                 205
   Arg Ser Lys Val Asn Gly Gln Ser Gly Arg Met Glu Phe Phe Trp Thr
       210                 215                 220
   Ile Leu Lys Ser Asn Asp Ala Ile Asn Phe Glu Ser Asn Gly Asn Phe
   225                 230                 235                 240
   Ile Ala Pro Glu Asn Ala Tyr Lys Ile Val Lys Lys Gly Asp Ser Thr
                   245                 250                 255
   Ile Met Lys Ser Glu Leu Glu Tyr Gly Asn Cys Asn Thr Lys Cys Gln
               260                 265                 270
   Thr Pro Ile Gly Ala Ile Asn Ser Ser Met Pro Phe His Asn Ile His
           275                 280                 285
   Pro Leu Thr Ile Gly Glu Cys Pro Lys Tyr Val Lys Ser Asn Arg Leu
       290                 295                 300
   Val Leu Ala Thr Gly Leu Arg Asn Ser Pro Gln Gly Glu Arg Arg Arg
   305                 310                 315                 320
   Lys Lys Arg Gly Leu Phe Gly Ala Ile Ala Gly Phe Ile Glu Gly Gly
                   325                 330                 335
   Trp Gln Gly Met Val Asp Gly Trp Tyr Gly Tyr His His Ser Asn Glu
               340                 345                 350
   Gln Gly Ser Gly Tyr Ala Ala Asp Lys Glu Ser Thr Gln Lys Ala Ile
           355                 360                 365
   Asp Gly Val Thr Asn Lys Val Asn Ser Ile Ile Asp Lys Met Asn Thr
       370                 375                 380
   Gln Phe Glu Ala Val Gly Arg Glu Phe Asn Asn Leu Glu Arg Arg Ile
   385                 390                 395                 400
   Glu Asn Leu Asn Lys Lys Met Glu Asp Gly Phe Leu Asp Val Trp Thr
   405 410 415
   Tyr Asn Ala Glu Leu Leu Val Leu Met Glu Asn Glu Arg Thr Leu Asp
   420 425 430
   Phe His Asp Ser Asn Val Lys Asn Leu Tyr Asp Lys Val Arg Leu Gln
   435 440 445
   Leu Arg Asp Asn Ala Lys Glu Leu Gly Asn Gly Cys Phe Glu Phe Tyr
   450 455 460
   His Arg Cys Asp Asn Glu Cys Met Glu Ser Val Arg Asn Gly Thr Tyr
   465 470 475 480
   Asp Tyr Pro Gln Tyr Ser Glu Glu Ala Arg Leu Lys Arg Glu Glu Ile
   485 490 495
   Ser Gly Val Lys Leu Glu Ser Ile Gly Thr Xaa Gln Ile Xaa
   500 505 510
<210> 37
   <211> 523
   <212> PRT
   <213> H5N1
<220>
   <221> misc_feature
   <222> (523)..(523)
   <223> Xaa can be any naturally occurring amino acid
<400> 37
   His Ala Asn Asn Ser Thr Glu Gln Val Asp Thr Ile Met Glu Lys Asn
   1 5 10 15
   Val Thr Val Thr His Ala Gln Asp Ile Leu Glu Lys Thr His Asn Gly
   20 25 30
   Lys Leu Cys Asp Leu Asp Gly Val Lys Pro Leu Ile Leu Arg Asp Cys
   35 40 45
   Ser Val Ala Gly Trp Leu Leu Gly Asn Pro Met Cys Asp Glu Phe Pro
   50 55 60
   Asn Val Ser Glu Trp Ser Tyr Ile Val Glu Lys Ile Asn Pro Ala Asn
   65 70 75 80
   Asp Leu Cys Tyr Pro Gly Asn Phe Asn Asn Tyr Glu Glu Leu Lys His
                   85                  90                  95
   Leu Leu Ser Arg Ile Asn Arg Phe Glu Lys Ile Lys Ile Ile Pro Lys
               100                 105                 110
   Ser Ser Trp Pro Asp His Glu Ala Ser Leu Gly Val Ser Ser Ala Cys
           115                 120                 125
   Pro Tyr Gln Gly Gly Pro Ser Phe Tyr Arg Asn Val Val Trp Leu Ile
       130                 135                 140
   Lys Lys Asn Asn Thr Tyr Pro Thr Ile Lys Lys Ser Tyr His Asn Thr
   145                 150                 155                 160
   Asn Gln Glu Asp Leu Leu Val Leu Trp Gly Ile His His Pro Asn Asp
                   165                 170                 175
   Glu Glu Glu Gln Thr Arg Ile Tyr Lys Asn Pro Thr Thr Tyr Ile Ser
               180                 185                 190
   Val Gly Thr Ser Thr Leu Asn Gln Arg Leu Val Pro Lys Ile Ala Thr
           195                 200                 205
   Arg Ser Lys Val Asn Gly Gln Ser Gly Arg Val Glu Phe Phe Trp Thr
       210                 215                 220
   Ile Leu Lys Ser Asn Asp Thr Ile Asn Phe Glu Ser Asn Gly Asn Phe
   225                 230                 235                 240
   Ile Ala Pro Glu Asn Ala Tyr Lys Ile Val Lys Lys Gly Asp Ser Thr
                   245                 250                 255
   Ile Met Lys Ser Glu Leu Glu Tyr Gly Asn Cys Asn Thr Lys Cys Gln
               260                 265                 270
   Thr Pro Ile Gly Ala Ile Asn Ser Ser Met Pro Phe His Asn Ile His
           275                 280                 285
   Pro Leu Thr Ile Gly Glu Cys Pro Lys Tyr Val Lys Ser Asn Arg Leu
       290                 295                 300
   Val Leu Ala Thr Gly Leu Arg Asn Ser Pro Gln Gly Glu Gly Arg Arg
   305                 310                 315                 320
   Lys Lys Arg Gly Leu Phe Gly Ala Ile Ala Gly Phe Ile Glu Gly Gly
                   325                 330                 335
   Trp Gln Gly Met Val Asp Gly Trp Tyr Gly Tyr His His Ser Asn Glu
   340 345 350
   Gln Gly Ser Gly Tyr Ala Ala Asp Lys Glu Ser Thr Gln Lys Ala Ile
   355 360 365
   Asp Gly Val Thr Asn Lys Val Asn Ser Ile Ile Asp Lys Met Asn Thr
   370 375 380
   Gln Phe Glu Ala Val Gly Arg Glu Phe Asn Asn Leu Glu Lys Arg Ile
   385 390 395 400
   Glu Asn Leu Asn Lys Lys Met Glu Asp Gly Phe Leu Asp Val Trp Thr
   405 410 415
   Tyr Asn Ala Glu Leu Leu Val Leu Met Glu Asn Glu Arg Thr Leu Asp
   420 425 430
   Phe His Asp Ser Asn Val Lys Asn Leu Tyr Asp Lys Val Arg Leu Gln
   435 440 445
   Leu Arg Asp Asn Ala Lys Glu Leu Gly Asn Gly Cys Phe Glu Phe Tyr
   450 455 460
   His Arg Cys Asp Asn Glu Cys Met Glu Ser Val Arg Asn Gly Thr Tyr
   465 470 475 480
   Asp Tyr Pro Gln Tyr Ser Glu Glu Ala Arg Leu Lys Arg Glu Glu Ile
   485 490 495
   Ser Gly Val Lys Leu Glu Ser Ile Gly Thr Tyr Gln Ile Leu Ile Tyr
   500 505 510
   Ser Thr Val Ala Ser Ser Leu Ala Leu Ala Xaa
   515 520
<210> 38
   <211> 541
   <212> PRT
   <213> H5N1
<400> 38
   Leu Leu Ala Ile Val Ser Leu Val Lys Ser Asp Gln Ile Cys Ile Gly
   1 5 10 15
   Tyr His Ala Asn Asn Ser Thr Glu Gln Val Asp Thr Ile Met Glu Lys
   20 25 30
   Asn Val Thr Val Thr His Ala Gln Asp Ile Leu Glu Lys Thr His Asn
           35                  40                  45
   Gly Lys Leu Cys Asp Leu Asp Gly Val Lys Pro Leu Ile Leu Arg Asp
       50                  55                  60
   Cys Ser Val Ala Gly Trp Leu Leu Gly Asn Pro Met Cys Asp Glu Phe
   65                  70                  75                  80
   Pro Asn Val Ser Glu Trp Ser Tyr Ile Val Glu Lys Ile Asn Pro Ala
                   85                  90                  95
   Asn Asp Leu Cys Tyr Pro Gly Asn Phe Asn Asn Tyr Glu Glu Leu Lys
               100                 105                 110
   His Leu Leu Ser Arg Ile Asn Arg Phe Glu Lys Ile Lys Ile Ile Pro
           115                 120                 125
   Lys Ser Ser Trp Pro Asp His Glu Ala Ser Leu Gly Val Ser Ser Ala
       130                 135                 140
   Cys Pro Tyr Gln Gly Gly Pro Ser Phe Tyr Arg Asn Val Val Trp Leu
   145                 150                 155                 160
   Ile Lys Lys Asn Asn Thr Tyr Pro Thr Ile Lys Glu Ser Tyr His Asn
                   165                 170                 175
   Ile Asn Lys Glu Asp Leu Leu Val Leu Trp Gly Ile His His Pro Asn
               180                 185                 190
   Asp Glu Glu Glu Gln Ile Arg Ile Tyr Lys Asn Pro Thr Thr Tyr Ile
           195                 200                 205
   Ser Val Gly Thr Ser Thr Leu Asn Gln Arg Leu Val Pro Lys Ile Ala
       210                 215                 220
   Thr Arg Ser Lys Val Asn Gly Gln Ser Gly Arg Val Glu Phe Phe Trp
   225                 230                 235                 240
   Thr Ile Leu Lys Ser Asn Asp Thr Ile Asn Phe Glu Ser Asn Gly Asn
                   245                 250                 255
   Phe Ile Ala Pro Glu Asn Ala Tyr Lys Ile Val Lys Lys Gly Asp Ser
               260                 265                 270
   Thr Ile Met Lys Ser Glu Leu Glu Tyr Gly Asn Cys Asn Thr Lys Cys
           275                 280                 285
   Gln Thr Pro Ile Gly Ala Ile Asn Ser Ser Met Pro Phe His Asn Ile
       290                 295                 300
   His Pro Leu Thr Ile Gly Glu Cys Pro Lys Tyr Val Lys Ser Asn Arg
   305                 310                 315                 320
   Leu Val Leu Ala Thr Gly Leu Arg Asn Ser Pro Gln Gly Glu Gly Arg
                   325                 330                 335
   Arg Lys Lys Arg Gly Leu Phe Gly Ala Ile Ala Gly Phe Ile Glu Gly
               340                 345                 350
   Gly Trp Gln Gly Met Val Asp Gly Trp Tyr Gly Tyr His His Ser Asn
           355                 360                 365
   Glu Gln Gly Ser Gly Tyr Ala Ala Asp Lys Glu Ser Thr Gln Lys Ala
       370                 375                 380
   Ile Asp Gly Val Thr Asn Lys Val Asn Ser Ile Ile Asp Lys Met Asn
   385                 390                 395                 400
   Thr Gln Phe Glu Ala Val Gly Arg Glu Phe Asn Asn Leu Glu Lys Arg
                   405                 410                 415
   Ile Glu Asn Leu Asn Lys Lys Met Glu Asp Gly Phe Leu Asp Val Trp
               420                 425                 430
   Thr Tyr Asn Ala Glu Leu Leu Val Leu Met Glu Asn Glu Arg Thr Leu
           435                 440                 445
   Asp Phe His Asp Ser Asn Val Lys Asn Leu Tyr Asp Lys Val Arg Leu
       450                 455                 460
   Gln Leu Arg Asp Asn Ala Lys Glu Leu Gly Asn Gly Cys Phe Glu Phe
   465                 470                 475                 480
   Tyr His Arg Cys Asp Asn Glu Cys Met Glu Ser Val Arg Asn Gly Thr
                   485                 490                 495
   Tyr Asp Tyr Pro Gln Tyr Ser Glu Glu Ala Arg Leu Lys Arg Glu Glu
               500                 505                 510
   Ile Ser Gly Val Lys Leu Glu Ser Ile Gly Thr Tyr Gln Ile Leu Ser
           515                 520                 525
   Ile Tyr Ser Thr Val Ala Ser Ser Leu Ala Leu Ala Ile
   530 535 540
<210> 39
   <211> 555
   <212> PRT
   <213> H5N1
<220>
   <221> misc_feature
   <222> (1)..(1)
   <223> Xaa can be any naturally occurring amino acid
<220>
   <221> misc_feature
   <222> (555)..(555)
   <223> Xaa can be any naturally occurring amino acid
<400> 39
   Xaa Leu Ala Ile Val Ser Leu Val Lys Ser Asp Gln Ile Cys Ile Gly
   1               5                   10                  15
   Tyr His Ala Asn Asn Ser Thr Glu Gln Val Asp Thr Ile Met Glu Lys
               20                  25                  30
   Asn Val Thr Val Thr His Ala Gln Asp Ile Leu Glu Lys Thr His Asn
           35                  40                  45
   Gly Lys Leu Cys Asp Leu Asp Gly Val Lys Pro Leu Ile Leu Arg Asp
       50                  55                  60
   Cys Ser Val Ala Gly Trp Leu Leu Gly Asn Pro Met Cys Asp Glu Phe
   65                  70                  75                  80
   Pro Asn Val Ser Glu Trp Ser Tyr Ile Val Glu Lys Ile Asn Pro Ala
                   85                  90                  95
   Asn Asp Leu Cys Tyr Pro Gly Asn Phe Asn Asn Tyr Glu Glu Leu Lys
               100                 105                 110
   His Leu Leu Ser Arg Ile Asn Arg Phe Glu Lys Ile Lys Ile Ile Pro
           115                 120                 125
   Lys Ser Ser Trp Pro Asp His Glu Ala Ser Leu Gly Val Ser Ser Ala
       130                 135                 140
   Cys Pro Tyr Gln Gly Gly Pro Ser Phe Tyr Arg Asn Val Val Trp Leu
   145                 150                 155                 160
   Thr Lys Lys Asn Asn Thr Tyr Pro Thr Ile Lys Lys Ser Tyr His Asn
                   165                 170                 175
   Ile Asn Lys Glu Asp Leu Leu Val Leu Trp Gly Ile His His Pro Asn
   180 185 190
   Asp Glu Glu Glu Gln Ile Arg Ile Tyr Lys Asn Pro Thr Thr Tyr Ile
   195 200 205
   Ser Val Gly Thr Ser Thr Leu Asn Gln Arg Leu Val Pro Lys Ile Ala
   210 215 220
   Thr Arg Ser Lys Val Asn Gly Gln Ser Gly Arg Val Glu Phe Phe Trp
   225 230 235 240
   Thr Ile Leu Lys Ser Asn Asp Thr Ile Asn Phe Glu Ser Asn Gly Asn
   245 250 255
   Phe Ile Ala Pro Glu Asn Ala Tyr Lys Ile Val Lys Lys Gly Asp Ser
   260 265 270
   Thr Ile Met Lys Ser Glu Leu Glu Tyr Gly Asn Cys Asn Thr Lys Cys
   275 280 285
   Gln Thr Pro Ile Gly Ala Ile Asn Ser Ser Met Pro Phe His Asn Ile
   290 295 300
   His Pro Leu Thr Ile Gly Glu Cys Pro Lys Tyr Val Lys Ser Asn Arg
   305 310 315 320
   Leu Val Leu Ala Thr Gly Leu Arg Asn Ser Pro Gln Gly Glu Gly Arg
   325 330 335
   Arg Lys Lys Arg Gly Leu Phe Gly Ala Ile Ala Gly Phe Ile Glu Gly
   340 345 350
   Gly Trp Gln Gly Met Val Asp Gly Trp Tyr Gly Tyr His His Ser Asn
   355 360 365
   Glu Gln Gly Ser Gly Tyr Ala Ala Asp Lys Glu Ser Thr Gln Lys Ala
   370 375 380
   Ile Asp Gly Val Thr Asn Lys Val Asn Ser Ile Ile Asp Lys Met Asn
   385 390 395 400
   Thr Gln Phe Glu Ala Val Gly Arg Glu Phe Asn Asn Leu Glu Lys Arg
   405 410 415
   Ile Glu Asn Leu Asn Lys Lys Met Glu Asp Gly Phe Leu Asp Val Trp
   420 425 430
   Thr Tyr Asn Ala Glu Leu Leu Val Leu Met Glu Asn Glu Arg Thr Leu
   435 440 445
   Asp Phe His Asp Ser Asn Val Lys Asn Leu Tyr Asp Lys Val Arg Leu
   450 455 460
   Gln Leu Arg Asp Asn Ala Lys Glu Leu Gly Asn Gly Cys Phe Glu Phe
   465 470 475 480
   Tyr His Arg Cys Asp Asn Glu Cys Met Glu Ser Val Arg Asn Gly Thr
   485 490 495
   Tyr Asp Tyr Pro Gln Tyr Ser Glu Glu Ala Arg Leu Lys Arg Glu Glu
   500 505 510
   Ile Ser Gly Val Lys Leu Glu Ser Ile Gly Thr Tyr Gln Ile Leu Ser
   515 520 525
   Ile Tyr Ser Thr Val Ala Ser Ser Leu Ala Leu Ala Ile Met Met Ala
   530 535 540
   Gly Leu Phe Leu Trp Met Cys Ser Asn Gly Xaa
   545 550 555
<210> 40
   <211> 568
   <212> PRT
   <213> Artificial
<220>
   <223> consensus sequence translated into protein
<400> 40
   Met Glu Lys Ile Val Leu Leu Leu Ala Ile Val Ser Leu Val Lys Ser
   1               5                   10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
   65                  70                  75                  80
   Pro Met Cys Asp Glu Phe Pro Asn Val Ser Glu Trp Ser Tyr Ile Val
   85 90 95
   Glu Lys Ile Asn Pro Ala Asn Asp Leu Cys Tyr Pro Gly Asn Phe Asn
   100 105 110
   Asn Tyr Glu Glu Leu Lys His Leu Leu Ser Arg Ile Asn Arg Phe Glu
   115 120 125
   Lys Ile Gln Ile Ile Pro Lys Ser Ser Trp Pro Asp His Glu Ala Ser
   130 135 140
   Leu Gly Val Ser Ser Ala Cys Pro Tyr Gln Gly Gly Pro Ser Phe Tyr
   145 150 155 160
   Arg Asn Val Val Trp Leu Ile Lys Lys Asn Asn Thr Tyr Pro Thr Ile
   165 170 175
   Lys Glu Ser Tyr His Asn Thr Asn Gln Glu Asp Leu Leu Val Leu Trp
   180 185 190
   Gly Ile His His Pro Asn Asp Glu Glu Glu Gln Thr Arg Ile Tyr Lys
   195 200 205
   Asn Pro Thr Thr Tyr Ile Ser Val Gly Thr Ser Thr Leu Asn Gln Arg
   210 215 220
   Leu Val Pro Lys Ile Ala Thr Arg Ser Lys Val Asn Gly Gln Ser Gly
   225 230 235 240
   Arg Val Glu Phe Phe Trp Thr Ile Leu Lys Ser Asn Asp Thr Ile Asn
   245 250 255
   Phe Glu Ser Asn Gly Asn Phe Ile Ala Pro Glu Asn Ala Tyr Lys Ile
   260 265 270
   Val Lys Lys Gly Asp Ser Thr Ile Met Lys Ser Glu Leu Glu Tyr Gly
   275 280 285
   Asn Cys Asn Thr Lys Cys Gln Thr Pro Ile Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
                   325                 330                 335
   Pro Gln Gly Glu Gly Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Lys Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Arg Cys Asp Asn Glu Cys Met Glu
                   485                 490                 495
   Ser Val Arg Asn Gly Thr Tyr Asp Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Met Val Ala Gly Leu Phe Leu Trp Met Cys Ser Asn Gly
   545                 550                 555                 560
   Ser Leu Gln Cys Arg Ile Cys Ile
                   565
<210> 41
   <211> 1620
   <212> **DNA**
   <213> H5N1
<400> **41**
   caatagtcag tattgttaaa agtgatcaga tttgcattgg ttaccatgca aacaactcga      60
   cagagcaggt tgacacaata atggaaaaga acgtcactgt tacacacgcc caagacatac     120
   tggaaaagac acacaacggg aaactctgca atctagatgg agtgaagcct ctaattttaa     180
   gagattgtag tgtagctgga tggctcctag ggaacccaat gtgcgacgaa ttcctcaatg     240
   tgccggaatg gtcttacata gtggagaaga tcaatccaac caatgacctc tgttatccag     300
   ggaatttcaa cgactatgaa gaactgaaac acctattgag cagaataaac cattttgaga     360
   aaattcagat cattcccaaa aattcttggt cagatcatga agcctcagga gtgagctcag     420
   catgtccata ccagggaaga tcctcctttt ttagaaatgt ggtatggctt accaaaaaga     480
   acaatgcata cccaacaata aagaaaagtt acaataatac caaccaagaa gatcttttgg     540
   tattatgggg gattcaccat ccaaatgatg cggcagagca gacaaggctt tatcaaaacc     600
   caactaccta tatttccgtt gggacatcaa cactaaacca gagattggta cccaaaatag     660
   ctactagatc taaggtaaac gggcaaagtg gaaggatgga gttcttttgg acaattttaa     720
   aatcgaatga tgcaataaac tttgagagta atggaaattt cattgctcca gaaaatgcat     780
   acaaaattgt caagaaaggg gactcaacaa ttatgaaaag tgagttggaa tatggtgact     840
   gcaacaccaa gtgtcagact ccaatagggg cgataaactc cagtatgcca ttccacaaca     900
   tccaccctct caccatcggg gaatgcccca aatatgtgaa atcaaacaga ttagtccttg     960
   ctactgggct cagaaatagc cctcaaggag agagaagaag aaaaaagaga ggactatttg    1020
   gagctatagc aggttttata gagggaggat ggcagggaat ggtagatggt tggtatgggt    1080
   accaccatag caacgagcag gggagtgggt acgctgcaga caaagaatcc actcaaaagg    1140
   caatagatgg agtcaccaat aaggtcaact cgatcattga caaaatgaac actcagtttg    1200
   aggctgttgg gagggaattt aataacttag aaaggagaat agaaaattta aacaagaaga    1260
   tggaagacgg attcctagat gtctggactt ataatgctga acttctggtt ctcatggaaa    1320
   atgagagaac tctagacttt catgactcaa atgtcaagaa cctttatgac aaggtccgac    1380
   tacagcttag ggataatgca aaggagcttg gtaacggttg tttcgagttc tatcacagat    1440
   gtgataatga atgtatggaa agtgtaagaa acggtacgta tgactacccg cagtattcag    1500
   aagaagcaag attaaaaaga gaggaaataa gtggagtaaa attggagtca ataggaactt    1560
   accaaatact gtcaatttat tcaacagtgg cgagctccct agcactggca atcatggtgg    1620
<210> 42
   <211> 1707
   <212> DNA
   <213> H5N1
<400> 42
   atggagaaaa tagtgcttct tcttgcaata gtcagtcttg ttaaaagtga tcagatttgc      60
   attggttacc atgcaaacaa ctcaacagag caggttgaca caataatgga aaagaacgtc     120
   actgttacac acgctcaaga catactggaa aagacacaca atgggaaact ctgcgatcta     180
   ggtggagtga agcctctaat tttaagagat tgtagtgtag ctggatggct cctcgggaac     240
   ccaatgtgtg acgaattccc caatgtgtcg gaatggtcct acatagtgga gaagatcaat     300
   ccagccaatg acctctgtta cccagggaat ttcaacaact atgaagaact gaaacatcta     360
   ttgagcagaa taaaccggtt tgagaaaatt cagatcatcc ccaaaagttc ttggccagat     420
   catgaagcct cattaggagt gagctcagca tgtccatacc agggaggacc ctccttttat     480
   agaaatgtgg tatggcttat caaaaagaac gatacatacc caacaataaa ggaaagttac     540
   cataatacca atcaagaaga tcttttggtg ctgtggggga tccaccatcc aaataatgag     600
   gaagaacaga aaaggatcta taaaaaccca actacctatg tttccgttgg gacatcaaca     660
   ctaaaccaga gattggtacc gaagatagcc actagatcta aggtaaacgg gcaaagtgga     720
   agagtggagt tcttttggac aattttaaaa tcaaatgata caataaactt tgagagtaat     780
   ggaaatttca ttgctccaga aaatgcatac aaaattgtca agaaagggga ctcaacaatt     840
   atgaaaagtg agttggaata tggtaactgc agcaccaagt gtcaaactcc aataggggcg     900
   ataaacacca gtatgccatt ccacaacatc caccctctca ccatcgggga atgccccaaa     960
   tatgtgaaat caaacagatt agtccttgct actgggctta gaaatagccc tcaaggagag    1020
   ggaagaagaa aaaagagagg actatttgga gctatagcag gttttataga gggaggatgg    1080
   cagggaatgg tagatggttg gtatgggtac caccatagta acgagcaggg gagtgggtac    1140
   gctgcagaca aagaatccac tcaaaaggca atagatggag tcaccaataa ggtcaactcg    1200
   atcattgaca aaatgaatac tcagtttgag gctgttggga gggaatttaa taacttggaa    1260
   aagagaatag aaaatttaaa caagaagatg gaagacgggt tcctagatgt ctggacttat    1320
   aatgctgaac ttctggttct catggaaaat gagagaactc tagactttca tgactcaaat    1380
   gtcaggaacc tttacgacaa ggtgcgacta cagcttaggg acaatgcaaa ggagcttggt    1440
   aacggttgtt tcgagttcta tcacagatgc gataatgaat gtatggaaag tgtaagaaac    1500
   ggaacgtatg actacccgca gtattcagaa gaagcaagat taaaaagaga ggaaataagt    1560
   ggagtaaaat tggaatcaat aggaacttac caaatactat caatttattc aacagtggca    1620
   agttccctag cactggcaat catggtggct ggtctatttt tatggatgtg ctccaatgga    1680
   tcgttacaat gcagaatttg catttaa                                       1707
<210> 43
   <211> 1707
   <212> DNA
   <213> Artificial
<220>
   <223> consensus of 38 H5 gene sequences
<400> 43
   atggagaaaa tagtgcttct tcttgcaata gtcagtcttg ttaaaagtga tcagatttgc      60
   attggttacc atgcaaacaa ctcaacagag caggttgaca caataatgga aaagaacgtc     120
   actgttacac acgctcaaga catactggaa aagacacaca acgggaaact ctgcgatcta     180
   gatggagtga agcctctaat tttaagagat tgtagtgtag ctggatggct cctcgggaac     240
   ccaatgtgtg acgaattccc caatgtgtcg gaatggtcct acatagtgga gaagatcaat     300
   ccagccaatg acctctgtta cccagggaat ttcaacaact atgaagaact gaaacaccta     360
   ttgagcagaa taaaccggtt tgagaaaatt cagatcatcc ccaaaagttc ttggccagat     420
   catgaagcct cattaggagt gagctcagca tgtccatacc agggaggacc ctccttttat     480
   agaaatgtgg tatggcttat caaaaagaac aatacatacc caacaataaa ggaaagttac     540
   cataatacca atcaagaaga tcttttggtg ctgtggggga ttcaccatcc aaatgatgag     600
   gaagagcaga caaggatcta taaaaaccca actacctata tttccgttgg gacatcaaca     660
   ctaaaccaga gattggtacc aaagatagcc actagatcta aggtaaacgg gcaaagtgga     720
   agagtggagt tcttttggac aattttaaaa tcaaatgata caataaactt tgagagtaat     780
   ggaaatttca ttgctccaga aaatgcatac aaaattgtca agaaagggga ctcaacaatt     840
   atgaaaagtg agttggaata tggtaactgc aacaccaagt gtcaaactcc aataggggcg     900
   ataaactcca gtatgccatt ccacaacatc caccctctca ccatcgggga atgccccaaa     960
   tatgtgaaat caaacagatt agtccttgct actgggctca gaaatagccc tcaaggagag    1020
   ggaagaagaa aaaagagagg actatttgga gctatagcag gttttataga gggaggatgg    1080
   cagggaatgg tagatggttg gtatgggtac caccatagca acgagcaggg gagtgggtac    1140
   gctgcagaca aagaatccac tcaaaaggca atagatggag tcaccaataa ggtcaactcg    1200
   atcattgaca aaatgaatac tcagtttgag gctgttggga gggaatttaa taacttggaa    1260
   aagagaatag aaaatttaaa caagaagatg gaagacgggt tcctagatgt ctggacttat    1320
   aatgctgaac ttctggttct catggaaaat gagagaactc tagactttca tgactcaaat    1380
   gtcaagaacc tttacgacaa ggtgcgacta cagcttaggg acaatgcaaa ggagcttggt    1440
   aacggttgtt tcgagttcta tcacagatgc gataatgaat gtatggaaag tgtaagaaac    1500
   ggaacgtatg actacccgca gtattcagaa gaagcaagat taaaaagaga ggaaataagt    1560
   ggagtaaaat tggaatcaat aggaacttac caaatactat caatttattc aacagtggca    1620
   agttccctag cactggcaat catggtggct ggtctatttt tatggatgtg ctccaatgga 1680
   tcgttacaat gcagaatttg catttaa 1707
<210> 44
   <211> 16
   <212> PRT
   <213> H5N1
<400> 44
   Met Glu Lys Ile Val Leu Leu Leu Ala Ile Val Ser Leu Val Lys Ser
   1 5 10 15
<210> 45
   <211> 532
   <212> PRT
   <213> H5N1
<400> 45
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Thr Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asp
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val
   530
<210> 46
   <211> 552
   <212> PRT
   <213> H5N1
<400> 46
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Gly Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asn Glu Glu Glu Gln Lys Arg Ile Tyr Lys
               180                 185                 190
   Asn Pro Thr Thr Tyr Val Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 47
   <211> 552
   <212> PRT
   <213> H5N1
<400> 47
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 48
   <211> 42
   <212> PRT
   <213> H5N1
<400> 48
   Ile Gly Thr Tyr Gln Ile Leu Ser Ile Tyr Ser Thr Val Ala Ser Ser
   1 5 10 15
   Leu Ala Leu Ala Ile Met Val Ala Gly Leu Phe Leu Trp Met Cys Ser
   20 25 30
   Asn Gly Ser Leu Gln Cys Arg Ile Cys Ile
   35 40
<210> 49
   <211> 4
   <212> PRT
   <213> H5N1
<400> 49
   Ile Met Val Ala
   1
<210> 50
   <211> 4
   <212> PRT
   <213> H5N1
<400> 50
   Ile Met Met Ala
   1
<210> 51
   <211> 42
   <212> PRT
   <213> H5N1
<400> 51
   Ile Gly Thr Tyr Gln Ile Leu Ser Ile Tyr Ser Thr Val Ala Ser Ser
   1 5 10 15
   Leu Ala Leu Ala Ile Met Val Ala Gly Leu Phe Leu Trp Met Cys Ser
   20 25 30
   Asn Gly Ser Leu Gln Cys Arg Ile Cys Ile
   35 40
<210> 52
   <211> 42
   <212> PRT
   <213> H5N1
<400> 52
   Ile Gly Thr Tyr Gln Ile Leu Ser Ile Tyr Ser Thr Val Ala Ser Ser
   1 5 10 15
   Leu Ala Leu Ala Ile Met Val Ala Gly Leu Ser Leu Trp Met Cys Ser
   20 25 30
   Asn Gly Ser Leu Gln Cys Arg Ile Cys Ile
   35 40
<210> 53
   <211> 42
   <212> PRT
   <213> H5N1
<400> 53
   Ile Gly Thr Tyr Gln Ile Leu Ser Ile Tyr Ser Thr Val Ala Ser Ser
   1 5 10 15
   Leu Ala Leu Ala Ile Met Met Ala Gly Leu Ser Leu Trp Met Cys Ser
   20 25 30
   Asn Gly Ser Leu Gln Cys Arg Ile Cys Ile
   35 40
<210> 54
   <211> 4
   <212> PRT
   <213> H5N1
<400> 54
   Ala Ser Gly Val
   1
<210> 55
   <211> 551
   <212> PRT
   <213> H5N1
<400> 55
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Lys Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Arg Asn Ser Trp Ser Asp His Glu Thr Ser
           115                 120                 125
   Gly Val Ser Ser Ala Cys Gln Tyr Gln Gly Arg Ser Ser Phe Phe Arg
       130                 135                 140
   Asn Val Val Trp Leu Thr Lys Lys Asp Asn Ala Tyr Ser Thr Ile Lys
   145                 150                 155                 160
   Arg Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Glu Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 56
   <211> 551
   <212> PRT
   <213> H5N1
<400> 56
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Gly Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Arg Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 57
   <211> 544
   <212> PRT
   <213> H5N1
<220>
   <221> misc_feature
   <222> (544)..(544)
   <223> Xaa can be any naturally occurring amino acid
<400> 57
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Tyr Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Ser Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asp Asn Ala Tyr Pro Thr Ile Lys
   145 150 155 160
   Lys Ser Tyr Asn Asn Thr Asn Gln Glu Asp Leu Leu Ile Leu Trp Gly
   165 170 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Met Val Ala Gly Leu Phe Leu Trp Met Cys Ser Asn Gly Xaa
       530                 535                 540
<210> 58
   <211> 533
   <212> PRT
   <213> H5N1
<220>
   <221> misc_feature
   <222> (533)..(533)
   <223> Xaa can be any naturally occurring amino acid
<400> 58
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asp Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Thr Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Thr Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   His Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Xaa
   530
<210> 59
   <211> 530
   <212> PRT
   <213> H5N1
<400> 59
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Glu Trp Ser Tyr Ile Val Glu
   65 70 75 80
   Lys Ile Asn Pro Thr Asn Asp Leu Cys Tyr Pro Gly Asn Phe Asn Asp
   85 90 95
   Tyr Glu Glu Leu Lys His Leu Leu Ser Arg Ile Asn His Phe Glu Lys
   100 105 110
   Ile Gln Ile Ile Pro Lys Asn Tyr Trp Ser Asp His Glu Thr Ser Gly
   115 120 125
   Val Ser Ser Ala Cys Pro Tyr Gln Gly Arg Pro Ser Phe Phe Arg Asn
   130 135 140
   Val Val Trp Leu Thr Lys Lys Asn Asn Ala Tyr Pro Thr Ile Lys Lys
   145 150 155 160
   Ser Tyr Asn Asn Thr Asn Gln Glu Asp Leu Leu Val Leu Trp Gly Ile
   165 170 175
   His His Pro Asn Asp Ala Ala Glu Gln Thr Arg Leu Tyr Gln Asn Pro
   180 185 190
   Thr Thr Tyr Ile Ser Val Gly Thr Ser Thr Leu Asn Gln Arg Leu Val
   195 200 205
   Pro Lys Ile Ala Thr Arg Ser Lys Val Asn Gly Gln Ser Gly Arg Met
   210 215 220
   Glu Phe Phe Trp Thr Ile Leu Lys Ser Asn Asp Ala Ile Asn Phe Glu
   225 230 235 240
   Ser Asn Gly Asn Phe Ile Ala Pro Glu Asn Ala Tyr Lys Ile Val Lys
   245 250 255
   Lys Gly Asp Ser Thr Ile Met Lys Ser Glu Leu Glu Tyr Gly Asn Cys
   260 265 270
   Asn Thr Lys Cys Gln Thr Pro Ile Gly Ala Ile Asn Ser Ser Met Pro
   275 280 285
   Phe His Asn Ile His Pro Leu Thr Ile Gly Glu Cys Pro Lys Tyr Val
   290 295 300
   Lys Ser Asn Arg Leu Val Leu Ala Thr Gly Leu Arg Asn Ser Pro Gln
   305 310 315 320
   Gly Glu Arg Arg Arg Lys Lys Arg Gly Leu Phe Gly Ala Ile Ala Gly
   325 330 335
   Phe Ile Glu Gly Gly Trp Gln Gly Met Val Asp Gly Trp Tyr Gly Tyr
   340 345 350
   His His Ser Asn Glu Gln Gly Ser Gly Tyr Ala Ala Asp Lys Glu Ser
   355 360 365
   Thr Gln Lys Ala Ile Asp Gly Val Thr Asn Lys Val Asn Ser Ile Ile
   370 375 380
   Asp Lys Met Asn Thr Gln Phe Glu Ala Val Gly Arg Glu Phe Asn Asn
   385 390 395 400
   Leu Glu Arg Arg Ile Glu Asn Leu Asn Lys Lys Met Glu Asp Gly Phe
                   405                 410                 415
   Leu Asp Val Trp Thr Tyr Asn Ala Glu Leu Leu Val Leu Met Glu Asn
               420                 425                 430
   Glu Arg Thr Leu Asp Phe His Asp Ser Asn Val Lys Asn Leu Tyr Asp
           435                 440                 445
   Lys Val Arg Leu Gln Leu Arg Asp Asn Ala Lys Glu Leu Gly Asn Gly
       450                 455                 460
   Cys Phe Glu Phe Tyr His Arg Cys Asp Asn Glu Cys Met Glu Ser Val
   465                 470                 475                 480
   Arg Asn Gly Thr Tyr Asp Tyr Pro Gln Tyr Ser Glu Glu Ala Arg Leu
                   485                 490                 495
   Lys Arg Glu Glu Ile Ser Gly Val Lys Leu Glu Ser Ile Gly Thr Tyr
               500                 505                 510
   Gln Ile Leu Ser Ile Tyr Ser Thr Val Ala Ser Ser Leu Ala Leu Ala
           515                 520                 525
   Ile Met
       530
<210> 60
   <211> 544
   <212> PRT
   <213> H5N1
<220>
   <221> misc_feature
   <222> (544)..(544)
   <223> Xaa can be any naturally occurring amino acid
<400> 60
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
   50 55 60
   Pro Met Cys Asp Lys Phe Leu Asn Val Pro Glu Trp Ser Tyr Ile Val
   65 70 75 80
   Glu Lys Ile Asn Pro Thr Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
   165 170 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asp
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Xaa
   530 535 540
<210> 61
   <211> 533
   <212> PRT
   <213> H5N1
<220>
   <221> misc_feature
   <222> (533)..(533)
   <223> Xaa can be any naturally occurring amino acid
<400> 61
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Thr Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asp
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Xaa
   530
<210> 62
   <211> 516
   <212> PRT
   <213> H5N1
<220>
   <221> misc_feature
   <222> (155)..(155)
   <223> Xaa can be any naturally occurring amino acid
<400> 62
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Xaa Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Lys Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu
   515
<210> 63
   <211> 531
   <212> PRT
   <213> H5N1
<220>
   <221> misc_feature
   <222> (531)..(531)
   <223> Xaa can be any naturally occurring amino acid
<400> 63
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Glu Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Xaa
   530
<210> 64
   <211> 520
   <212> PRT
   <213> H5N1
<220>
   <221> misc_feature
   <222> (520)..(520)
   <223> Xaa can be any naturally occurring amino acid
<400> 64
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Asn Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Xaa
   515 520
<210> 65
   <211> 531
   <212> PRT
   <213> H5N1
<220>
   <221> misc_feature
   <222> (531)..(531)
   <223> Xaa can be any naturally occurring amino acid
<400> 65
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
       130                 135                 140
   Asn Val Val Trp Leu Thr Lys Lys Asp Asn Ala Tyr Pro Thr Ile Lys
   145                 150                 155                 160
   Arg Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Xaa
   530
<210> 66
   <211> 552
   <212> PRT
   <213> H5N1
<400> 66
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Gly Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asn Glu Glu Glu Gln Lys Arg Ile Tyr Lys
               180                 185                 190
   Asn Pro Thr Thr Tyr Val Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 67
   <211> 552
   <212> PRT
   <213> H5N1
<220>
   <221> misc_feature
   <222> (359)..(359)
   <223> Xaa can be any naturally occurring amino acid
<400> 67
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Thr Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Val Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Xaa Gln Gly Ser Gly Tyr Ala Ala Asp Arg
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Ile Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Ile Val Ala Gly Leu Phe Leu Trp Met Cys Ser Asn Gly
   530 535 540
   Ser Leu Gln Cys Arg Ile Cys Ile
   545 550
<210> 68
   <211> 552
   <212> PRT
   <213> H5N1
<400> 68
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Arg Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Met Ala Gly Leu Phe Leu Trp Met Cys Ser Asn Gly
       530                 535                 540
   Ser Leu Gln Cys Arg Ile Cys Ile
   545                 550
<210> 69
   <211> 552
   <212> PRT
   <213> H5N1
<400> 69
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Arg Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Met Ala Gly Leu Phe Leu Trp Met Cys Ser Asn Gly
       530                 535                 540
   Ser Leu Gln Cys Arg Ile Cys Ile
   545 550
<210> 70
   <211> 552
   <212> PRT
   <213> H5N1
<400> 70
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Thr Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Ile Cys Ile
   545 550
<210> 71
   <211> 540
   <212> PRT
   <213> H5N1
<220>
   <221> misc_feature
   <222> (540)..(540)
   <223> Xaa can be any naturally occurring amino acid
<400> 71
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Thr Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Arg Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Ile Gly Thr Ser Thr Leu Asn Gln Arg
   195 200 205
   Leu Ile Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Xaa
   530 535 540
<210> 72
   <211> 543
   <212> PRT
   <213> H5N1
<220>
   <221> misc_feature
   <222> (543)..(543)
   <223> Xaa can be any naturally occurring amino acid
<400> 72
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145 150 155 160
   Lys Glu Asn Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Pro Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Lys Asn Ala Tyr Lys Ile
   245 250 255
   Val Lys Lys Gly Ser Ser Thr Ile Met Lys Ser Glu Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Xaa
   530 535 540
<210> 73
   <211> 552
   <212> PRT
   <213> H5N1
<400> 73
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145 150 155 160
   Lys Glu Asn Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Ser Leu Trp Met Cys Ser Asn Gly
       530                 535                 540
   Ser Leu Gln Cys Arg Ile Cys Ile
   545                 550
<210> 74
   <211> 540
   <212> PRT
   <213> H5N1
<400> 74
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Gly Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met
   530 535 540
<210> 75
   <211> 545
   <212> PRT
   <213> H5N1
<220>
   <221> misc_feature
   <222> (545)..(545)
   <223> Xaa can be any naturally occurring amino acid
<400> 75
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu His Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Asn Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Xaa
   545
<210> 76
   <211> 547
   <212> PRT
   <213> H5N1
<220>
   <221> misc_feature
   <222> (120)..(120)
   <223> Xaa can be any naturally occurring amino acid
<400> 76
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Xaa Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Ile Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Gly Ser Leu Ala
   515 520 525
   Leu Ala Ile Met Met Ala Gly Leu Phe Leu Trp Met Cys Ser Asn Gly
   530 535 540
   Ser Leu Gln
   545
<210> 77
   <211> 534
   <212> PRT
   <213> H5N1
<220>
   <221> misc_feature
   <222> (534)..(534)
   <223> Xaa can be any naturally occurring amino acid
<400> 77
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Glu Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Xaa
       530
<210> 78
   <211> 534
   <212> PRT
   <213> H5N1
<220>
   <221> misc_feature
   <222> (141)..(141)
   <223> Xaa can be any naturally occurring amino acid
<220>
   <221> misc_feature
   <222> (316)..(316)
   <223> Xaa can be any naturally occurring amino acid
<220>
   <221> misc_feature
   <222> (534)..(534)
   <223> Xaa can be any naturally occurring amino acid
<400> 78
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Xaa Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
   180 185 190
   Asn Pro Thr Thr Tyr Ile Ser Ile Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Xaa Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
                   420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Gly Ser Leu Ala
           515                 520                 525
   Leu Ala Ile Met Val Xaa
       530
<210> 79
   <211> 547
   <212> PRT
   <213> H5N1
<400> 79
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145 150 155 160
   Lys Glu Asn Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Ile Ser Val Gly Thr Ser Thr Leu Thr Gln Arg
   195 200 205
   Leu Val Pro Lys Ile Ala Thr Arg Pro Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Lys Asn Ala Tyr Lys Ile
   245 250 255
   Val Lys Lys Gly Ser Ser Thr Ile Met Lys Ser Glu Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Asn Gly
   530 535 540
   Ser Leu Gln
   545
<210> 80
   <211> 550
   <212> PRT
   <213> H5N1
<220>
   <221> misc_feature
   <222> (550)..(550)
   <223> Xaa can be any naturally occurring amino acid
<400> 80
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
   245 250 255
   Val Lys Lys Gly Ser Ser Thr Ile Met Lys Ser Glu Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Ser Leu Trp Met Cys Ser Asn Gly
       530                 535                 540
   Ser Leu Gln Cys Arg Xaa
   545                 550
<210> 81
   <211> 535
   <212> PRT
   <213> H5N1
<220>
   <221> misc_feature
   <222> (535)..(535)
   <223> Xaa can be any naturally occurring amino acid
<400> 81
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Xaa
   530 535
<210> 82
   <211> 552
   <212> PRT
   <213> H5N1
<400> 82
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Asn Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Glu Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 83
   <211> 517
   <212> PRT
   <213> H5N1
<220>
   <221> misc_feature
   <222> (7)..(7)
   <223> Xaa can be any naturally occurring amino acid
<220>
   <221> misc_feature
   <222> (188)..(188)
   <223> Xaa can be any naturally occurring amino acid
<220>
   <221> misc_feature
   <222> (206)..(206)
   <223> Xaa can be any naturally occurring amino acid
<220>
   <221> misc_feature
   <222> (517)..(517)
   <223> Xaa can be any naturally occurring amino acid
<400> 83
   Asp Gln Ile Cys Ile Gly Xaa His Ala Asn Asn Ser Thr Glu Gln Val
   1               5                   10                  15
   Asp Thr Ile Met Glu Lys Asn Ile Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Ser Lys Asn Ser Trp Pro Asp His Glu Ala Ser
           115                 120                 125
   Leu Gly Val Ser Ala Ala Cys Pro Tyr Gln Gly Gly Leu Ser Phe Tyr
       130                 135                 140
   Arg Asn Val Val Trp Leu Ile Glu Lys Asn Asn Thr Tyr Pro Leu Ile
   145                 150                 155                 160
   Lys Lys Asn Tyr His Asn Thr Asn Gln Glu Asp Leu Leu Val Leu Trp
                   165                 170                 175
   Gly Ile His His Pro Asn Asp Glu Ala Glu Gln Xaa Arg Leu Tyr Lys
               180                 185                 190
   Asn Ser Thr Thr Tyr Ile Ser Val Gly Thr Ser Thr Leu Xaa Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Val Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Asp Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Arg Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Arg Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Ala Lys Leu Glu Ser Ile Gly
   500 505 510
   Thr Tyr Gln Ile Xaa
   515
<210> 84
   <211> 532
   <212> PRT
   <213> H5N1
<400> 84
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Arg Gly Pro Ser Phe Tyr
   130 135 140
   Arg Asn Val Val Trp Leu Ile Lys Lys Asn Asn Thr Tyr Pro Thr Ile
   145 150 155 160
   Lys Lys Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Lys Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met
       530
<210> 85
   <211> 532
   <212> PRT
   <213> H5N1
<400> 85
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asp Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Ser Thr Lys Cys Gln Thr Pro Ile Gly Ala Ile Asn Thr Ser
   275 280 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asn Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met
       530
<210> 86
   <211> 532
   <212> PRT
   <213> H5N1
<220>
   <221> misc_feature
   <222> (307)..(307)
   <223> Xaa can be any naturally occurring amino acid
<400> 86
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Thr Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Val Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Xaa Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Arg
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Ile Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met
   530
<210> 87
   <211> 510
   <212> PRT
   <213> H5N1
<220>
   <221> misc_feature
   <222> (510)..(510)
   <223> Xaa can be any naturally occurring amino acid
<400> 87
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Val Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Ile Gly Ala Ile Asn Thr Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
               355                 360                 365
   Glu Ser Thr Gln Lys Ala Ile Asp Gly Val Thr Asn Lys Val Asn Ser
           370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Xaa
               500                 505                 510
<210> 88
   <211> 515
   <212> PRT
   <213> H5N1
<220>
   <221> misc_feature
   <222> (513)..(513)
   <223> Xaa can be any naturally occurring amino acid
<400> 88
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
   50 55 60
   Pro Met Cys Asp Lys Phe Leu Asn Val Pro Glu Trp Ser Tyr Ile Val
   65 70 75 80
   Glu Lys Ile Asn Pro Ala Asn Asp Leu Cys Tyr Pro Gly Asp Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
   165 170 175
   Ile His His Pro Asn Asp Ala Ala Glu Gln Thr Met Leu Tyr Gln Asn
   180 185 190
   Pro Thr Thr Tyr Val Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Xaa Gln Ile
           515
<210> 89
   <211> 516
   <212> PRT
   <213> H5N1
<220>
   <221> misc_feature
   <222> (7)..(7)
   <223> Xaa can be any naturally occurring amino acid
<220>
   <221> misc_feature
   <222> (513)..(513)
   <223> Xaa can be any naturally occurring amino acid
<220>
   <221> misc_feature
   <222> (516)..(516)
   <223> Xaa can be any naturally occurring amino acid
<400> 89
   Asp Gln Ile Cys Ile Gly Xaa His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Leu Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Arg Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Xaa Gln Ile Xaa
   515
<210> 90
   <211> 530
   <212> PRT
   <213> H5N1
<220>
   <221> misc_feature
   <222> (530)..(530)
   <223> Xaa can be any naturally occurring amino acid
<400> 90
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Lys Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ile Tyr Ser Thr Val Ala Ser Ser Leu Ala Leu
           515                 520                 525
   Ala Xaa
       530
<210> 91
   <211> 531
   <212> PRT
   <213> H5N1
<400> 91
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Ile Asn Lys Glu Asp Leu Leu Val Leu Trp
                   165                 170                 175
   Gly Ile His His Pro Asn Asp Glu Glu Glu Gln Ile Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile
       530
<210> 92
   <211> 545
   <212> PRT
   <213> H5N1
<220>
   <221> misc_feature
   <222> (545)..(545)
   <223> Xaa can be any naturally occurring amino acid
<400> 92
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Thr Lys Lys Asn Asn Thr Tyr Pro Thr Ile
   145                 150                 155                 160
   Lys Lys Ser Tyr His Asn Ile Asn Lys Glu Asp Leu Leu Val Leu Trp
                   165                 170                 175
   Gly Ile His His Pro Asn Asp Glu Glu Glu Gln Ile Arg Ile Tyr Lys
               180                 185                 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Xaa
   545
<210> 93
   <211> 551
   <212> PRT
   <213> H5N1
<400> 93
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Ala Asn Asp Leu Cys Tyr Pro Gly Lys Phe Asn
   85 90 95
   Asp Tyr Glu Glu Leu Lys His Leu Leu Ser Arg Ile Asn His Phe Glu
   100 105 110
   Lys Ile Gln Ile Ile Pro Arg Asn Ser Trp Ser Asp His Glu Thr Ser
           115                 120                 125
   Gly Val Ser Ser Ala Cys Gln Tyr Gln Gly Arg Ser Ser Phe Phe Arg
       130                 135                 140
   Asn Val Val Trp Leu Thr Lys Lys Asp Asn Ala Tyr Ser Thr Ile Lys
   145                 150                 155                 160
   Arg Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Glu Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Met Val Ala Gly Leu Phe Leu Trp Met Cys Ser Asn Gly Ser
       530                 535                 540
   Leu Gln Cys Arg Ile Cys Ile
   545                 550
<210> 94
   <211> 551
   <212> PRT
   <213> H5N1
<400> 94
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Gly Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Arg Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 95
   <211> 551
   <212> PRT
   <213> H5N1
<220>
   <221> misc_feature
   <222> (544)..(544)
   <223> Xaa can be any naturally occurring amino acid
<400> 95
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Tyr Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Ser Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asp Asn Ala Tyr Pro Thr Ile Lys
   145                 150                 155                 160
   Lys Ser Tyr Asn Asn Thr Asn Gln Glu Asp Leu Leu Ile Leu Trp Gly
                   165                 170                 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Xaa
   530 535 540
   Leu Gln Cys Arg Ile Cys Ile
   545 550
<210> 96
   <211> 551
   <212> PRT
   <213> H5N1
<220>
   <221> misc_feature
   <222> (533)..(533)
   <223> Xaa can be any naturally occurring amino acid
<400> 96
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asp Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Thr Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Thr Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   His Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Xaa Gly Leu Phe Leu Trp Met Cys Ser Asn Gly Ser
   530 535 540
   Leu Gln Cys Arg Ile Cys Ile
   545 550
<210> 97
   <211> 550
   <212> PRT
   <213> H5N1
<400> 97
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Glu Trp Ser Tyr Ile Val Glu
   65                  70                  75                  80
   Lys Ile Asn Pro Thr Asn Asp Leu Cys Tyr Pro Gly Asn Phe Asn Asp
                   85                  90                  95
   Tyr Glu Glu Leu Lys His Leu Leu Ser Arg Ile Asn His Phe Glu Lys
               100                 105                 110
   Ile Gln Ile Ile Pro Lys Asn Tyr Trp Ser Asp His Glu Thr Ser Gly
           115                 120                 125
   Val Ser Ser Ala Cys Pro Tyr Gln Gly Arg Pro Ser Phe Phe Arg Asn
       130                 135                 140
   Val Val Trp Leu Thr Lys Lys Asn Asn Ala Tyr Pro Thr Ile Lys Lys
   145                 150                 155                 160
   Ser Tyr Asn Asn Thr Asn Gln Glu Asp Leu Leu Val Leu Trp Gly Ile
                   165                 170                 175
   His His Pro Asn Asp Ala Ala Glu Gln Thr Arg Leu Tyr Gln Asn Pro
               180                 185                 190
   Thr Thr Tyr Ile Ser Val Gly Thr Ser Thr Leu Asn Gln Arg Leu Val
           195                 200                 205
   Pro Lys Ile Ala Thr Arg Ser Lys Val Asn Gly Gln Ser Gly Arg Met
       210                 215                 220
   Glu Phe Phe Trp Thr Ile Leu Lys Ser Asn Asp Ala Ile Asn Phe Glu
   225 230 235 240
   Ser Asn Gly Asn Phe Ile Ala Pro Glu Asn Ala Tyr Lys Ile Val Lys
   245 250 255
   Lys Gly Asp Ser Thr Ile Met Lys Ser Glu Leu Glu Tyr Gly Asn Cys
   260 265 270
   Asn Thr Lys Cys Gln Thr Pro Ile Gly Ala Ile Asn Ser Ser Met Pro
   275 280 285
   Phe His Asn Ile His Pro Leu Thr Ile Gly Glu Cys Pro Lys Tyr Val
   290 295 300
   Lys Ser Asn Arg Leu Val Leu Ala Thr Gly Leu Arg Asn Ser Pro Gln
   305 310 315 320
   Gly Glu Arg Arg Arg Lys Lys Arg Gly Leu Phe Gly Ala Ile Ala Gly
   325 330 335
   Phe Ile Glu Gly Gly Trp Gln Gly Met Val Asp Gly Trp Tyr Gly Tyr
   340 345 350
   His His Ser Asn Glu Gln Gly Ser Gly Tyr Ala Ala Asp Lys Glu Ser
   355 360 365
   Thr Gln Lys Ala Ile Asp Gly Val Thr Asn Lys Val Asn Ser Ile Ile
   370 375 380
   Asp Lys Met Asn Thr Gln Phe Glu Ala Val Gly Arg Glu Phe Asn Asn
   385 390 395 400
   Leu Glu Arg Arg Ile Glu Asn Leu Asn Lys Lys Met Glu Asp Gly Phe
   405 410 415
   Leu Asp Val Trp Thr Tyr Asn Ala Glu Leu Leu Val Leu Met Glu Asn
   420 425 430
   Glu Arg Thr Leu Asp Phe His Asp Ser Asn Val Lys Asn Leu Tyr Asp
   435 440 445
   Lys Val Arg Leu Gln Leu Arg Asp Asn Ala Lys Glu Leu Gly Asn Gly
   450 455 460
   Cys Phe Glu Phe Tyr His Arg Cys Asp Asn Glu Cys Met Glu Ser Val
   465 470 475 480
   Arg Asn Gly Thr Tyr Asp Tyr Pro Gln Tyr Ser Glu Glu Ala Arg Leu
   485 490 495
   Lys Arg Glu Glu Ile Ser Gly Val Lys Leu Glu Ser Ile Gly Thr Tyr
   500 505 510
   Gln Ile Leu Ser Ile Tyr Ser Thr Val Ala Ser Ser Leu Ala Leu Ala
   515 520 525
   Ile Met Val Ala Gly Leu Phe Leu Trp Met Cys Ser Asn Gly Ser Leu
   530 535 540
   Gln Cys Arg Ile Cys Ile
   545 550
<210> 98
   <211> 551
   <212> PRT
   <213> H5N1
<220>
   <221> misc_feature
   <222> (544)..(544)
   <223> Xaa can be any naturally occurring amino acid
<400> 98
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Lys Phe Leu Asn Val Pro Glu Trp Ser Tyr Ile Val
   65                  70                  75                  80
   Glu Lys Ile Asn Pro Thr Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
   165 170 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asp
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Xaa
   530 535 540
   Leu Gln Cys Arg Ile Cys Ile
   545 550
<210> 99
   <211> 551
   <212> PRT
   <213> H5N1
<220>
   <221> misc_feature
   <222> (533)..(533)
   <223> Xaa can be any naturally occurring amino acid
<400> 99
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Thr Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asp
               260                 265                 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Xaa Gly Leu Phe Leu Trp Met Cys Ser Asn Gly Ser
   530 535 540
   Leu Gln Cys Arg Ile Cys Ile
   545 550
<210> 100
   <211> 551
   <212> PRT
   <213> H5N1
<220>
   <221> misc_feature
   <222> (155)..(155)
   <223> Xaa can be any naturally occurring amino acid
<400> 100
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Xaa Tyr Pro Thr Ile Lys
   145 150 155 160
   Lys Ser Tyr Asn Asn Thr Asn Gln Glu Asp Leu Leu Val Leu Trp Gly
   165 170 175
   Ile His His Pro Asn Asp Ala Ala Glu Gln Thr Lys Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
   340 345 350
   Tyr His His Ser Asn Glu Gln Gly Ser Gly Tyr Ala Ala Asp Lys Glu
   355 360 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 101
   <211> 551
   <212> PRT
   <213> H5N1
<220>
   <221> misc_feature
   <222> (531)..(531)
   <223> Xaa can be any naturally occurring amino acid
<400> 101
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Glu Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile Gln Gly Met Val Asp Gly Trp Tyr Gly Tyr His
       290                 295                 300
   His Ser Asn Glu Gln Gly Ser Gly Tyr Ala Ala Asp Lys Glu Ser Thr
   305 310 315 320
   Gln Lys Ala Ile Asp Gly Val Thr Asn Lys Val Asn Ser Ile Ile Asp
   325 330 335
   Lys Met Asn Thr Gln Phe Glu Ala Val Gly Arg Glu Phe Asn Asn Leu
   340 345 350
   Glu His Pro Leu Thr Ile Gly Glu Cys Pro Lys Tyr Val Lys Ser Asn
   355 360 365
   Arg Leu Val Leu Ala Thr Gly Leu Arg Asn Ser Pro Gln Gly Glu Arg
   370 375 380
   Arg Arg Lys Lys Arg Gly Leu Phe Gly Ala Ile Ala Gly Phe Ile Glu
   385 390 395 400
   Gly Gly Trp Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Ser Thr Val Ala Ser Ser Leu Ala Leu
   515 520 525
   Ala Ile Xaa Val Ala Gly Leu Phe Leu Trp Met Cys Ser Asn Gly Ser
   530 535 540
   Leu Gln Cys Arg Ile Cys Ile
   545 550
<210> 102
   <211> 551
   <212> PRT
   <213> H5N1
<220>
   <221> misc_feature
   <222> (520)..(520)
   <223> Xaa can be any naturally occurring amino acid
<400> 102
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asn Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Pro Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Asn Ser Trp Ser Asp His Glu Ala Ser
           115                 120                 125
   Gly Val Ser Ser Ala Cys Pro Tyr Gln Gly Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Asn Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Tyr Gln Ile Leu Ser Ile Tyr Xaa Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 103
   <211> 551
   <212> PRT
   <213> H5N1
<220>
   <221> misc_feature
   <222> (531)..(531)
   <223> Xaa can be any naturally occurring amino acid
<400> 103
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Leu Asn Val Pro Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asp Asn Ala Tyr Pro Thr Ile Lys
   145 150 155 160
   Arg Ser Tyr Asn Asn Thr Asn Gln Glu Asp Leu Leu Val Leu Trp Gly
   165 170 175
   Ile His His Pro Asn Asp Ala Ala Glu Gln Thr Arg Leu Tyr Gln Asn
   180 185 190
   Pro Thr Thr Tyr Ile Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
   260 265 270
   Cys Asn Thr Lys Cys Gln Thr Pro Ile Gly Ala Ile Asn Ser Ser Met
   275 280 285
   Pro Phe His Asn Ile His Pro Leu Thr Ile Gly Glu Cys Pro Lys Tyr
   290 295 300
   Val Lys Ser Asn Arg Leu Val Leu Ala Thr Gly Leu Arg Asn Ser Pro
   305 310 315 320
   Gln Gly Glu Arg Arg Arg Lys Lys Arg Gly Leu Phe Gly Ala Ile Ala
   325 330 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile Leu Ser Ile Tyr Ser Thr Val Ala Ser Ser Leu Ala Leu
           515                 520                 525
   Ala Ile Xaa Val Ala Gly Leu Phe Leu Trp Met Cys Ser Asn Gly Ser
       530                 535                 540
   Leu Gln Cys Arg Ile Cys Ile
   545                 550
<210> 104
   <211> 552
   <212> PRT
   <213> H5N1
<400> 104
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Gly Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asp Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asn Glu Glu Glu Gln Lys Arg Ile Tyr Lys
               180                 185                 190
   Asn Pro Thr Thr Tyr Val Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
   260 265 270
   Asn Cys Ser Thr Lys Cys Gln Thr Pro Ile Gly Ala Ile Asn Thr Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 105
   <211> 552
   <212> PRT
   <213> H5N1
<220>
   <221> misc_feature
   <222> (359)..(359)
   <223> Xaa can be any naturally occurring amino acid
<400> 105
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Thr Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Val Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Xaa Gln Gly Ser Gly Tyr Ala Ala Asp Arg
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Ile Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Ile Val Ala Gly Leu Phe Leu Trp Met Cys Ser Asn Gly
       530                 535                 540
   Ser Leu Gln Cys Arg Ile Cys Ile
   545                 550
<210> 106
   <211> 552
   <212> PRT
   <213> H5N1
<400> 106
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Arg Gly Pro Ser Phe Tyr
   130 135 140
   Arg Asn Val Val Trp Leu Ile Lys Lys Asn Asn Thr Tyr Pro Thr Ile
   145 150 155 160
   Lys Lys Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Ser Leu Gln Cys Arg Ile Cys Ile
   545 550
<210> 107
   <211> 552
   <212> PRT
   <213> H5N1
<400> 107
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Arg Gly
       210                 215                 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Ser Leu Gln Cys Arg Ile Cys Ile
   545 550
<210> 108
   <211> 552
   <212> PRT
   <213> H5N1
<400> 108
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Pro Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Lys Asn Ala Tyr Lys Ile
   245 250 255
   Val Lys Lys Gly Ser Ser Thr Ile Met Lys Ser Glu Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Thr Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Ile Cys Ile
   545 550
<210> 109
   <211> 552
   <212> PRT
   <213> H5N1
<220>
   <221> misc_feature
   <222> (540)..(540)
   <223> Xaa can be any naturally occurring amino acid
<400> 109
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Thr Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Arg Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Ile Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Met Ala Gly Leu Phe Leu Trp Xaa Cys Ser Asn Gly
       530                 535                 540
   Ser Leu Gln Cys Arg Ile Cys Ile
   545 550
<210> 110
   <211> 552
   <212> PRT
   <213> H5N1
<220>
   <221> misc_feature
   <222> (543)..(543)
   <223> Xaa can be any naturally occurring amino acid
<400> 110
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Glu Pro Ser Phe Tyr
   130 135 140
   Arg Asn Val Val Trp Leu Ile Lys Lys Asn Asn Thr Tyr Pro Thr Ile
   145 150 155 160
   Lys Glu Asn Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Xaa Gly
   530 535 540
   Ser Leu Gln Cys Arg Ile Cys Ile
   545 550
<210> 111
   <211> 552
   <212> PRT
   <213> H5N1
<400> 111
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Ile Cys Ile
   545 550
<210> 112
   <211> 552
   <212> PRT
   <213> H5N1
<400> 112
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Gly Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Ile Cys Ile
   545 550
<210> 113
   <211> 552
   <212> PRT
   <213> H5N1
<220>
   <221> misc_feature
   <222> (545)..(545)
   <223> Xaa can be any naturally occurring amino acid
<400> 113
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu His Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Asn Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Xaa Leu Gln Cys Arg Ile Cys Ile
   545 550
<210> 114
   <211> 552
   <212> PRT
   <213> H5N1
<220>
   <221> misc_feature
   <222> (120)..(120)
   <223> Xaa can be any naturally occurring amino acid
<400> 114
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Xaa Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Gly Ser Leu Ala
           515                 520                 525
   Leu Ala Ile Met Met Ala Gly Leu Phe Leu Trp Met Cys Ser Asn Gly
       530                 535                 540
   Ser Leu Gln Cys Arg Ile Cys Ile
   545                 550
<210> 115
   <211> 552
   <212> PRT
   <213> H5N1
<220>
   <221> misc_feature
   <222> (534)..(534)
   <223> Xaa can be any naturally occurring amino acid
<400> 115
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Glu Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Xaa Gly Leu Phe Leu Trp Met Cys Ser Asn Gly
   530 535 540
   Ser Leu Gln Cys Arg Ile Cys Ile
   545 550
<210> 116
   <211> 552
   <212> PRT
   <213> H5N1
<220>
   <221> misc_feature
   <222> (141)..(141)
   <223> Xaa can be any naturally occurring amino acid
<220>
   <221> misc_feature
   <222> (316)..(316)
   <223> Xaa can be any naturally occurring amino acid
<220>
   <221> misc_feature
   <222> (534)..(534)
   <223> Xaa can be any naturally occurring amino acid
<400> 116
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Xaa Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Xaa Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Gly Ser Leu Ala
   515 520 525
   Leu Ala Ile Met Val Xaa Gly Leu Phe Leu Trp Met Cys Ser Asn Gly
   530 535 540
   Ser Leu Gln Cys Arg Ile Cys Ile
   545 550
<210> 117
   <211> 552
   <212> PRT
   <213> H5N1
<400> 117
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Thr Gln Arg
           195                 200                 205
   Leu Val Pro Lys Ile Ala Thr Arg Pro Lys Val Asn Gly Gln Ser Gly
           210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Ser Leu Trp Met Cys Ser Asn Gly
       530                 535                 540
   Ser Leu Gln Cys Arg Ile Cys Ile
   545                 550
<210> 118
   <211> 552
   <212> PRT
   <213> H5N1
<220>
   <221> misc_feature
   <222> (550)..(550)
   <223> Xaa can be any naturally occurring amino acid
<400> 118
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Arg Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Val Arg Asn Gly Thr Tyr Asp Tyr Pro Gln Tyr Ser Lys Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Ser Leu Trp Met Cys Ser Asn Gly
   530 535 540
   Ser Leu Gln Cys Arg Xaa Cys Ile
   545 550
<210> 119
   <211> 552
   <212> PRT
   <213> H5N1
<220>
   <221> misc_feature
   <222> (535)..(535)
   <223> Xaa can be any naturally occurring amino acid
<400> 119
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Asn Tyr His Asn Thr Asn Gln Glu Asp Leu Leu Val Leu Trp
                   165                 170                 175
   Gly Ile His His Pro Asn Asp Glu Glu Glu Gln Lys Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Lys Asn Ala Tyr Lys Ile
                   245                 250                 255
   Val Lys Lys Gly Ser Ser Thr Ile Met Lys Ser Glu Leu Glu Tyr Gly
               260                 265                 270
   Asn Cys Ser Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Thr Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Lys Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Xaa Leu Phe Leu Trp Met Cys Ser Asn Gly
   530 535 540
   Ser Leu Gln Cys Arg Ile Cys Ile
   545 550
<210> 120
   <211> 552
   <212> PRT
   <213> H5N1
<400> 120
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Asn Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Glu Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 121
   <211> 552
   <212> PRT
   <213> H5N1
<220>
   <221> misc_feature
   <222> (7)..(7)
   <223> Xaa can be any naturally occurring amino acid
<220>
   <221> misc_feature
   <222> (188)..(188)
   <223> Xaa can be any naturally occurring amino acid
<220>
   <221> misc_feature
   <222> (206)..(206)
   <223> Xaa can be any naturally occurring amino acid
<220>
   <221> misc_feature
   <222> (517)..(517)
   <223> Xaa can be any naturally occurring amino acid
<400> 121
   Asp Gln Ile Cys Ile Gly Xaa His Ala Asn Asn Ser Thr Glu Gln Val
   1               5                   10                  15
   Asp Thr Ile Met Glu Lys Asn Ile Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Leu Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Ser Lys Asn Ser Trp Pro Asp His Glu Ala Ser
           115                 120                 125
   Leu Gly Val Ser Ala Ala Cys Pro Tyr Gln Gly Gly Leu Ser Phe Tyr
       130                 135                 140
   Arg Asn Val Val Trp Leu Ile Glu Lys Asn Asn Thr Tyr Pro Leu Ile
   145                 150                 155                 160
   Lys Lys Asn Tyr His Asn Thr Asn Gln Glu Asp Leu Leu Val Leu Trp
                   165                 170                 175
   Gly Ile His His Pro Asn Asp Glu Ala Glu Gln Xaa Arg Leu Tyr Lys
               180                 185                 190
   Asn Ser Thr Thr Tyr Ile Ser Val Gly Thr Ser Thr Leu Xaa Gln Arg
           195                 200                 205
   Leu Val Pro Lys Ile Ala Thr Arg Pro Lys Val Asn Gly Gln Ser Gly
       210                 215                 220
   Arg Val Glu Phe Phe Trp Thr Ile Leu Lys Ser Asn Asp Val Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Asp Leu Glu Tyr Gly
   260 265 270
   Asn Cys Ser Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Arg Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Arg Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Ala Lys Leu Glu Ser Ile Gly
   500 505 510
   Thr Tyr Gln Ile Xaa Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 122
   <211> 552
   <212> PRT
   <213> H5N1
<400> 122
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Arg Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Lys Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Lys Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 123
   <211> 552
   <212> PRT
   <213> H5N1
<400> 123
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asp Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Ser Thr Lys Cys Gln Thr Pro Ile Gly Ala Ile Asn Thr Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
                   340                 345                 350
   Gly Tyr His His Ser Asn Glu Gln Gly Ser Gly Tyr Ala Ala Asp Lys
               355                 360                 365
   Glu Ser Thr Gln Lys Ala Ile Asn Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Lys Glu Ala
                   485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 124
   <211> 552
   <212> PRT
   <213> H5N1
<220>
   <221> misc_feature
   <222> (307)..(307)
   <223> Xaa can be any naturally occurring amino acid
<400> 124
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Thr Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Glu Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Ser Thr Lys Cys Gln Thr Pro Val Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Xaa Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Arg
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Ile Glu
   465                 470                 475                 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 125
   <211> 552
   <212> PRT
   <213> H5N1
<220>
   <221> misc_feature
   <222> (510)..(510)
   <223> Xaa can be any naturally occurring amino acid
<400> 125
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Gln Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asp Thr Tyr Pro Thr Ile
   145 150 155 160
   Lys Glu Ser Tyr His Asn Thr Asn Gln Glu Asp Leu Leu Val Leu Trp
   165 170 175
   Gly Ile His His Pro Asn Asp Glu Glu Glu Gln Lys Arg Ile Tyr Lys
   180 185 190
   Asn Pro Thr Thr Tyr Val Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Ser Thr Lys Cys Gln Thr Pro Ile Gly Ala Ile Asn Thr Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Arg Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                   485                 490                 495
   Arg Leu Lys Arg Glu Glu Ile Ser Gly Val Lys Leu Glu Xaa Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550
<210> 126
   <211> 551
   <212> PRT
   <213> H5N1
<220>
   <221> misc_feature
   <222> (513)..(513)
   <223> Xaa can be any naturally occurring amino acid
<400> 126
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Lys Phe Leu Asn Val Pro Glu Trp Ser Tyr Ile Val
   65 70 75 80
   Glu Lys Ile Asn Pro Ala Asn Asp Leu Cys Tyr Pro Gly Asp Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
   115 120 125
   Gly Val Ser Ser Ala Cys Pro Tyr Gln Gly Arg Ser Ser Phe Phe Arg
   130 135 140
   Asn Val Val Trp Leu Thr Lys Lys Asn Asn Ala Tyr Pro Thr Ile Lys
   145 150 155 160
   Lys Ser Tyr Asn Asn Thr Asn Gln Glu Asp Leu Leu Val Leu Trp Gly
   165 170 175
   Ile His His Pro Asn Asp Ala Ala Glu Gln Thr Met Leu Tyr Gln Asn
   180 185 190
   Pro Thr Thr Tyr Val Ser Val Gly Thr Ser Thr Leu Asn Gln Arg Leu
   195 200 205
   Val Pro Lys Ile Ala Thr Arg Ser Lys Val Asn Gly Gln Ser Gly Arg
   210 215 220
   Met Glu Phe Phe Trp Thr Ile Leu Lys Ser Asn Asp Ala Ile Asn Phe
   225 230 235 240
   Glu Ser Asn Gly Asn Phe Ile Ala Pro Glu Asn Ala Tyr Lys Ile Val
   245 250 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Arg Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Xaa Gln Ile Leu Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 127
   <211> 551
   <212> PRT
   <213> H5N1
<220>
   <221> misc_feature
   <222> (7)..(7)
   <223> Xaa can be any naturally occurring amino acid
<220>
   <221> misc_feature
   <222> (513)..(513)
   <223> Xaa can be any naturally occurring amino acid
<220>
   <221> misc_feature
   <222> (516)..(516)
   <223> Xaa can be any naturally occurring amino acid
<400> 127
   Asp Gln Ile Cys Ile Gly Xaa His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asn Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Leu Glu Trp Ser Tyr Ile Val
   65 70 75 80
   Glu Lys Ile Asn Pro Ala Asn Asp Leu Cys Tyr Pro Gly Asn Phe Asn
   85 90 95
   Asp Tyr Glu Glu Leu Lys His Leu Leu Ser Arg Ile Asn His Phe Glu
   100 105 110
   Lys Ile Gln Ile Ile Pro Lys Asn Ser Trp Ser Asp His Glu Ala Ser
           115                 120                 125
   Gly Val Ser Ser Ala Cys Pro Tyr Gln Arg Arg Ser Ser Phe Phe Arg
       130                 135                 140
   Asn Val Val Trp Leu Thr Lys Lys Asn Asn Ala Tyr Pro Thr Ile Lys
   145                 150                 155                 160
   Lys Ser Tyr Asn Asn Thr Asn Gln Glu Asp Leu Leu Val Leu Trp Gly
                   165                 170                 175
   Ile His His Pro Asn Asp Ala Ala Glu Gln Thr Arg Leu Tyr Gln Asn
               180                 185                 190
   Pro Thr Thr Tyr Ile Ser Val Gly Thr Ser Thr Leu Asn Gln Arg Leu
           195                 200                 205
   Val Pro Lys Ile Ala Thr Arg Ser Lys Val Asn Gly Gln Ser Gly Arg
       210                 215                 220
   Met Glu Phe Phe Trp Thr Ile Leu Lys Ser Asn Asp Ala Ile Asn Phe
   225                 230                 235                 240
   Glu Ser Asn Gly Asn Phe Ile Ala Pro Glu Asn Ala Tyr Lys Ile Val
                   245                 250                 255
   Lys Lys Gly Asp Ser Thr Ile Met Lys Ser Glu Leu Glu Tyr Gly Asn
               260                 265                 270
   Cys Asn Thr Lys Cys Gln Thr Pro Ile Gly Ala Ile Asn Ser Ser Met
           275                 280                 285
   Pro Phe His Asn Ile His Pro Leu Thr Ile Gly Glu Cys Pro Lys Tyr
       290                 295                 300
   Val Lys Ser Asn Arg Leu Val Leu Ala Thr Gly Leu Arg Asn Ser Pro
   305                 310                 315                 320
   Gln Gly Glu Arg Arg Arg Lys Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
   370 375 380
   Ile Asp Lys Met Asn Thr Gln Phe Glu Ala Val Gly Arg Glu Phe Asn
   385 390 395 400
   Asn Leu Glu Arg Arg Ile Glu Asn Leu Asn Lys Lys Met Glu Asp Gly
   405 410 415
   Phe Leu Asp Val Trp Thr Tyr Asn Ala Glu Leu Leu Val Leu Met Glu
   420 425 430
   Asn Glu Arg Thr Leu Asp Phe His Asp Ser Asn Val Lys Asn Leu Tyr
   435 440 445
   Asp Lys Val Arg Leu Gln Leu Arg Asp Asn Ala Lys Glu Leu Gly Asn
   450 455 460
   Gly Cys Phe Glu Phe Tyr His Arg Cys Asp Asn Glu Cys Met Glu Ser
   465 470 475 480
   Val Arg Asn Gly Thr Tyr Asp Tyr Pro Gln Tyr Ser Glu Glu Ala Arg
   485 490 495
   Leu Lys Arg Glu Glu Ile Ser Gly Val Lys Leu Glu Ser Ile Gly Thr
   500 505 510
   Xaa Gln Ile Xaa Ser Ile Tyr Ser Thr Val Ala Ser Ser Leu Ala Leu
   515 520 525
   Ala Ile Met Val Ala Gly Leu Phe Leu Trp Met Cys Ser Asn Gly Ser
   530 535 540
   Leu Gln Cys Arg Ile Cys Ile
   545 550
<210> 128
   <211> 551
   <212> PRT
   <213> H5N1
<220>
   <221> misc_feature
   <222> (530)..(530)
   <223> Xaa can be any naturally occurring amino acid
<400> 128
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Lys Ser Tyr His Asn Thr Asn Gln Glu Asp Leu Leu Val Leu Trp
   165 170 175
   Gly Ile His His Pro Asn Asp Glu Glu Glu Gln Thr Arg Ile Tyr Lys
   180 185 190
   Asn Pro Thr Thr Tyr Ile Ser Val Gly Thr Ser Thr Leu Asn Gln Arg
   195 200 205
   Leu Val Pro Lys Ile Ala Thr Arg Ser Lys Val Asn Gly Gln Ser Gly
   210 215 220
   Arg Val Glu Phe Phe Trp Thr Ile Leu Lys Ser Asn Asp Thr Ile Asn
   225 230 235 240
   Phe Glu Ser Asn Gly Asn Phe Ile Ala Pro Glu Asn Ala Tyr Lys Ile
   245 250 255
   Val Lys Lys Gly Asp Ser Thr Ile Met Lys Ser Glu Leu Glu Tyr Gly
   260 265 270
   Asn Cys Asn Thr Lys Cys Gln Thr Pro Ile Gly Ala Ile Asn Ser Ser
   275 280 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ile Tyr Ser Thr Val Ala Ser Ser Leu Ala Leu
   515 520 525
   Ala Xaa Met Val Ala Gly Leu Phe Leu Trp Met Cys Ser Asn Gly Ser
   530 535 540
   Leu Gln Cys Arg Ile Cys Ile
   545 550
<210> 129
   <211> 552
   <212> PRT
   <213> H5N1
<400> 129
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65 70 75 80
   Glu Lys Ile Asn Pro Ala Asn Asp Leu Cys Tyr Pro Gly Asn Phe Asn
   85 90 95
   Asn Tyr Glu Glu Leu Lys His Leu Leu Ser Arg Ile Asn Arg Phe Glu
   100 105 110
   Lys Ile Lys Ile Ile Pro Lys Ser Ser Trp Pro Asp His Glu Ala Ser
   115 120 125
   Leu Gly Val Ser Ser Ala Cys Pro Tyr Gln Gly Gly Pro Ser Phe Tyr
   130 135 140
   Arg Asn Val Val Trp Leu Ile Lys Lys Asn Asn Thr Tyr Pro Thr Ile
   145 150 155 160
   Lys Glu Ser Tyr His Asn Ile Asn Lys Glu Asp Leu Leu Val Leu Trp
   165 170 175
   Gly Ile His His Pro Asn Asp Glu Glu Glu Gln Ile Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Val Ala Gly Leu Phe Leu Trp Met Cys Ser Asn Gly
   530 535 540
   Ser Leu Gln Cys Arg Ile Cys Ile
   545 550
<210> 130
   <211> 552
   <212> PRT
   <213> H5N1
<220>
   <221> misc_feature
   <222> (545)..(545)
   <223> Xaa can be any naturally occurring amino acid
<400> 130
   Asp Gln Ile Cys Ile Gly Tyr His Ala Asn Asn Ser Thr Glu Gln Val
   1 5 10 15
   Asp Thr Ile Met Glu Lys Asn Val Thr Val Thr His Ala Gln Asp Ile
   20 25 30
   Leu Glu Lys Thr His Asn Gly Lys Leu Cys Asp Leu Asp Gly Val Lys
   35 40 45
   Pro Leu Ile Leu Arg Asp Cys Ser Val Ala Gly Trp Leu Leu Gly Asn
   50 55 60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Lys Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Thr Lys Lys Asn Asn Thr Tyr Pro Thr Ile
   145                 150                 155                 160
   Lys Lys Ser Tyr His Asn Ile Asn Lys Glu Asp Leu Leu Val Leu Trp
                   165                 170                 175
   Gly Ile His His Pro Asn Asp Glu Glu Glu Gln Ile Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Lys Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Arg Cys Asp Asn Glu Cys Met Glu
   465 470 475 480
   Ser Val Arg Asn Gly Thr Tyr Asp Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile Leu Ser Ile Tyr Ser Thr Val Ala Ser Ser Leu Ala
   515 520 525
   Leu Ala Ile Met Met Ala Gly Leu Phe Leu Trp Met Cys Ser Asn Gly
   530 535 540
   Xaa Leu Gln Cys Arg Ile Cys Ile
   545 550
<210> 131
   <211> 552
   <212> PRT
   <213> H5N1
<400> 131
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Pro Asn Val Ser Glu Trp Ser Tyr Ile Val
   65                  70                  75                  80
   Glu Lys Ile Asn Pro Ala Asn Asp Leu Cys Tyr Pro Gly Asn Phe Asn
                   85                  90                  95
   Asn Tyr Glu Glu Leu Lys His Leu Leu Ser Arg Ile Asn Arg Phe Glu
               100                 105                 110
   Lys Ile Gln Ile Ile Pro Lys Ser Ser Trp Pro Asp His Glu Ala Ser
           115                 120                 125
   Leu Gly Val Ser Ser Ala Cys Pro Tyr Gln Gly Gly Pro Ser Phe Tyr
       130                 135                 140
   Arg Asn Val Val Trp Leu Ile Lys Lys Asn Asn Thr Tyr Pro Thr Ile
   145                 150                 155                 160
   Lys Glu Ser Tyr His Asn Thr Asn Gln Glu Asp Leu Leu Val Leu Trp
                   165                 170                 175
   Gly Ile His His Pro Asn Asp Glu Glu Glu Gln Thr Arg Ile Tyr Lys
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Val Glu Phe Phe Trp Thr Ile Leu Lys Ser Asn Asp Thr Ile Asn
   225                 230                 235                 240
   Phe Glu Ser Asn Gly Asn Phe Ile Ala Pro Glu Asn Ala Tyr Lys Ile
                   245                 250                 255
   Val Lys Lys Gly Asp Ser Thr Ile Met Lys Ser Glu Leu Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Arg Leu Val Leu Ala Thr Gly Leu Arg Asn Ser
   305                 310                 315                 320
   Pro Gln Gly Glu Gly Arg Arg Lys Lys Arg Gly Leu Phe Gly Ala Ile
                   325                 330                 335
   Ala Gly Phe Ile Glu Gly Gly Trp Gln Gly Met Val Asp Gly Trp Tyr
               340                 345                 350
   Gly Tyr His His Ser Asn Glu Gln Gly Ser Gly Tyr Ala Ala Asp Lys
           355                 360                 365
   Glu Ser Thr Gln Lys Ala Ile Asp Gly Val Thr Asn Lys Val Asn Ser
       370                 375                 380
   Ile Ile Asp Lys Met Asn Thr Gln Phe Glu Ala Val Gly Arg Glu Phe
   385                 390                 395                 400
   Asn Asn Leu Glu Lys Arg Ile Glu Asn Leu Asn Lys Lys Met Glu Asp
                   405                 410                 415
   Gly Phe Leu Asp Val Trp Thr Tyr Asn Ala Glu Leu Leu Val Leu Met
               420                 425                 430
   Glu Asn Glu Arg Thr Leu Asp Phe His Asp Ser Asn Val Lys Asn Leu
           435                 440                 445
   Tyr Asp Lys Val Arg Leu Gln Leu Arg Asp Asn Ala Lys Glu Leu Gly
       450                 455                 460
   Asn Gly Cys Phe Glu Phe Tyr His Arg Cys Asp Asn Glu Cys Met Glu
   465                 470                 475                 480
   Ser Val Arg Asn Gly Thr Tyr Asp Tyr Pro Gln Tyr Ser Glu Glu Ala
                       485                 490                 495
   Arg Leu Lys Arg Glu Glu Ile Ser Gly Val Lys Leu Glu Ser Ile Gly
               500                 505                 510
   Thr Tyr Gln Ile Leu Ser Ile Tyr Ser Thr Val Ala Ser Ser Leu Ala
           515                 520                 525
   Leu Ala Ile Met Val Ala Gly Leu Phe Leu Trp Met Cys Ser Asn Gly
       530                 535                 540
   Ser Leu Gln Cys Arg Ile Cys Ile
   545                 550